# EUROPEAN PATENT SPECIFICATION

(11) **EP 1 723 235 B1**
(45) Date of publication and mention of the grant of the patent: **27.06.2012**
(21) Application number: 05700191.9
(22) Date of filing: 11.02.2005
(51) Int. Cl.: C12N 15/12, C12N 15/90, C12N 15/63

(54) **MODIFIED CELLS THAT CO-EXPRESS BLIMP1 AND A REPORTER MOLECULE AND METHODS OF USING THE SAME**
MODIFIZIERTE, GLEICHZEITIG BLIMP1 UND EIN REPORTERMOLEKÜL EXPRIMIERENDE ZELLEN UND VERFAHREN ZU DEREN VERWENDUNG
CELLULES MODIFIEES CO-EXPRIMANT BLIMP1 ET MOLECULE RAPPORTRICE ET PROCEDES D'UTILISATION DE CELLES-CI

(30) Priority: 12.02.2004 AU 2004900673
(43) Date of publication of application: 22.11.2006
(73) Proprietor: THE WALTER AND ELIZA HALL INSTITUTE OF MEDICAL RESEARCH, Parkville, VIC 3052 (AU)
(72) Inventor: KALLIES, Axel, St Kilda, VIC 3182 (AU); HASBOLD, Jhagvaral, North Balwyn, VIC 3104 (AU); TARLINTON, David, Blackburn, VIC 3130 (AU); CORCORAN, Lynn, East Kew, VIC 3102 (AU); HODGKIN, Philip, Desmond, Coburg, VIC 3058 (AU); NUTT, Stephen, Laurence, Ascot Vale, VIC 3032 (AU)
(74) Representative: Leathley, Anna Elisabeth
(86) International application number: PCT/AU2005/000168
(87) International publication number: WO 2005/078098

(56) References cited:
- SHAPIRO-SHELEF MIRIAM ET AL: "Blimp-1 is required for the formation of immunoglobulin secreting plasma cells and pre-plasma memory B cells." IMMUNITY OCT 2003, vol. 19, no. 4, October 2003 (2003-10), pages 607-620, XP002446620 ISSN: 1074-7613
- MOUNTFORD P ET AL: "DICISTRONIC TARGETING CONSTRUCTS: REPORTERS AND MODIFIERS OF GENE EXPRESSION" PROCEEDINGS OF THE NATIONAL ACADEMY OF SCIENCES OF USA, NATIONAL ACADEMY OF SCIENCE, WASHINGTON, DC, US, vol. 91, no. 10, 10 May 1994 (1994-05-10), pages 4303-4307, XP000563920 ISSN: 0027-8424
- WEILER-GUETTLER H ET AL: "Developmentally regulated gene expression of thrombomodulin in postimplantation mouse embryos." DEVELOPMENT (CAMBRIDGE, ENGLAND) JUL 1996, vol. 122, no. 7, July 1996 (1996-07), pages 2271-2281, XP002446621 ISSN: 0950-1991
- KALLIES A. ET AL.: 'Plasma cell ontogeny defined by quantitative changes in blimp expression.' EXPERIMENTAL MEDICINE vol. 200, no. 8, 2004, pages 967 - 977, XP003014669
- KNöDEL M. ET AL.: 'Reversal of blomp-1 mediated apoptosis by A1, a member of the Bel-2 family.' EUROPEAN JOURNAL OF IMMUNOLOGY vol. 29, 1999, pages 2988 - 2998, XP003014670
- BAXENDALE S. ET AL: 'The B-cell maturation factor Blimp-1 specifies vertebrate slow-twitch muscle fiber identity in response to Hedgehog signaling.' NATURE GENETICS vol. 36, no. 1, January 2004, pages 88 - 93, XP003014671
- TUNYAPLIN C. ET AL.: 'Characterisation of the B lymphocyte-induced naturation protein-1 (Blimp-1) gene, mRBA isoforms and basal promoter.' NUCLEIC ACIDS RESEARCH vol. 28, no. 24, 2000, pages 4846 - 4855, XP003014672
- RELJIC R. ET AL.: 'Suppression of signal transducer and activator of transcription 3-dependent B lymhocyte terminal differentiation by BCL-6.' EXPERIMENTAL MEDICINE vol. 192, no. 12, 2000, pages 1841 - 1947, XP002248599
- CHANG D.H. ET AL.: 'BLIMP-1 trigger for differentiation of myeloid lineage.' NATURE IMMUNOLOGY vol. 1, no. 2, 2000, pages 169 - 176, XP003014673
- ANGELIN-DUCLOS C. ET AL: 'Role of B-lymphocyte-induced maturation protein-1 in terminal differentiation of b cells lineages.' COLD SPRING HARBOR SYMPOSIA ON QUANTITATIVE BIOLOGY. vol. 64, 1999, pages 61 - 70, XP008083213

## Description

### BACKGROUND OF THE INVENTION

### FIELD OF THE INVENTION

The present invention relates generally to a model system to identify haematopoietic cells of particular lineages and their stage of differentiation. More particularly, the present invention provides genetically modified cells and non-human animals comprising such cells which carry a genetic marker of terminal differentiation modified to co-produce a reporter molecule capable of eliciting an identifiable signal and their use in identifying molecules capable of modulating the differentiation or transformation status of cells, such as, without limitation, embryonic cells during development, cells with aberrant differentiation such as cancer cells, and cells of the haematopoietic cell lineages such as, for example, B and/or T cells. Identified molecules form the basis for pharmaceutical compositions for therapeutic and prophylactic application.

### DESCRIPTION OF THE PRIOR ART

Bibliographic details of references in the subject specification are also listed at the end of the specification.

Reference to any prior art in this specification is not, and should not be taken as, an acknowledgement or any form of suggestion that this prior art forms part of the common general knowledge in any country.

Cellular life involves a myriad of alternative and highly regulated biochemical pathways directing changes in cell division, differentiation, morphogenesis and apoptosis. Cells vary in their potential to divide and/or differentiate. For example, the embryo comprises totipotent cells retaining the ability to differentiate into any cell type. Other cell types including stem cells are pluripotent and may ultimately differentiate into a range of but not all cell phenotypes. Some cells become committed to one final form: they are terminally differentiated.

Changes which block normal maturation of cells into terminally differentiated cells or which prevent apoptosis can act as triggers for tumor development characterized by uncontrolled cell division without differentiation or cell death. Thus, agents which promote differentiation and normal apoptosis may switch off tumor development.

Molecules which are expressed during the time of terminal differentiation of particular cell types have been intensely studied. However, in order to understand the sequence of events during this period at a molecular level it is necessary to understand the temporal and spatial expression patterns of molecules which are expressed in this phase of development.

B-lymphocyte-induced maturation factor (Blimp) is a 98kDa transcription factor which was originally identified as being induced during the differentiation of a B-cell lymphoma cell line (Turner et al., Cell 77:297, 1994). The corresponding factor from human cells is referred to as PRDM-1. It has been proposed that Blimp-1 has a pre-eminent role in regulating B-cell terminal differentiation. Specifically, *Blimp-1* is expressed in antibody secreting cells (ASC) from man and mouse but it is not expressed in memory cells (Angelin-Duclos et al., J Immunol 165:5462, 2000). Ectopic expression of *Blimp-1* is sufficient to drive terminal differentiation of lymphomas and primary B-cells into ASC cells (Turner *et al., (supra)*, Schliephake et al., Eur J Immunol 26:268, 1996; Messika et al., J Exp Med 188:515, 1998; Knodel et al., Eur J Immunol 31:1972, 2001). Blocking expression of *Blimp-1* through antisense or dominant-interfering approaches suppresses cell-cycle exit which is thought to be essential for full ASC differentiation (Soro et al., J Immunol 163:611, 1999; Angelin-Duclos et al., J Immunol 165:5462, 2000; Johnson et al., Eur J Immunol 32:3765, 2002). Also, mice which lack Blimp-1 in B-cells produce very little immunoglobulin and have a markedly reduced ASC compartment. (Shapiro-Shelef et al., Immunity 19:607, 2003.).

It was initially reported that Blimp-1 is only produced in cells of the B-cell lineage, however, it is now evident that Blimp-1 is also produced during myeloid differentiation (Keller et al., Genes Dev 5:868, 1991, Chang et al., Nat Immunol 1:169, 2000). Blimp-1 is required for the repression of *c-myc* which is involved in myeloid differentiation (Chang *et al*., *(supra),* 2000; Marcu et al., Annu Rev Biochem 61:809, 1992). Over production of Blimp-1 in U937 cells for example is sufficient to induce macrophage differentiation (Chang *et al., (supra),* 2000). Thus, repression of *c-myc* by Blimp-1 in macrophages and B-cells is a feature of terminal differentiation in these two lineages. Blimp-1 is also broadly produced during mouse and *Xenopus* embryonic development (de Souza et al., Embo J 18: 6062, 1999; Rosenbaum et al., Embo J, 9:897, 1990).

B-lymphocytes are among the most intensively studied eukaryotic cell types but while the early steps of B-cell development are relatively well characterized, much less is known about the processes which control the final differentiation of B-lymphocytes into ASC. ASC (plasma cells) are the direct mediators of the humoral immune response. They secrete a large amount of serum immunoglobulin essential for protective immunity. The terminal differentiation of B-lymphocytes into ASC is, therefore, a subject of intense therapeutic interest. For example, terminal differentiation to ASC is a crucial element in effective vaccination strategies. Furthermore, multiple myeloma results from the failure of an ASC to complete the differentiation pathway.

However, ASC represent a very rare population of highly specialised cells located mostly in the bone marrow and spleen. ASC populations in mice and man comprise cells of heterogeneous life span and cell surface phenotype making a definitive prospective isolation of pure ASC impossible (Fong et al., Proc Natl Acad Sci U S A 11:11, 2003; Medina et al., Blood 99:2154, 2002; O'Connor et al., J. Exp Med 195:737, 2002; Manz et al., Curr Opin Immunol 14:517, 2002; Underhill et al., Blood 2-1:24, 2003).

T-cell terminal differentiation programs are poorly understood (Sprent et al., Immunol Lett 85:145-149, 2003). In response to infection, antigen-specific T cells differentiate into effector cells and undergo massive clonal expansion. Homeostasis of T cell numbers is maintained by the subsequent contraction phase where >90% of effector cells are eliminated with a small fraction becoming memory T cells (Sprent et al., Annu Rev Immunol 20:551-579, 2002). This process has been proposed to be under genetic control as the contraction is independent of the dose or duration of infection (Badovinac et al., Nat Immunol 5:809-817, 2004; Badovinac et al., Nat Immunol 3:619-626, 2002). The ability to control T cell numbers is essential as enhanced expansion due to the lack of T-regulator cells (Khattri et al., Nat Immunol 4:337-342, 2003; Hori et al., Science 299:1057-1061, 2003; Fontenot et al., Nat Immunol 4:330-336, 2003), the loss of the down-regulatory molecule CTLA-4 (Chambers et al., Immunity 7:885-895, 1997) or genetic deficiencies in non-obese diabetic (NOD) mice result in autoimmunity.

The ability to monitor terminal differentiation of ASC, T-cells and other cells of the haematopoietic system in a wide range of contexts and under various stimuli would be extremely valuable in developing strategies and reagents for use in the treatment and/or prophylaxis of a range of conditions associated with aberrant differentiation, such cancer autoimmune disease, or with harnessing normal developmental programs such as in the development of an appropriate immune response.

### SUMMARY OF THE INVENTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Nucleotide and amino acid sequences are referred to by a sequence identifier number (SEQ ID NO:). The SEQ ID NOs: correspond numerically to the sequence identifiers <400>1 (SEQ ID NO:1), <400>2 (SEQ ID NO:2), etc. A summary of sequence identifiers is provided in Table 1. A sequence listing is provided after the claims.

Genes and other genetic material (eg mRNA, constructs etc) are represented in italics and their proteinaceous expression products are represented in non-italicised form. Thus, the transcription factor Blimp is the expression product of *Blimp.* The term "Blimp" or "*Blimp*" is used to denote all homologs or variant molecules derived from any animal or mammalian species, including a human homolog. Accordingly, human *PRDM-1* and its product, PRDM-1 are encompassed in the terms *Blimp* or Blimp. Unless otherwise stated, reference to Blimp is a reference to a functional form of the polypeptide and reference to a modified *Blimp* is a reference to the gene or allele sequences encoding a functional form of Blimp.

The present invention is predicated, in part, on the identification of the role of Blimp in the differentiation of haematopoietic and embryonic cells. By screening for the presence of Blimp, or the level of Blimp, a determination can be made as to the stage of terminal differentiation of a cell. The identification of the role of Blimp further enables substantially homogeneous populations of particular haematopoietic cells to be identified such as, but not limited to, ASC (plasma cells).

More particularly, the present invention provides a genetically modified cell or an *in vivo* or *in vitro* system comprising cells which co-express genetic material which encodes Blimp and a reporter molecule. Detection of reporter activity in cells of a haematopoietic lineage, such as but not limited to a lymphocyte lineage, is indicative that cells having reporter activity and producing functional Blimp are committed to terminal differentiation. Thus, the detection of reporter-active B-cells producing functional Blimp is an indication that these cells are committed to differentiate into an antibody secreting cells (ASC). Also, as described herein the detection of reporter activity in T cells expressing a functional *Blimp,* is indicative that these cells are activated/memory T cells, such as activated CD4⁺ T-cells or effector CD8⁺ T-cells. The present invention provides therefore, genetically modified cells or non-human animals comprising such cells which facilitate monitoring the differentiation or transformation status of particular cells under various conditions or in the presence of various stimuli or agents. The present invention further provides screening methods, including high through-put screening methods, for identifying molecules capable of modulating the differentiation or transformation status of cells, such as, without limitation, embryonic cells including stem cells during development, cells with aberrant differentiation such as cancer cells, and cells of the haematopoietic cell lineages such as, for example B and/or T cells.

Specifically, a genetically modified cell, or a non-human organism comprising such cells, is provided by the present invention. In one embodiment, the cells produce Blimp translated from an mRNA modified to encode a reporter molecule. Preferably, the reporter molecule encoding sequence is inserted into an intron of a Blimp allele. When the modified Blimp allele is present in heterozygous form, the other allele will express a functional Blimp. In some embodiments, the modified allele may express a functional Blimp polypeptide or a functional form thereof. In other embodiment the modified allele expressed a non-functional Blimp polypeptide. In one embodiment the modified cells are useful in *in vivo* or *in vitro* cellular model systems to identify and isolate, *inter alia,* ASC. In another embodiment, the modified cells are useful for monitoring the differentiation status of haematopoietic such as T-cells and/or B-cells in a wide range of assays.

In one aspect, the present invention provides a genetically modified cell or non-human organism comprising such cells comprising modified genetic material which when expressed produces a polypeptide co-expressed with a reporter molecule and wherein the polypeptide is associated with terminal differentiation of a haematopoietic cell. Preferably, the genetic material is a *Blimp* gene or a part, fragment, homolog, derivative or functional form thereof. Furthermore, the identification of the reporter molecule in B-cell lineage cells indicates that such cells are committed to differentiate or have differentiated into ASC. Alternatively, reporter molecule activity in cells of a T cell lineage indicates that these cells are activated. Thus, as described herein, the presence of Blimp in a lymphocyte indicates that the cell is terminally differentiated or is committed to terminal differentiation. Exemplary T-cells include CD4⁺ T-cells and CD8⁺ T-cells and exemplary B-cells are ASC. Where a non-functional Blimp polypeptide is produced, detection of reporter-active cells indicates that the cells have been exposed to conditions sufficient to render them terminally differentiated if they had been able to produce a functional Blimp polypeptide.

Genetically modified non-human organisms may be provided in the form of gametes, embryos or ES cells for transplantation. Embryos are preferably maintained in a frozen state and may optionally be sold with instructions for use. Targeting constructs and genetically modified cells are also preferably maintained in a frozen state and may optionally be sold with instructions for use. All such cells are referred to herein as an *in vivo* or *in vitro* cellular model system.

The present invention provides a system for monitoring gene expression and differentiation fate in cells *in vivo* and *in vitro* at the single cell, tissue and organism level. Thus, reporter activity may be monitored in live cells and gene expression monitored in fixed tissues. Preferably, the reporter expression cassette encodes a fluorescent or other light emitting moiety. The availability of organisms and cells which report the expression of *Blimp-1* for example as a marker for terminal differentiation of a particular lineage or cell will be an extremely useful tool in a wide range of applications. In relation to cells of the B-cell lineage, this system finds broad application in the study, isolation and monitoring of ASC. As previously mentioned, ASC have not hitherto been available for study although these cells are crucial for an effective antibody response. Furthermore, aberrant differentiation in ASC causes multiple myeloma in man making them an important cell type to study for this reason.

In a related embodiment, the present invention provides a method for phenotyping and/or monitoring a cell of the haematopoietic system comprising screening a genetically modified cell or non-human animal comprising such cells comprising a modified *Blimp,* gene encoding a Blimp protein which when expressed co-expresses Blimp or a part, fragment, variant, homolog or functional or non-functional form thereof and a reporter molecule, wherein detection of reporter activity is indicative or predictive of a cellular phenotype and/or commitment of a cell to terminally differentiate. Haematopoietic cells include without limitation B-cells, T-cells, dendritic cells, macrophages, natural killer cells, granulocytes, erythrocytes, eosinophils, megakaryocytes, bone marrow, splenic, dermal, or stromal cells or their derivatives. In one particular embodiment, the haematopoietic cells are lymphocytes such as B and/or T cells.

In a further embodiment, cells which exhibit reporter activity or changes in reporter activity are isolated or selected from among cells which do not exhibit reporter activity. Isolation of reporter-active cells may be by any convenient method. For example, flow cytometry, laser scanning cytometry, chromatography and/or other equivalent procedures are conveniently employed. Flow cytometric procedures are particularly preferred. Additionally, further selection markers such as for example drug selection markers, may be used to isolate or select the modified cells of the present invention.

The present invention also provides antagonists and agonists of *Blimp-1* expression or Blimp-1 activity. One example of an agonist of Blimp-1 expression is a cytokine such as but not limited to IL-21. Pharmaceutical compositions are further contemplated comprising recombinant, synthetic or isolated forms of the present agonists and antagonists and one or more pharmaceutically acceptable carriers, diluents or excipients. Reference to *Blimp-1* expression or production of Blimp-1 protein includes in a single cell or within a population of cells.

**TABLE 1**

| **Summary of sequence identifiers** | |
|---|---|
| **SEQUENCE ID NO:** | **DESCRIPTION** |
| 1 | Nucleotide sequence encoding murine Blimp-1 |
| 2 | Amino acid sequence of murine Blimp-1 |
| 3 | Nucleotide sequence encoding human Blimp-1 (PRDM-1) |
| 4 | Amino acid sequence of human Blimp-1 (PRDM-1) |
| 5 | Genomic nucleotide sequence of murine Blimp-1 |
| 6 | Genomic nucleotide sequence of human Blimp-1 (PRDM-1) |

### BRIEF DESCRIPTION OF THE FIGURES

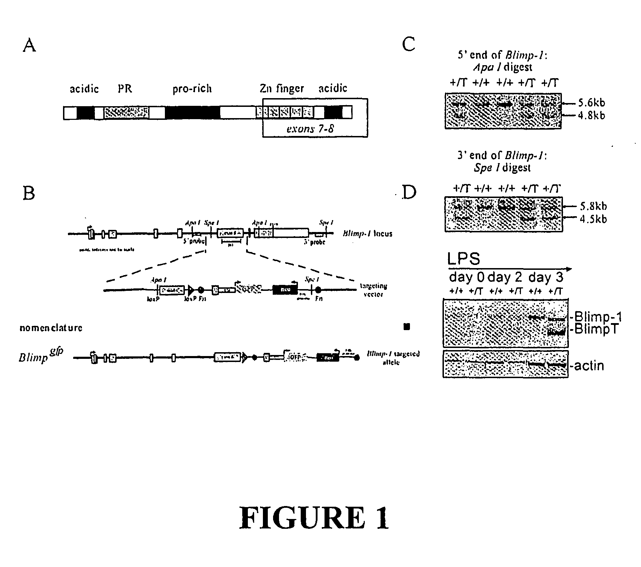

**Figure 1** is a diagrammatic representation of the Blimp-1 locus and a targeting strategy. A) Structural domains of the Blimp-1 protein. The segment of the protein encoded by exons 7-8 are indicated. Acidic, N and C terminal acidic regions; PR, region of homology to the retinoblastoma interacting zinc finger protein RIZ; Pro, proline rich region; Zn, 5 Zinc fingers. B) Genomic locus of Blimp-1, indicating the 8 exons as boxes and introns as black lines. Coding regions are in grey, non-translated regions are white. Restriction enzymes used for Southern hybridisations are marked, along with the 5' and 3' probes. Targeted allele derived from the homologous recombination event and subsequent manipulations is indicated C) Southern hybridisation on targeted and control ES cell DNA, using 5' and 3' ends of the Blimp-1 locus, to show expected products of the targeting event (4.8kb 5' arm and 4.5kb 3' arm). Expression of Blimp-1 in blimp^{gfp/+} LPS stimulated B cells cultured for 0-3 days *ex vivo* in IL-15 ^{+/-}IL-21. Blimp-1 expression was detected using a monoclonal antibody against mouse Blimp-1, a goat polyclonal antibody against α-actin was used as a loading control. ^{+/+}, wild type C57B1/6 mice; ^{+/T} blimp^{gfp/+} mice.

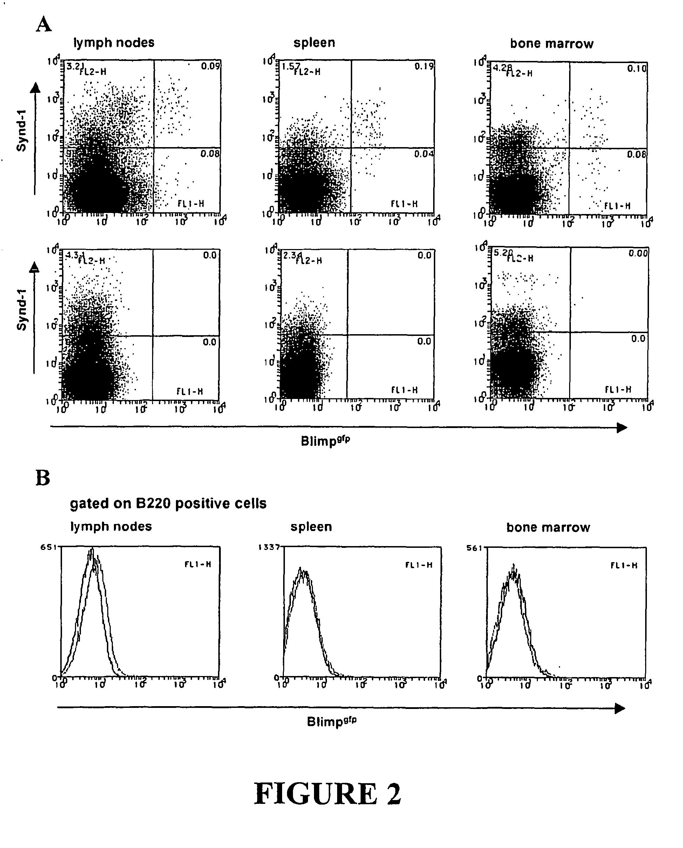

**Figure 2** is a graphical representation showing the results of FACS analysis of Blimp^{gfP} expression in B-cells *in vivo.* A) Syndecan-1 and Blimp^{gfp} expression in lymph nodes, spleen and bone marrow in Blimp^{gfp/+} mice (upper panel) and controls (lower panel). B) Expression of Blimp^{gfp} in B220 positive B cells.

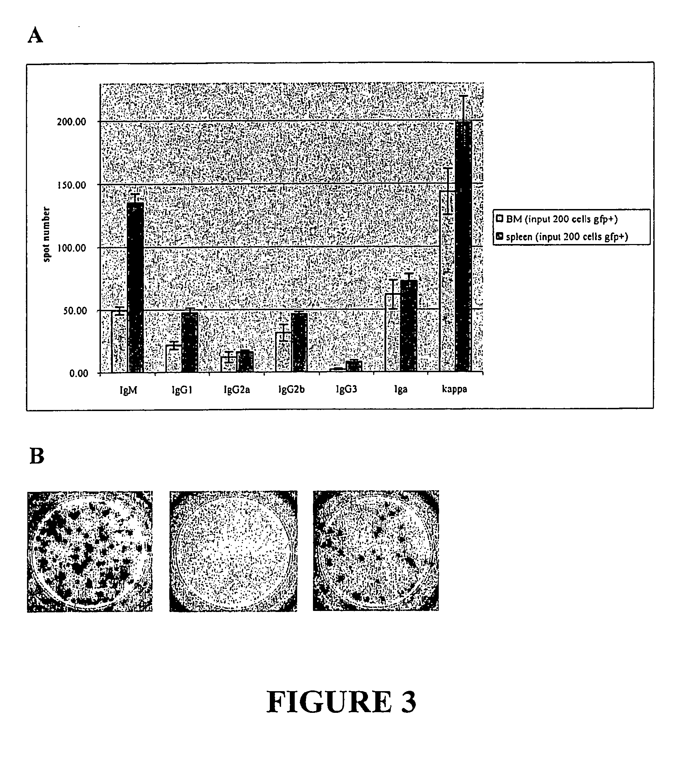

**Figure 3** is a graphical and photographic representation showing the results of ELIspot analysis of Blimp^{gfp} sorted cells. Gfp positive cells were sorted from bone marrow (BM) and spleen of an untreated Blimp^{gfp/+} mouse and analysed in an Elispot assay. Isotype specific antibodies or anti kappa antibodies were used to coat the elispot plate and to detect secreted immunglobulins. A) Distribution of isotype specific immunglobulins in 200 gfp-positive sorted cells (one representative experiment of three). B) Detection of kappa chain in a single representative well of an ELIspot plate (sample: sorted bone marrow cells). left, input 200 gfp-positive cells; middle, input 100 000 gfp-negative cells; right, input 100 000 unsorted cells.

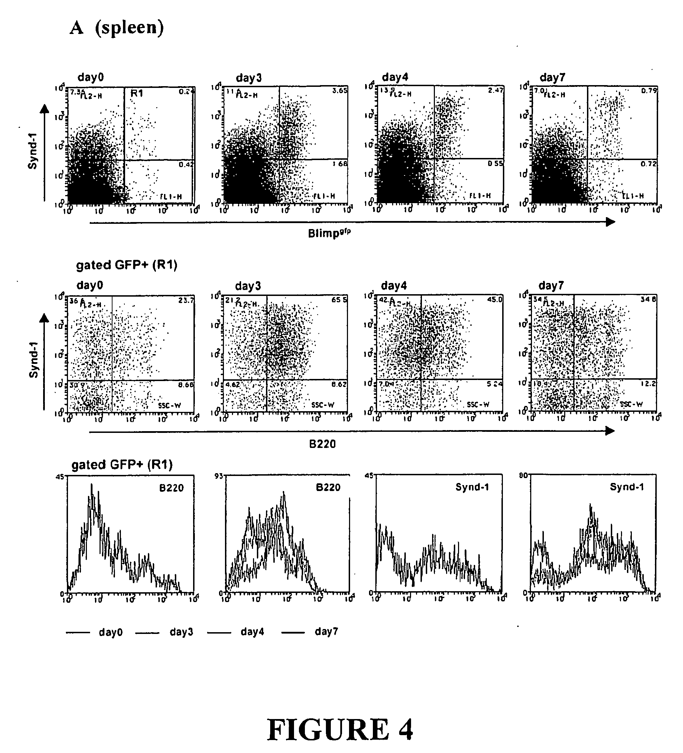

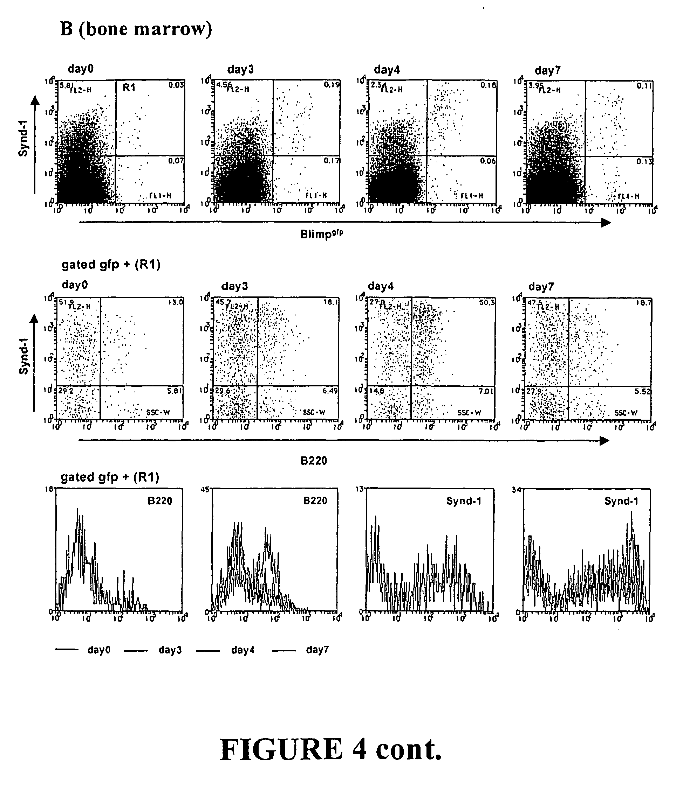

**Figure 4** is a graphical representation of the results of FACS analysis showing induction of antibody secreting cells with LPS *in vivo.* Blimp^{gfp/+} mice were i.v. injected with 2ug E. coli LPS. Spleens A) and bone marrows B) of these mice were analysed at indicated time points after LPS treatment. LPS induces the formation of ASC, increasing the frequency from about 0.5% to about 5% at day 3 in spleen and from about 0.05% to about 0.25% at day 4 in the bone marrow, respectively. upper panel, FACS scans for syndecan-1 and Blimp^{gfp}. middle panel, syndecan-1 and B220 in gfp-positive gated cells. lower panel, histograms for syndecan-1 and B220 expression in GFP-positive cells at indicated time points.

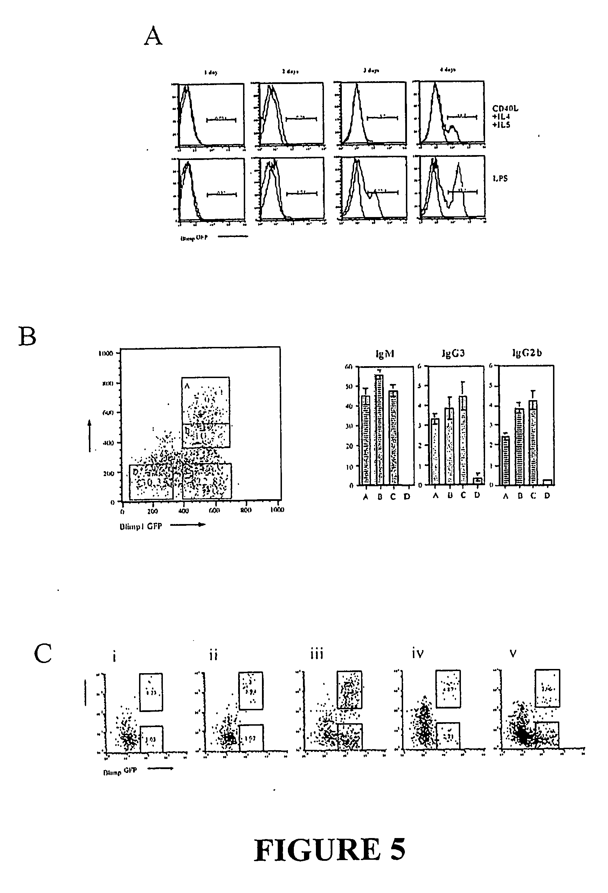

**Figure 5A** is a graphical representation of the kinetics of Blimp^{gfp} expression. Flow cytometry histograms of Blimp^{gfp} expression by stimulated B cells from Blimp^{gfp/+} mice (red line) and wild type C 57B1/6 mice (blue me) are shown. Histogram gates show a percentage of Blimp^{gfp} positive populations. Highly purified small resting B cells were stimulated recombinant CD40L, IL-4 and IL-5 (top panels) or LPS (20ug/ml) (bottom panels). Cells were harvested different days of culture time and analysed on flow cytometry. LPS stimulated cells start to express Blimp at 2 days, while in response to CD40L and IL-4/IL-5 Blimp expression become evident 3 days.
**Figure 5B** is a graphical representation showing that Blimp^{gfp} positive cells secrete antibodies. Blimp^{gfp/+} B cells were stimulated with LPS for four days. Cells were harvested and stained with Syndecan-1 (Synd-1) specific antibodies and GFP expressing (left panel, A-C) and non-expressing regions (left panel, D) were sorted directly to the Elispot plates coated with various isotype specific antibodies, using automated cell deposition unit. Sorted cells were processed according to the standart Elispot method. Right panels show number of Ig secreting cells in sorted regions. Most Blimp^{gfp} cells secrete Ig, while all Blimp^{gfp} negative cells do not secrete any of Ig isotypes tested.
**Figure 5C** is a graphical representation showing the different expression of Blimp^{gfp} in response to various stimuli. Highly purified small resting B cells were stimulated with i) re combinant CD40L and IL-4; ii) CD40L, IL-4 and IL-5; iii) LPS; iv) LPS and IL-4; v) LPS and anti-IgD monoclonal antibody. After four days of culture cells were harvested, stained with Synd-1 specific antibody and analysed on flow cytometry. Shown here are two parameter dot plots of flow cytometry analysis.

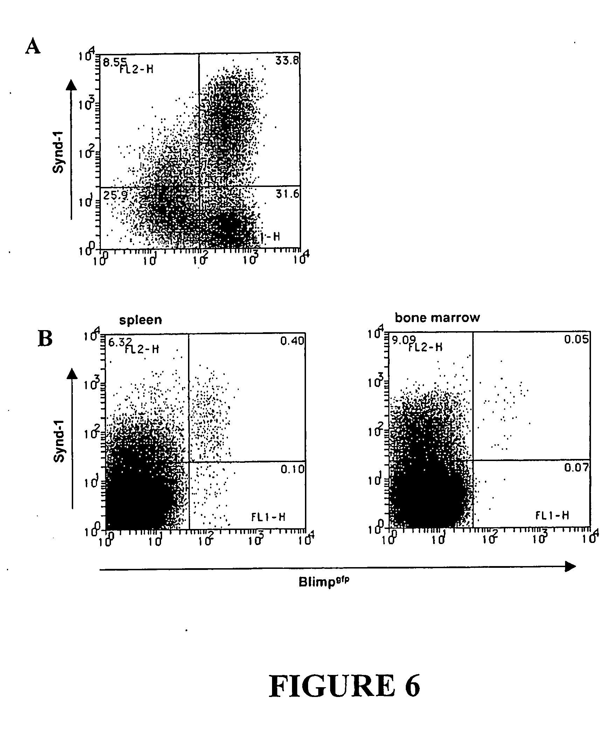

**Figure 6** is a graphical representation showing the results of analyses of mice transplanted with activated B-cells. Purified resting splenic B-cells of Blimp^{gfp/+} mice were activated for three days in the presence of 20ug/ml LPS. 3x10⁶ cells (containing about 2x10⁶ gfp positive cells, i.e. antibody secreting cells, A) were washed three times with LPS and transplanted into WT recipients by i.v. injection. After 7 days the recipient mice were analysed for the presence of donor ASC (B).

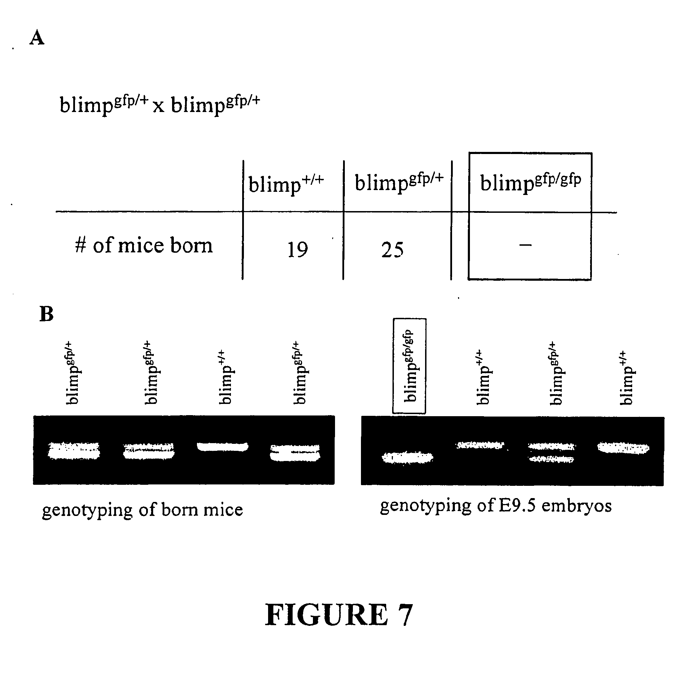

**Figure 7A** is a tabulated summary of genotyping results of mice bom from Blimp^{gfp/+} x blimp^{gfp/+} matings. **Figure 7B** is a photographical representation of representative PCR results of genotyping of mice weaned (left) or embryos at day E9.5 (right).

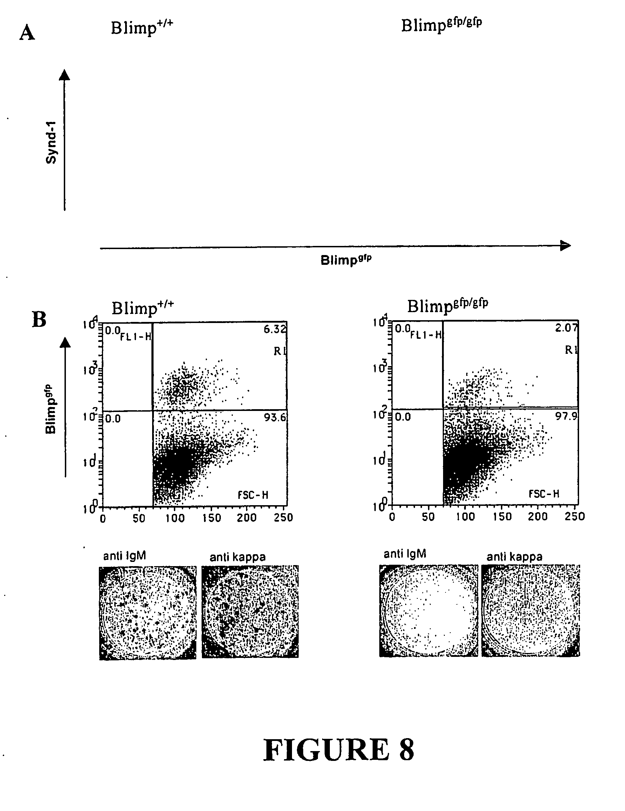

**Figure 8** is a photographic and graphical representation of splenocytes of Blimp^{gfp/gft} and Blimp^{gfp/+} reconstituted mice were cultured in the presence of 20ug/ml LPS and analysed for the presence of GFP positive, i.e. antibody secreting cells, at day three (A). GFP positive cells of both cultures were than sorted (B, gate R1) and analysed in an ELIspot assay. While Blimp^{gfp/+} cells yielded 60-70% antibody secreting cells (B, lower panel left), Blimp^{gfp/gft} gave only 5-7% antibody secreting cells which produced only tiny ELIspot's (B, lower panel, right) compared to spots produced by heterozygous cells. Detection of IgM and kappa chain in single representative wells of an ELISPOT plate (input 200 gfp-positive cells).

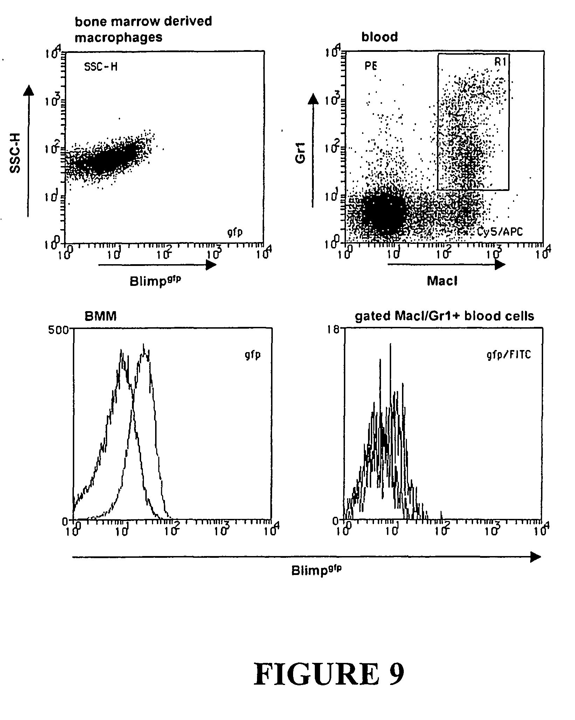

**Figure 9** is a graphical representation of the results of FACS analysis of bone marrow derived macrophages (BMM) and blood monocytes. Bone marrow cells were cultured for 7 days in the presence of 10ng/ml rMCSF, medium was changed and non-adherent cells were removed at day 3 and 5 of culture. Adherent cells (BMM) were analysed for Blimp^{gfp} expression (left panel). Further, MacI/Grl double positive blood cells were analysed in FACS (right panel) (black line - wildtype, red line - Blimp^{gfp/+}).

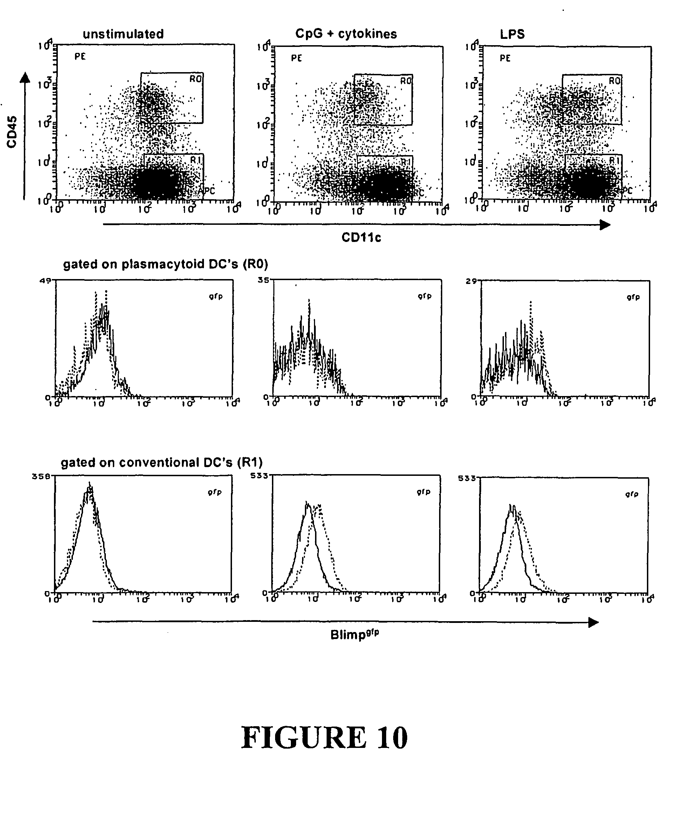

**Figure 10** is a graphical representation showing FACS analysis *in vitro* generated dendritic cells (DC's). Bone marrow cells were cultured for 8 days in the presence of 100ng/ml Flt3 ligand. Cells were than cultured for another 24 hours (left column) or were stimulated with CpG (1.5uM), GMCSF (50ng/ml), gIFN (20ng/ml) and IL-4 (20ng/ml) (middle column) or with 1ug/ml LPS (right column). Blimp^{gfp} expression is shown in a histograms for plasmacytoid DC's and conventional DC's (solid line - wildtype, dotted line - *Blimp*^{*gfp*/}*⁺).*

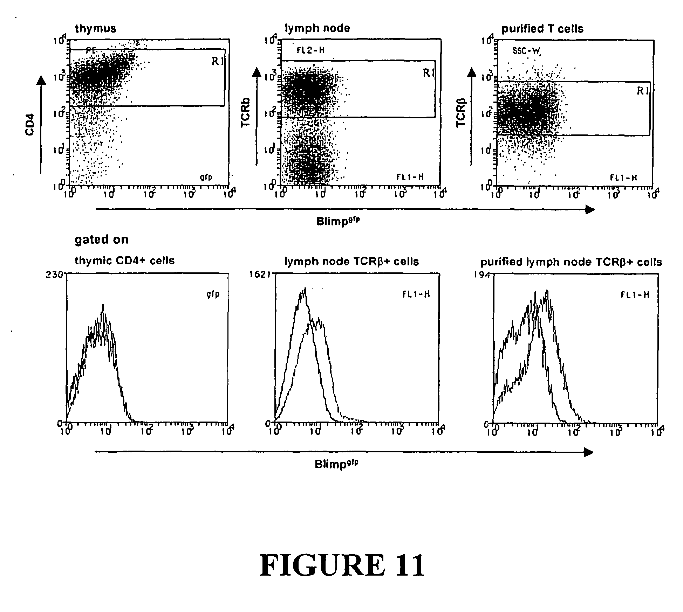

**Figure 11** is a graphical representation showing FACS analysis of T cells *in vivo* and 111 *in vitro.* Thymic (left) and lymph node (middle) T cells, and *in vitro* activated CD4⁺/CD8⁺ purified lymph node cells (right) of *Blimp*^{*gfp*/*+*} mice were analysed in FACS. *Blimp^{gfp}* expression levels of gated T cell populations are shown in histograms (lower panel; black line - wildtype, red line - *Blimp*^{*gfp*/*+*}).

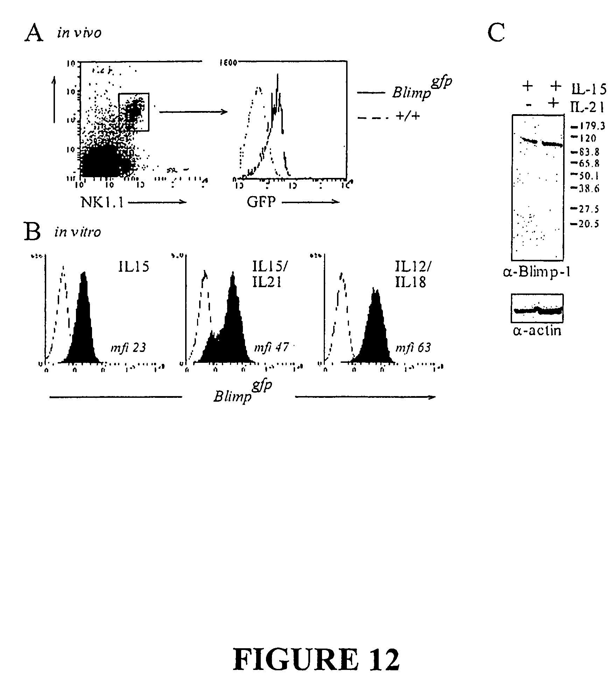

**Figure 12** is a graphical representation showing *Blimp-1* expression in the NK lineage can be detected in the *Blimp*^{*gfp*/*+*} reporter mice and induced by maturation stimuli. A) *in vivo* splenic NK cells are GFP⁺. B) Sorted NK cells from *Blimp*^{*gfp*/*+*} spleens were cultured for 4 days in IL-15, followed by 2 days in the indicated cytokine. mfi, mean fluorescence index of *Blimp*^{gfp}*.* C) Expression of *Blimp-1* in ⁺/⁺ NK cells cultured for 7 days *ex vivo* in IL-15 ^{+/-}IL-21. *Blimp*-1 expression was detected using a monoclonal antibody against mouse Blimp-1, a goat polyclonal antibody against α-actin was used as a loading control.

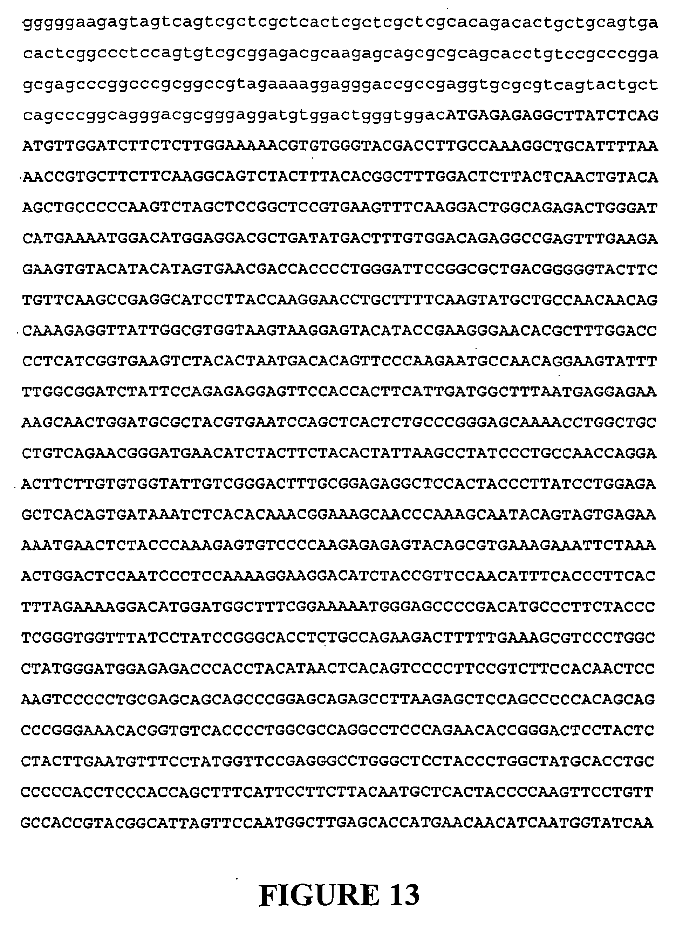

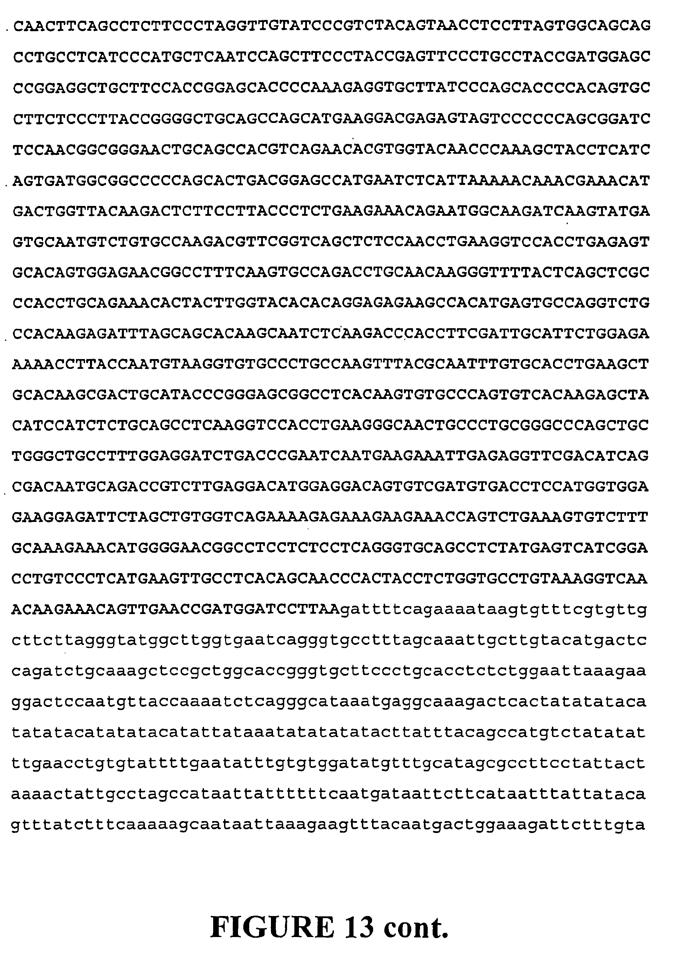

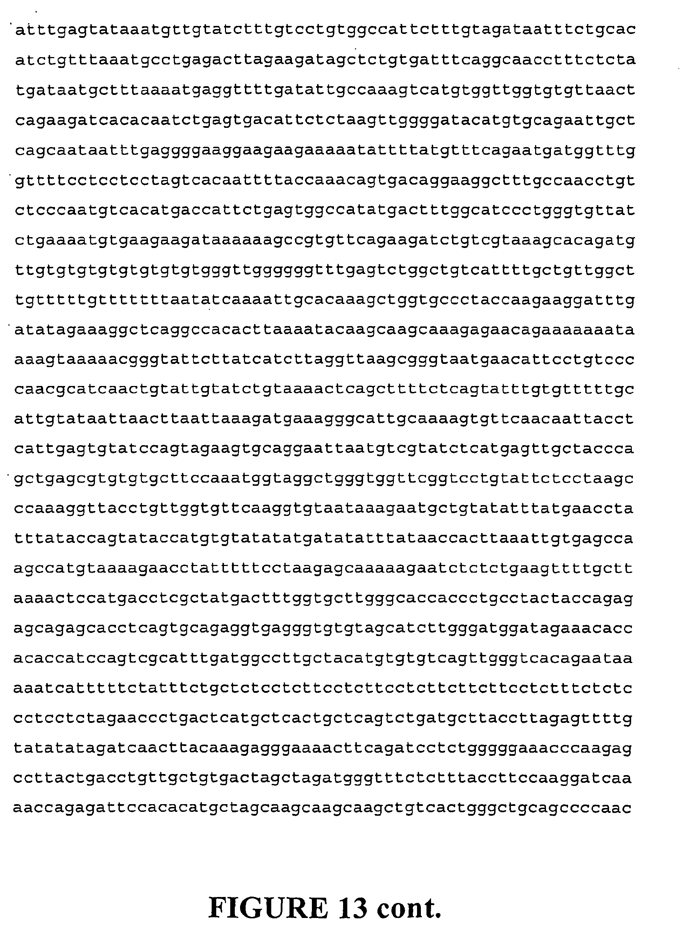

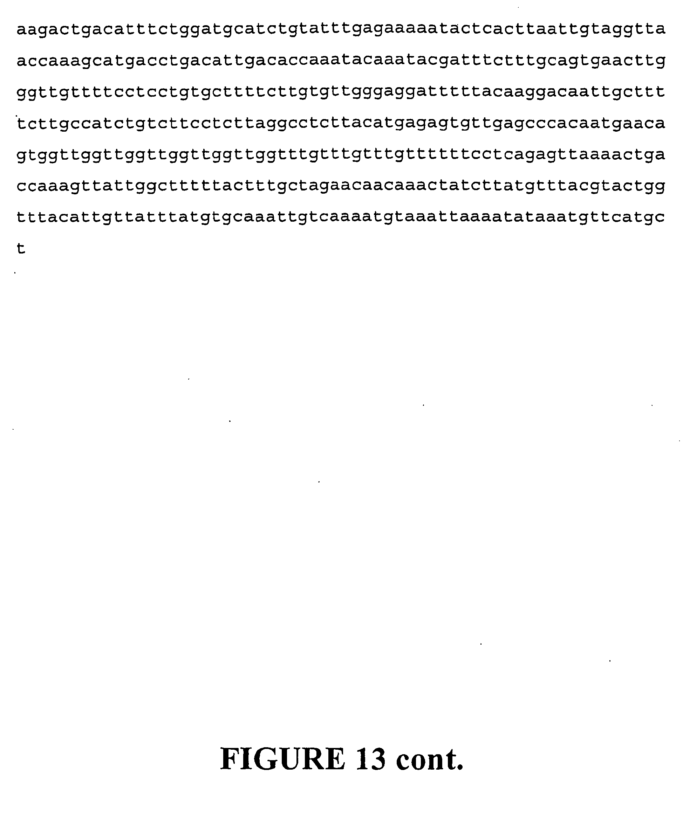

**Figure 13** is a representation showing the cDNA and predicted amino acid sequence of mouse Blimp-1/PRDM-1. The coding sequence is shown in upper case.

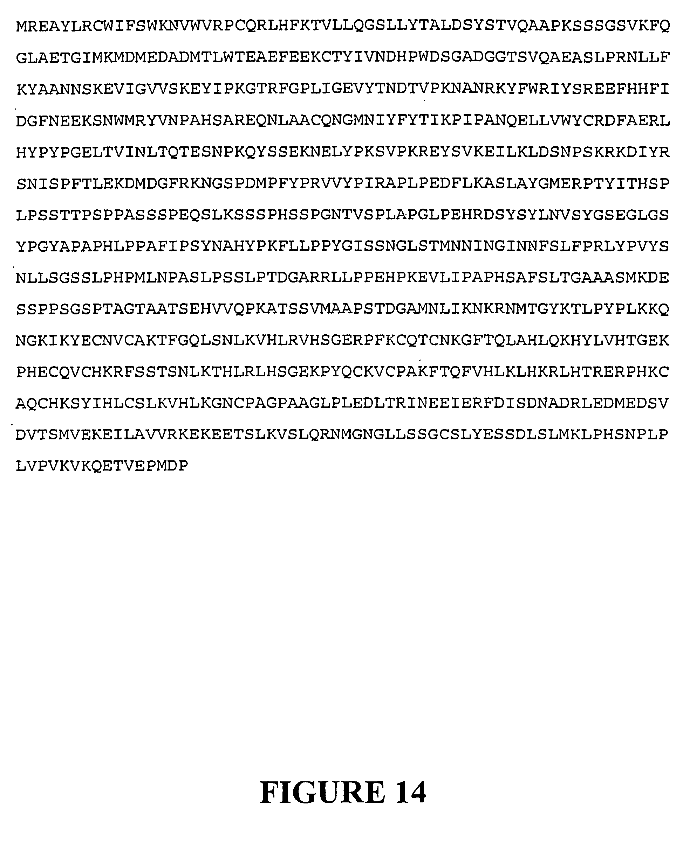

**Figure 14** is a representation showing the amino acid sequence of mouse Blimp-1/PRDM-1 derived from the nucleotide sequence (upper case) in Figure 13.

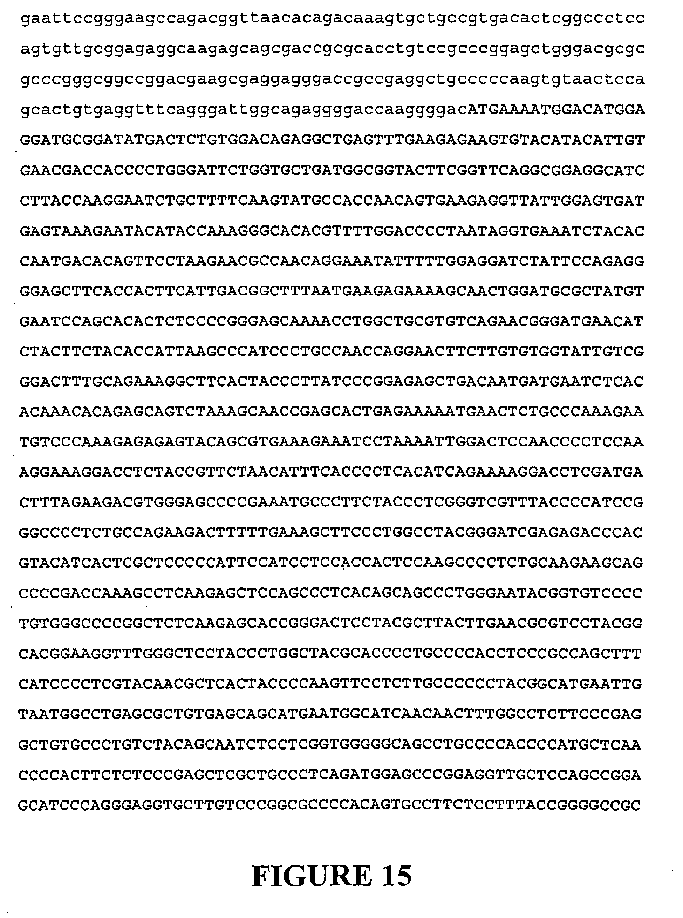

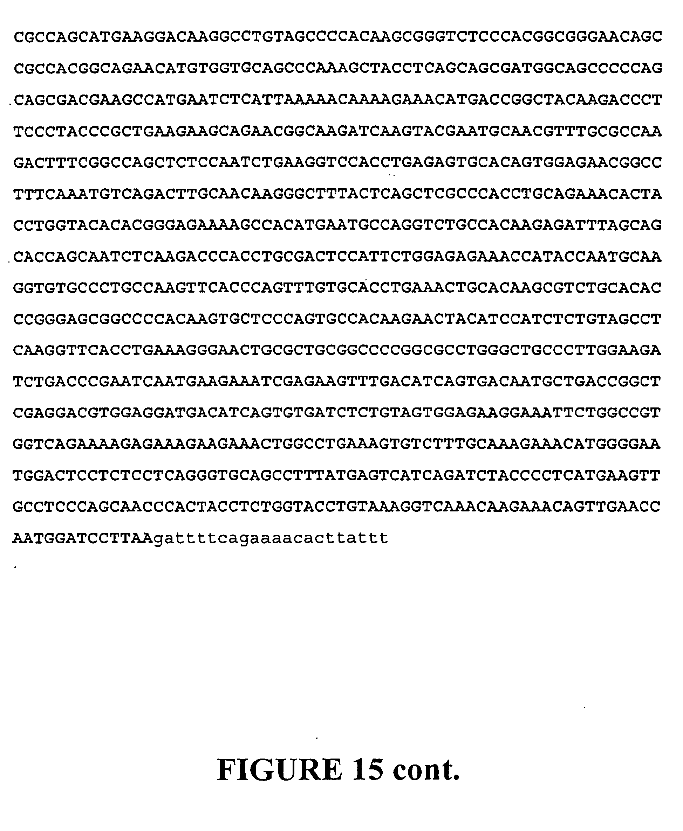

**Figure 15** is a representation showing the cDNA and predicted amino acid sequence of human Blimp-1/PRDM-1. The coding sequence is shown in upper case.

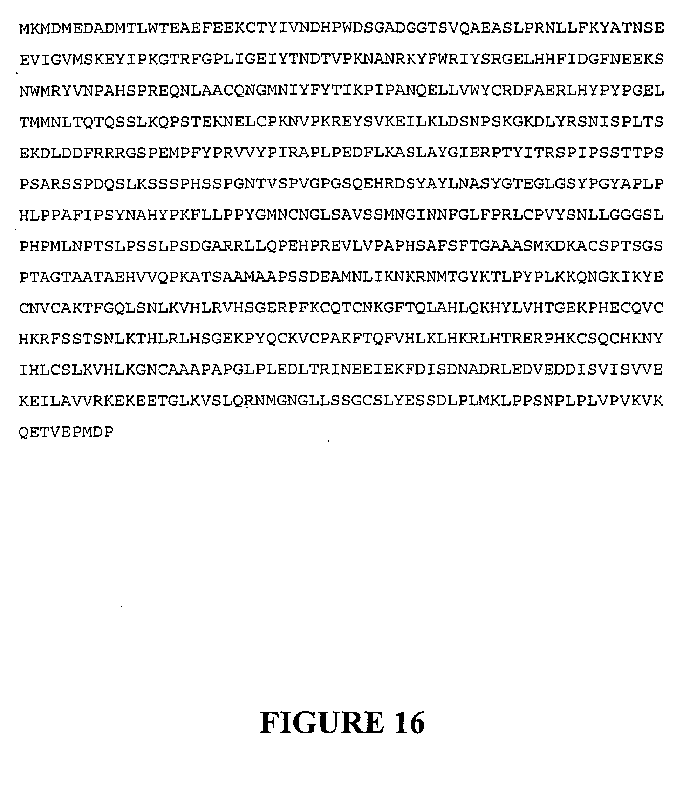

**Figure 16** is a representation showing the amino acid sequence of human Blimp-1/PRDM-1 derived from the nucleotide sequence (upper case) in Figure 15.

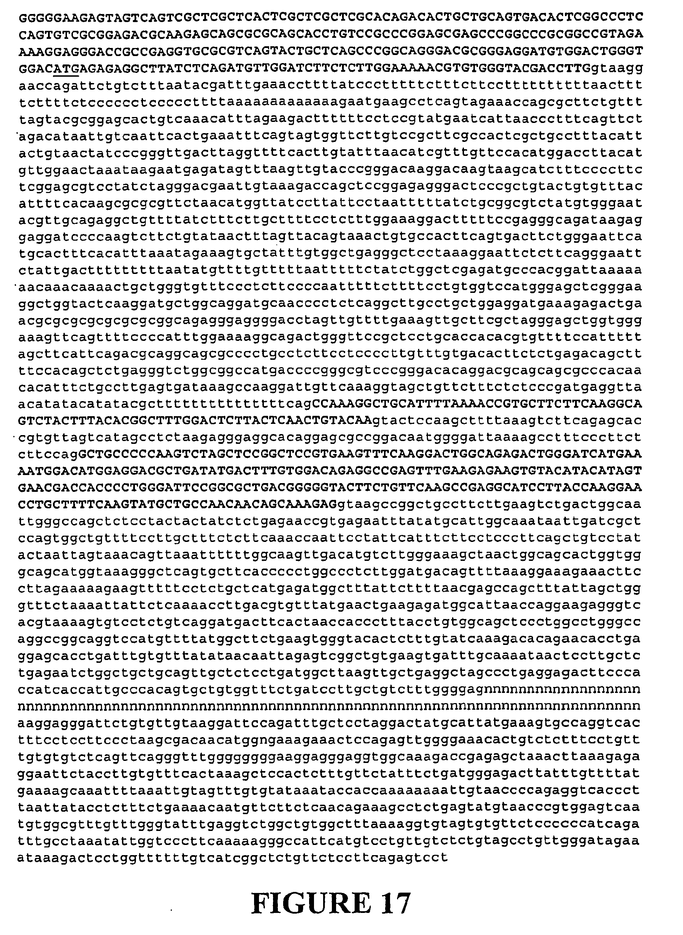

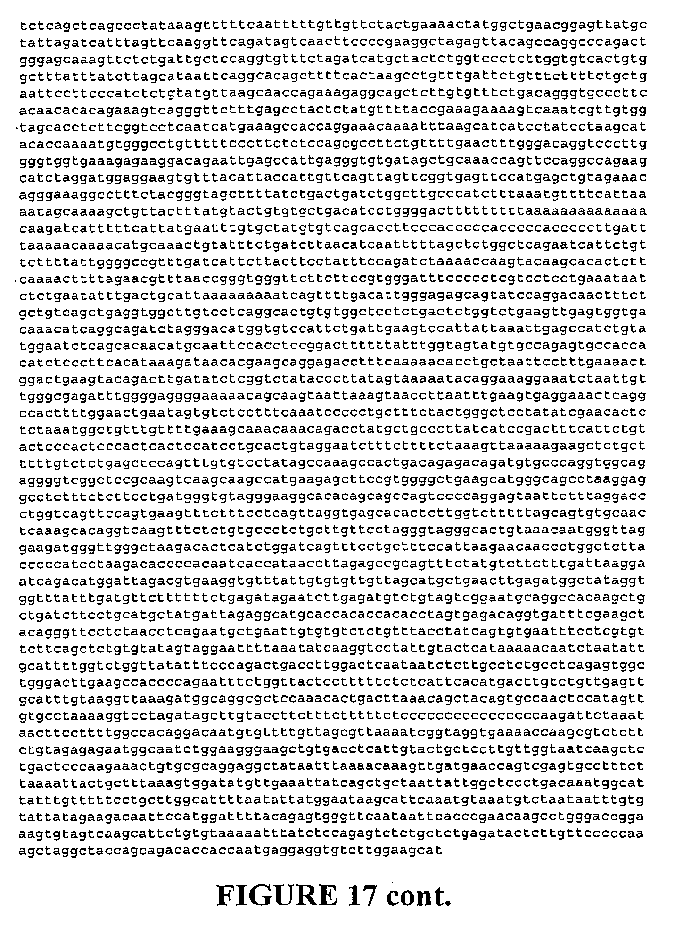

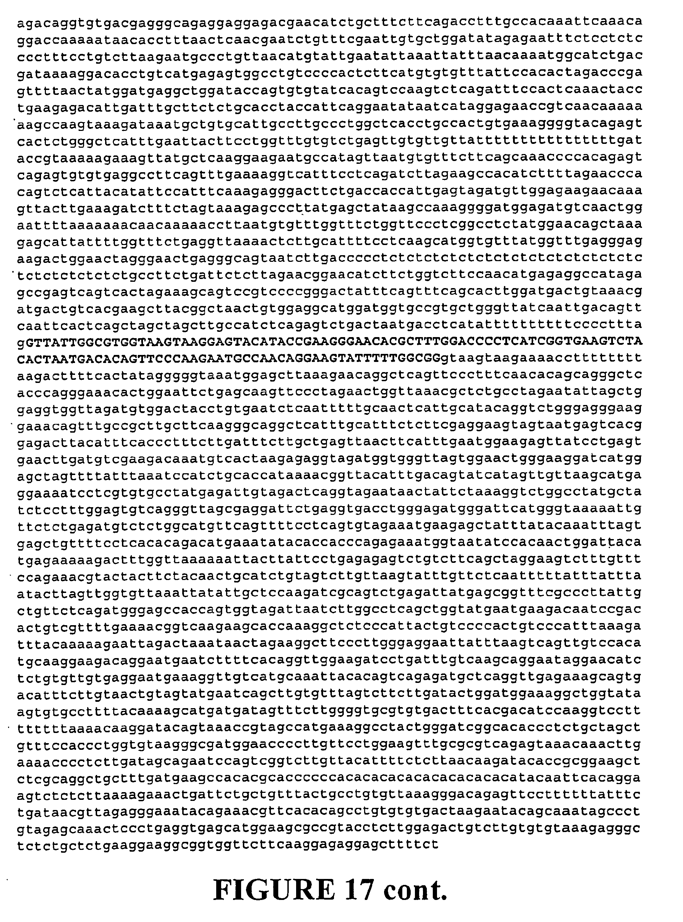

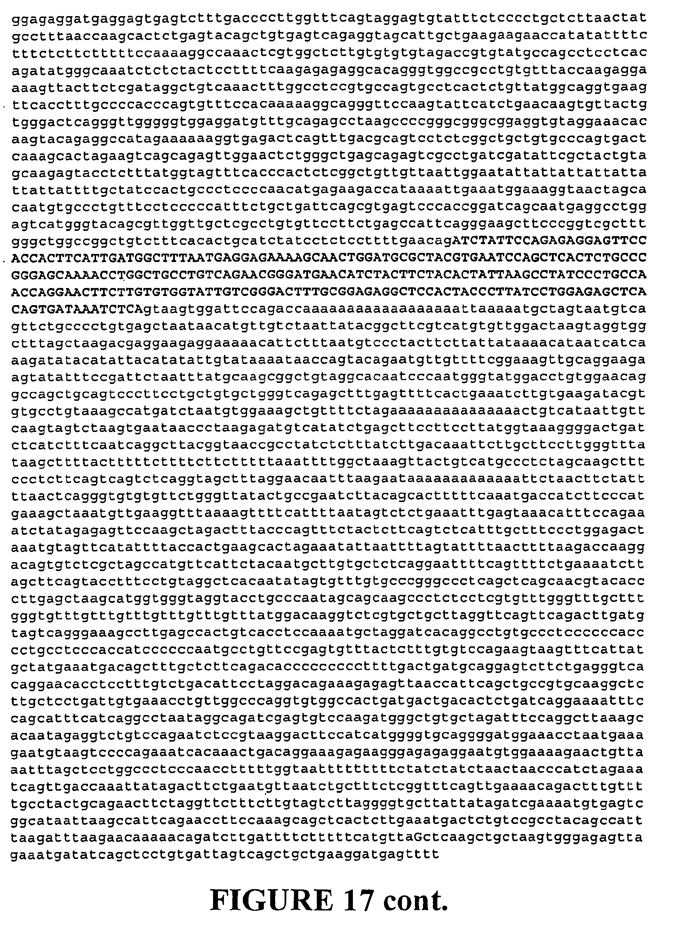

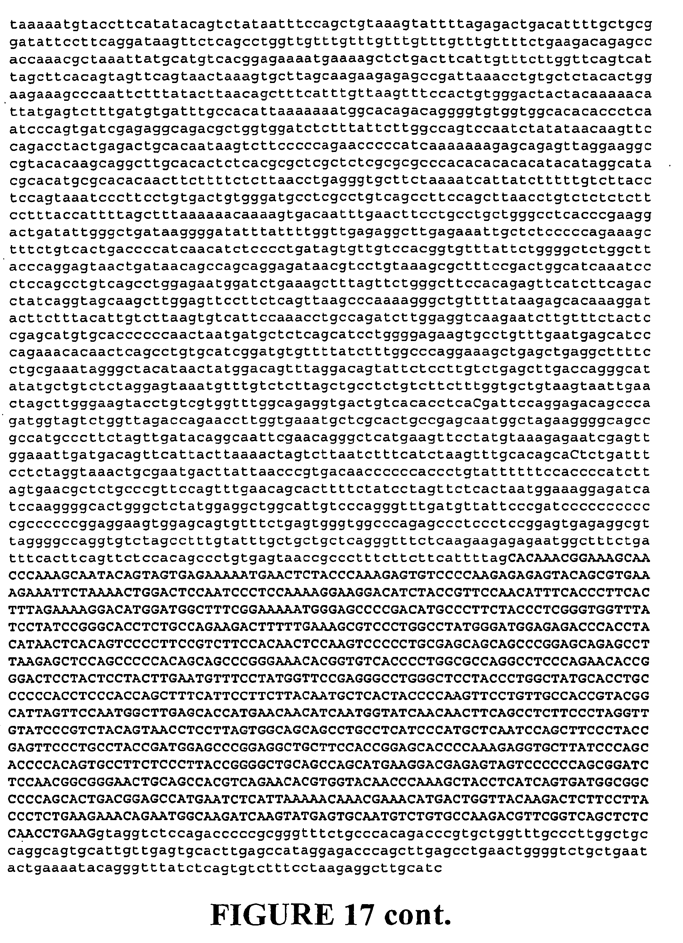

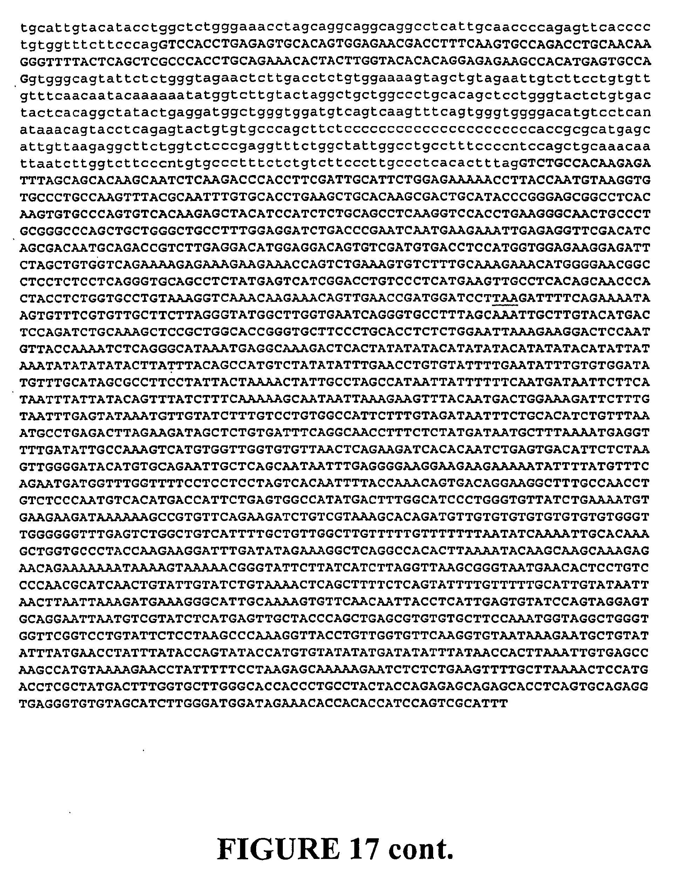

**Figure 17** is a representation showing the genomic nucleotide sequence of mouse *Blimp-1.*
The genomic locus comprises 8 exons in bold upper case. ATG and stop codons are underlined.

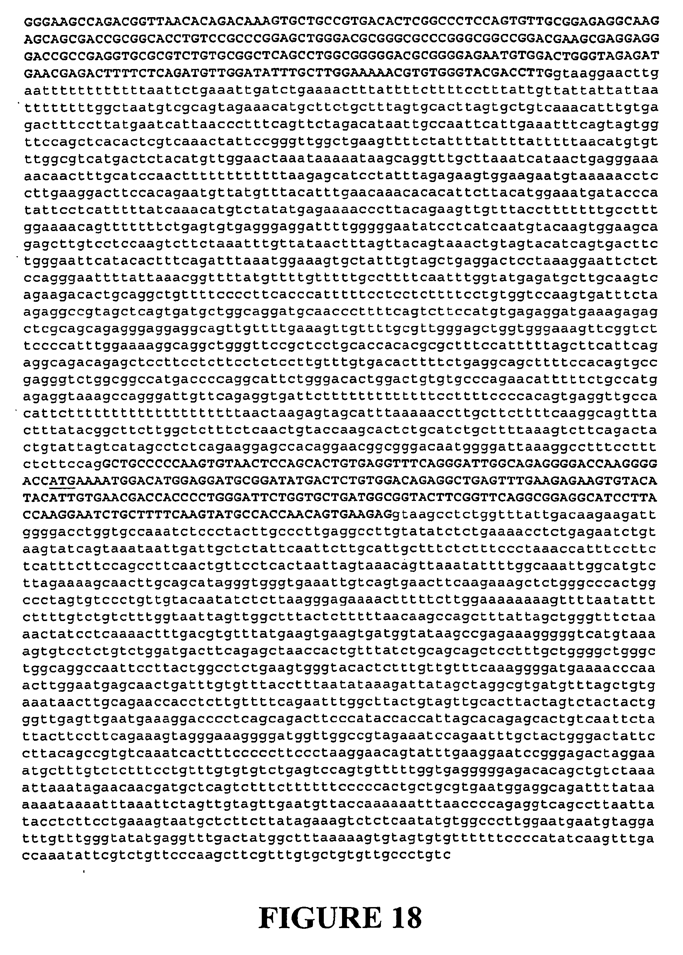

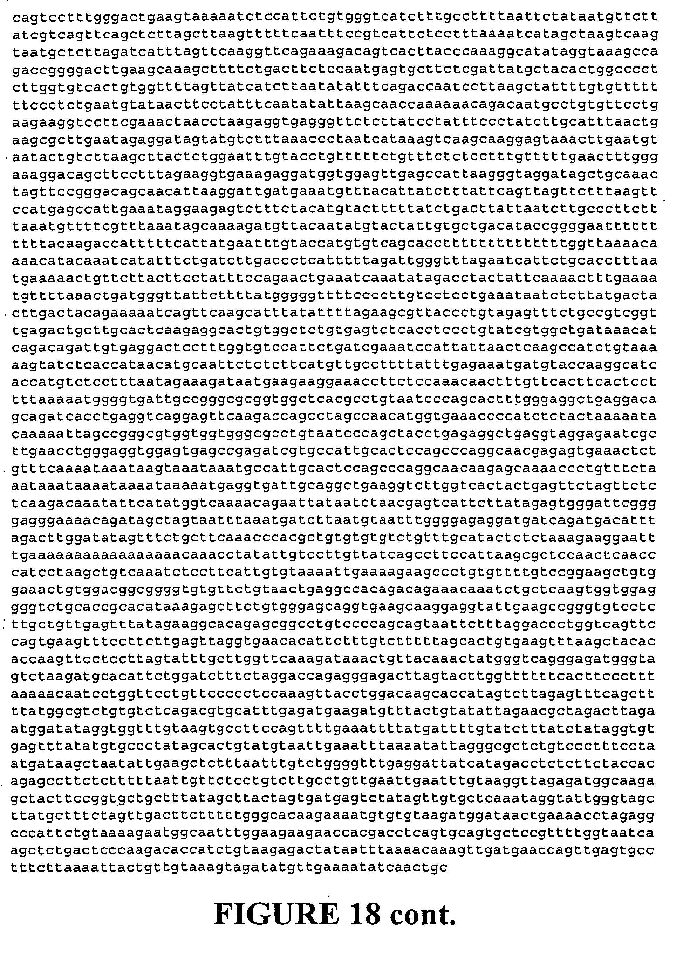

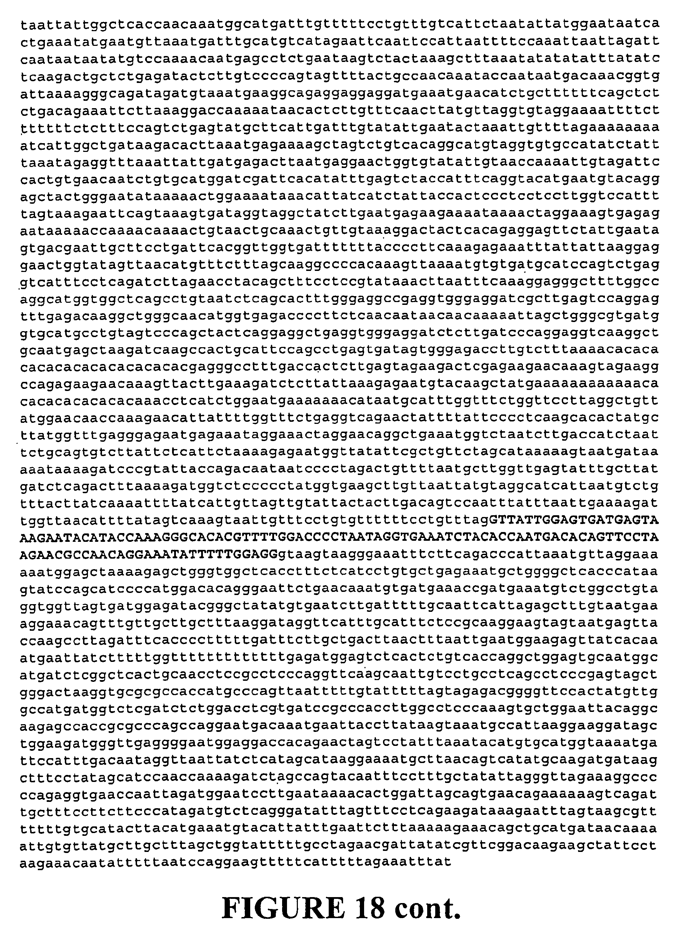

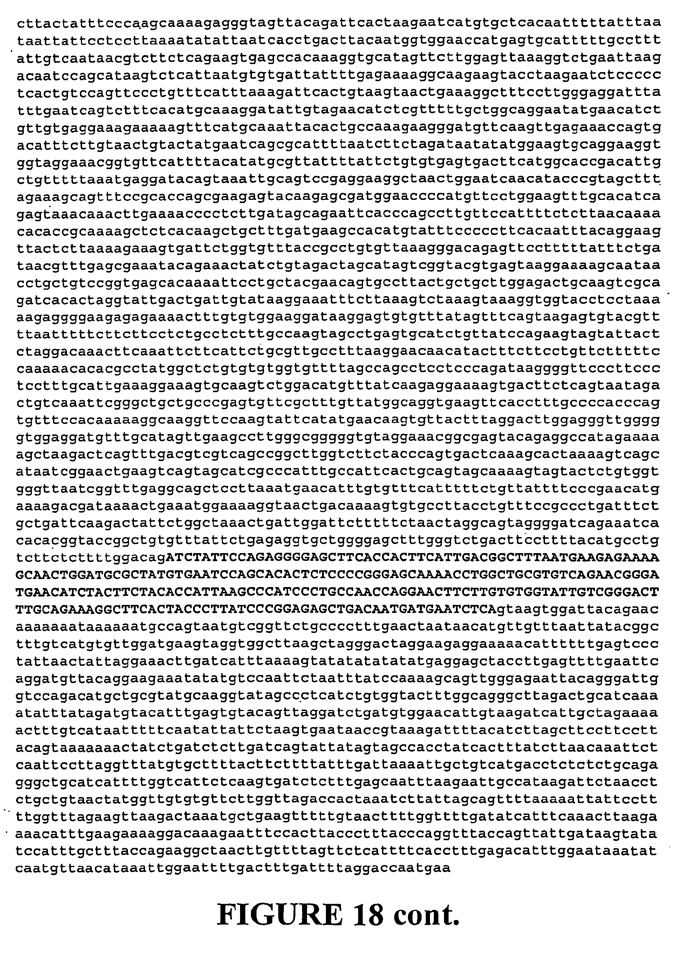

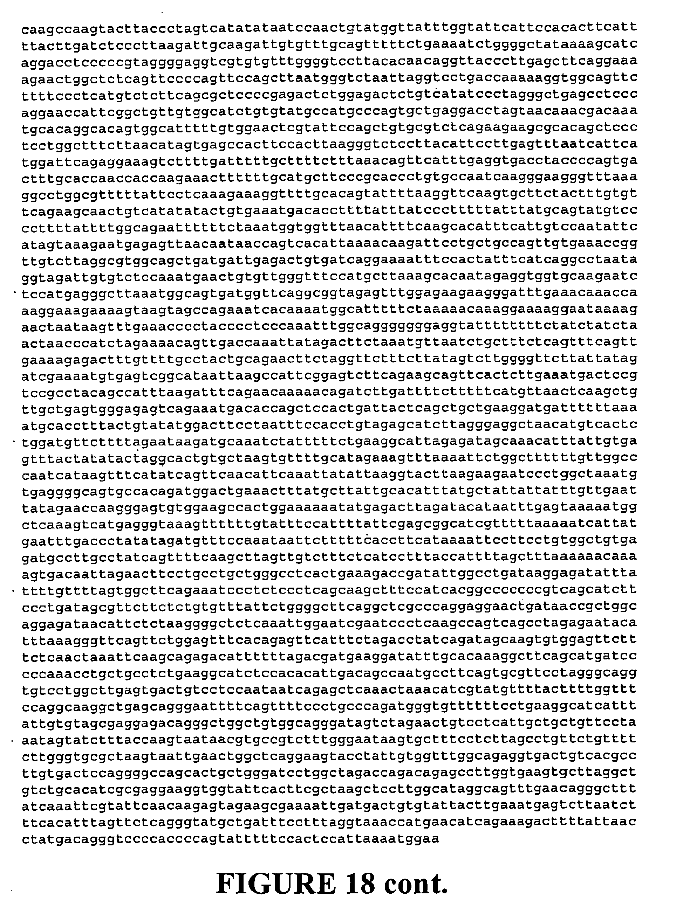

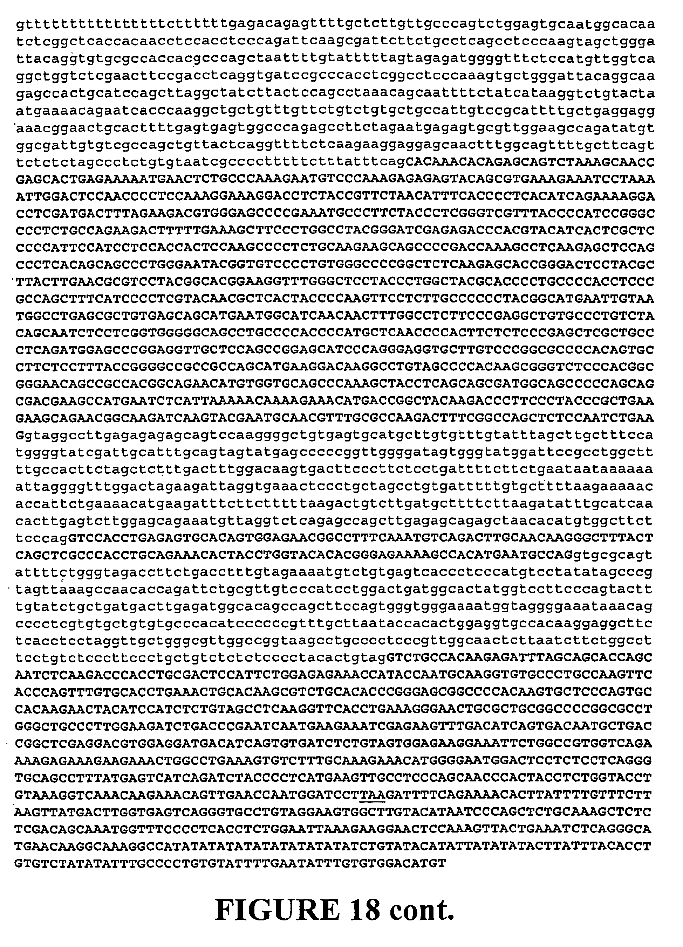

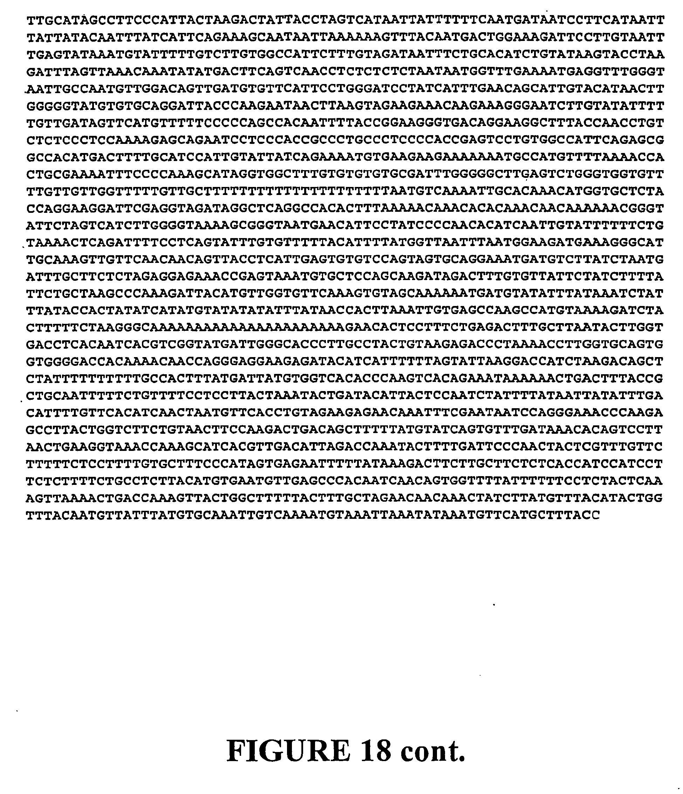

**Figure 18** is a representation showing the genomic nucleotide sequence of human *Blimp-1.*
The genomic locus comprises 8 exons in upper case, bold. ATG and stop codons are underlined.

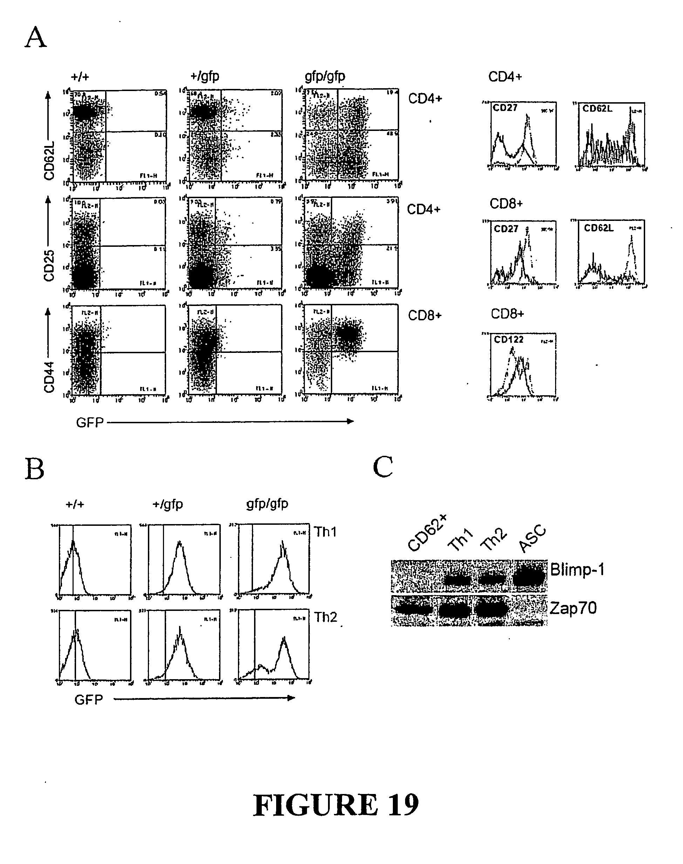

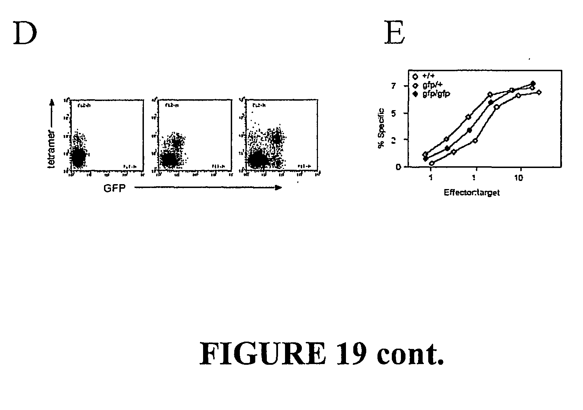

**Figure 19** is a graphical representation showing that Blimp-1 is expressed in activated/memory T cells. Blimp-1 deficient T cells have an activated/memory phenotype. A) Gated CD4⁺ or CD8⁺ splenic T cells of the indicated genotype were examined for GFP fluorescence and activation state. The majority of CD62L^{low} CD4⁺ T cells and CD44^{high} CD8⁺ T cells from *Blimp*^{*gfp*/+} mice is low for GFP, only a small number of CD4⁺ T cells (CD62L low or high) is GFP high Blimp GFP is strongly expressed in the same population in *Blimp*^{*gfp*/*gfp*} T cells (dot blots); phenotype of Blimp-1 deficient CD4⁺ and CD8⁺ splenic T cells (histograms, solid line blimp-1⁺/⁺, dotted line blimp-1gfp/gfp). B) *in vitro* culture induces Blimp-1 expression. Naïve CD4⁺ T cells were grown for two rounds in Th1/Th2 polarizing conditions. C) Western blotting for wild type Blimp-1 protein in CD4⁺ cells grown as above in Th1 or Th2 conditions. Anti-Zap-70 is used as a loading control. B cells stimulated for four days with LPS to induce plasma cell differentiation were used as a positive control. D) Mice reconstituted with the indicated genotypes were analysed post-HSV infection for the appearance of CD8⁺ T cells specific for the dominant gB₄₉₈₋₅₀₅ epitope using a gB specific tetramer. E) *in vitro* cultured gB-specific CTL show normal cytotoxic function. The HSV infection data is representative of at least three mice of each genotype.

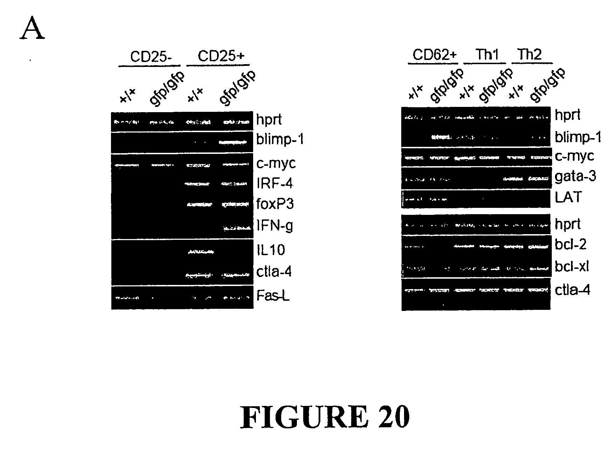

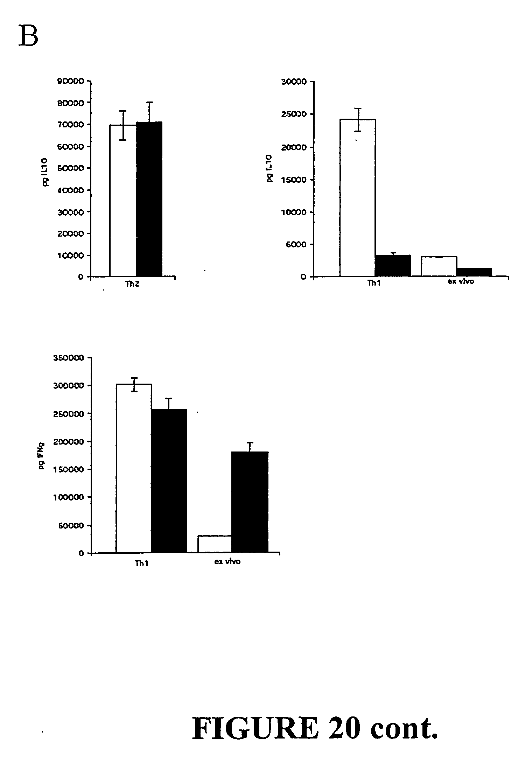

**Figure 20** is a representation of the molecular analysis of Blimp-1 positive CD4⁺ T cell populations. A) Blimp-1 is expressed in CD25⁺ suppressor T cells. CD25⁺ and CD25-CD4⁺ T cells were sorted (left panel), and naïve CD4⁺ were differentiated under Th1 and Th2 polarizing conditions (right panel), all populations were subjected to RT-PCR analysis. B) Blimp-1 deficient CD4⁺ cells secrete high amounts of IFNg and show defective IL-10 secretion. CD4⁺ cells were either sorted ex vivo from the spleen and restimulated with plate bound anti CD3 and CD28 for 24h or differentiated into Th1 or Th2 cells *in vitro* and subjected to re-stimulation. IL-10 and IFNg in the supernatant was detected in an ELISA.

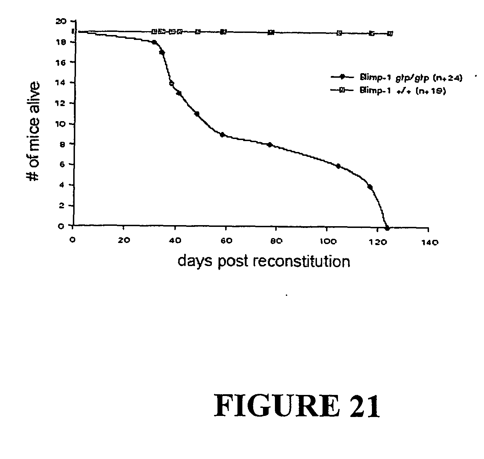

**Figure 21** is a representation showing that Blimp-1 deficient mice develop a lethal lymphocyte hyperproliferative syndrome. A) Histological examination of *Ragl*^{*-*/*-*} mice reconstituted with control or *Blimp*^{*gfp*/*gfp*} foetal liver derived stem cells. *Blimp*^{*gfg*/*gfp*} reconstituted mice were sacrificed when moribund. The normal histological appearance of the organs from *Blimp*^{*gfp*/*+*} mice is contrasted with the lymphocyte infiltration observed in *Blimp*^{*gfp*/*gfp*} mice. B) rapid onset of morbidity in *Blimp*^{*gfp*/*gfp*} but not *Blimp*^{*gfp*/*+*} reconstituted mice. The number of animals of each genotype examined is indicated.

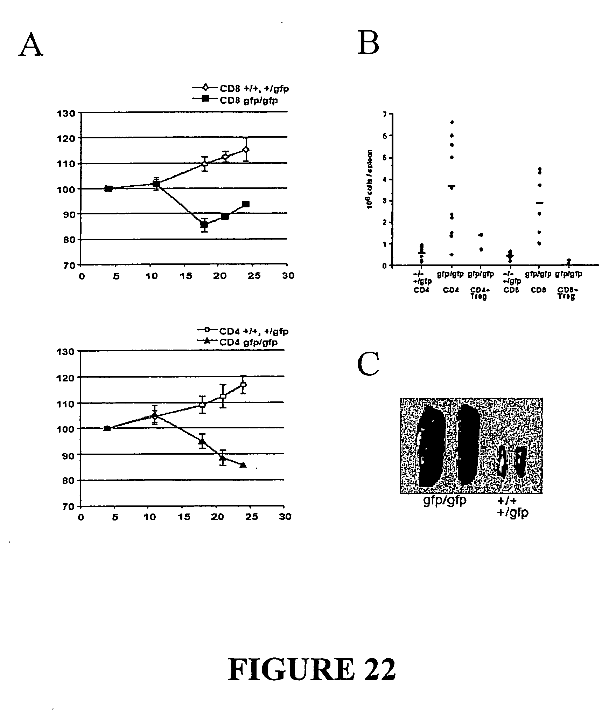

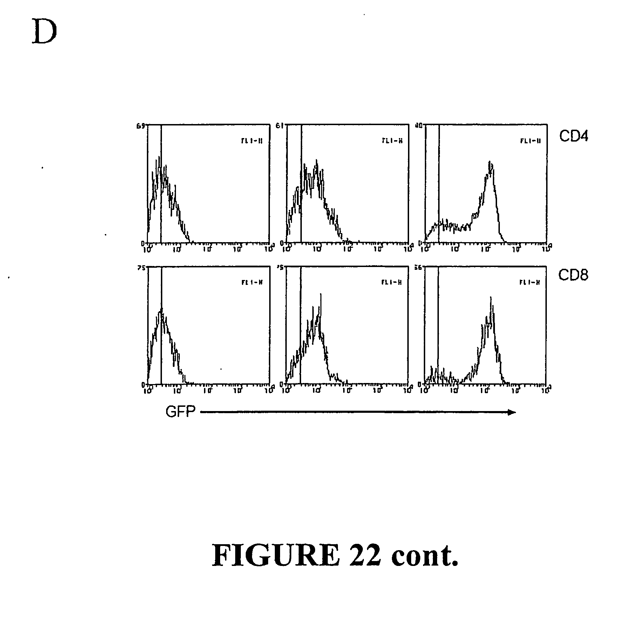

**Figure 22** is a graphical representation of data showing that Blimp-1 regulates homeostatic proliferation of T cells. A) 3x10⁶ Naïve CD4⁺ or CD8⁺ splenic T cells from the indicated genotypes were adoptively transferred into non-irradiated *Rag2*^{*-*/*-*} recipients. Recipients were monitored for weight loss and signs of distress. Graph indicates the percentage changes in weight over the 3-4 week period. 4-6 mice were reconstituted with cells of each genotype. B) Mice that lost >10% body weight were sacrificed and splenic T cell numbers determined. C) splenomegaly of representative *Rag2^{-l-}* mice after transfer of *Blimp*^{*gfp*/*gfp*} CD8⁺ T cells. D) Flow cytometric analysis of Blimp^{gfp} expression in donor T cells 3 weeks after transfer.

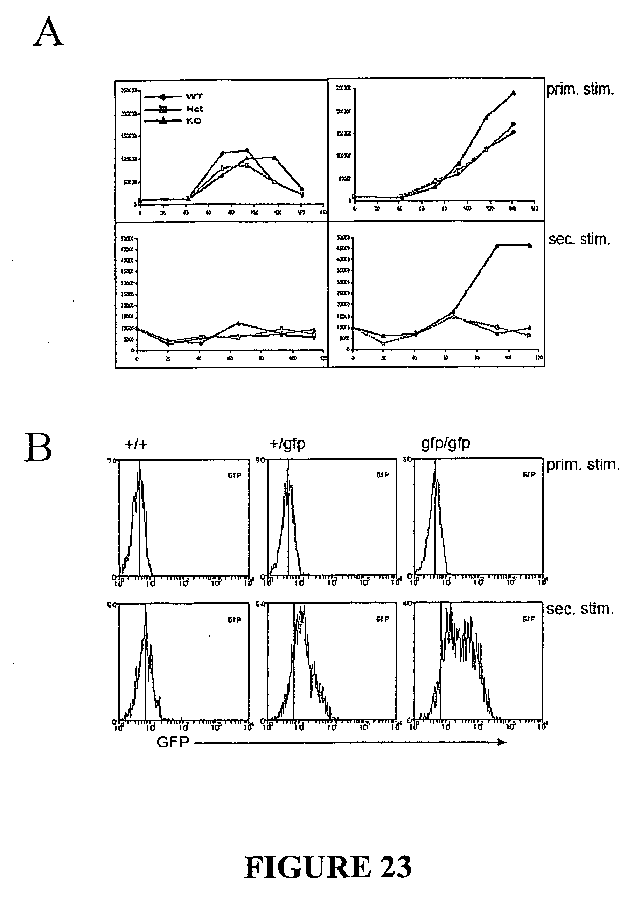

**Figure 23** is a representation of data showing that Blimp-1 is induced after secondary stimulation and regulates cytokine responsiveness. A) Naïve CD8⁺ T cells of the indicated genotypes were cultured in the presence of anti-CD3/CD28 and IL-2 for 5 days before being re-seeded into secondary cultures containing IL-21 for 5 days. B) Identical cell cultures to above were stimulated with anti-CD3/CD28 with or without the indicated cytokine combinations. Total live cell number was determined at the indicated time-points in primary or secondary cultures. CD44^{high} activated/memory CD8⁺ cells were subjected only to primary culture.

### DETAILED DESCRIPTION OF THE PREFERRED EMBODIMENTS

The present invention is predicated, in part, by the development of a method for identifying and isolating cells of the haematopoietic system or embryonic cells and/or monitoring the differentiation of haematopoietic or embryonic cells, the method comprising detecting or quantifying the presence of a polypeptide (via a reporter) whose presence is associated with terminal differentiation of the cells.

In a particularly preferred embodiment, the polypeptide is Blimp or a part, fragment or functional form thereof which is co-expressed with a reporter molecule.

Accordingly, one aspect of the present invention provides a genetically modified cell or non-human organism comprising such cells comprising genetic material encoding a polypeptide which when expressed produces the polypeptide co-expressed with a reporter molecule and which polypeptide is associated with a cellular phenotype including a commitment in the cell to terminally differentiate.

In a further aspect, the present invention provides a genetically modified cell or non-human organism comprising such cells comprising a modified *Blimp* gene encoding a Blimp polypeptide which when expressed produces Blimp or a part, fragment or functional form thereof co-expressed with a reporter molecule and wherein the presence of Blimp is associated with a cellular phenotype including a commitment in the cell to terminally differentiate.

In a further preferred aspect, the present invention provides a genetically modified cell or non-human organism comprising such cells comprising a modified *Blimp* gene encoding a Blimp mRNA transcript comprising a Blimp coding sequence or a part, fragment or functional form thereof and a reporter molecule encoding sequence, wherein the presence of Blimp is associated with a cellular phenotype including a commitment in the cell to terminally differentiate.

Preferably, the reporter molecule encoding sequence is inserted with an intron of the *Blimp* allele. In this way, the modified *Blimp* allele co-produces the reporter from a bicistronic RNA under the control of endogenous *Blimp* regulatory elements.

The terms "co-expression" and "co-production" are used herein in a broad sense to refer to the transcription of two or more nucleic acid regions (expressed as one or more RNAs) at the same time or at substantially the same time and their subsequent translation (produced as one or more polypeptides) at the same or substantially the same time. Preferably, one transcript is expressed which encodes both Blimp or a part, fragment or functional form thereof and a reporter molecule. In each case, the expression of the reporter is operatively linked to the expression of the molecule to be reported.

Reference to "cellular phenotype" herein encompasses the molecular or functional characteristics of a cell. For example, ASC cells express *Blimp-1* (a molecular marker) and are functionally distinguished from other B-cells by exhibiting, *inter alia,* a high rate of Ig secretion, the absence of MHC class II molecules and low levels of surface Ig. As used herein, the term is a reference to the full range of molecular or functional characteristics, or any particular molecules or functional characteristic in addition to the molecular characteristic of modulated levels of *Blimp-1* expression.

The genetically modified cell or non-human organism comprising such cells may comprise cells or genetic material from any organism such as, but not limited to, humans, non-human primates, livestock, companion or laboratory test organism, reptilian or amphibian species. Preferably the genetically modified organism is a mouse or other laboratory test animal such as a rat, guinea pig, pig, rabbit or sheep.

As used herein the singular forms "a", "an" and "the" include plural aspects unless the context clearly dictates otherwise. Thus, for example, reference to a "cell" includes a single cell, as well as two or more cells; reference to "a gene" includes a gene, as well as two or more gene; and so forth.

The modified gene of the present invention is a marker for terminal differentiation in cells of the haematopoietic system, such as B-cell lineage cells.

Reference to a "genetically modified cell" is a reference to any cell which has been engineered to comprise a sequence of nucleotides from a coding or non-coding region of the genome which is altered relative to its pre-modified form, and its progeny. In particular, the cell is genetically modified to co-express a genetic marker of terminal differentiation and a reporter molecule encoding sequence. Preferably, the cell is genetically modified to co-express Blimp or a part, fragment or functional part thereof and a reporter molecule. The reporter molecule may be any molecule capable of directly or indirectly providing an identifiable signal. A fluorescent or other light emitting reporter molecule is particularly preferred.

Conveniently, targeting constructs are initially used to generate the modified genetic sequences in the cell or organism. Targeting constructs generally but not exclusively modify a target sequence by homologous recombination. Alternatively, a modified genetic sequence may be introduced using artificial chromosomes. Targeting or other constructs are produced and introduced into target cells using methods well known in the art which are described in molecular biology laboratory manuals such as, for example, in Sambrook, Molecular Cloning: A Laboratoy Manual, 3rd Edition, CSHLP, CSH, NY, 2001; Ausubel (Ed) Current Protocols in Molecular Biology, 5th Edition, John Wiley & Sons, Inc, NY, 2002. Targeting constructs may be introduced into cells by any method such as electroporation, viral mediated transfer or microinjection. Selection markers are generally employed to initially identify cells which have successfully incorporated the targeting construct. As the skilled artisan will appreciate, the subject modified organisms may be genetically modified to express the *Blimp* allele and reporter molecule in only certain cells.

In one particular embodiment the present invention provides a nucleic acid construct suitable for use as a targeting construct said construct comprising all or a portion of an allele of *Blimp-1* and a reporter construct. The construct comprise genetic material which encodes a functionally active Blimp-1 polypeptide or a functionally inactive Blimp-1 polypeptide. In a particular embodiment, the construct encodes a partial Blimp-1 polypeptide which lacks a zinc finger domain comprising a DNA binding motif. In a particularly preferred embodiment, the construct is flanked by sites to facilitate recombinase mediated deletion and homologous recombination of the nucleic acid construct into a target genetic sequence. Alternatively, the construct may be introduced into a host cell where it replicates episomally.

Genetically modified organisms are generated using techniques well known in the art such as described in Hogan et al., Manipulating the Mouse Embryo: A Laboratory Manual, Cold Spring Harbour Laboratory Press, CSH NY, 1986; Mansour et al., Nature 336:348-352, 1988; Pickert, Transgenic Animal Technology: A Laboratory Handbook, Academic Press, San Diago, CA, 1994. Stem cells including embryonic stem cells (ES cells) are introduced into the embryo of a recipient organism at the blastocyst stage of development. There they are capable of integration into the inner cell mass where they develop and contribute to the germ line of the recipient organism. ES cells are conveniently obtained from pre-implantation embryos maintained *in vitro* (Robertson et al., Nature 322:445-448, 1986). Once correct targeting has been verified, modified cells are injected into the blastocyst or morula or other suitable developmental stage, to generate a chimeric organism. Alternatively, modified cells are allowed to aggregate with dissociated embronic cells to form aggregation chimera. The chimeric organism is then implanted into a suitable female foster organism and the embryo allowed to develop to term. Chimeric progeny are bred to obtain offspring in which the genome of each cell contains the nucleotide sequences conferred by the targeting construct. Genetically modified organism may comprise a heterozygous modification or alternatively both alleles may be affected.

In accordance with the present invention it is surprisingly determined that Blimp-1 is essential for the production of antibody by ASC but not the commitment to differentiate down the ASC pathway. Accordingly, the identification of Blimp (eg via a reporter molecule co-expressed therewith) in B-cell lineage cells indicates that the cells are committed to differentiate or have differentiated into ASC.

Furthermore, as disclosed herein, Blimp is essential for lymphocyte homeostasis including T-cell homeostasis and the agility of T-cells to become terminally differentiated. The absence of Blimp in adult mammals leads to aggressive multi-organ lymphproliferative disease.

Accordingly, another aspect of the present invention provides a genetically modified cell or non-human organism comprising such cells comprising genetic material encoding a polypeptide which when expressed produces the polypeptide co-expressed with a reporter molecule wherein detection of said reporter molecule is indicative of a cellular phenotype and/or commitment of a cell to terminally differentiate.

In a further aspect, the present invention provides a genetically modified cell or non-human organism comprising such cells comprising a modified *Blimp* gene encoding a Blimp polypeptide which when expressed produces Blimp or a part, fragment or functional form thereof co-expressed with a reporter molecule and wherein detection of said reporter molecule is indicative of a cellular phenotype and/or commitment of a cell to terminally differentiate.

In a further preferred aspect, the present invention provides a genetically modified cell or non-human organism comprising such cells comprising a modified *Blimp* gene encoding a Blimp mRNA transcript comprising a Blimp coding sequence or a part, fragment or functional form thereof and a reporter molecule encoding sequence, wherein detection of said reporter molecule is indicative of a cellular phenotype and/or commitment of a cell to terminally differentiate

Preferably, the reporter molecule encoding sequence is inserted with an intron of the *Blimp,* allele. In this way, the modified *Blimp* allele co-produces the reporter from a bicistronic RNA under the control of endogenous *Blimp* regulatory elements.

Accordingly, another aspect of the present invention provides a genetically modified cell or non-human organism comprising such cells comprising genetic material encoding a polypeptide which when expressed produces the polypeptide co-expressed with a reporter molecule and wherein detection of said reporter molecule in cells of the haematopoietic system is indicative of a cellular phenotype and/or commitment of a cell to terminally differentiate.

In a further aspect, the present invention provides a genetically modified cell or non-human organism comprising such cells comprising a modified *Blimp* gene encoding a Blimp polypeptide which when expressed produces Blimp or a part, fragment or functional form thereof co-expressed with a reporter molecule and wherein detection of said reporter molecule in B-cells is indicative that cells having reporter molecule activity are committed to differentiation into ASC.

In a further preferred aspect, the present invention provides a genetically modified cell or non-human organism comprising such cells comprising a modified *Blimp* gene encoding a Blimp mRNA transcript comprising a Blimp coding sequence or a part, fragment or functional form thereof and a reporter molecule encoding sequence, wherein detection of said reporter molecule in T-cells is indicative that cells having reporter molecule activity are activated T-cells.

Preferably, the reporter molecule encoding sequence is inserted with an intron of the *Blimp* allele. In this way, the modified *Blimp* allele co-produces the reporter from a bicistronic RNA under the control of endogenous *Blimp* regulatory elements.

Reference herein to a *Blimp-1* gene or nucleic acid expression product thereof (RNA) includes homologs, parts, fragments, functional forms thereof including functional variants or derivatives which hybridize thereto under low stringency conditions or comprise significant sequence similarity to all or a functional part such as at least about 60% sequence similarity, after optimal alignment. Reference to a Blimp-1 polypeptide or protein is used in a broad sense to include all homologs, parts, fragments or functional forms thereof including functional variants or derivatives bearing at least about 60% amino acid sequence similarity after optimal alignment.

Functional parts of the instant molecules include portions of the full length molecule which are important for the particular functions thereof such as substrate binding, tertiary conformation or transcriptional activity. Transcription initiation sites are readily mapped and sites conferring promoter activity readily identified (see for example Tunyaplin et al., Nucleic Acid Research 28(24):4846-4855, 2000). Functional parts are important for regulating the expression and activity of the molecule. Functional variants or derivatives retain at least one of the functional activities important for regulating expression and activity of a reference molecule. With reference to *Blimp-1,* its expression is associated with terminal differentiation, induction of Ig secretion by ASC cells and activation of T-cells.

The modified *Blimp* gene may encode a functionally active Blimp polypeptide, a functionally inactive Blimp polypeptide and/or partial Blimp polypeptide such as a polypeptide or peptide, for example, lacking a zinc finger domain comprising a DNA binding motif. The terms "polypeptide" and "protein" are used interchangeably herein.

A "part" in peptide form may be as small as an epitope comprising less than 5 amino acids or as large as several hundred kilodaltons. The length of the polypeptide sequences compared for homology will generally be at least about 16 amino acids, usually at least about 20 residues, more usually at least about 24 residues, typically at least about 28 residues and preferably more than about 35 residues. A "part" of a nucleic acid molecule is defined a having a minimal size of at least about 10 nucleotides or preferably about 13 nucleotides or more preferably at least about 20 nucleotides and may have a minimal size of at least about 35 nucleotides. This definition includes all sizes in the range of 10-35 nucleotides as well as greater than 35 nucleotides including 50, 100, 300, 500, 600 nucleotides or nucleic acid molecules having any number of nucleotides within these values.

The present invention also contemplates modified Blimp alleles encoding variant Blimp polypeptides. "Variant" polypeptides include proteins derived from the native protein by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Variant proteins encompassed by the present invention are biologically active, that is, they continue to possess the desired biological activity of the native protein (i.e, they are transcriptional repressors of for example c-myc and/or CIITA). Alternatively, the variant Blimp polypeptides are non-functional. Such variants may result from, for example, genetic polymorphism or from human manipulation. Biologically active variants of a native Blimp polypeptide will have at least 40%, 50%, 60%, 70%, generally at least 75%, 80%, 85%, preferably about 90% to 95% or more, and more preferably about 98% or more sequence similarity with the amino acid sequence for the native protein as determined by sequence alignment programs described elsewhere herein using default parameters. A biologically active variant of a Blimp polypeptide may differ from that polypeptide generally by as much 100, 50 or 20 amino acid residues or suitably by as few as 1-15 amino acid residues, as few as 1-10, such as 6-10, as few as 5, as few as 4, 3, 2, or even 1 amino acid residue.

A Blimp polypeptide may be altered in various ways including amino acid substitutions, deletions, truncations, and insertions. Methods for such manipulations are generally known in the art. For example, amino acid sequence variants of a Blimp polypeptide can be prepared by mutations in the encoding nucleic acid sequence. Methods for mutagenesis and nucleotide sequence alterations are well known in the art. See, for example, Kunkel (Proc. Natl. Acad. Sci. USA 82:488-492, 1985), Kunkel et al., (Methods in Enzymol. 154:367-382, 1987), U.S. Pat. No. 4,873,192, Watson et al. ("Molecular Biology of the Gene", Fourth Edition, Benjamin/Cummings, Menlo Park, Calif., 1987) and the references cited therein. Guidance as to appropriate amino acid substitutions that do or do not affect biological activity of the protein of interest may be found in the model of Dayhoff et al., (Natl. Biomed. Res. Found 5:345-358,1978). For example deletion of all or part of the zinc finger domains containing the DNA binding motif will produce a Blimp variant which is functionally inactive. In some embodiments, animal models are heterozygous in some or all tissues for a genetically modified non-functional Blimp allele, while the other allele comprises a functional Blimp allele capable of expressing a functional Blimp polypeptide. In other embodiments, homozygous animals are produced which do not express Blimp in particular cells or tissues. Alternatively functional Blimp may be produced by one or two modified Blimp alleles in a cell, tissue or non-human organism. Methods for screening gene products of combinatorial libraries made by point mutations or truncation, and for screening cDNA libraries for gene products having a selected property are known in the art. Such methods are adaptable for rapid screening of the gene libraries generated by combinatorial mutagenesis of Blimp polypeptides. Recursive ensemble mutagenesis (REM), a technique which enhances the frequency of functional mutants in the libraries, can be used in combination with the screening assays to identify Blimp polypeptide variants (Arkin et al., Proc. Natl. Acad. Sci. USA 89:7811-7815, 1992; Delgrave et al. Protein Engineering 6:327-331, 1993). Conservative substitutions, such as exchanging one amino acid with another having similar properties, may be desirable as discussed in more detail below.

Variant Blimp polypeptides may contain conservative amino acid substitutions at various locations along their sequence, as compared to the parent Blimp amino acid sequence. A "conservative amino acid substitution" is one in which the amino acid residue is replaced with an amino acid residue having a similar side chain. Families of amino acid residues having similar side chains have been defined in the art, which can be generally sub-classified as follows:
Acidic: The residue has a negative charge due to loss of H ion at physiological pH and the residue is attracted by aqueous solution so as to seek the surface positions in the conformation of a peptide in which it is contained when the peptide is in aqueous medium at physiological pH. Amino acids having an acidic side chain include glutamic acid and aspartic acid.
Basic: The residue has a positive charge due to association with H ion at physiological pH or within one or two pH units thereof (e.g., histidine) and the residue is attracted by aqueous solution so as to seek the surface positions in the conformation of a peptide in which it is contained when the peptide is in aqueous medium at physiological pH. Amino acids having a basic side chain include arginine, lysine and histidine.
Charged: The residues are charged at physiological pH and, therefore, include amino acids having acidic or basic side chains (i.e., glutamic acid, aspartic acid, arginine, lysine and histidine).
Hydrophobic: The residues are not charged at physiological pH and the residue is repelled by aqueous solution so as to seek the inner positions in the conformation of a peptide in which it is contained when the peptide is in aqueous medium. Amino acids having a hydrophobic side chain include tyrosine, valine, isoleucine, leucine, methionine, phenylalanine and tryptophan.
Neutral/polar: The residues are not charged at physiological pH, but the residue is not sufficiently repelled by aqueous solutions so that it would seek inner positions in the conformation of a peptide in which it is contained when the peptide is in aqueous medium. Amino acids having a neutral/polar side chain include asparagine, glutamine, cysteine, histidine, serine and threonine.

This description also characterises certain amino acids as "small" since their side chains are not sufficiently large, even if polar groups are lacking, to confer hydrophobicity. With the exception of proline, "small" amino acids are those with four carbons or less when at least one polar group is on the side chain and three carbons or less when not. Amino acids having a small side chain include glycine, serine, alanine and threonine. The gene-encoded secondary amino acid proline is a special case due to its known effects on the secondary conformation of peptide chains. The structure of proline differs from all the other naturally-occurring amino acids in that its side chain is bonded to the nitrogen of the α-amino group, as well as the α-carbon. Several amino acid similarity matrices (e.g., PAM120 matrix and PAM250 matrix as disclosed for example by Dayhoff *et al.,* 1978 (*supra*); and by Gonnet et al., Science 256(5062):1443-1445, 1992), however, include proline in the same group as glycine, serine, alanine and threonine. Accordingly, for the purposes of the present invention, proline is classified as a "small" amino acid.

The degree of attraction or repulsion required for classification as polar or nonpolar is arbitrary and, therefore, amino acids specifically contemplated by the invention have been classified as one or the other. Most amino acids not specifically named can be classified on the basis of known behaviour.

Amino acid residues can be further sub-classified as cyclic or noncyclic, and aromatic or nonaromatic, self-explanatory classifications with respect to the side-chain substituent groups of the residues, and as small or large. The residue is considered small if it contains a total of four carbon atoms or less, inclusive of the carboxyl carbon, provided an additional polar substituent is present; three or less if not. Small residues are, of course, always nonaromatic. Dependent on their structural properties, amino acid residues may fall in two or more classes. For the naturally-occurring protein amino acids, sub-classification according to this scheme is presented in the Table A.

**Table A**

| ***Amino acid sub-classification*** | |
|---|---|
| **Sub-classes** | **Amino acids** |
| Acidic | Aspartic acid, Glutamic acid |
| Basic | Noncyclic: Arginine, Lysine; Cyclic: Histidine |
| Charged | Aspartic acid, Glutamic acid, Arginine, Lysine, Histidine |
| Small | Glycine, Serine, Alanine, Threonine, Proline |
| Polar/neutral | Asparagine, Histidine, Glutamine, Cysteine, Serine, |
| | Threonine |
| Polar/large | Asparagine, Glutamine |
| Hydrophobic | Tyrosine, Valine, Isoleucine, Leucine, Methionine, |
| | Phenylalanine, Tryptophan |
| Aromatic | Tryptophan, Tyrosine, Phenylalanine |
| Residues that influence chain orientation | Glycine and Proline |

Conservative amino acid substitution also includes groupings based on side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. For example, it is reasonable to expect that replacement of a leucine with an isoleucine or valine, an aspartate with a glutamate, a threonine with a serine, or a similar replacement of an amino acid with a structurally related amino acid will not have a major effect on the properties of the resulting variant polypeptide. Whether an amino acid change results in a functional Blimp polypeptide can readily be determined by assaying its activity. Conservative substitutions are shown in Table B below under the heading of exemplary substitutions. More preferred substitutions are shown under the heading of preferred substitutions. Amino acid substitutions falling within the scope of the invention, are, in general, accomplished by selecting substitutions that do not differ significantly in their effect on maintaining (a) the structure of the peptide backbone in the area of the substitution, (b) the charge or hydrophobicity of the molecule at the target site, or (c) the bulk of the side chain. After the substitutions are introduced, the variants are screened for biological activity.

**Table B**

| ***Exemplary and Preferred Amino Acid Substitutions*** | | |
|---|---|---|
| **Original Residue** | **EXEMPLARY SUBSTITUTIONS** | **PREFERRED SUBSTITUTIONS** |
| Ala | Val, Leu, Ile | Val |
| Arg | Lys, Gln, Asn | Lys |
| Asn | Gln, His, Lys, Arg | Gln |
| Asp | Glu | Glu |
| Cys | Ser | Ser |
| Gln | Asn, His, Lys, | Asn |
| Glu | Asp, Lys | Asp |
| Gly | Pro | Pro |
| His | Asn, Gln, Lys, Arg | Arg |
| Ile | Leu, Val, Met, Ala, Phe, Norleu | Leu |
| Leu | Norleu, Ile, Val, Met, Ala, Phe | Ile |
| Lys | Arg, Gln, Asn | Arg |
| Met | Leu, Ile, Phe | Leu |
| Phe | Leu, Val, Ile, Ala | Leu |
| Pro | Gly | Gly |
| Ser | Thr | Thr |
| Thr | Ser | Ser |
| Trp | Tyr | Tyr |
| Tyr | Trp, Phe, Thr, Ser | Phe |
| Val | Ile, Leu, Met, Phe, Ala, Norleu | Leu |

Alternatively, similar amino acids for making conservative substitutions can be grouped into three categories based on the identity of the side chains. The first group includes glutamic acid, aspartic acid, arginine, lysine, histidine, which all have charged side chains; the second group includes glycine, serine, threonine, cysteine, tyrosine, glutamine, asparagine; and the third group includes leucine, isoleucine, valine, alanine, proline, phenylalanine, tryptophan, methionine, as described in Zubay, G., Biochemistry, third edition, Wm.C. Brown Publishers (1993).

Thus, a predicted non-essential amino acid residue in a Blimp polypeptide is typically replaced with another amino acid residue from the same side chain family. Alternatively, mutations can be introduced randomly along all or part of a Blimp polynucleotide coding sequence, such as by saturation mutagenesis, and the resultant mutants can be screened for an activity of the parent polypeptide to identify mutants which retain that activity. Following mutagenesis of the coding sequences, the encoded peptide can be expressed recombinantly and the activity of the peptide can be determined.

Accordingly, the present invention also contemplates variants of the naturally-occurring Blimp polypeptide sequences or their biologically-active fragments, wherein the variants are distinguished from the naturally-occurring sequence by the addition, deletion, or substitution of one or more amino acid residues. In general, variants will display at least about 30, 40, 50, 55, 60, 65, 70, 75, 80, 85, 90, 91, 92, 93, 94, 95, 96, 97, 98, 99 % similarity to a parent Blimp polypeptide sequence as, for example, set forth in any one of SEQ ID NO: 2 and 4. Desirably, variants will have at least 30, 40, 50, 55, 60, 65, 70, 75, 80, 85, 90, 91, 92, 93, 94, 95, 96, 97, 98, 99% sequence identity to a reference Blimp polypeptide sequence as, for example, set forth in any one of SEQ ID NO: 2 and 4. Moreover, sequences differing from the native or parent sequences by the addition, deletion, or substitution of 1, 2, 3, 4, 5, 6, 7, 8, 9, 10, 11, 12, 13, 14, 15, 16, 17, 18, 19, 20, 30, 40, 50, 60, 70, 80, 90, 100 or more amino acids but which retain the properties of the parent Blimp polypeptide are contemplated. Blimp polypeptides also include polypeptides that are encoded by polynucleotides that hybridise under stringency conditions as defined herein, especially high stringency conditions, to Blimp polynucleotide sequences, or the non-coding strand thereof.

In some embodiments, variant polypeptides differ from an Blimp sequence by at least one but by less than 50, 40, 30, 20, 15, 10, 8, 6, 5, 4, 3 or 2 amino acid residue(s). In another, variant polypeptides differ from the corresponding sequence in any one of SEQ ID NO: 2 and 4 by at least 1% but less than 20%, 15%, 10% or 5% of the residues. (If this comparison requires alignment the sequences should be aligned for maximum similarity. "Looped" out sequences from deletions or insertions, or mismatches, are considered differences.) The differences are, suitably, differences or changes at a non-essential residue or a conservative substitution.

A "non-essential" amino acid residue is a residue that can be altered from the wild-type sequence of an embodiment polypeptide without abolishing or substantially altering one or more of its activities. Suitably, the alteration does not substantially alter one of these activities, for example, the activity is at least 20%, 40%, 60%, 70% or 80% of wild-type. An "essential" amino acid residue is a residue that, when altered from the wild-type sequence of an Blimp polypeptide of the invention, results in abolition of an activity of the parent molecule such that less than 20% of the wild-type activity is present.

In other embodiments, a variant polypeptide includes an amino acid sequence having at least about 50%, 55%, 60%, 65%, 70%, 75%, 80%, 85%, 90%, 91%, 92%, 93%, 94% 95%, 96%, 97%, 98% or more similarity to a corresponding sequence of a Blimp polypeptide as, for example, set forth in any one of SEQ ID NO: 2 and 4.

The present invention encompasses Blimp from any mammal or animal (including avian species) subject such as from humans, non-human primates, livestock, laboratory, companion or wild animals. Reference to "Blimp" includes Blimp or *Blimp* from any of the above species as well as structural or evolutionary equivalents or homologs thereof. for example, the present invention encompasses Blimp or a *Blimp* having an amino acid sequence which has substantially at least about 60% similarity to SEQ ID NO: 2 or 4 or at least about 60% identity to SEQ ID NO:1, 3, 5 or 6. Reference to at least about 60% includes 60, 61, 62, 63, 64% and all following consecutive numbers in the series to 100%.

Function derivatives of molecules in nucleic acid form include nucleic acid molecules comprising a nucleotide sequence capable of hybridising to the molecule or its complementary form under low stringency conditions.

The terms "similarity" or identity as used herein includes exact identity between compared sequences at the nucleotide or amino acid level. Where there is non-identity at the nucleotide level, "similarity" includes differences between sequences which result in different amino acids that are nevertheless related to each other at the structural, functional, biochemical and/or conformational levels. Where there is non-identity at the amino acid level, "similarity" includes amino acids that are nevertheless related to each other at the structural, functional, biochemical and/or conformational levels. In a particularly preferred embodiment, nucleotide and amino acid sequence comparisons are made at the level of identity rather than similarity.

Terms used to describe sequence relationships between two or more polynucleotides or polypeptides include "reference sequence", "comparison window", "sequence similarity", "sequence identity", "percentage of sequence similarity", "percentage of sequence identity", "substantially similar" and "substantial identity". A "reference sequence" is at least 12 but frequently 1 to 18 and often at least 25 or above, such as 30 monomer units, inclusive of nucleotides and amino acid residues, in length. Because two polynucleotides may each comprise (1) a sequence (i.e. only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of typically 12 contiguous residues that is compared to a reference sequence. The comparison window may comprise additions or deletions (i.e. gaps) of about 20% or less as compared to the reference sequence (which does not comprise additions or deletions) for optimal alignment of the two sequences. Optimal alignment of sequences for aligning a comparison window may be conducted by computerised implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e. resulting in the highest percentage homology over the comparison window) generated by any of the various methods selected. Reference also may be made to the BLAST family of programs as, for example, disclosed by Altschul et al., Nucl. Acids Res. 25: 3389, 1997. A detailed discussion of sequence analysis can be found in Unit 19.3 of Ausubel et al., Current Protocols in Molecular Biology John Wiley & Sons Inc, 1994-1998, Chapter 15).

The terms "sequence similarity" and "sequence identity" as used herein refer to the extent that sequences are identical or functionally or structurally similar on a nucleotide-by-nucleotide basis or an amino acid-by-amino acid basis over a window of comparison. Thus, a "percentage of sequence identity", for example, is calculated by comparing two optimally aligned sequences over the window of comparison, determining the number of positions at which the identical nucleic acid base (e.g. A, T, C, G, I) or the identical amino acid residue (e.g. Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. For the purposes of the present invention, "sequence identity" will be understood to mean the "match percentage" calculated by the DNASIS computer program (Version 2.5 for windows; available from Hitachi Software engineering Co., Ltd., South San Francisco, California, USA) using standard defaults as used in the reference manual accompanying the software. Similar comments apply in relation to sequence similarity.

Furthermore, a *Blimp* homolog or derivative may be defined as being capable of hybridising to SEQ ID NO: 1, 3, 5 or 6 or to a complementary form thereof under low stringency conditions.

Reference herein to a low stringency includes and encompasses from at least about 0 to at least about 15% v/v formamide and from at least about 1 M to at least about 2 M salt for hybridization, and at least about 1 M to at least about 2 M salt for washing conditions. Generally, low stringency is at from about 25-30°C to about 42°C. The temperature may be altered and higher temperatures used to replace formamide and/or to give alternative stringency conditions. Alternative stringency conditions may be applied where necessary, such as medium stringency, which includes and encompasses from at least about 16% v/v to at least about 30% v/v formamide and from at least about 0.5 M to at least about 0.9 M salt for hybridization, and at least about 0.5 M to at least about 0.9 M salt for washing conditions, or high stringency, which includes and encompasses from at least about 31% v/v to at least about 50% v/v formamide and from at least about 0.01 M to at least about 0.15 M salt for hybridization, and at least about 0.01 M to at least about 0.15 M salt for washing conditions. In general, washing is carried out Tₘ = 69.3 + 0.41 (G+C)% (Marmur et al., J. Mol. Biol. 5: 109, 1962). However, the Tₘ of a duplex DNA decreases by 1 °C with every increase of 1% in the number of mismatch base pairs (Bonner et al., Eur. J. Biochem. 46: 83, 1974). Formamide is optional in these hybridization conditions. Accordingly, particularly preferred levels of stringency are defined as follows: low stringency is 6 x SSC buffer, 0.1% w/v SDS at 25-42°C; a moderate stringency is 2 x SSC buffer, 0.1% w/v SDS at a temperature in the range 20°C to 65°C; high stringency is 0.1 x SSC buffer, 0.1% w/v SDS at a temperature of at least 65°C.

Preferably the modified *Blimp* gene is modified using a nucleic acid construct comprising all or a portion of an allele of *Blimp* into which a nucleotide sequence encoding a reporter molecule is inserted.

The reporter molecule is conveniently encoded by a reporter expression cassette or reporter construct. The reporter construct can be brought under the control of the *Blimp-1* regulatory elements and faithfully report the *Blimp-1* expression pattern in cells, tissues or organisms.

By "reporter" is meant any molecule, protein or polypeptide which is typically encoded by a reporter gene and measured in a reporter assay. Reporters provide a detectable signal which permit an understanding of the activity of genetic sequences. They may report an activity directly or may indirectly monitor activity by monitoring the activity of down stream targets. A reporter protein should be distinguishable from other proteins and ideally, readily quantified. The reactivity between an epitope and an antibody determined thereby may readily be employed optionally together with second or further antibodies. Common reporter proteins include luciferase, chloramphenicol transferase (CAT), Beta-galactosidase (B-gal), or fluorescent proteins such as green fluorescent proteins (GFP). Reference herein to GFP is meant to encompass any fluorescent or light-emitting protein including those derived from jelly fish or other organisms and all homologues, derivatives, analogues including colour variants such as DSRed, HcRed, Clontech; or hrGFP, Stratagene). Preferably said reporter expression cassette encodes a fluorescent or other light emitting GFP. GFP reporters are readily detectable in live cells and are particularly useful and preferred in cell sorting applications.

Examples of fluorescent or light emitting markers may be selected from among those included, but are not limited to those, in the following Table 2.

**TABLE 2**

| **Probe** | **Ex¹ (nm)** | **Em² (nm)** |
|---|---|---|
| **Reactive and conjugated probes** | | |
| Hydroxycoumarin | 325 | 386 |
| Aminocoumarin | 350 | 455 |
| Methoxycoumarin | 360 | 410 |
| Cascade Blue | 375; 400 | 423 |
| Lucifer Yellow | 425 | 528 |
| NBD | 466 | 539 |
| R-Phycoerythrin (PE) | 480; 565 | 578 |
| PE-Cy5 conjugates | 480; 565; 650 | 670 |
| PE-Cy7 conjugates | 480; 565; 743 | 767 |
| APC-Cy7 conjugates | 650; 755 | 767 |
| Red 613 | 480; 565 | 613 |
| Fluorescein | 495 | 519 |
| FluorX | 494 | 520 |
| BODIPY-FL | 503 | 512 |
| TRITC | 547 | 574 |
| X-Rhodamine | 570 | 576 |
| Lissamine Rhodamine B | 570 | 590 |
| PerCP | 490 | 675 |
| Texas Red | 589 | 615 |
| Allophycocyanin (APC) | 650 | 660 |
| TruRed | 490,675 | 695 |
| Alexa Fluor 350 | 346 | 445 |
| Alexa Fluor 430 | 430 | 545 |
| Alexa Fluor 488 | 494 | 517 |
| Alexa Fluor 532 | 530 | 555 |
| Alexa Fluor 546 | 556 | 573 |
| Alexa Fluor 555 | 556 | 573 |
| Alexa Fluor 568 | 578 | 603 |
| Alexa Fluor 594 | 590 | 617 |
| Alexa Fluor 633 | 621 | 639 |
| Alexa Fluor 647 | 650 | 688 |
| Alexa Fluor 660 | 663 | 690 |
| Alexa Fluor 680 | 679 | 702 |
| Alexa Fluor 700 | 696 | 719 |
| Alexa Fluor 750 | 752 | 779 |
| Cy2 | 489 | 506 |
| Cy3 | (512); 550 | 570;(615) |
| Cy3,5 | 581 | 596; (640) |
| Cy5 | (625); 650 | 670 |
| Cy5,5 | 675 | 694 |
| Cy7 | 743 | 767 |

| **Nucleic acid probes** | | |
|---|---|---|
| Hoeschst 33342 | 343 | 483 |
| DAPI | 345 | 455 |
| Hoechst 33258 | 345 | 478 |
| SYTOX Blue | 431 | 480 |
| Chromomycin A3 | 445 | 575 |
| Mithramycin | 445 | 575 |
| YOYO-1 | 491 | 509 |
| SYTOX Green | 504 | 523 |
| SYTOX Orange | 547 | 570 |
| Ethidium Bormide | 493 | 620 |
| 7-AAD | 546 | 647 |
| Acridine Orange | 503 | 530/640 |
| TOTO-1, TO-PRO-1 | 509 | 533 |
| Thiazole Orange | 510 | 530 |

| **Probe** | **Ex¹ (nm)** | **Em² (nm)** |
|---|---|---|
| Propidium Iodide (PI) | 536 | 617 |
| TOTO-3, TO-PRO-3 | 642 | 661 |
| LDS 751 | 543; *590* | 712; *607* |

| **Cell function probes** | | |
|---|---|---|
| Indo-1 | 361/330 | 490/405 |
| Fluo-3 | 506 | 526 |
| DCFH | 505 | 535 |
| DHR | 505 | 534 |
| SNARF | 548/579 | 587/635 |

| **Fluorescent Proteins** | | |
|---|---|---|
| Y66F | 360 | 508 |
| Y66H | 360 | 442 |
| EBFP | 380 | 440 |
| Wild-type | 396,475 | 50, 503 |
| GFPuv | 385 | 508 |
| ECFP | 434 | 477 |
| Y66W | 436 | 485 |
| S65A | 471 | 504 |
| S65C | 479 | 507 |
| S65L | 484 | 510 |
| S65T | 488 | 511 |
| EGFP | 489 | 508 |
| EYFP | 514 | 527 |
| DsRed | 558 | 583 |

| **Other probes** | | |
|---|---|---|
| Monochlorobimane | 380 | 461 |
| Calcein | 496 | 517 |

| | | |
|---|---|---|
| ¹ Ex: Peak excitation wavelength (nm) ² Em: Peak emission wavelength (nm) | | |

Any suitable method of analyzing fluorescence emission is encompassed by the present invention. In this regard, the invention contemplates techniques including but not restricted to 2-photon and 3-photon time resolved fluorescence spectroscopy as, for example, disclosed by Lakowicz *et al., Biophys. J. 72*: 567, 1997, fluorescence lifetime imaging as, for example, disclosed by Eriksson *et al., Biophys. J. 2:* 64, 1993, incorporated herein by reference) and fluorescence resonance energy transfer as, for example, disclosed by Youvan *et al., Biotechnology et Elia 3:* 1-18, 1997).

Exemplary fluorophores which may be used in accordance with the present invention include those discussed by Dower *et al.* (International Patent Publication No. WO 93/06121). Preferably, fluorescent dyes are employed. Any suitable fluorescent dye may be used for incorporation into the instant reporter molecule. For example, reference may be made to U.S. Patent Nos. 5,573,909 (Singer et al.) and 5,326,692 (Brinkley et al.) which describe a plethora of fluorescent dyes. Reference may also be made to fluorescent dyes described in U.S. Patent Nos. 5,227,487, 5,274,113, 5,405,975, 5,433,986, 5,442,045, 5,451,663, 5,453,517, 5,459,276, 5,515,864, 5,648,270 and 5,723,218.

A modern flow cytometer is able to perform these tasks up to 100,000 cells/particles s⁻¹. Through the use of an optical array of filters and dichroic mirrors, different wavelengths of fluorescent light can be separated and detected simultaneously. In addition, a number of lasers with different excitation wavelengths may be used. Hence, a variety of fluorophores can be used to target and examine, for example, intra- and extra-cellular properties of individual cells. The scattered light measurements can also classify an individual cells's size, shape, granularity and/or complexity and, hence, belonging to a particular population of interest (Shapiro, Practical flow cytometry, 3rd Ed., Brisbane, Wiley-Liss, 1995).

Suitable flow cytometers which may be used in the methods of the present invention include those which measure five to nine optical parameters (see Table 3) using a single excitation laser, commonly an argon ion air-cooled laser operating at 15 mW on its 488 nm spectral line. More advanced flow cytometers are capable of using multiple excitation lasers such as a HeNe laser (633 nm) or a HeCd laser (325 nm) in addition to the argon ion laser (488 or 514 nm). Optical parameters, corresponding to different optically detectable/quantifiable attributes, for a carrier, may be measured by a flow cytometer to provide a matrix of qualitative and/or quantitative information, providing a code (or addressability in a multi-dimensional space) for the carrier.

For example, Biggs et al. (Cytometry 36: 36-45, 1999) have constructed an 11-parameter flow cytometer using three excitation lasers and have demonstrated the use of nine distinguishable fluorophores in addition to forward and side scatter measurements for purposes of immunophenotyping (i.e. classifying) cells. The maximum number of parameters commercially available currently is 17: forward scatter, side scatter and three excitation lasers each with five fluorescence detectors. Whether all of the parameters can be adequately used depends heavily on the extinction coefficients, quantum yields and amount of spectral overlap between all fluorophores (Malemed et al., "Flow cytometry and sorting", 2nd Ed., New York, Wiley-Liss, 1990). However, it will be understood that the present invention is not restricted to any particular flow cytometer or any particular set of parameters. In this regard, the invention also contemplates use in place of a conventional flow cytometer, a microfabricated flow cytometer as, for example, disclosed by Fu et al., Nature Biotechnology 17: 1109-1111, 1999.

**TABLE 3**

| ***Exemplary optical parameters which may be measured by a flow cytometer.*** | | | |
|---|---|---|---|
| **Parameter** | **Acronym** | **Detection angle form incident laser beam** | **Wavelength (nm)** |
| Forward scattered light | FS | 2-5° | 488* |
| Side scattered light | SS | 90° | 488* |
| "Green" fluorescence | FL1 | 90° | 510-540^{†} |
| "Yellow" fluorescence | FL2 | 90° | 560-580^{†} |
| "Red" fluorescence | FL3 | 90° | >650^{#} |

| | | | |
|---|---|---|---|
| * using a 488 nm excitation laser ^{†} width of bandpass filter ^{#} longpass filter | | | |

A flow cytometer with this capacity to sort is known as a "fluorescence-activated cell sorter" (FACS). Accordingly, the step of sorting in the present method of obtaining a population of detectably unique carriers may be effected by flow cytometric techniques such as by fluorescence activated cell sorting (FACS) although with respect to the present invention, FACS is more accurately "fluorescence activated carrier or solid support sorting" (see, for example, *"*Methods in Cell Biology" Vol. 33, Darzynkiewica, Z. and Crissman, H.A., eds., Academic Press).

In a further embodiment the present invention provides a method for phenotyping and/or monitoring a cell of the haematopoietic system comprising screening a genetically modified cell or non-human organism comprising such cells comprising a modified *Blimp* gene wherein expression or activity of said gene is indicative of a cellular phenotype and/or a commitment of said cell to terminally differentiate. Haematopoietic cells include but are not limited to B-cells, T-cells, dendritic cells, macrophages and natural killer cells, granulocytes, eosinophils, erythrocytes, megakaryocytes, bone marrow, stromal, splenic precursor cells and their derivatives.

Preferably the modified *Blimp* gene encodes a Blimp mRNA transcript comprising a Blimp coding sequence or a part, fragment or functional form thereof and a reporter molecule encoding sequence which when expressed produces Blimp or a part, fragment or functional form thereof co-expressed with a reporter molecule and wherein detection of the reporter molecule is indicative of cellular phenotype and/or commitment of a cell to terminally differentiate.

In a further embodiment, cells which exhibit reporter activity or changes in reporter activity are isolated or selected from among cells which do not exhibit reporter activity. Isolation of reporter-active cells may be by flow cytometry, laser scanning cytometry, chromatography and/or other equivalent procedures. Additionally, further selection markers may be used to isolate or select the modified cells of the present invention. Flow cytometric isolation is particularly preferred.

Preferably the cells are ASC identified or isolated in a population of cells of a B-cell lineage.

Accordingly, the present invention provides a method for isolating a substantially purified population of ASC from a population of substantially B-cells said method comprising contacting a genetically modified cell or non-human organism comprising such cells comprising a modified *Blimp* gene with an agent or composition capable of inducing differentiation to ASC wherein expression or activity of said gene is reported by a reporter construct and wherein detection of said reporter activity is indicative that cells with reporter molecule activity are ASC, where necessary isolating B-cells from said organism and isolating ASC based on the activity of the reporter molecule.

Preferably the modified cell comprises a modified *Blimp* gene encoding a Blimp mRNA transcript comprising a Blimp coding sequence or a part, fragment or functional form thereof and a reporter molecule encoding sequence which when expressed produces Blimp or a part, fragment or functional form thereof co-expressed with a reporter molecule and wherein reporter activity is indicative that cells with reporter molecule activity are ASC.

Preferably, screening of cells is achieved by flow cytometric analysis of a fluroescent reporter molecule.

B-cells are conveniently isolated from an organism or sample for example by density gradient centrifugation, flow cytometry or using magnetic beads. Any agent or composition which selectively, clonally or polyclonally of otherwise effectively activates B-cells and induces their differentiation to ASC is encompassed. An example of a polyclonal activator is LPS.

In one embodiment the reporter is a GFP and said ASC are isolated by flow cytometry.

Substantially purified means that the ASC comprise at least about 60 to 95%, preferably at least about 97%, more preferably at least about 99% of the cells, such as at least about 60, 61, 62, 63, 64 and following subsequent numbers in the series to 100%. Alternatively, enrichment of approximately 100,000 fold over unsorted cells is contemplated.

The present invention also provides a method for testing the antigenicity of a vaccine or the ability of agents to enhance or suppress antibody production by ASC wherein reduced reporter activity is indicative of an agent which down regulates or inhibits an antibody response and reporter activity or enhanced reporter activity relative to controls is indicative of agents which are positive regulators of the antibody response. In accordance with this aspect, the method comprises:
(i) administering an agent or vaccine to a genetically modified cell or non-human animal comprising such cells wherein the cell or organism comprises a modified *Blimp-1* gene which encodes a Blimp polypeptide which when expressed produces Blimp or a part or fragment or functional form thereof co-expressed with a reporter molecule;
(ii) testing the cell or organism for the reporter molecule the presence of which is indicative of cellular phenotype and the ability of said agent or vaccine to regulate antibody production by ASC.

In another embodiment, the present invention provides a method for testing the antigenicity or immunogenicity of a vaccine comprising a genetic or proteinaceous composition, the method comprising;
(i) administering the vaccine to a genetically modified cell or non-human animal comprising such cells wherein the cell or organism comprises a modified *Blimp-1* gene which encodes a Blimp polypeptide which when expressed produces Blimp or a part or fragment or functional form thereof co-expressed with a reporter molecule; and
(ii) testing the cell or organism for the reporter molecule the presence of which is indicative of the ability of the vaccine to regulate the activation of T-cells and/or B-cells.

In some embodiments, the *Blimp* gene encodes a *Blimp* mRNA transcript comprising a Blimp coding sequence or a part, fragment or functional form thereof and a reporter molecule coding sequence. In other embodiments, the reporter molecule coding sequence is inserted within an intron of a *Blimp* allele. In further embodiments, the modified *Blimp* allele is present in homozygous or heterozygous form. Depending upon the purpose of the assay in some embodiments, the modified *Blimp* allele encodes a functional Blimp transcription factor or a functional part, form, homolog or variant thereof. In other embodiments, the modified *Blimp* allele encodes a non-functional Blimp transcription factor or a non-functional part, form, homolog or variant thereof. In an illustrative embodiment, the cells or genetic material are derived from man, a non-human primate, a livestock, companion or laboratory test organisms, reptilian or amphibian species. Examples of laboratory test animal include a rodent (including mice), guinea pig, pig, duck, rabbit or sheep.

In some illustrative embodiments of the methods disclosed herein the cell is a haematopoietic or embryonic cell. As disclosed herein Blimp is essential for both B-cell and T-cell terminal differentiation and accordingly a preferred cell lineage is is a lymphocytic cell. In particular embodiments the lymphocytic cell types is selected from a B-cell and a T-cell. Where a B-cell, the terminally differentiated form is an ASC and these cell can furthermore be substantially purified using the methods disclosed herein. When the cell is a B-cell, the terminally differentiated T-cells include without limitation CD4⁺ T-cells and CD8⁺ T-cells. Conveniently, the detection of the reporter molecule is indicative or predictive of a cellular phenotype and/or commitment of a cell to terminally differentiate under particular conditions or in the presence of test agents. Still more conveniently, the reporter molecule is a fluorescent or light emitting reporter molecule.

The present invention also directed to antagonists and agonists of terminal differentiation of cells such as, but not limited to ASC including antagonists and agonists of *Blimp-1* expression or Blimp-1 activity, identified by the herein described method, for use in modulating cellular differentiation. The molecules to which the instant modulators, agonists or antagonists are directed are collectively referred to herein as "targets" or "target molecules".

In another aspect therefore, the present invention provides methods for *in vitro* or *in vivo* screening for agonists or antagonists of terminal differentiation in haematopoietic cells comprising exposing one or more agent/s to a genetically modified cell or non-human animal comprising such cells wherein the cell or organism comprises a modified *Blimp-1* gene which encodes a Blimp polypeptide which when expressed produces Blimp or a part or fragment or functional form thereof co-expressed with a reporter molecule; and testing the cell or organism for the presence or a change in the level of the reporter molecule the presence of which is indicative of the ability of the one or more agent/s to agonise or antagonise terminal differentiation. Agonists of Blimp directly or indirectly induce terminal differentiation of haematopoietic cells and are useful, for example, in the treatment or prevention of cancer and/or autoimmune disease and in promoting appropriate immune responses to pathological infections. Molecules which inhibit generation of terminally differentiated cells are useful in autoimmune patents such as lupus patients or in treating immune dysfunction such as cases of allegy.

Preferably the modified cell is a haematopoietic cell which comprises a modified *Blimp* gene encoding a Blimp mRNA transcript comprising a Blimp coding sequence or a part, fragment or functional form thereof and a reporter molecule encoding sequence which when expressed produces Blimp or a part, fragment, homolog, variant, derivative or functional or non-functional form thereof co-expressed with a reporter molecule and wherein reporter activity is indicative that cells with reporter molecule activity are terminally differentiated or committed to terminal differentiation. More preferably, the haematopoietic cell is a lymphocyte lineage cell. In some embodiments the terminally differentiated cells are ASC: in other embodiments, the terminally differentiated cells are CD4 T-cell and/or CD8 T-cells. The modified Blimp allele is present in the cell, tissue or non-human organism in homozygous or heterozygous form. Furthermore, depending upon the particular application, the *Blimp* allele expresses a transcriptionally (functionally) active Blimp polypeptide. Thus in some assays it will be useful to have a functional Blimp polypeptide to modulate or induce terminal differentiation in a cell. In other embodiments, the modified Blimp allele does not express a functional Blimp and it will be sufficient to determine, via detection of the reporter activity whether a Blimp allele would have been expressed or whether the level of Blimp expression would have been modulated in a cell capable of producing a function Blimp polypeptide.

Cellular (*in vitro*) assays are particularly convenient and, when coupled with a reporter molecule whose activity can readily be detected in cells, the assays are ideally suited to high throughput screening. A large number of different formats are available as known to the skilled artisan. One useful example is described in Ulleras et al., Toxicology 206(2):245-256, 2005.

"Modulation" of a molecule or differentiation status includes completely or partially inhibiting or reducing or down regulating all or part of its functional activity or differentiation and enhancing or up regulating all or part its functional activity or differentiation. Where the molecule is a genetic sequence its functional activity may be modulated by, for example, modulating its binding capabilities or transcriptional or translational activity, or its half-life. Where the molecule is an encoded polypeptide, its functional activity may be modulated by, for example, modulating its binding capabilities, its half-life, location in a cell or membrane or its enzymatic capability. Modulators are agonists or antagonists which achieve modulation. Enhanced differentiation can also be indicative of reduced cell division.

An example of an antagonist or agonist is a protein, polypeptide or peptide. These terms may be used interchangeably. These terms refer to a polymer of amino acids and its equivalent and does not refer to a specific length of the product, thus, polypeptides, peptides, oligopeptides and proteins are included within the one definition of a polypeptide. These terms also do not exclude modifications of the polypeptide, for example, glycosylations, aceylations, phosphorylations and the like. Included within the definition are, for example, polypeptides containing one or more analogs of an amino acid including, for example, unnatural amino acids such as those given in Table 4 or polypeptides with substituted linkages. Such polypeptides may need to be able to enter the cell. Polypeptides carrying chemical analogs of the amino acids may be more resistant to protease mediated digestion. One example of an antagonist or agonist is a chemical analog of Blimp. Antagonists and agonists may affect the molecules with which Blimp interacts, such as, for example *c-myc* expression is repressed by Blimp-1.

Genetic molecules are also developed into agonist and antagonist modulators. The terms "genetic molecule" "nucleic acids", "nucleotide" and "polynucleotide" include RNA, cDNA, genomic DNA, synthetic forms and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog (such as the morpholine ring), internucleotide modifications such as uncharged linkages (e.g. methyl phosphonates, phosphotriesters, phosphoamidates, carbamates, etc.), charged linkages (e.g. phosphorothioates, phosphorodithioates, etc.), pendent moieties (e.g. polypeptides), intercalators (e.g. acridine, psoralen, etc.), chelators, alkylators and modified linkages (e.g. α-anomeric nucleic acids, etc.). Also included are synthetic molecules that mimic polynucleotides in their ability to bind to a designated sequence via hydrogen binding and other chemical interactions. Such molecules are known in the art and include, for example, those in which peptide linkages substitute for phosphate linkages in the backbone of the molecule. Modifications of antisense molecules are well known and are summarised in Kurrek, Eur. J. Biochem. 270:1628-1644,2003.

Antisense polynucleotide sequences, for example, are useful in silencing transcripts. Furthermore, polynucleotide vectors containing all or a part of a Blimp gene locus may be placed under the control of a promoter in an antisense orientation and introduced into a cell. Expression of such an antisense construct within a cell will interfere with target transcription and/or translation. Such molecules may be particularly useful in dampening the immune response in autoimmune conditions. Furthermore, co-suppression and mechanisms to induce RNAi or siRNA may also be employed. Alternatively, antisense or sense molecules may be directly administered. In this latter embodiment, the antisense or sense molecules may be formulated in a composition and then administered by any number of means to target cells.

A variation on antisense and sense molecules involves the use of morpholinos, which are oligonucleotides composed of morpholine nucleotide derivatives and phosphorodiamidate linkages (for example, Summerton and Weller, Antisense and Nucleic Acid Drug Development 7: 187-195, 1997). Such compounds are injected into embryos and the effect of interference with mRNA is observed.

In one embodiment, the present invention employs compounds such as oligonucleotides and similar species for use in modulating the function or effect of nucleic acid molecules encoding Blimp i.e. the oligonucleotides induce transcriptional or post-transcriptional gene silencing. This is accomplished by providing oligonucleotides which specifically hybridize with one or more nucleic acid molecules encoding the endogenous ligands. The oligonucleotides may be provided directly to a cell or generated within the cell. As used herein, the terms "target nucleic acid" and "nucleic acid molecule encoding an inhibitor" have been used for convenience to encompass DNA encoding the inhibitor, RNA (including pre-mRNA and mRNA or portions thereof) transcribed from such DNA, and also cDNA derived from such RNA. The hybridization of a compound of the subject invention with its target nucleic acid is generally referred to as "antisense". Consequently, the preferred mechanism believed to be included in the practice of some preferred embodiments of the invention is referred to herein as "antisense inhibition." Such antisense inhibition is typically based upon hydrogen bonding-based hybridization of oligonucleotide strands or segments such that at least one strand or segment is cleaved, degraded, or otherwise rendered inoperable. In this regard, it is presently preferred to target specific nucleic acid molecules and their functions for such antisense inhibition.

The functions of DNA to be interfered with can include replication and transcription. Replication and transcription, for example, can be from an endogenous cellular template, a vector, a plasmid construct or otherwise. The functions of RNA to be interfered with can include functions such as translocation of the RNA to a site of protein translation, translocation of the RNA to sites within the cell which are distant from the site of RNA synthesis, translation of protein from the RNA, splicing of the RNA to yield one or more RNA species, and catalytic activity or complex formation involving the RNA which may be engaged in or facilitated by the RNA. In one example, the result of such interference with target nucleic acid function is reduced levels of Blimp. In the context of the present invention, "modulation" and "modulation of expression" mean either an increase (stimulation) or a decrease (inhibition) in the amount or levels of a nucleic acid molecule encoding the gene, e.g., DNA or RNA. Inhibition is often the preferred form of modulation of expression and mRNA is often a preferred target nucleic acid.

An antisense compound is specifically hybridizable when binding of the compound to the target nucleic acid interferes with the normal function of the target nucleic acid to cause a loss of activity, and there is a sufficient degree of complementarity to avoid non-specific binding of the antisense compound to non-target nucleic acid sequences under conditions in which specific binding is desired, i.e. under physiological conditions in the case of in vivo assays or therapeutic treatment, and under conditions in which assays are performed in the case of in vitro assays.

"Complementary" as used herein, refers to the capacity for precise pairing between two nucleobases of an oligomeric compound. For example, if a nucleobase at a certain position of an oligonucleotide (an oligomeric compound), is capable of hydrogen bonding with a nucleobase at a certain position of a target nucleic acid, said target nucleic acid being a DNA, RNA, or oligonucleotide molecule, then the position of hydrogen bonding between the oligonucleotide and the target nucleic acid is considered to be a complementary position. The oligonucleotide and the further DNA, RNA, or oligonucleotide molecule are complementary to each other when a sufficient number of complementary positions in each molecule are occupied by nucleobases which can hydrogen bond with each other. Thus, "specifically hybridizable" and "complementary" are terms which are used to indicate a sufficient degree of precise pairing or complementarity over a sufficient number of nucleobases such that stable and specific binding occurs between the oligonucleotide and a target nucleic acid.

According to the present invention, compounds include antisense oligomeric compounds, antisense oligonucleotides, ribozymes, external guide sequence (EGS) oligonucleotides, alternate splicers, primers, probes, and other oligomeric compounds which hybridize to at least a portion of the target nucleic acid. As such, these compounds may be introduced in the form of single-stranded, double-stranded, circular or hairpin oligomeric compounds and may contain structural elements such as internal or terminal bulges or loops. Once introduced to a system, the compounds of the invention may elicit the action of one or more enzymes or structural proteins to effect modification of the target nucleic acid. One non-limiting example of such an enzyme is RNAse H, a cellular endonuclease which cleaves the RNA strand of an RNA:DNA duplex. It is known in the art that single-stranded antisense compounds which are "DNA-like" elicit RNAse H. Activation of RNase H, therefore, results in cleavage of the RNA target, thereby greatly enhancing the efficiency of oligonucleotide-mediated inhibition of gene expression. Similar roles have been postulated for other ribonucleases such as those in the RNase III and ribonuclease L family of enzymes.

While the preferred form of antisense compound is a single-stranded antisense oligonucleotide, in many species the introduction of double-stranded structures, such as double-stranded RNA (dsRNA) molecules, has been shown to induce potent and specific antisense-mediated reduction of the function of a gene or its associated gene products. This phenomenon occurs in both plants and animals.

In the context of the subject invention, the term "oligomeric compound" refers to a polymer or oligomer comprising a plurality of monomeric units. In the context of this invention, the term "oligonucleotide" refers to an oligomer or polymer of ribonucleic acid (RNA) or deoxyribonucleic acid (DNA) or mimetics, chimeras, analogs and homologs thereof. This term includes oligonucleotides composed of naturally occurring nucleobases, sugars and covalent internucleoside (backbone) linkages as well as oligonucleotides having non-naturally occurring portions which function similarly. Such modified or substituted oligonucleotides are often preferred over native forms because of desirable properties such as, for example, enhanced cellular uptake, enhanced affinity for a target nucleic acid and increased stability in the presence of nucleases.

While oligonucleotides are a preferred form of the compounds of this invention, the present invention contemplates other families of compounds as well, including but not limited to oligonucleotides, analogs and mimetics such as those herein described.

The open reading frame (ORF) or "coding region" which is known in the art to refer to the region between the translation initiation codon and the translation termination codon, is a region which may be effectively targeted. Within the context of the present invention, one region is the intragenic region encompassing the translation initiation or termination codon of the open reading frame (ORF) of a gene.

Other target regions include the 5' untranslated region (5'UTR), known in the art to refer to the portion of an mRNA in the 5' direction from the translation initiation codon, and thus including nucleotides between the 5' cap site and the translation initiation codon of an mRNA (or corresponding nucleotides on the gene), and the 3' untranslated region (3'UTR), known in the art to refer to the portion of an mRNA in the 3' direction from the translation termination codon, and thus including nucleotides between the translation termination codon and 3' end of an mRNA (or corresponding nucleotides on the gene). The 5' cap site of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap site. It is also preferred to target the 5' cap region.

As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn, the respective ends of this linear polymeric compound can be further joined to form a circular compound, however, linear compounds are generally preferred. In addition, linear compounds may have internal nucleobase complementarity and may, therefore, fold in a manner as to produce a fully or partially double-stranded compound. Within oligonucleotides, the phosphate groups are commonly referred to as forming the internucleoside backbone of the oligonucleotide. The normal linkage or backbone of RNA and DNA is a 3' to 5' phosphodiester linkage.

Specific examples of preferred antisense compounds useful in this invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

Preferred modified oligonucleotide backbones containing a phosphorus atom therein include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Preferred oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included.

The isolated or recombinant agonists and antagonists of the instant invention are used directly or they may be further modified by methods well known in the art in order to improve their effectiveness as pharmaceutical or other reagents. Important considerations for an active compound include formulations and methods of delivery.

An agonist or antagonist includes molecules determined by all or part of the target in genetic or proteinaceous form, such as antibodies, mimetics or antisense molecules.

Antibodies including anti-idiotypic antibodies, chaemeric antibodies and humanised antibodies are useful in this regard and their generation is now routine to those of skill in the art. Peptide or non-peptide mimetics can be developed as agonists of the targets by identifying those residues of the target molecule which are important for function. Modelling can be used to design molecules which interact with the target molecule and which have improved pharmacological properties. All such molecules will need to be modified to permit entry into a cell.

Rational drug design permits the production of structural analogs of biologically active polypeptides of interest or of small molecules with which they interact (e.g. agonists, antagonists, inhibitors or enhancers) in order to fashion drugs which are, for example, more active or stable forms of the polypeptide, or which, e.g. enhance or interfere with the function of a polypeptide *in vivo.* See, e.g. Hodgson (Bio/Technology 9: 19-21, 1991). In one approach, one first determines the three-dimensional structure of a protein of interest by x-ray crystallography, by computer modeling or most typically, by a combination of approaches. Useful information regarding the structure of a polypeptide may also be gained by modeling based on the structure of homologous proteins. An example of rational drug design is the development of HIV protease inhibitors (Erickson et al., Science 249: 527-533, 1990). In addition, target molecules may be analyzed by an alanine scan (Wells, Methods Enzymol. 202: 2699-2705, 1991). In this technique, an amino acid residue is replaced by Ala and its effect on the peptide's activity is determined. Each of the amino acid residues of the peptide is analyzed in this manner to determine the important regions of the peptide.

It is also possible to isolate a target-specific antibody, selected by a functional assay and then to solve its crystal structure. In principle, this approach yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced banks of peptides. Selected peptides would then act as the pharmacore.

Analogs contemplated herein include but are not limited to modification to side chains, incorporating of unnatural amino acids and/or their derivatives during peptide, polypeptide or protein synthesis and the use of crosslinkers and other methods which impose conformational constraints on the proteinaceous molecule or their analogs.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation via O-acylisourea formation followed by subsequent derivitization, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 4.

**TABLE 4**

| ***Codes for non-conventional amino acids*** | | | |
|---|---|---|---|
| Non-conventional amino acid | Code | Non-conventional amino acid | Code |
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | Chexa | L-Nmethylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(p-hydroxyphenyl)glycine | Nhtyr |
| L-t-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-t-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Mom |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) carbamylmethyl)glycine | Nnbhm | N-(N-(3,3-diphenylpropyl) carbamylmethyl)glycine | Nnbhe |
| 1-carboxy-1-(2,2-diphenyl-ethylamino)cyclopropane | Nmbc | | |

Crosslinkers can be used, for example, to stabilize 3D conformations, using homobifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of C_{α} and N _{α}-methylamino acids and the introduction of double bonds between C_{α} and C_{β} atoms of amino acids.

Natural product, combinatorial or phage display technologies are all available for screening for modulators. A huge choice of high through put screening methods are available which may be adapted to employ the cells of the present invention.

Two-hybrid screening is also useful in identifying other members of the genetic network acting with of Blimp-1. Target interactions and screens for modulators can be carried out using the yeast two-hybrid system, which takes advantage of transcriptional factors that are composed of two physically separable, functional domains. The most commonly used is the yeast GAL4 transcriptional activator consisting of a DNA binding domain and a transcriptional activation domain. Two different cloning vectors are used to generate separate fusions of the GAL4 domains to genes encoding potential binding proteins. The fusion proteins are co-expressed, targeted to the nucleus and if interactions occur, activation of a reporter gene *(*e.g. *lacZ)* produces a detectable phenotype. In the present case, for example, S. *cerevisiae is* co-transformed with a library or vector expressing a cDNA GAL4 activation domain fusion and a vector expressing a Myb pathway component fused to GAL4. If *lacZ is* used as the reporter gene, co-expression of the fusion proteins will produce a blue colour. Small molecules or other candidate compounds which interact with a target will result in loss of colour of the cells. Reference may be made to the yeast two-hybrid systems as disclosed by Munder et al., (Appl. Microbiol. Biotechnol. 52(3): 311-320, 1999) and Young et al., Nat. Biotechnol. 16(10): 946-950, 1998). Molecules thus identified by this system are then re-tested in the genetically modified organisms or genetically modified cells of the present invention.

Pharmaceutical compositions for therapy are further contemplated comprising recombinant, synthetic or isolated forms of the present agonists and antagonists and one or more pharmaceutically acceptable carriers, diluents or excipients. The treatment of cancer or the modulation of an immune response are particularly contemplated.

The term therapy should be taken as a reference to treatment or prophylaxis of a condition or disease. The term "treating" and "ameliorating" are used interchangeably.

The terms "composition" or "agent" or "medicament" refer to a chemical compound that induces a desired pharmacological and/or physiological effect. The term also encompass pharmaceutically acceptable and pharmacologically active ingredients of those compounds specifically mentioned herein including but not limited to salts, esters, amides, prodrugs, active metabolites, analogs and the like. When the above term is used, then it is to be understood that this includes the active agent *per se* as well as pharmaceutically acceptable, pharmacologically active salts, esters, amides, prodrugs, metabolites, analogs, etc. The term "compound" is not to be construed narrowly but extends to peptides, polypeptides and proteins as well as genetic molecules such as RNA, DNA and mimetics and chemical analogs thereof.

The phrases "ameliorating a disease or condition" or "treatment" or "therapeutic" are used in the broadest context and include any measurable or statistically significant improvement in a disease or condition or one or more symptoms or frequency of symptoms of a disease or condition as well as complete recovery from the disease or elimination of a condition, its symptoms or its underlying cause. The present invention is applicable to a large range of diseases or conditions and the skilled addressee must determine the precise parameters of the assessment of phenotypes on a case by case basis. Conditions may be associated with one or more diseased or they may not be so linked. The amelioration of a condition encompasses any desired physiological or psychological change.

An effective amount of the instant compositions is established best by those skilled in the art. The term "effective amount" of a compound as used herein mean a sufficient amount of the agent to provide the desired therapeutic or physiological effect. Undesirable effects, e.g. side effects, are sometimes manifested along with the desired therapeutic effect; hence, a practitioner balances the potential benefits against the potential risks in determining what is an appropriate "effective amount". The exact amount required will vary from subject to subject, depending on the species, age and general condition of the subject, mode of administration and the like. Thus, it may not be possible to specify an exact "effective amount". However, an appropriate "effective amount" in any individual case may be determined by one of ordinary skill in the art using only routine experimentation.

Pharmaceutical compositions for therapy are further contemplated comprising recombinant, synthetic or isolated forms of the present agonists and antagonists and one or more pharmaceutically acceptable carriers, diluents or excipients. The treatment of cancer or the modulation of an immune response are particularly contemplated.

The term therapy should be taken as a reference to treatment or prophylaxis of a condition or disease. The term "treating" and "ameliorating" are used interchangeably.

The terms "composition" or "agent" or "medicament" refer to a chemical compound that induces a desired pharmacological and/or physiological effect. The term also encompass pharmaceutically acceptable and pharmacologically active ingredients of those compounds specifically mentioned herein including but not limited to salts, esters, amides, prodrugs, active metabolites, analogs and the like. When the above term is used, then it is to be understood that this includes the active agent *per se* as well as pharmaceutically acceptable, pharmacologically active salts, esters, amides, prodrugs, metabolites, analogs, etc. The term "compound" is not to be construed narrowly but extends to peptides, polypeptides and proteins as well as genetic molecules such as RNA, DNA and mimetics and chemical analogs thereof.

The phrases "ameliorating a disease or condition" or "treatment" or "therapeutic" are used in the broadest context and include any measurable or statistically significant improvement in a disease or condition or one or more symptoms or frequency of symptoms of a disease or condition as well as complete recovery from the disease or elimination of a condition, its symptoms or its underlying cause. The present invention is applicable to a large range of diseases or conditions and the skilled addressee must determine the precise parameters of the assessment of phenotypes on a case by case basis. Conditions may be associated with one or more diseased or they may not be so linked. The amelioration of a condition encompasses any desired physiological or psychological change.

An effective amount of the instant compositions is established best by those skilled in the art. The term "effective amount" of a compound as used herein mean a sufficient amount of the agent to provide the desired therapeutic or physiological effect. Undesirable effects, e.g. side effects, are sometimes manifested along with the desired therapeutic effect; hence, a practitioner balances the potential benefits against the potential risks in determining what is an appropriate "effective amount". The exact amount required will vary from subject to subject, depending on the species, age and general condition of the subject, mode of administration and the like. Thus, it may not be possible to specify an exact "effective amount". However, an appropriate "effective amount" in any individual case may be determined by one of ordinary skill in the art using only routine experimentation.

The polypeptides, nucleic acids, antibodies, peptides, chemical analogs, agonists, antagonists or mimetics of the present invention can be formulated in pharmaceutic compositions which are prepared according to conventional pharmaceutical compounding techniques. See, for example, Remington's Pharmaceutical Sciences, 18th Ed. (1990, Mack Publishing, Company, Easton, PA, U.S.A.). The composition may contain the active agent or pharmaceutically acceptable salts of the active agent. These compositions may comprise, in addition to one of the active substances, a pharmaceutically acceptable excipient, carrier, buffer, stabilizer or other materials well known in the art. Such materials should be non-toxic and should not interfere with the efficacy of the active ingredient. The carrier may take a wide variety of forms depending on the form of preparation desired for administration, e.g. intravenous, oral, intrathecal, epineural or parenteral.

For oral administration, the compounds can be formulated into solid or liquid preparations such as capsules, pills, tablets, lozenges, powders, suspensions or emulsions. In preparing the compositions in oral dosage form, any of the usual pharmaceutical media may be employed, such as, for example, water, glycols, oils, alcohols, flavoring agents, preservatives, coloring agents, suspending agents, and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are obviously employed. If desired, tablets may be sugar-coated or enteric-coated by standard techniques. The active agent can be encapsulated to make it stable to passage through the gastrointestinal tract while at the same time allowing for passage across the blood brain barrier. See for example, International Patent Publication No. WO 96/11698.

For parenteral administration, the compound may dissolved in a pharmaceutical carrier and administered as either a solution or a suspension. Illustrative of suitable carriers are water, saline, dextrose solutions, fructose solutions, ethanol, or oils of animal, vegetative or synthetic origin. The carrier may also contain other ingredients, for example, preservatives, suspending agents, solubilizing agents, buffers and the like. When the compounds are being administered intrathecally, they may also be dissolved in cerebrospinal fluid.

The active agent is preferably administered in a therapeutically effective amount. The actual amount administered and the rate and time-course of administration will depend on the nature and severity of the condition being treated. Prescription of treatment, e.g. decisions on dosage, timing, etc. is within the responsibility of general practitioners or specialists and typically takes account of the disorder to be treated, the condition of the individual patient, the site of delivery, the method of administration and other factors known to practitioners. Examples of techniques and protocols can be found in Remington's Pharmaceutical Sciences, *(supra).*

Alternatively, targeting therapies may be used to deliver the active agent more specifically to certain types of cell, by the use of targeting systems such as antibodies or cell specific ligands. Targeting may be desirable for a variety of reasons, e.g. if the agent is unacceptably toxic or if it would otherwise require too high a dosage or if it would not otherwise be able to enter the target cells.

Instead of administering these agents directly, they could be produced in the target cell, e.g. in a viral vector such as described above or in a cell based delivery system such as described in U.S. Patent No. 5,550,050 and International Patent Publication Nos. WO 92/19195, WO 94/25503, WO 95/01203, WO 95/05452, WO 96/02286, WO 96/02646, WO 96/40871, WO 96/40959 and WO 97/12635. The vector could be targeted to the target cells or expression of expression products could be limited to specific cells, stages of decelopment or cell cycle stages. The cell based delivery system is designed to be implanted in a patient's body at the desired target site and contains a coding sequence for the target agent. Alternatively, the agent could be administered in a precursor form for conversion to the active form by an activating agent produced in, or targeted to, the cells to be treated. See, for example, European Patent Application No. 0 425 731A and International Patent Publication No. WO 90/07936.

The present invention is further described by the following non-limiting Examples.

### EXAMPLE 1

### Generation of a Blimp-1 mutant allele (Blimp^{gfp})

*A Blimp-1* targeting construct was produced in which, inserted into the intron 3' to exon 6, is an *eGFP* expression cassette consisting of a splice acceptor, stop codons in all three reading frames, an internal ribosome entry site (IRES), the cDNA encoding *eGFP,* and the SV40 polyadenylation signal to terminate transcription. Also inserted into the intron is a *PGK-Neo^{r}* gene to allow for the selection of embryonic stem (ES) cells with an integrated targeting vector. The *eGFP* and *Neo^{r}* cassettes are flanked by *Frt* sites to allow flp recombinase-mediated deletion of the inserted DNA. C57BL/6 ES cells were electroporated with the *Blimp-1* targeting construct, resistant clones selected by G418 resistance and screened by Southern hybridisation to 5' and 3' genomic DNA probes (Figure 1C). Four correctly targeted clones carrying the *Blimp*^{gfp} allele (Figure 1C) were identified from 300 screened colonies. These were injected into BALB/c blastocysts to obtain chimeric founders. These chimeras have been bred, and germ-line transmission has been achieved with one clone (4F3).

### EXAMPLE 2

### A GFP reporter that allows the description of the expression pattern of Blimp-1

*Blimp-1* was initially reported to be expressed solely in B-lymphocytes that have been induced to undergo ASC differentiation (Turner *et al., (supra)*). However, subsequent studies have revealed a broader expression pattern of Blimp-1 during embryogenesis *(*Chang et al., Mech Dev 117:305, 2002) and in myeloid cells (Chang *et al., (supra),* 2000). The *Blimp*^{gfp} allele permits a fuller definition of the expression pattern of *Blimp-1,* both within the haematopoietic lineage and more broadly in the organism. The targeting strategy outlined above results in a *Blimp*^{gfp} allele that expresses GFP from a bicistronic mRNA under the control of the endogenous Blimp-1 regulatory elements and is thus predicted to recapitulate the full *Blimp-1* expression pattern. In addition this strategy interrupts the *Blimp-1* mRNA transcript to produce a truncated version of the Blimp-1 protein (exons 1-6) that lacks the Zinc finger domains containing the DNA binding motif. In agreement with this, Western blotting of *Blimp*^{gfp/+} B cells induced to differentiate with LPS *in vitro* demonstrated both the wild type and truncated Blimp-1 protein bands (Figure 1D). By monitoring *GFP* expression in live cells and Blimp-1 protein in fixed tissue, the gene activity and differentiation fate of B-lymphocytes *in vivo* and *in vitro* at the single cell level can be monitored.

### EXAMPLE 3

### In vivo expression of Blimp-1 in ASC

Examination of lymphoid tissues in *Blimp*^{gfpl+} mice demonstrated a small population of high Blimp-1 is expressing cells in the bone marrow (0.1-0.2%), spleen (0.4-0.6%) and lymph node (0.1%) (Figure 2). Further, phenotypic analysis of the GFP⁺ cells indicated that they represented the previously defined Synd-1 high/B220 low ASC population as well as a previously poorly characterized Synd-1 low to negative phenotype (Figure 2, (Underhill *et al., (supra)*)). To confirm that these cells were ASC, GFP⁺ cells from *Blimp*^{gfp/+} bone marrow and spleen were sorted and subjected to ELISpot analysis for Ig production. As can be seen in Figure 3, 75-100% of cells were Ig secreting cells (representative of 3 independent experiments). Moreover sorting of the GFP negative fraction, revealed a frequency of ASC of 0.001% (<1 per 100,000 cells), whereas the frequency of these cells in unsorted bone marrow was between 0.05-0.09% (50-90 per 100,000). Therefore the isolation of *Blimp*^{gfp} expressing ASC gives an enrichment of 100,000 fold over unsorted cells and provides a virtually definitive method to isolate these rare cells. In addition all Ig isotypes were represented in the GFP⁺ ASC populations (Figure 3).

To further examine the production of ASC in *Blimp*^{gfP/+} mice using the GFP reporter mice were injected with 2µg lipopolysaccharide (LPS) intravenously and analysed for *GFP* expression 1-7 days post-injection (LPS injection results in the polyclonal activation of mature B cells). LPS injection resulted in a dramatic increase in the numbers of splenic GFP⁺ cells peaking at day 3 post-injection (∼5% of total cells) before declining to steady state levels around day 7 (Figure 4A). Analysis of gated GFP⁺ cells indicated that ASC differentiation occurred in a synchronous wave with the appearance of Synd-1⁺/B220⁺ cells followed by Synd-1⁺B220⁻ and finally a fraction of ASC become Synod-1⁻/B220⁻. This differentiation is also mirrored in the bone marrow where GFP⁺ cells appear at day 4 post-injection as Synd-1⁺/B220⁺ cells that rapidly generate the Synd-1^{+/-} /B220⁻ steady state populations (Figure 4B). In summary, LPS treatment induces a wave of ASC differentiation that can be for the first time phenotypically defined using the *Blimp*^{gfp/+} mouse stain.

### EXAMPLE 4

### Expression of Blimp-1 in ASC derived in vitro

A methodology was developed to quantitatively analyse the parameters affecting the commitment to and progression through the ASC lineage *in vitro.* This system involves the isolation of small resting B cells that are purified by Percoll gradient centrifugation and magnetic bead enrichment and cultured in the presence of a variety of stimuli that induce B cell proliferation and differentiation to ASC. These conditions include mimicking a T-dependent response using IL-4 and anti-CD40 or a T-independent reaction using LPS. In addition IL-5 can be titrated into these cultures to accelerate the rate of differentiation and anti-IgD (1.19) crosslinking carried out to activate an antigen specific response. Cultures were assayed on days 1-5 by flow cytometry to measure the frequency of *Blimp*^{gfp} and *Synd-1*⁺ expressing cells. The number of ASC in the culture was determined by ELIspot.

Analysis of the time course of *Blimp*^{gfp} induction using CD40L/IL-4/IL-5 or LPS (Figure 5A) indicated that the first GFP⁺ cells are observed in LPS cultures after 2 days. Thereafter, the numbers of positive cells increases until a peak at day 4 of approximately 50% GFP⁺ cells. In contrast CD40L/IL-4/IL-5 treatment results in a delayed induction of fewer GFP expressing cells. Interestingly, whereas the majority of CD40L/IL-4/IL-5 induced GFP expressing cells are also *Synd-1*⁺, LPS induces both *Synd-1*⁺ and *Synd-1*⁻GFP expressing cells (Figure 5B). To determine if all the GFP⁺ cells were actually ASC four fractions from LPS treated *Blimp*^{gfp} B cells were sorted (Figure 5C). ELIspot assays for IgM, IgG3 and IgG2b clearly show that all the ASC in the cultures are represented by the GFP⁺ fractions (A, B, C) and is not correlated with the levels of *Synd-1* expression. Moreover, the frequency of ASC did not vary between the GFP⁺ populations (Figure 5C). Thus these data clearly show that whilst *Blimp*^{gfp} is a marker of the ASC fate, *Synd-1*⁺ is only indicative of a sub-population of the ASC activity *in vitro* as it is *in vivo* (Figure 2). The regulation of *Blimp*^{gfp} and *Synd-1* expression was examined using the variety of stimuli outlined above. Interestingly, the frequency of GFP⁺/Synd-1⁺ and GFP⁺/Synd-1⁻ASC formation can be modulated by different stimuli as outlined in Figure 5C. Thus, following *Blimp*^{gfp} expression provides a simple and definitive methodology to identify the modulators of ASC induction *in vitro* and *in vivo.*

Finally, a transplantation model was developed to demonstrate that *in vitro* derived *Blimp*^{gfp} positive ASC can be detected in the bone marrow or spleen of non-irradiated hosts 7 days after intravenous injection (Figure 6). Therefore, the *Blimp^{gfp}* allele provides a method to examine the effects of *in vitro* treatments of ASC on their survival, migration and functional properties *in vivo.*

### EXAMPLE 5

### Blimp-1 is required for embryogenesis

To produce homozygous *Blimp*^{gfp/gfp} animals *Blimp*^{gfp/+} individuals were intercrossed. Offspring from these crosses were genotyped at day 21 post-birth using *Blimp-1* wild type and *Blimp*^{gfp} specific PCR primers. Whereas *Bliinp*^{gfp/+} mice were alive and healthy, no *Blimp*^{gfp/gfp} individuals were identified indicating that *Blimp*-1 deficiency results in embryonic or early post-partum lethality (Figure 7). To examine more closely the stage at which *Blimp*^{gfp/gfp} animals die, embryos produced from timed matings of *Blimp*^{gfp/+} mice were examiner. These data indicate that *Blimp*^{gfp/gfp} embryos are alive as late as embryonic stage E15.5. However, no viable older individuals have been documented. *Blimp*-1 is known to be widely expressed during embryogenesis, a finding that is supported by the analysis using the *Blimp*^{gfp} mouse.

### EXAMPLE 6

### Blimp-1 is essential for antibody production

To circumvent the embryonic lethality of *Blimp*^{gfp/gfp} animals, and examine directly the importance of *Blimp*-1 in antibody production fetal liver stem cell reconstitution of lethally irradiated syngenic mice was used to produce adult mice that lack functional a functional Blimp-1 protein throughout the haematopoietic system. These *Blimp*^{gfp/gfp} chimeric animals are healthy and contain relatively normal numbers of all the haematological lineages examined. *In vitro* analysis of the ASC population in these mice following stimulation with either LPS or CD40L/IL-4 and IL-5 revealed that the presence of GFP⁺ *Blimp* deficient cells that were predominantly synd-1⁺ (Figure 8A). Importantly, these cells failed to secrete antibody as assessed by ELIspot assay (Figure 8B). Therefore, the *Blimp*^{gfp} the mouse model described here not only provides a definitive tool to isolate ASC but enables the identification of the population of *Blimp-1* expressing cells from homozygous mutant *Blimp*^{gfp/gfp} splenocytes, thereby greatly facilitating the analysis of the mechanism underlying the phenotype of Blimp-1 deficiency.

### EXAMPLE 7

### Expression of Blimp-1 in other haematopoietic lineages

The *Blimp*^{gfp} reporter system has also enabled for the first time define the expression pattern of *Blimp-1* in haematopoiesis. As stated above analysis of the lymphoid organs of *Blimp*^{gfp} mice revealed that the GFP high producing populations are almost exclusively ASC. However, lower level GFP producing cells were also apparent.

*Blimp*-1 has been reported to be expressed by human and mouse macrophages and granulocytes. Flow cytometric analysis of blood monocytic cells and and bone marrow derived macrophages cultures in the presence of MCSF-1 revealed clear *Blimp*^{gfp} expression in these cell types (Figure 9). However, no GFP fluorescence was observed in granulocytes. *In vivo* isolated dendritic cells in contrast lack *Blimp*-1 mRNA expression. Similarly, plasmacytoid and conventional dendritic cells derived from the culture of bone marrow cells with flt3L lack *Blimp*^{gfp} fluorescence. However, the *ex vivo* activation of sorted dendritic cells or the *in vitro* activation of the flt3L cultures by CpG DNA results in *Blimp*-1 expression predominantly conventional dendritic cells (Figure 10).

Analysis of thymus and resting spleen from *Blimp*^{gfpl+} mice demonstrated that *Blimp*-1 is not expressed during T cell development. However, a small population of *Blimp*^{gfp} expressing T cells were present in lymph nodes. As these cells could represent the small population of activated T cells present we have stimulated lymph node T cells *in vitro* with an anti-CD3 monoclonal antibody in the presence or absence of concanavalin A, conditions known to strongly activate T cells. In support of the *in vivo* analysis, *in vitro* activated T cells expressed *Blimp*^{gfp} (Figure 12C).

Examination of the NK lineage in *Blimp*^{gfp} mice revealed that unlike the other haematopoietic lineages examined NK cells constitutively express *Blimp*-1*.* NK cells were identified from blood, spleen and bone marrow as NK1.1⁺/CD122⁺ cells and demonstrated to be uniformly GFP⁺ (Figure 12A). This expression was maintained *in vitro* as mature NK cells cultured in the presence of IL-15 are GFP⁺ and can be further induced by cytokines such as IL-21 or IL-12/IL-18 that induce NK cell terminal differentiation (Figure 12B). The expression of *Blimp*-1 in NK cells was also confirmed by Western blotting with a Blimp-1 specific monoclonal antibody.

In summary, the *Blimp*^{gfp} reporter mouse has revealed that Blimp-1 is induced in the late stages of a variety of haematopoietic lineages thereby providing a method of identifying the regulators of the maturation of these cell types. Importantly, the relatively lower production levels of GFP in non-B lymphoid cell types does not interfere with the isolation of homogenous populations of ASC.

### EXAMPLE 8

### Examining the role of Blimp-1 in cancer using the Blimp^{gfp} mouse

In addition to its utility in examining ASC differentiation, the *Blimp*^{gfp} reporter mouse can be used to examine the malignant transformation of this cell type. Tumors of ASC, designated plasmacytomas in mice and multiple myeloma in humans, are specifically and frequently elicited in Eµ*-*v-*abl* transgenic mice (Rosenbaum *et al., (supra)*), which express the v-*abl* oncogene in the B cell lineage, under the control of the IgH intronic enhancer. These mice were crossed with *Blimp*^{gfp} mutant mice, to determine the affect of loss of one or both copies of the *Blimp-1* gene on latency and incidence of tumors. Two outcomes are envisaged: Blimp-1, by inducing the plasma cell differentiation program, might be required to open the window of opportunity for v*-abl* transformation. This transgene induces only plasmacytomas, despite expression in earlier B cells (Rosenbaum *et al., (supra)*). Therefore, loss of functional *Blimp-1* alleles would be predicted to decrease tumor incidence or increase latency. Alternatively, as a large proportion of v-abl-induced plasmacytomas also bear a rearranged and activated *c-myc* gene, it may be that Myc is an essential cooperating activity in the transformation (Rosenbaum *et al., (supra)*). Blimp- is believed normally to repress *c-myc* expression during terminal ASC differentiation (Lin et al., Science 276:596, 1997). In this scenario, loss of functional Blimp-1 should allow continued *c-myc* expression, which may accelerate plasmacytoma development.

If Blimp-1 is indeed playing a role in ASC tumorogenesis, the *Blimp*^{***g***fp} reporter strain provides, therefore, a useful animal model to determe the effects of inhibiting/inducing Blimp-1 on tumor progression.

### EXAMPLE 9

### Methods for the Assessment of the role of Blimp in regulating terminal differentiation in T-cells

### Mice

*Blimp^{gfp}* (Kallies et al., J Exp Med 200:967-977, 2004), *Rag1*^{*-*/}*⁻,* and *Rag2*^{*-*/*-*} mice were maintained on a C57BL/6 background. *Blimp^{gfp}* genotyping and foetal liver chimeras were generated as described (Kallies *et al.*, 2004 (*supra*)).

### Flow cytometry and ELISAs

Monoclonal antibodies (mAbs) against CD4 (GK1.5), CD8 (53.6.7), TCRβ (H57-597), Ly5.2 (ALI-4A2) were purified from hybridoma supernatants on Protein G-sepharose columns (Amersham Pharmacia Biotech) and conjugated to biotin (Pierce Chemical Company), allophycocyanin (APC) and phycoerythrin (PE) (ProZyme) as recommended. Biotinlyated anti-CD25 (7D4) and CD122 (Tm-β1) and PE conjugated anti-CD44 (IM7), CD62L (MEL-14) IFNγ. □□□ 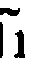 □, IL-4 (11B11) were obtained from PharMingen. Biotinylated mAbs were revealed with Streptavidin-PE or Cy5 (Southern Biotechnologies Inc). Cells were analyzed on a LSR cytometer (BD Biosciences) and cell sorting was carried out on high-speed flow cytometers (Moflo cytomation and BD Biosciences). Intracellular staining for cytokines was carried out according tostandard procedures known in the art. ELISA for IFNγ and IL-10 production was performed as described (Brady et al., J Immunol 172: 2048-2058, 2004). IL-4 ELISA used one monoclonal antibody as a capture reagent and a second monoclonal antibody for detection. ELISAs were performed in triplicate and quantified using recombinant protein standards.

### Western blotting

Total protein extracts were produced from equivalent numbers of cells and Western blotting was carried out for example as described by Rosenbauer et al., Embo J 21:211-20, 2002). Anti-Blimp-1 mAb has been previously described (Kallies *et al.*, 2004 (*supra*)). Equal protein loading was confirmed using anti-Vav1.

### HSV infection

Mice were infected with 4 × 10⁵ Herpes Simplex Virus (HSV-1 KOS strain) diluted in 20 µl PBS. Administration was by subcutaneous injection into the hindleg between the footpad and heal. Spleen and popliteal lymph nodes of infected mice were subsequently harvested for analysis.

### In vitro cytotoxicity

gB-specific cytotoxic T-Cell lymphoctyes (CTL) were generated by routine procedures.[Belz, 2001]. Spleens were removed from infected mice and single cells were cultured with 10⁸ 1000 Gy-irradiated gB₄₉₈₋₅₀₅-coated C57BL/6 spleen cells for 5 days. Cytotoxicity was assessed in a conventional ⁵¹Cr-release assay. The EL4 (H-2^{b}) target cells were labelled with Na⁵¹Cr for 1 h and pulsed with gB peptide 60 min, washed twice, and plated at 5,000 targets/well. They were then incubated with the effector populations for 5 h before harvesting supernatants for gamma counting. Two-fold lymphocyte dilutions were assayed in triplicate, while untreated and Triton X-100-disrupted controls were measured in quadruplicate. The percent specific lysis was calculated as 100 x (⁵¹Cr release from targets with effectors - ⁵¹Cr release from targets alone)/(51Cr release from targets with Triton X-100). The level of ⁵¹Cr release from targets incubated in the absence of T cells was <10% of the total Triton X-100-mediated ⁵¹Cr release.

### Tetramer staining of gB-specific CD8⁺ T cells

Virus-specific CD8⁺ T cells were identified using MHC class I tetrameric complexes [Altman, 1996 #102;Allan, 2003 #100] of the H-2K^{b} glycoprotein and peptide (SSIEFARL) derived from the glycoprotein B of herpes simplex virus (gB₄₉₈₋₅₀₅). Recombinant H-2K^{b} molecules with a birA biotinylation motif substituted for the carboxyl-terminal transmembrane domain were refolded with human β₂-microglobulin plus the viral peptide, biotinylated with birA and complexed at a 4:1 molar ratio with neutravidin-PE (Molecular Probes, Eugene, OR). Lymphocytes were stained for 60 minutes at room temperature with the tetrameric complexes in PBS/BSA/azide, followed by staining with anti-CD8αAPC, washed twice, and analyzed by flow cytometry.

### Histology

Organs were fixed in 10% buffered formalin, embedded in paraffin, sectioned and stained with hematoxylin/eosin.

### EXAMPLE 10

### Blimp expression can be induced by IL-21, is a key component of the maturation of naïve CD4⁺ and CD8⁺ T-cells into activated effector cells and is essential for normal lymphocyte homeostasis

Examination of the lymphoid organs from *Blimp*^{*gfp*/+} mice revealed a high level GFP in plasma cells. Furthermore, a population of T cells expressed low-levels of GFP (Figure 19A). Further analysis of *Rag1*^{*-*/*-*} mice reconstituted with *Blimp*^{*gfp*/*gfp*} cells, expressing no functional *Blimp* alleles revealed a pronounced expansion of this same population and a significant increase in GFP fluorescence (Figure 20). More extensive flow cytometric analysis of the T cell compartment of these mice revealed no GFP fluorescence in thymocytes (data not shown) or naive T cells (Figure 20).

In contrast and as shown herein, Blimp-1 is expressed specifically in activated/memory type CD4⁺ (TCRβ⁺CD62L^{low}) and CD8⁺ (TCRP⁺CD44^{high}) T cells (Figure 20). In a more detailed FACS analysis, alternate markers have been used to determine the nature of the expanded T cell pool in Blimp-1 deficient mice. In contrast to mice reconstituted with wildtype fetal liver, *Rag1*^{*-*/*-*} mice reconstituted with *Blimp*^{*gfp*/*gfp*} cells showed a strong expansion of the CD62L low GFP positive T cell population. Further, these mice had strongly elevated numbers of CD27 low to negative T cells, an increase in CD25 positive CD4 cells as well as a lack of distinct CD122 high population. Analysis using alternate markers, such as CD69, confirmed the activated status of the GFP⁺ cells (data not shown). The increased numbers of effector CD4 T cells in *Blimp*^{*gfp*/*gfp*} reconstituted *Rag1*^{*-*/*-*} mice was confirmed by *ex vivo* stimulation of isolated splenic CD4 T cells, resulting in 8 to 10 times higher IFN secretion, while secretion of IL-10 was decreased.

*In vitro* culture of naive T cells in the presence of anti-CD3/CD28 and IL-2 (for CD8⁺ cell) or Th1/Th2 polarizing conditions (for CD4⁺ cells) showed a low level GFP in primary stimulated cultures. However, upon re-stimulation CD4 and CD8 T cells from *Blimp*^{*gfp*/+} and *Blimp*^{*gfp*/*gfp*} mice rapidly became GFP⁺ (Figure 19B). Importantly, Blimp-1 expression in wildtype CD4⁺ effector T cells was confirmed by Western blotting (Figure. 19C). These data demonstrate that the induction of Blimp-1 expression is a component of the maturation of naïve CD4⁺ and CD8⁺ into effector cells.

To further evaluate the Blimp-1 expression after specific antigen stimulation, use was of an infectious disease model. Mice were infected with herpes simplex virus (HSV) and monitored at various time-points post infection for virus specific CD8⁺ T cell primary and memory response using a tetramer specific to the dominant epitope and standard cytotoxic function assays.

These experiments confirm the expression of Blimp-1 in antigen specific T cells and indicate that *Blimp*^{*gfp*/*gfp*} cells are fully functional in their ability to lyse antigen specific target cells.

The expanded pool of activated T cells observed in the *Blimp*^{*gfp*/*gfp*} reconstituted mice suggested aberrant responsiveness or deregulated homeostasis in the absence of Blimp-1. In keeping with this conclusion, *Blimp*^{*gfp*/*gfp*} reconstituted mice, displayed pronounced weight loss, ruffled coat and diarrhoea and had to be sacrificed from 6 weeks post-reconstitution. Histological analysis of these mice revealed extensive lymphocyte infiltration and inflammation of a variety of organs including lung, liver and gastrointestinal tract (Figure 21A). The tissue damage was most pronounced in the lung and intestine, implicating this process in the weight loss, diarrhoea and death observed in these mice. Flow cytometric analysis of lymphoid organs and liver of the reconstituted mice revealed a large expansion of fully activated CD4⁺ and CD8⁺ T cells in all organs (Figure 2B). The CD4⁺ cells were primarily Th1 biased. Moreover, reconstitution of *Rag1^{-l-}* mice with *Blimp*^{*gfp*/}*^{gfp} Rag2*^{*-*/*-*} foetal liver did not result in any deaths, strongly implicating T cells in the pathology observed (Figure 21).

To determine if the uncontrolled T cell expansion is intrinsic to the T cells, adoptive transfer experiments were performed of sorted T cells into *Rag1*^{*-*/}*⁻.* In these assays wild-type T cells respond to the lymphopenic environment by undergoing limited homeostatic expansion (Figure 22A). T-cells, 3x10⁶ CD4⁺ or CD8⁺ C57BL/6 or *Blimp*^{*gfp*/*gfp*} were injected into *Rag1*^{*-*/*-*} recipients and the numbers of splenic T cells assessed 3 weeks post-transfer. As shown in Figure 22A, *Blimp*^{*gfp*/*gfp*} T cells of both lineages had a dramatically expanded homeostatic expansion capacity. Moreover, all the resulting T cells expressed GFP and like the wild-type counterparts displayed an activated phenotype (Figure 22). The recipients that received *Blimp*^{*gfp*/*gfp*} T cells rapidly developed weight loss, splenomegaly (Figure 22) and a similar range of lymphocyte infiltration phenotypes as described in Figure 21. These data demonstrate that Blimp-1 deficient T cells display a dysregulated expansion that results in multi-organ infiltration and death.

Autoimmunity has been associated with the loss of functional CD4⁺CD25⁺ suppressor T cells. Analysis of this population in *Blimp*^{*+*/*gfp*} and *Blimp*^{*gfp*/*gfp*} CD4⁺ T cells revealed moderate to strong expression of GFP in a fraction of CD25⁺ CD4 T cells suggesting a function for Blimp-1 in the regulatory T cell population. Functional analyses *in vitro* and *in vivo* confirmed unchanged expression of FoxP3, a key factor for Treg, and other genes associated with Tregs. Blimp deficient cells CD4 cells secrete high levels of IFN and show defective IL-10 secretion.

To determine if the dysregulated homeostasis seen in Blimp-1 deficient T cells *in vivo* also manifests itself as enhanced proliferation *in vitro*, cultured naïve CD8⁺ T cells were incubated in the presence of anti-CD3/CD28 and cytokines known to regulate cell proliferation and homeostasis (including IL-2, IL-15 and IL-21). Naïve CD8⁺ cells grown for 7 days in the above conditions showed little GFP expression and similar proliferation profiles between the genotypes (Figure 23A). Similar, results were observed for naive CD4⁺ cells and those stimulated by PMA/ionomycin combination (data not shown). In contrast, secondary stimulation of the CD8⁺ cells in the presence of IL-2, IL-15 or IL-21 resulted in the strong induction of Blimp-1 expression (Figure 23A). In agreement with this expression profile, a significant increase in the cumulative cell number was observed in the proliferative response of the *Blimp*^{*gfp*/*gfp*} cells as compared to Blimp-1 sufficient cells. As expected, a similar strong difference in proliferative potential was observed when activated/memory CD44^{high} cells were used for the primary response. This increase was observed in all cytokine conditions but was most pronounced in the presence of IL-21 (Figure 23B). IL-21 is a T-helper cytokine that was recently shown to be a candidate diabetes susceptibility gene in NOD mice (King, et al. Cell 117:265-277, 2004). In that model IL-21 increased effector T cell turnover resulting in lymphopenia induced homeostatic proliferation and diabetes (King, *et al.*, 2004(*supra*)). Interestingly, IL-21 is a potent stimulator of Blimp-1 in B cell terminal differentiation, and was the most efficient inducer of GFP in CD8⁺ T cells suggesting a common role for this cytokine in lymphocyte differentiation.

Molecular and *in vitro* studies have shown that Blimp-1 is a potent transcriptional repressor that can recruit histone methyl-transferases (Gyory et al., Nat Immunol 5: 299-308, 2004), deacetylases (Yu et al., Mol Cell Biol 20:2592-3603, 2000) and co-repressors of the Groucho family (Ren et al., Genes Dev 13:125-137, 1999) to silence targets. Promoter and microarray studies have identified a number of Blimp-1 targets in the B lineage (Shaffer et al., Immunity 17:51-62, 2002; Shaffer et al., Immunity 21:81-93, 2004). Of these a number including c-myc (Lin et al., Science 276:596, 1997), CIITA (Piskurich et al., Nat Immunol 1:526-532, 2000), are expressed in the T lineage.

### EXAMPLE 11

### Blimp is the master regulator of a conserved terminal differentiation program in all lymphocytes.

In summary, the data described herein demonstrate that Blimp-1 is expressed in activated conventional T cells in a variety of contexts. Blimp expression is essential for normal lymphocyte homeostasis as mice injected with Blimp-1 deficient T cells or reconstituted with mutant stem cells die as a result of an aggressive multi-organ lymphoproliferative disease. Additionally, a cytokines such as IL-21 known to regulate the homeostasis of differentiating T cells, was a strong inducer of Blimp-1 expression and supported enhanced proliferation *in vitro* in the absence of Blimp-1. Accordingly, Blimp-1 expression may be induced in differentiating effector T cells, not by the initial stimulation but towards the completion of the immune response. Blimp-1 is therefore the first transcription factor identified which functions to regulate the genetic program of T cell contraction and/or memory formation that is essential for immune homeostasis.

B and T lymphocytes share many cellular and genetic similarities during their development such as a common progenitor, the ordered VDJ recombination and similar developmental checkpoints, however whilst the terminal differentiation of B cells to plasma cells is a clear functional end-point (Calame et al., Annu Rev Immunol 8:8, 2003), the final stages of T cell ontogeny are less defined. The similar functions and expression profile of Blimp-1 within the B and T cell lineages raises the intriguing possibility that despite the outwardly dissimilar appearance, Blimp-1 is the master regulator of a conserved terminal differentiation program in all lymphocytes.

Blimp deficiency causes hyperplasia and uncontrolled proliferation while expression of Blimp permits lymphocyte homeostasis and terminal differentiation of haematopoietic cells including ASC, T-cells and B-cells. Accordingly, Cytokines and other immunomodulatory, chemicals, peptides or other small or medium molecular agents which can be screened in the herein described *in vitro* and *in vivo* cellular model systems to determine their potential as therapeutic or prophylactic agents.

Accordingly, the present model reporter systems will be useful in assessing the ability of agents to modulate terminal differentiation in cells of the immune system such as T-cells and B-cells

### BIBLIOGRAPHY

Arkin et al., Proc. Natl. Acad. Sci. USA 89:7811-7815, 1992.
Altschul et al., Nucl. Acids Res. 25: 3389, 1997.
Angelin-Duclos et al., J Immunol 165:5462, 2000.
Angelin-Duclos et al., Eur J Immunol 32: 3765, 2002.
Ausubel (Ed) Current Protocols in Molecular Biology, 5th Edition, John Wiley & Sons, Inc, NY, 2002.
Badovinac et al., Nat Immunol 3:619-626, 2002.
Badovinac et al., Nat Immunol 5:809-817, 2004.
Bonner and Laskey, Eur. J. Biochem. 46: 83, 1974.
Brady et al., J Immunol 172: 2048-2058, 2004.
Calame et al., Annu Rev Immunol 8:8, 2003.
Chambers et al., Immunity 7:885-895, 1997.
Chang et al., Nat Immunol 1:169, 2000.
Chang et al., Mech Dev 117:305, 2002.
Dayhoff et al., Natl. Biomed. Res. Found 5:345-358,1978.
Delgrave et al., Protein Engineering 6:327-331, 1993.
de Souza et al., Embo J 18: 6062, 1999.
Erickson et al., Science 249: 527-533, 1990.
Fong et al., Proc Natl Acad Sci U S A 11: 11,2003.
Fontenot et al., Nat Immunol 4:330-336, 2003.
Gonnet et al., Science 256(5062):1443-1445, 1992.
Gyory et al., Nat Immunol 5: 299-308, 2004.
Hodgson, Bio/Technology 9: 19-21, 1991.
Hogan et al., Manipulating the Mouse Embryo: A Laboratory Manual, Cold Spring Harbour Laboratory Press, CSH NY, 1986.
Hori et al., Science 299:1057-1061, 2003.
Kallies et al., J Exp Med 200:967-977, 2004.
Keller et al., Genes Dev 5:868, 1991.
Khattri et al., Nat Immunol 4:337-342, 2003.
King, et al. Cell 117:265-277, 2004.
Knodel et al., Eur J Immunol 31:1972, 2001.
Kunkel, Proc. Natl. Acad. Sci. USA 82:488-492, 1985.
Kunkel et al., Methods in Enzymol. 154:367-382, 1987.
Kurreck et al, Eur. J. Biochem., 270:1628-1644, 2003.
Lin et al., Science 276:596, 1997.
Mansour et al., Nature 336:348-352, 1988.
Manz et al., Curr Opin Immunol 14:517, 2002.
Marcu et al., Annu Rev Biochem 61:809, 1992.
Marmur et al., J. Mol. Biol. 5: 109, 1962.
Medina et al., Blood 99:2154, 2002.
Messika et al., J Exp Med 188:515, 1998.
Munder et al., Appl., Microbiol. Biotechnol. 52(3): 311-320, 1999.
O'Connor et al., J Exp Med 195: 737, 2002.
Ozaki et al., J Immunol 173:5361-5371, 2004.
Pickert, Transgenic Animal Technology: A Laboratory Handbook, Academic Press, San Diago, CA, 1994.
Piskurich et al., Nat Immunol 1:526-532, 2000.
Remington's Pharmaceutical Sciences, 18th Ed., 1990, Mack Publishing, Company, Easton, PA, U.S.A.
Ren et al., Genes Dev 13:125-137, 1999.
Robertson et al., Nature 322:445-448, 1986.
Rosenbaum et al., Embo J 9:897, 1990.
Rosenbauer et al., Embo J 21:211-20, 2002.
Sambrook, Molecular Cloning: A Laboratoy Manual, 3rd Edition, CSHLP, CSH, NY, 2001.
Schliephake et al., Eur J Immunol 26: 268, 1996.
Shaffer et al., Immunity 17:51-62, 2002.
Shaffer et al., Immunity 21:81-93, 2004.
Shapiro, Practical flow cytometry, 3rd Ed., Brisbane, Wiley-Liss, 1995.
Shapiro-Shelef et al., Immunity 19: 607, 2003.
Soro et al., J Immunol 163:611, 1999.
Sprent et al., Annu Rev Immunol 20:551-579, 2002.
Sprent et al., Immunol Lett 85:145-149, 2003.
Summerton and Weller, Antisense and Nucleic Acid Drug Development 7: 187-195, 1997.
Tunyaplin et al., Nucleic Acid Research 28(24):4846-4855, 2000.
Turner et al., Cell 77:207, 1994.
Watson et al. "Molecular Biology of the Gene", Fourth Edition, Benjamin/Cummings, Menlo Park, Calif., 1987.
Ulleras et al., Toxicology 206(2):245-256, 2005*.*
Underhill et al., Blood 24:24, 2003.
Wells, Methods Enzymol. 202: 2699-2705, 1991.
Young et al., Nat. Biotechnol. 16(10): 946-950, 1998.
Youvan et al., Biotechnology et Elia 3: 1-18, 1997.
Yu et al., Mol Cell Biol 20:2592-3603, 2000.

## Claims

1. A genetically modified cell, which is not a human embryonic stem cell, or non-human organism comprising said cell, comprising a modified *Blimp. (PRDM-1)* gene which encodes a *Blimp* mRNA transcript comprising a Blimp coding sequence or encoding a functional or non-functional part thereof and a reporter molecule coding sequence and which is expressed under the control of endogenous *Blimp* regulatory elements and wherein the presence of Blimp in the cell is associated with a cellular phenotype and/or a commitment in the cell to terminally differentiate.

2. The cell or organism of claim 1, wherein the reporter molecule coding sequence is inserted within an intron of a *Blimp* allele.

3. The cell or non-human organism of claim 2, wherein the modified *Blimp* allele is present in homozygous or heterozygous form.

4. The cell or non-human organism of claim 3, wherein the modified *Blimp* allele is present in heterozygous form.

5. The cell or non-human organism of any one of claims 1 to 4, wherein the modified *Blimp* allele encodes a functional Blimp transcription factor or a functional part thereof.

6. The cell or non-human organism of any one of claims 1 to 4, wherein the modified *Blimp* allele encodes a non-functional Blimp transcription factor or a non-functional part thereof.

7. The cell or non-human organism of claim 1, comprising cells or genetic material derived from man, non-human primates, livestock, companion or laboratory test organisms, reptilian or amphibian species.

8. The cell or organism of claim 7, derived from a rodent, guinea pig, pig, duck, rabbit or sheep.

9. The cell or organism of claim 8, wherein the rodent is a mouse or rat.

10. The cell or organism of any one of claims 1 to 4, wherein the cell is a haematopoietic or embryonic cell.

11. The cell or organism of claim 10, wherein the cell is a haematopoietic cell.

12. The cell or organism of claim 11, wherein the cell is a lymphocytic cell.

13. The cell or organism of claim 12 wherein the presence of Blimp in a lymphocyte indicates that the cell is terminally differentiated or is committed to terminal differentiation.

14. The cell or organism of claim 12, wherein the cell is a cell of the lymphocyte lineage selected from a B-cell and a T-cell.

15. The cell or organism of claim 14 wherein the presence of Blimp in a B-cell lineage cell indicates that such cells are committed to differentiate or have differentiated into ASCs.

16. The cell or organism of claim 14 wherein the presence of Blimp in a T-cell lineage cell indicates that these cells are activated.

17. The cell or organism of claim 14, wherein the B-cells are ASCs.

18. The B-cells of claim 17, which are a substantially purified population of ASC.

19. The cell or organism of claim 14, wherein the T-cells are selected from CD4⁺ T-cells and CD8⁺ T-cells.

20. The cell or organism of any one of claims 1 to 19, wherein the detection of the reporter molecule is indicative of a cellular phenotype and/or commitment of a cell to terminally differentiate.

21. The non-human organism of any one of claims 1 to 17, 19 and 21, wherein the organism is provided in the form of embryos, gametes or ES cells.

22. The cell or organism of any one of claims 1 to 21, wherein the reporter molecule is a fluorescent or light emitting reporter molecule.

23. A method for phenotyping and/or monitoring a cell of the haematopoietic system comprising screening a genetically modified haematopoietic cell or non-human animal comprising such cells comprising a modified *Blimp* gene as defined in any one of claims 1, 2, 5 or 6, wherein detection of reporter activity is indicative of a cellular phenotype and/or commitment of the cell to terminally differentiate.

24. The method of claim 23, wherein the haematopoietic cell is a cell selected from B-cells, T-cells, dendritic cells, macrophages, natural killer cells, granulocytes, erythrocytes, eosinophils, megakaryocytes, bone marrow, splenic, dermal, or stromal cells and their precursors and derivatives.

25. The method of claim 24 wherein the B-cells are ASCs.

26. The method of claim 23 or 24, wherein phenotyping and/or monitoring of cells is achieved by cytometric analysis of a fluorescent or light emitting reporter molecule.

27. The method of claim 23, further comprising isolating or selecting cells which exhibit reporter activity or changes in reporter activity or level from among cells which do not exhibit reporter activity.

28. The method of claim 23, wherein the isolation of reporter-active cells is by flow cytometry, laser scanning cytometry, chromatography and/or other equivalent procedure.

29. The method of claim 27, further comprising selecting reporter-active cells using further selection markers.

30. The method of any one of claims 23 to 29, wherein the cells are ASC identified or isolated in a population of cells of a B-cell lineage.

31. The method of any one of claims 23, 24 or 26 to 29, wherein the cells are activated/terminally differentiated T-cells identified or isolated in a mixed population of T-cells.

32. A method for testing the antigenicity or immunogenicity of a vaccine, the method comprising;
(i) administering the vaccine to a genetically modified haematopoietic cell or non-human animal comprising such cells wherein the cell or organism comprises a modified *Blimp-1* gene as defined in any one of claims 1, 2, 5 or 6; and
(ii) testing the cell or organism for the reporter molecule the presence of which is indicative of the ability of the vaccine to induce terminal differentiation in haematopoietic cells.

33. The method of claim 32, wherein the presence of reporter activity is indicative of the ability of the vaccine to promote terminal differentiation in T-cells and/or B-cells.

34. A method for *in vitro* or *in vivo* screening for agonists or antagonists of terminal differentiation in haematopoietic cells comprising exposing one or more agent/s to a genetically modified cell or non-human animal comprising such cells wherein the cell or organism comprises a modified *Blimp-1* gene as defined in any one of claims 1, 2, 5 or 6 and testing the cell or organism for the presence or a change in the level of the reporter molecule the presence of which is indicative of the ability of the one or more agent/s to agonise or antagonise terminal differentiation.

35. The method of claim 23, 32 or 34, wherein the modified *Blimp* allele is present in homozygous or heterozygous form.

36. The method of claim 35, wherein the modified *Blimp* allele is present in heterozygous form.

37. The method of claim 33 or 34 wherein the cells or genetic material are derived from an organism as defined in any one of claims 7 to 9.

38. The method of claim 32 or 34, wherein the cell is as defined in any one of claims 12 to 17 or 19.

39. A targeting vector comprising a modified *Blimp (PRDM-1)* gene encoding a Blimp polypeptide and a reporter molecule which is expressed in a target cell under the control of endogenous *Blimp* regulatory elements and produces Blimp (PRDM-1) or a functional or non-functional part thereof co-expressed with the reporter molecule.

40. The targeting vector of claim 39, wherein the *Blimp* gene encodes a *Blimp* mRNA transcript comprising a Blimp coding sequence or encoding a functional or non-functional part thereof, and a reporter molecule coding sequence.

41. The targeting vector of claim 40, wherein the reporter molecule coding sequence is inserted within an intron of a *Blimp* allele.

42. The targeting vector of claim 39, wherein the reporter molecule is a GFP.

## Patentansprüche

1. Gentechnisch veränderte Zelle, bei der es sich nicht um eine menschliche embryonale Stammzelle handelt, oder die Zelle umfassender nichtmenschlicher Organismus, umfassend ein modifiziertes *Blimp-(PRDM-1-)-Gen,* das für ein eine Blimp-Codierungssequenz umfassendes oder für einen funktionellen oder nichtfunktionellen Teil davon codierendes *Blimp*-mRNA-Transkript und eine Reportermolekül-Codierungssequenz codiert und das unter der Kontrolle endogener Blimp-Regulationselemente exprimiert wird, und wobei das Vorliegen von Blimp in der Zelle mit einem zellulären Phänotyp und/oder einer Festlegung in der Zelle auf Ausdifferenzierung assoziiert ist.

2. Zelle oder Organismus nach Anspruch 1, wobei die Reportermolekül-Codierungssequenz in einem Intron eines *Blimp*-Allels inseriert ist.

3. Zelle oder nichtmenschlicher Organismus nach Anspruch 2, wobei das modifizierte *Blimp*-Allel in homozygoter oder heterozygoter Form vorliegt.

4. Zelle oder nichtmenschlicher Organismus nach Anspruch 3, wobei das modifizierte *Blimp*-Allel in heterozygoter Form vorliegt.

5. Zelle oder nichtmenschlicher Organismus nach einem der Ansprüche 1 bis 4, wobei das modifizierte *Blimp-Allel* für einen funktionellen Blimp-Transkriptionsfaktor oder einen funktionellen Teil davon codiert.

6. Zelle oder nichtmenschlicher Organismus nach einem der Ansprüche 1 bis 4, wobei das modifizierte *Blimp-Allel* für einen nichtfunktionellen Blimp-Transkriptionsfaktor oder einen nichtfunktionellen Teil davon codiert.

7. Zelle oder nichtmenschlicher Organismus nach Anspruch 1, umfassend Zellen oder genetisches Material aus Mensch, nichtmenschlichen Primaten, Vieh, Begleit- oder Labortestorganismen, Reptilien- oder Amphibienspezies.

8. Zelle oder Organismus nach Anspruch 7, aus einem Nager, Meerschweinchen, Schwein, Ente, Kaninchen oder Schaf.

9. Zelle oder Organismus nach Anspruch 8, wobei es sich bei dem Nager um eine Maus oder Ratte handelt.

10. Zelle oder Organismus nach einem der Ansprüche 1 bis 4, wobei es sich bei der Zelle um eine hämatopoetische oder embryonale Zelle handelt.

11. Zelle oder Organismus nach Anspruch 10, wobei es sich bei der Zelle um eine hämatopoetische Zelle handelt.

12. Zelle oder Organismus nach Anspruch 11, wobei es sich bei der Zelle um eine lymphozytische Zelle handelt.

13. Zelle oder Organismus nach Anspruch 12, wobei das Vorliegen von Blimp in einem Lymphozyten anzeigt, dass die Zelle ausdifferenziert oder auf Ausdifferenzierung festgelegt ist.

14. Zelle oder Organismus nach Anspruch 12, wobei es sich bei der Zelle um eine Zelle der aus einer B-Zelle und einer T-Zelle ausgewählten Lymphozytenlinie handelt.

15. Zelle oder Organismus nach Anspruch 14, wobei das Vorliegen von Blimp in einer Zelle der B-Zelllinie anzeigt, dass solche Zellen auf Differenzierung zu ASC-Zellen festgelegt oder zu ASC-Zellen differenziert sind.

16. Zelle oder Organismus nach Anspruch 14, wobei das Vorliegen von Blimp in einer Zelle der T-Zelllinie anzeigt, dass diese Zellen aktiviert sind.

17. Zelle oder Organismus nach Anspruch 14, wobei es sich bei den B-Zellen um ASC-Zellen handelt.

18. B-Zellen nach Anspruch 17, bei denen es sich um eine weitgehend aufgereinigte ASC-Population handelt.

19. Zelle oder Organismus nach Anspruch 14, wobei die T-Zellen aus CD4⁺-T-Zellen und CD8⁺-T-Zellen ausgewählt sind.

20. Zelle oder Organismus nach einem der Ansprüche 1 bis 19, wobei der Nachweis des Reportermoleküls ein Anzeichen für einen zellulären Phänotyp und/oder die Festlegung einer Zelle auf Ausdifferenzierung ist.

21. Nichtmenschlicher Organismus nach einem der Ansprüche 1 bis 17, 19 und 21, wobei der Organismus in Form von Embryos, Gameten oder ES-Zellen bereitgestellt wird.

22. Zelle oder Organismus nach einem der Ansprüche 1 bis 21, wobei es sich bei dem Reportermolekül um ein fluoreszierendes oder Licht emittierendes Reportermolekül handelt.

23. Verfahren zur Phänotypisierung und/oder Überwachung einer Zelle des hämatopoetischen Systems, umfassend das Screening einer gentechnisch veränderten hämatopoetischen Zelle oder eines solche Zellen umfassenden nichtmenschlichen Tiers, umfassend ein modifiziertes *Blimp-*Gen mit der in einem der Ansprüche 1, 2, 5 oder 6 angegebenen Bedeutung, wobei der Nachweis von Reporteraktivität ein Anzeichen für einen zellulären Phänotyp und/oder Festlegung der Zelle auf Ausdifferenzierung ist.

24. Verfahren nach Anspruch 23, wobei es sich bei der hämatopoetischen Zelle um eine aus B-Zellen, T-Zellen, dendritischen Zellen, Makrophagen, natürlichen Killerzellen, Granulozyten, Erythrozyten, Eosinophilen, Megakaryozyten, Knochenmark-, Milz-, Haut- oder Stromazellen und deren Vorläufern und Derivaten ausgewählte Zelle handelt.

25. Verfahren nach Anspruch 24, wobei es sich bei den B-Zellen um ASC-Zellen handelt.

26. Verfahren nach Anspruch 23 oder 24, wobei die Phänotypisierung und/oder Überwachung von Zellen mittels zytometrischer Analyse eines fluoreszierenden oder Licht emittierenden Reportermoleküls erreicht wird.

27. Verfahren nach Anspruch 23, bei dem man ferner Zellen, die Reporteraktivität oder Änderungen der Reporteraktivität oder -konzentration zeigen, unter Zellen, die keine Reporteraktivität zeigen, isoliert oder auswählt.

28. Verfahren nach Anspruch 23, wobei die Isolierung reporteraktiver Zellen mittels Durchflusszytometrie, Laser-Scanning-Zytometrie, Chromatographie und/oder einer gleichwertigen Verfahrensweise erfolgt.

29. Verfahren nach Anspruch 27, bei dem man ferner reporteraktive Zellen unter Verwendung weiterer Selektionsmarker auswählt.

30. Verfahren nach einem der Ansprüche 23 bis 29, wobei es sich bei den Zellen um in einer Population von Zellen einer B-Zelllinie identifizierte oder isolierte ASC-Zellen handelt.

31. Verfahren nach einem der Ansprüche 23, 24 oder 26 bis 29, wobei es sich bei den Zellen um in einer gemischten Population von T-Zellen identifizierte oder isolierte aktivierte/ausdifferenzierte T-Zellen handelt.

32. Verfahren zum Testen der Antigenität oder Immunogenität eines Impfstoffs, wobei man
(i) den Impfstoff an eine gentechnisch veränderte hämatopoetische Zelle oder ein solche Zellen umfassendes nichtmenschliches Tier verabreicht, wobei die Zelle bzw. der Organismus ein modifiziertes *Blimp-1-*Gen mit der in einem der Ansprüche 1, 2, 5 oder 6 angegebenen Bedeutung umfasst, und
(ii) die Zelle bzw. den Organismus auf das Reportermolekül testet, dessen Vorliegen ein Anzeichen für das Vermögen des Impfstoffs ist, Ausdifferenzierung bei hämatopoetischen Zellen zu induzieren.

33. Verfahren nach Anspruch 32, wobei das Vorliegen von Reporteraktivität ein Anzeichen für das Vermögen des Impfstoffs ist, Ausdifferenzierung bei T-Zellen und/oder B-Zellen zu fördern.

34. Verfahren zur In-vitro- oder In-vivo-Suche nach Agonisten oder Antagonisten der Ausdifferenzierung bei hämatopoetischen Zellen, bei dem man ein Agens oder mehrere Agentien mit einer gentechnisch veränderten Zelle oder einem solche Zellen umfassenden nichtmenschlichen Tier in Kontakt bringt, wobei die Zelle bzw. der Organismus ein modifiziertes *Blimp-1-*Gen mit der in einem der Ansprüche 1, 2, 5 oder 6 angegebenen Bedeutung umfasst, und die Zelle bzw. den Organismus auf das Vorliegen oder eine Änderung der Konzentration des Reportermoleküls testet, dessen Vorliegen ein Anzeichen für das Vermögen des einen Agens bzw. der mehreren Agentien ist, als Agonist oder Antagonist der Ausdifferenzierung zu wirken.

35. Verfahren nach Anspruch 23, 32 oder 34, wobei das modifizierte *Blimp-Allel* in homozygoter oder heterozygoter Form vorliegt.

36. Verfahren nach Anspruch 35, wobei das modifizierte *Blimp-Allel* in heterozygoter Form vorliegt.

37. Verfahren nach Anspruch 33 oder 34, wobei die Zellen oder das genetische Material aus einem Organismus mit der in einem der Ansprüche 7 bis 9 angegebenen Bedeutung stammen bzw. stammt.

38. Verfahren nach Anspruch 32 oder 34, wobei die Zelle die in einem der Ansprüche 12 bis 17 oder 19 angegebene Bedeutung aufweist.

39. Zielvektor, umfassend ein für ein Blimp-Polypeptid und ein Reportermolekül codierendes modifiziertes *Blimp-(PRDM-1-)-Gen,* das in einer zielzelle unter der Kontrolle endogener *Blimp*-Regulationselemente exprimiert wird, wobei bei gleichzeitiger Expression mit dem Reportermolekül Blimp (PRDM-1) oder ein funktioneller oder nichtfunktioneller Teil davon produziert wird.

40. Zielvektor nach Anspruch 39, wobei das *Blimp*-Gen für ein eine Blimp-Codierungssequenz umfassendes oder für einen funktionellen oder nichtfunktionellen Teil davon codierendes *Blimp-*mRNA-Transkript und eine Reportermolekül-Codierungssequenz codiert.

41. Zielvektor nach Anspruch 40, wobei die Reportermolekül-Codierungssequenz in einem Intron eines *Blimp*-Allels inseriert ist.

42. Zielvektor nach Anspruch 39, wobei es sich bei dem Reportermolekül um ein GFP handelt.

## Revendications

1. Cellule génétiquement modifiée, qui n'est pas une cellule souche embryonnaire humaine, ou organisme non humain comprenant ladite cellule comprenant un gène *Blimp* (*PRDM*-1) modifié, qui code pour un transcrit d'ARNm de *Blimp* comprenant une séquence codante de Blimp ou codant pour une partie fonctionnelle ou non fonctionnelle de celle-ci, et une séquence codante de molécule rapporteur et qui est exprimée sous le contrôle d'éléments de régulation de *Blimp* endogènes, et dans laquelle la présence de Blimp dans la cellule est associée à un phénotype cellulaire et/ou à un engagement dans la cellule pour se différencier en phase terminale.

2. Cellule ou organisme selon la revendication 1, dans laquelle la séquence codant pour la molécule rapporteur est insérée dans un intron d'un allèle de *Blimp.*

3. Cellule ou organisme non humain selon la revendication 2, dans laquelle l'allèle de *Blimp* modifié est présent sous forme homozygote ou hétérozygote.

4. Cellule ou organisme non humain selon la revendication 3, dans laquelle l'allèle de *Blimp* modifié est présent sous forme hétérozygote.

5. Cellule ou organisme non humain selon l'une quelconque des revendications 1 à 4, dans laquelle l'allèle de *Blimp* modifié code pour un facteur de transcription de Blimp fonctionnel ou une partie fonctionnelle de celui-ci.

6. Cellule ou organisme non humain selon l'une quelconque des revendications 1 à 4, dans laquelle l'allèle de *Blimp* modifié code pour un facteur de transcription de Blimp non fonctionnel ou une partie non fonctionnelle de celui-ci.

7. Cellule ou organisme non humain, selon la revendication 1, comprenant des cellules ou du matériel génétique dérivé de l'Homme, de primates non humains, du bétail, d'organismes de compagnie ou de test pour laboratoire, d'espèces reptiliennes ou amphibiennes.

8. Cellule ou organisme, selon la revendication 7, dérivé(e) d'un rongeur, d'un cobaye, d'un porc, d'un canard, d'un lapin ou d'un mouton.

9. Cellule ou organisme selon la revendication 8, dans laquelle le rongeur est une souris ou un rat.

10. Cellule ou organisme selon l'une quelconque des revendications 1 à 4, dans laquelle la cellule est une cellule hématopoïétique ou embryonnaire.

11. Cellule ou organisme selon la revendication 10, dans laquelle la cellule est une cellule hématopoïétique.

12. Cellule ou organisme selon la revendication 11, dans laquelle la cellule est une cellule lymphocytaire.

13. Cellule ou organisme selon la revendication 12, dans laquelle la présence de Blimp dans un lymphocyte indique que la cellule est différentiée en phase terminale ou qu'elle est engagée vers une différentiation terminale.

14. Cellule ou organisme selon la revendication 12, dans laquelle la cellule est une cellule de la lignée lymphocytaire choisie parmi un lymphocyte B et un lymphocyte T.

15. Cellule ou organisme selon la revendication 14, dans laquelle la présence de Blimp dans une cellule de lignée de lymphocytes B indique que de telles cellules sont engagées pour se différentier ou se sont différentiées en ASC.

16. Cellule ou organisme selon la revendication 14, dans laquelle la présence de Blimp dans une cellule de lignée de lymphocytes T indique que ces cellules sont activées.

17. Cellule ou organisme selon la revendication 14, dans laquelle les lymphocytes B sont des ASC.

18. Lymphocytes B selon la revendication 17, qui sont une population substantiellement purifiée d'ASC.

19. Cellule ou organisme selon la revendication 14, dans laquelle les lymphocytes T sont choisis parmi des lymphocytes T CD4⁺ et des lymphocytes T CD8⁺.

20. Cellule ou organisme selon l'une quelconque des revendications 1 à 19, dans laquelle la détection de la molécule rapporteur est une indication d'un phénotype cellulaire et/ou de l'engagement d'une cellule pour se différentier en phase terminale.

21. Organisme non humain selon l'une quelconque des revendications 1 à 17, 19 et 21, dans laquelle l'organisme est fourni sous forme d'embryons, de gamètes ou de cellules ES.

22. Cellule ou organisme selon l'une quelconque des revendications 1 à 21, dans laquelle la molécule rapporteur est une molécule rapporteur fluorescente ou qui émet une lumière.

23. Procédé de phénotypage et/ou de suivi d'une cellule du système hématopoïétique comprenant le criblage d'une cellule hématopoïétique ou d'un animal non humain génétiquement modifié(e) comprenant de telles cellules, qui comprennent un gène *Blimp.*modifié, tel que défini dans l'une quelconque des revendications 1, 2, 5 ou 6, dans lequel une détection de l'activité de rapporteur est une indication d'un phénotype cellulaire et/ou d'un engagement de la cellule pour se différentier en phase terminale.

24. Procédé selon la revendication 23, dans lequel la cellule hématopoïétique est une cellule choisie parmi les lymphocytes B, lymphocytes T, cellules dendritiques, macrophages, cellules tueuses naturelles, granulocytes, érythrocytes, polynucléaires éosinophiles, mégacaryocytes, la moelle osseuse, des cellules de la rate, du derme ou du stroma ainsi que leurs précurseurs et dérivés.

25. Procédé selon la revendication 24, dans lequel les lymphocytes B sont des ASC.

26. Procédé selon la revendication 23 ou la 24, dans lequel le phénotypage et/ou le suivi de cellules est accompli par une analyse cytométrique d'une molécule rapporteur fluorescente ou qui émet une lumière.

27. Procédé, selon la revendication 23, comprenant en outre l'isolement ou la sélection de cellules qui présentent une activité de rapporteur ou des changements de l'activité ou du taux de rapporteur provenant de cellules qui ne présentent pas d'activité de rapporteur.

28. Procédé selon la revendication 23, dans laquelle l'isolement de cellules à rapporteur actif se fait par cytométrie en flux, cytométrie par balayage laser, chromatographie et/ou autre procédure équivalente.

29. Procédé, selon la revendication 27, comprenant en outre la sélection de cellules à rapporteur actif en utilisant des marqueurs de sélection supplémentaires.

30. Procédé selon l'une quelconque des revendications 23 à 29, dans lequel les cellules sont des ASC identifiées ou isolées dans une population de cellules d'une lignée de lymphocytes B.

31. Procédé selon l'une quelconque des revendications 23, 24 ou 26 à 29, dans lequel les cellules sont des lymphocytes T activés/différentiés en phase terminale, qui sont identifiés ou isolés dans une population mélangée de lymphocytes T.

32. Procédé de test de l'antigénicité ou de l'immunogénicité d'un vaccin, le procédé comprenant les étapes consistant à :
(i) administrer le vaccin à une cellule hématopoïétique ou un animal non humain génétiquement modifié(e) comprenant de telles cellules, dans lequel la cellule ou l'organisme comprend un gène *Blimp-1* modifié, tel que défini dans l'une quelconque des revendications 1, 2, 5 ou 6 ; et
(ii) tester la cellule ou l'organisme pour la molécule rapporteur, dont la présence est une indication de la faculté du vaccin à induire une différentiation terminale dans des cellules hématopoïétiques.

33. Procédé selon la revendication 32, dans lequel la présence d'une activité de rapporteur est une indication de la faculté du vaccin à favoriser une différentiation terminale dans des lymphocytes T et/ou des lymphocytes B.

34. Procédé de criblage *in vitro* ou *in vivo* en vue d'agonistes ou d'antagonistes d'une différentiation terminale dans des cellules hématopoïétiques, comprenant l'exposition d'un ou plusieurs agent(s) à une cellule ou un animal non humain génétiquement modifié(e) comprenant de telles cellules, dans lequel la cellule ou l'organisme comprend un gène *Blimp-1* modifié, tel que défini dans l'une quelconque des revendications 1, 2, 5 ou 6, et le test de la cellule ou de l'organisme pour la présence ou un changement dans le taux de la molécule rapporteur, dont la présence est une indication de la faculté d'un ou plusieurs agent(s) à être en agonisme ou en antagonisme avec une différentiation terminale.

35. Procédé selon la revendication 23, la 32 ou la 34, dans lequel l'allèle de *Blimp* modifié est présent sous forme homozygote ou hétérozygote.

36. Procédé selon la revendication 35, dans lequel l'allèle de *Blimp* modifié est présent sous forme hétérozygote.

37. Procédé selon la revendication 33 ou la 34, dans lequel les cellules ou le matériel génétique sont (est) dérivé(es) d'un organisme tel que défini dans l'une quelconque des revendications 7 à 9.

38. Procédé selon la revendication 32 ou la 34, dans lequel la cellule est telle que définie dans l'une quelconque des revendications 12 à 17 ou 19.

39. Vecteur de ciblage comprenant un gène *Blimp* (*PRDM-*1) modifié, qui code pour un polypeptide Blimp, et une molécule rapporteur qui est exprimée dans une cellule cible sous le contrôle d'éléments de régulation de *Blimp* endogènes et produit un Blimp (PRDM-1) ou une partie fonctionnelle ou non fonctionnelle de celui-ci, qui est exprimée conjointement avec la molécule rapporteur.

40. Vecteur de ciblage selon la revendication 39, dans lequel le gène Blimp code pour un transcrit d'ARNm de *Blimp* comprenant une séquence codante de Blimp ou codant pour une partie fonctionnelle ou non fonctionnelle de celle-ci, et une séquence codante de molécule rapporteur.

41. Vecteur de ciblage selon la revendication 40, dans lequel la séquence codante de molécule rapporteur est insérée dans un intron d'un allèle de *Blimp.*

42. Vecteur de ciblage selon la revendication 39, dans lequel la molécule rapporteur est une GFP.


### SEQUENCE LISTING

<110> The Walter and Eliza Hall Institute of Medical Research
<120> Modified cells and methods of using same
<130> 12567320
<150> AU 2004900673
   <151> 2004-02-12
<160> 6
<170> Patent In version 3.1
<210> 1
   <211> 2571
   <212> DNA
   <213> murine
<400> 1
   atgagagagg cttatctcag atgttggatc ttctcttgga aaaacgtgtg ggtacgacct      60
   tgccaaaggc tgcattttaa aaccgtgctt cttcaaggca gtctacttta cacggctttg     120
   gactcttact caactgtaca agctgccccc aagtctagct ccggctccgt gaagtttcaa     180
   ggactggcag agactgggat catgaaaatg gacatggagg acgctgatat gactttgtgg     240
   acagaggccg agtttgaaga gaagtgtaca tacatagtga acgaccaccc ctgggattcc     300
   ggcgctgacg ggggtacttc tgttcaagcc gaggcatcct taccaaggaa cctgcttttc      360
   aagtatgctg ccaacaacag caaagaggtt attggcgtgg taagtaagga gtacataccg     420
   aagggaacac gctttggacc cctcatcggt gaagtctaca ctaatgacac agttcccaag     480
   aatgccaaca ggaagtattt ttggcggatc tattccagag aggagttcca ccacttcatt     540
   gatggcttta atgaggagaa aagcaactgg atgcgctacg tgaatccagc tcactctgcc     600
   cgggagcaaa acctggctgc ctgtcagaac gggatgaaca tctacttcta cactattaag     660
   cctatccctg ccaaccagga acttcttgtg tggtattgtc gggactttgc ggagaggctc     720
   cactaccctt atcctggaga gctcacagtg ataaatctca cacaaacgga aagcaaccca     780
   aagcaataca gtagtgagaa aaatgaactc tacccaaaga gtgtccccaa gagagagtac     840
   agcgtgaaag aaattctaaa actggactcc aatccctcca aaaggaagga catctaccgt     900
   tccaacattt cacccttcac tttagaaaag gacatggatg gctttcggaa aaatgggagc    960
   cccgacatgc ccttctaccc tcgggtggtt tatcctatcc gggcacctct gccagaagac    1020
   tttttgaaag cgtccctggc ctatgggatg gagagaccca cctacataac tcacagtccc    1080
   cttccgtctt ccacaactcc aagtccccct gcgagcagca gcccggagca gagccttaag 1140
   agctccagcc cccacagcag cccgggaaac acggtgtcac ccctggcgcc aggcctccca 1200
   gaacaccggg actcctactc ctacttgaat gtttcctatg gttccgaggg cctgggctcc 1260
   taccctggct atgcacctgc cccccacctc ccaccagctt tcattccttc ttacaatgct 1320
   cactacccca agttcctgtt gccaccgtac ggcattagtt ccaatggctt gagcaccatg 1380
   aacaacatca atggtatcaa caacttcagc ctcttcccta ggttgtatcc cgtctacagt 1440
   aacctcctta gtggcagcag cctgcctcat cccatgctca atccagcttc cctaccgagt 1500
   tccctgccta ccgatggagc ccggaggctg cttccaccgg agcaccccaa agaggtgctt 1560
   atcccagcac cccacagtgc cttctccctt accggggctg cagccagcat gaaggacgag 1620
   agtagtcccc ccagcggatc tccaacggcg ggaactgcag ccacgtcaga acacgtggta 1680
   caacccaaag ctacctcatc agtgatggcg gcccccagca ctgacggagc catgaatctc 1740
   attaaaaaca aacgaaacat gactggttac aagactcttc cttaccctct gaagaaacag 1800
   aatggcaaga tcaagtatga gtgcaatgtc tgtgccaaga cgttcggtca gctctccaac 1860
   ctgaaggtcc acctgagagt gcacagtgga gaacggcctt tcaagtgcca gacctgcaac 1920
   aagggtttta ctcagctcgc ccacctgcag aaacactact tggtacacac aggagagaag 1980
   ccacatgagt gccaggtctg ccacaagaga tttagcagca caagcaatct caagacccac 2040
   cttcgattgc attctggaga aaaaccttac caatgtaagg tgtgccctgc caagtttacg 2100
   caatttgtgc acctgaagct gcacaagcga ctgcataccc gggagcggcc tcacaagtgt 2160
   gcccagtgtc acaagagcta catccatctc tgcagcctca aggtccacct gaagggcaac 2220
   tgccctgcgg gcccagctgc tgggctgcct ttggaggatc tgacccgaat caatgaagaa 2280
   attgagaggt tcgacatcag cgacaatgca gaccgtcttg aggacatgga ggacagtgtc 2340
   gatgtgacct ccatggtgga gaaggagatt ctagctgtgg tcagaaaaga gaaagaagaa 2400
   accagtctga aagtgtcttt gcaaagaaac atggggaacg gcctcctctc ctcagggtgc 2460
   agcctctatg agtcatcgga cctgtccctc atgaagttgc ctcacagcaa cccactacct 2520
   ctggtgcctg taaaggtcaa acaagaaaca gttgaaccga tggatcctta a 2571
<210> 2
   <211> 856
   <212> PRT
   <213> murine
<400> 2
   Met Arg Glu Ala Tyr Leu Arg Cys Trp Ile Phe Ser Trp Lys Asn Val
   1               5                   10                  15
   Trp Val Arg Pro Cys Gln Arg Leu His Phe Lys Thr Val Leu Leu Gln
               20                  25                  30
   Gly Ser Leu Leu Tyr Thr Ala Leu Asp Ser Tyr Ser Thr Val Gln Ala
           35                  40                  45
   Ala Pro Lys Ser Ser Ser Gly Ser Val Lys Phe Gln Gly Leu Ala Glu
       50                  55                  60
   Thr Gly Ile Met Lys Met Asp Met Glu Asp Ala Asp Met Thr Leu Trp
   65                  70                  75                  80
   Thr Glu Ala Glu Phe Glu Glu Lys Cys Thr Tyr Ile Val Asn Asp His
                   85                  90                  95
   Pro Trp Asp Ser Gly Ala Asp Gly Gly Thr Ser Val Gln Ala Glu Ala
               100                 105                 110
   Ser Leu Pro Arg Asn Leu Leu Phe Lys Tyr Ala Ala Asn Asn Ser Lys
           115                 120                 125
   Glu Val Ile Gly Val Val Ser Lys Glu Tyr Ile Pro Lys Gly Thr Arg
       130                 135                 140
   Phe Gly Pro Leu Ile Gly Glu Val Tyr Thr Asn Asp Thr Val Pro Lys
   145                 150                 155                 160
   Asn Ala Asn Arg Lys Tyr Phe Trp Arg Ile Tyr Ser Arg Glu Glu Phe
                   165                 170                 175
   His His Phe Ile Asp Gly Phe Asn Glu Glu Lys Ser Asn Trp Met Arg
               180                 185                 190
   Tyr Val Asn Pro Ala His Ser Ala Arg Glu Gln Asn Leu Ala Ala Cys
           195                 200                 205
   Gln Asn Gly Met Asn Ile Tyr Phe Tyr Thr Ile Lys Pro Ile Pro Ala
       210                 215                 220
   Asn Gln Glu Leu Leu Val Trp Tyr Cys Arg Asp Phe Ala Glu Arg Leu
   225 230 235 240
   His Tyr Pro Tyr Pro Gly Glu Leu Thr Val Ile Asn Leu Thr Gln Thr
   245 250 255
   Glu Ser Asn Pro Lys Gln Tyr Ser Ser Glu Lys Asn Glu Leu Tyr Pro
   260 265 270
   Lys Ser Val Pro Lys Arg Glu Tyr Ser Val Lys Glu Ile Leu Lys Leu
   275 280 285
   Asp Ser Asn Pro Ser Lys Arg Lys Asp Ile Tyr Arg Ser Asn Ile Ser
   290 295 300
   Pro Phe Thr Leu Glu Lys Asp Met Asp Gly Phe Arg Lys Asn Gly Ser
   305 310 315 320
   Pro Asp Met Pro Phe Tyr Pro Arg Val Val Tyr Pro Ile Arg Ala Pro
   325 330 335
   Leu Pro Glu Asp Phe Leu Lys Ala Ser Leu Ala Tyr Gly Met Glu Arg
   340 345 350
   Pro Thr Tyr Ile Thr His Ser Pro Leu Pro Ser Ser Thr Thr Pro Ser
   355 360 365
   Pro Pro Ala Ser Ser Ser Pro Glu Gln Ser Leu Lys Ser Ser Ser Pro
   370 375 380
   His Ser Ser Pro Gly Asn Thr Val Ser Pro Leu Ala Pro Gly Leu Pro
   385 390 395 400
   Glu His Arg Asp Ser Tyr Ser Tyr Leu Asn Val Ser Tyr Gly Ser Glu
   405 410 415
   Gly Leu Gly Ser Tyr Pro Gly Tyr Ala Pro Ala Pro His Leu Pro Pro
   420 425 430
   Ala Phe Ile Pro Ser Tyr Asn Ala His Tyr Pro Lys Phe Leu Leu Pro
   435 440 445
   Pro Tyr Gly Ile Ser Ser Asn Gly Leu Ser Thr Met Asn Asn Ile Asn
   450 455 460
   Gly Ile Asn Asn Phe Ser Leu Phe Pro Arg Leu Tyr Pro Val Tyr Ser
   465 470 475 480
   Asn Leu Leu Ser Gly Ser Ser Leu Pro His Pro Met Leu Asn Pro Ala
   485 490 495
   Ser Leu Pro Ser Ser Leu Pro Thr Asp Gly Ala Arg Arg Leu Leu Pro
   500 505 510
   Pro Glu His Pro Lys Glu Val Leu Ile Pro Ala Pro His Ser Ala Phe
   515 520 525
   Ser Leu Thr Gly Ala Ala Ala Ser Met Lys Asp Glu Ser Ser Pro Pro
   530 535 540
   Ser Gly Ser Pro Thr Ala Gly Thr Ala Ala Thr Ser Glu His Val Val
   545 550 555 560
   Gln Pro Lys Ala Thr Ser Ser Val Met Ala Ala Pro Ser Thr Asp Gly
   565 570 575
   Ala Met Asn Leu Ile Lys Asn Lys Arg Asn Met Thr Gly Tyr Lys Thr
   580 585 590
   Leu Pro Tyr Pro Leu Lys Lys Gln Asn Gly Lys Ile Lys Tyr Glu Cys
   595 600 605
   Asn Val Cys Ala Lys Thr Phe Gly Gln Leu Ser Asn Leu Lys Val His
   610 615 620
   Leu Arg Val His Ser Gly Glu Arg Pro Phe Lys Cys Gln Thr Cys Asn
   625 630 635 640
   Lys Gly Phe Thr Gln Leu Ala His Leu Gln Lys His Tyr Leu Val His
   645 650 655
   Thr Gly Glu Lys Pro His Glu Cys Gln Val Cys His Lys Arg Phe Ser
   660 665 670
   Ser Thr Ser Asn Leu Lys Thr His Leu Arg Leu His Ser Gly Glu Lys
   675 680 685
   Pro Tyr Gln Cys Lys Val Cys Pro Ala Lys Phe Thr Gln Phe Val His
       690                 695                 700
   Leu Lys Leu His Lys Arg Leu His Thr Arg Glu Arg Pro His Lys Cys
   705                 710                 715                 720
   Ala Gln Cys His Lys Ser Tyr Ile His Leu Cys Ser Leu Lys Val His
                   725                 730                 735
   Leu Lys Gly Asn Cys Pro Ala Gly Pro Ala Ala Gly Leu Pro Leu Glu
               740                 745                 750
   Asp Leu Thr Arg Ile Asn Glu Glu Ile Glu Arg Phe Asp Ile Ser Asp
           755                 760                 765
   Asn Ala Asp Arg Leu Glu Asp Met Glu Asp Ser Val Asp Val Thr Ser
       770                 775                 780
   Met Val Glu Lys Glu Ile Leu Ala Val Val Arg Lys Glu Lys Glu Glu
   785                 790                 795                 800
   Thr Ser Leu Lys Val Ser Leu Gln Arg Asn Met Gly Asn Gly Leu Leu
                   805                 810                 815
   Ser Ser Gly Cys Ser Leu Tyr Glu Ser Ser Asp Leu Ser Leu Met Lys
               820                 825                 830
   Leu Pro His Ser Asn Pro Leu Pro Leu Val Pro Val Lys Val Lys Gln
           835                 840                 845
   Glu Thr Val Glu Pro Met Asp Pro
       850                 855
<210> 3
   <211> 2370
   <212> DNA
   <213> human
<400> 3
   atgaaaatgg acatggagga tgcggatatg actctgtgga cagaggctga gtttgaagag      60
   aagtgtacat acattgtgaa cgaccacccc tgggattctg gtgctgatgg cggtacttcg     120
   gttcaggcgg aggcatcctt accaaggaat ctgcttttca agtatgccac caacagtgaa     180
   gaggttattg gagtgatgag taaagaatac ataccaaagg gcacacgttt tggaccccta 240
   ataggtgaaa tctacaccaa tgacacagtt cctaagaacg ccaacaggaa atatttttgg 300
   aggatctatt ccagagggga gcttcaccac ttcattgacg gctttaatga agagaaaagc 360
   aactggatgc gctatgtgaa tccagcacac tctccccggg agcaaaacct ggctgcgtgt 420
   cagaacggga tgaacatcta cttctacacc attaagccca tccctgccaa ccaggaactt 480
   cttgtgtggt attgtcggga ctttgcagaa aggcttcact acccttatcc cggagagctg 540
   acaatgatga atctcacaca aacacagagc agtctaaagc aaccgagcac tgagaaaaat 600
   gaactctgcc caaagaatgt cccaaagaga gagtacagcg tgaaagaaat cctaaaattg 660
   gactccaacc cctccaaagg aaaggacctc taccgttcta acatttcacc cctcacatca 720
   gaaaaggacc tcgatgactt tagaagacgt gggagccccg aaatgccctt ctaccctcgg 780
   gtcgtttacc ccatccgggc ccctctgcca gaagactttt tgaaagcttc cctggcctac 840
   gggatcgaga gacccacgta catcactcgc tcccccattc catcctccac cactccaagc 900
   ccctctgcaa gaagcagccc cgaccaaagc ctcaagagct ccagccctca cagcagccct 960
   gggaatacgg tgtcccctgt gggccccggc tctcaagagc accgggactc ctacgcttac 1020
   ttgaacgcgt cctacggcac ggaaggtttg ggctcctacc ctggctacgc acccctgccc 1080
   cacctcccgc cagctttcat cccctcgtac aacgctcact accccaagtt cctcttgccc 1140
   ccctacggca tgaattgtaa tggcctgagc gctgtgagca gcatgaatgg catcaacaac 1200
   tttggcctct tcccgaggct gtgccctgtc tacagcaatc tcctcggtgg gggcagcctg 1260
   ccccacccca tgctcaaccc cacttctctc ccgagctcgc tgccctcaga tggagcccgg 1320
   aggttgctcc agccggagca tcccagggag gtgcttgtcc cggcgcccca cagtgccttc 1380
   tcctttaccg gggccgccgc cagcatgaag gacaaggcct gtagccccac aagcgggtct 1440
   cccacggcgg gaacagccgc cacggcagaa catgtggtgc agcccaaagc tacctcagca 1500
   gcgatggcag cccccagcag cgacgaagcc atgaatctca ttaaaaacaa aagaaacatg 1560
   accggctaca agacccttcc ctacccgctg aagaagcaga acggcaagat caagtacgaa 1620
   tgcaacgttt gcgccaagac tttcggccag ctctccaatc tgaaggtcca cctgagagtg 1680
   cacagtggag aacggccttt caaatgtcag acttgcaaca agggctttac tcagctcgcc 1740
   cacctgcaga aacactacct ggtacacacg ggagaaaagc cacatgaatg ccaggtctgc 1800
   cacaagagat ttagcagcac cagcaatctc aagacccacc tgcgactcca ttctggagag 1860
   aaaccatacc aatgcaaggt gtgccctgcc aagttcaccc agtttgtgca cctgaaactg 1920
   cacaagcgtc tgcacacccg ggagcggccc cacaagtgct cccagtgcca caagaactac 1980
   atccatctct gtagcctcaa ggttcacctg aaagggaact gcgctgcggc cccggcgcct 2040
   gggctgccct tggaagatct gacccgaatc aatgaagaaa tcgagaagtt tgacatcagt 2100
   gacaatgctg accggctcga ggacgtggag gatgacatca gtgtgatctc tgtagtggag 2160
   aaggaaattc tggccgtggt cagaaaagag aaagaagaaa ctggcctgaa agtgtctttg 2220
   caaagaaaca tggggaatgg actcctctcc tcagggtgca gcctttatga gtcatcagat 2280
   ctacccctca tgaagttgcc tcccagcaac ccactacctc tggtacctgt aaaggtcaaa 2340
   caagaaacag ttgaaccaat ggatccttaa 2370
<210> 4
   <211> 789
   <212> PRT
   <213> human
<400> 4
   Met Lys Met Asp Met Glu Asp Ala Asp Met Thr Leu Trp Thr Glu Ala
   1 5 10 15
   Glu Phe Glu Glu Lys Cys Thr Tyr Ile Val Asn Asp His Pro Trp Asp
   20 25 30
   Ser Gly Ala Asp Gly Gly Thr Ser Val Gln Ala Glu Ala Ser Leu Pro
   35 40 45
   Arg Asn Leu Leu Phe Lys Tyr Ala Thr Asn Ser Glu Glu Val Ile Gly
   50 55 60
   Val Met Ser Lys Glu Tyr Ile Pro Lys Gly Thr Arg Phe Gly Pro Leu
   65 70 75 80
   Ile Gly Glu Ile Tyr Thr Asn Asp Thr Val Pro Lys Asn Ala Asn Arg
   85 90 95
   Lys Tyr Phe Trp Arg Ile Tyr Ser Arg Gly Glu Leu His His Phe Ile
   100 105 110
   Asp Gly Phe Asn Glu Glu Lys Ser Asn Trp Met Arg Tyr Val Asn Pro
   115 120 125
   Ala His Ser Pro Arg Glu Gln Asn Leu Ala Ala Cys Gln Asn Gly Met
       130                 135                 140
   Asn Ile Tyr Phe Tyr Thr Ile Lys Pro Ile Pro Ala Asn Gln Glu Leu
   145                 150                 155                 160
   Leu Val Trp Tyr Cys Arg Asp Phe Ala Glu Arg Leu His Tyr Pro Tyr
                   165                 170                 175
   Pro Gly Glu Leu Thr Met Met Asn Leu Thr Gln Thr Gln Ser Ser Leu
               180                 185                 190
   Lys Gln Pro Ser Thr Glu Lys Asn Glu Leu Cys Pro Lys Asn Val Pro
           195                 200                 205
   Lys Arg Glu Tyr Ser Val Lys Glu Ile Leu Lys Leu Asp Ser Asn Pro
       210                 215                 220
   Ser Lys Gly Lys Asp Leu Tyr Arg Ser Asn Ile Ser Pro Leu Thr Ser
   225                 230                 235                 240
   Glu Lys Asp Leu Asp Asp Phe Arg Arg Arg Gly Ser Pro Glu Met Pro
                   245                 250                 255
   Phe Tyr Pro Arg Val Val Tyr Pro Ile Arg Ala Pro Leu Pro Glu Asp
               260                 265                 270
   Phe Leu Lys Ala Ser Leu Ala Tyr Gly Ile Glu Arg Pro Thr Tyr Ile
           275                 280                 285
   Thr Arg Ser Pro Ile Pro Ser Ser Thr Thr Pro Ser Pro Ser Ala Arg
       290                 295                 300
   Ser Ser Pro Asp Gln Ser Leu Lys Ser Ser Ser Pro His Ser Ser Pro
   305                 310                 315                 320
   Gly Asn Thr Val Ser Pro Val Gly Pro Gly Ser Gln Glu His Arg Asp
                   325                 330                 335
   Ser Tyr Ala Tyr Leu Asn Ala Ser Tyr Gly Thr Glu Gly Leu Gly Ser
               340                 345                 350
   Tyr Pro Gly Tyr Ala Pro Leu Pro His Leu Pro Pro Ala Phe Ile Pro
   355 360 365
   Ser Tyr Asn Ala His Tyr Pro Lys Phe Leu Leu Pro Pro Tyr Gly Met
   370 375 380
   Asn Cys Asn Gly Leu Ser Ala Val Ser Ser Met Asn Gly Ile Asn Asn
   385 390 395 400
   Phe Gly Leu Phe Pro Arg Leu Cys Pro Val Tyr Ser Asn Leu Leu Gly
   405 410 415
   Gly Gly Ser Leu Pro His Pro Met Leu Asn Pro Thr Ser Leu Pro Ser
   420 425 430
   Ser Leu Pro Ser Asp Gly Ala Arg Arg Leu Leu Gln Pro Glu His Pro
   435 440 445
   Arg Glu Val Leu Val Pro Ala Pro His Ser Ala Phe Ser Phe Thr Gly
   450 455 460
   Ala Ala Ala Ser Met Lys Asp Lys Ala Cys Ser Pro Thr Ser Gly Ser
   465 470 475 480
   Pro Thr Ala Gly Thr Ala Ala Thr Ala Glu His Val Val Gln Pro Lys
   485 490 495
   Ala Thr Ser Ala Ala Met Ala Ala Pro Ser Ser Asp Glu Ala Met Asn
   500 505 510
   Leu Ile Lys Asn Lys Arg Asn Met Thr Gly Tyr Lys Thr Leu Pro Tyr
   515 520 525
   Pro Leu Lys Lys Gln Asn Gly Lys Ile Lys Tyr Glu Cys Asn Val Cys
   530 535 540
   Ala Lys Thr Phe Gly Gln Leu Ser Asn Leu Lys Val His Leu Arg Val
   545 550 555 560
   His Ser Gly Glu Arg Pro Phe Lys Cys Gln Thr Cys Asn Lys Gly Phe
   565 570 575
   Thr Gln Leu Ala His Leu Gln Lys His Tyr Leu Val His Thr Gly Glu
   580 585 590
   Lys Pro His Glu Cys Gln Val Cys His Lys Arg Phe Ser Ser Thr Ser
   595 600 605
   Asn Leu Lys Thr His Leu Arg Leu His Ser Gly Glu Lys Pro Tyr Gln
   610 615 620
   Cys Lys Val Cys Pro Ala Lys Phe Thr Gln Phe Val His Leu Lys Leu
   625 630 635 640
   His Lys Arg Leu His Thr Arg Glu Arg Pro His Lys Cys Ser Gln Cys
   645 650 655
   His Lys Asn Tyr Ile His Leu Cys Ser Leu Lys Val His Leu Lys Gly
   660 665 670
   Asn Cys Ala Ala Ala Pro Ala Pro Gly Leu Pro Leu Glu Asp Leu Thr
   675 680 685
   Arg Ile Asn Glu Glu Ile Glu Lys Phe Asp Ile Ser Asp Asn Ala Asp
   690 695 700
   Arg Leu Glu Asp Val Glu Asp Asp Ile Ser Val Ile Ser Val Val Glu
   705 710 715 720
   Lys Glu Ile Leu Ala Val Val Arg Lys Glu Lys Glu Glu Thr Gly Leu
   725 730 735
   Lys Val Ser Leu Gln Arg Asn Met Gly Asn Gly Leu Leu Ser Ser Gly
   740 745 750
   Cys Ser Leu Tyr Glu Ser Ser Asp Leu Pro Leu Met Lys Leu Pro Pro
   755 760 765
   Ser Asn Pro Leu Pro Leu Val Pro Val Lys Val Lys Gln Glu Thr Val
   770 775 780
   Glu Pro Met Asp Pro
   785
<210> 5
   <211> 20894
   <212> DNA
   <213> murine
<220>
   <221> misc_feature
   <222> (2935)..(3024)
   <223> n is any nucleotide
<220>
   <221> misc_feature
   <222> (3125)..(3125)
   <223> n is any nucleotide
<220>
   <221> misc_feature
   <222> (18390)..(18390)
   <223> n is any nucleotide
<220>
   <221> misc feature
   <222> (18519)..(18519)
   <223> n is any nucleotide
<220>
   <221> misc feature
   <222> (18552)..(18552)
   <223> n is any nucleotide
<400> 5
   gggggaagag tagtcagtcg ctcgctcact cgctcgctcg cacagacact gctgcagtga      60
   cactcggccc tccagtgtcg cggagacgca agagcagcgc gcagcacctg tccgcccgga     120
   gcgagcccgg cccgcggccg tagaaaagga gggaccgccg aggtgcgcgt cagtactgct     180
   cagcccggca gggacgcggg aggatgtgga ctgggtggac atgagagagg cttatctcag     240
   atgttggatc ttctcttgga aaaacgtgtg ggtacgacct tggtaaggaa ccagattctg     300
   tctttaatac gatttgaaac cttttatccc tttttctttc ttcctttttt ttttaacttt     360
   tcttttctcc cccctccccc ttttaaaaaa aaaaaaagaa tgaagcctca gtagaaacca     420
   gcgcttctgt tttagtacgc ggagcactgt caaacattta gaagactttt ttcctccgta     480
   tgaatcatta accctttcag ttctagacat aattgtcaat tcactgaaat ttcagtagtg     540
   gttcttgtcc gcttcgccac tcgctgcctt tacattactg taactatccc gggttgactt     600
   aggttttcac ttgtatttaa catcgtttgt tccacatgga ccttacatgt tggaactaaa     660
   taagaatgag atagtttaag ttgtacccgg gacaaggaca agtaagcatc tttccccttc     720
   tcggagcgtc ctatctaggg acgaattgta aagaccagct ccggagaggg actcccgctg     780
   tactgtgttt acattttcac aagcgcgcgt tctaacatgg ttatccttat tcctaatttt     840
   tatctgcggc gtctatgtgg gaatacgttg cagaggctgt tttatctttc ttgcttttcc 900
   tctttggaaa ggactttttc cgagggcaga taagaggagg atccccaagt cttctgtata 960
   actttagtta cagtaaactg tgccacttca gtgacttctg ggaattcatg cactttcaca 1020
   tttaaataga aagtgctatt tgtggctgag ggctcctaaa ggaattctct tcagggaatt 1080
   ctattgactt tttttttaat atgttttgtt tttaattttt ctatctggct cgagatgccc 1140
   acggattaaa aaaacaaaca aaactgctgg gtgtttccct cttccccaat ttttcttttc 1200
   ctgtggtcca tgggagctcg ggaaggctgg tactcaagga tgctggcagg atgcaacccc 1260
   tctcaggctt gcctgctgga ggatgaaaga gactgaacgc gcgcgcgcgc gcggcagagg 1320
   gaggggacct agttgttttg aaagttgctt cgctagggag ctggtgggaa agttcagttt 1380
   tccccatttg gaaaaggcag actgggttcc gctcctgcac cacacgtgtt ttccattttt 1440
   agcttcattc agacgcaggc agcgcccctg cctcttcctc cccttgtttg tgacacttct 1500
   ctgagacagc ttttccacag ctctgagggt ctggcggcca tgaccccggg cgtcccggga 1560
   cacaggacgc agcagcgccc acaacacatt tctgccttga gtgataaagc caaggattgt 1620
   tcaaaggtag ctgttctttc tctcccgatg aggttaacat atacatatac gctttttttt 1680
   tttttttcag ccaaaggctg cattttaaaa ccgtgcttct tcaaggcagt ctactttaca 1740
   cggctttgga ctcttactca actgtacaag tactccaagc ttttaaagtc ttcagagcac 1800
   cgtgttagtc atagcctcta agagggaggc acaggagcgc cggacaatgg ggattaaaag 1860
   cctttccctt ctcttccagg ctgcccccaa gtctagctcc ggctccgtga agtttcaagg 1920
   actggcagag actgggatca tgaaaatgga catggaggac gctgatatga ctttgtggac 1980
   agaggccgag tttgaagaga agtgtacata catagtgaac gaccacccct gggattccgg 2040
   cgctgacggg ggtacttctg ttcaagccga ggcatcctta ccaaggaacc tgcttttcaa 2100
   gtatgctgcc aacaacagca aagaggtaag ccggctgcct tcttgaagtc tgactggcaa 2160
   ttgggccagc tctcctacta ctatctctga gaaccgtgag aatttatatg cattggcaaa 2220
   taattgatcg ctccagtggc tgttttcctt gctttctctt caaaccaatt cctattcatt 2280
   tcttcctccc ttcagctgtc ctatactaat tagtaaacag ttaaattttt tggcaagttg 2340
   acatgtcttg ggaaagctaa ctggcagcac tggtgggcag catggtaaag ggctcagtgc 2400
   ttcaccccct ggccctcttg gatgacagtt ttaaaggaaa gaaacttcct tagaaaaaga 2460
   agtttttcct ctgctcatga gatggcttta ttcttttaac gagccagctt tattagctgg 2520
   gtttctaaaa ttattctcaa aaccttgacg tgtttatgaa ctgaagagat ggcattaacc 2580
   aggaagaggg tcacgtaaaa gtgtcctctg tcaggatgac ttcactaacc accctttacc 2640
   tgtggcagct ccctggcctg ggccaggccg gcaggtccat gttttatggc ttctgaagtg 2700
   ggtacactct ttgtatcaaa gacacagaac acctgaggag cacctgattt gtgtttatat 2760
   aacaattaga gtcggctgtg aagtgatttg caaaataact ccttgctctg agaatctggc 2820
   tgctgcagtt gctctcctga tggcttaagt tgctgaggct agccctgagg agacttccca 2880
   ccatcaccat tgcccacagt gctgtggttt ctgatccttg ctgtctttgg ggagnnnnnn 2940
   nnnnnnnnnn nnnnnnnnnn nnnnnnnnnn nnnnnnnnnn nnnnnnnnnn nnnnnnnnnn 3000
   nnnnnnnnnn nnnnnnnnnn nnnnaaggag ggattctgtg ttgtaaggat tccagatttg 3060
   ctcctaggac tatgcattat gaaagtgcca ggtcactttc ctccttccct aagcgacaac 3120
   atggngaaag aaactccaga gttggggaaa cactgtctct ttcctgtttg tgtgtctcag 3180
   ttcagggttt ggggggggaa ggagggaggt ggcaaagacc gagagctaaa cttaaagaga 3240
   ggaattctac cttgtgtttc actaaagctc cactctttgt tctatttctg atgggagact 3300
   tatttgtttt atgaaaagca aattttaaat tgtagtttgt gtataaatac caccaaaaaa 3360
   aattgtaacc ccagaggtca cccttaatta tacctctttc tgaaaacaat gttcttctca 3420
   acagaaagcc tctgagtatg taacccgtgg agtcaatgtg gcgtttgttt gggtatttga 3480
   ggtctggctg tggctttaaa aggtgtagtg tgttctcccc ccatcagatt tgcctaaata 3540
   ttggtccctt caaaaagggc cattcatgtc ctgttgtctc tgtagcctgt tgggatagaa 3600
   ataaagactc ctggtttttt gtcatcggct ctgttctcct tcagagtcct tctcagctca 3660
   gccctataaa gtttttcaat ttttgttgtt ctactgaaaa ctatggctga acggagttat 3720
   gctattagat catttagttc aaggttcaga tagtcaactt ccccgaaggc tagagttaca 3780
   gccaggccca gactgggagc aaagttctct gattgctcca ggtgtttcta gatcatgcta 3840
   ctctggtccc tcttggtgtc actgtggctt tatttatctt agcataattc aggcacagct 3900
   tttcactaag cctgtttgat tctgtttctt ttctgctgaa ttccttccca tctctgtatg 3960
   ttaagcaacc agaaagaggc agctcttgtg tttctgacag ggtgcccttc acaacacaca 4020
   gaaagtcagg gttctttgag cctactctat gttttaccga aagaaaagtc aaatcgttgt 4080
   ggtagcacct cttcggtcct caatcatgaa agccaccagg aaacaaaatt taagcatcat 4140
   cctatcctaa gcatacacca aaatgtgggc ctgtttttcc cttctctcca gcgccttctg 4200
   ttttgaactt tgggacaggt ccccttgggt ggtgaaagag aaggacagaa ttgagccatt 4260
   gagggtgtga tagctgcaaa ccagttccag gccagaagca tctaggatgg aggaagtgtt 4320
   tacattacca ttgttcagtt agttcggtga gttccatgag ctgtagaaac agggaaaggc 4380
   ctttctacgg gtagctttta tctgactgat ctggcttgcc catctttaaa tgttttcatt 4440
   aaaatagcaa aagctgttac tttatgtact gtgtgctgac atcctgggga cttttttttt 4500
   aaaaaaaaaa aaaacaagat catttttcat tatgaatttg tgctatgtgt cagcaccttc 4560
   ccacccccac ccccaccccc ttgatttaaa aacaaaacat gcaaactgta tttctgatct 4620
   taacatcaat ttttagctct ggctcagaat cattctgttc ttttattggg gccgtttgat 4680
   cattcttact tcctatttcc agatctaaaa ccaagtacaa gcacactctt caaaactttt 4740
   agaacgttta accgggtggg ttcttcttcc gtgggatttc cccctcgtcc tcctgaaata 4800
   atctctgaat atttgactgc attaaaaaaa aatcagtttt gacattggga gagcagtatc 4860
   caggacaact ttctgctgtc agctgaggtg gcttgtcctc aggcactgtg tggctcctct 4920
   gactctggtc tgaagttgag tggtgacaaa catcaggcag atctagggac atggtgtcca 4980
   ttctgattga agtccattat taaattgagc catctgtatg gaatctcagc acaacatgca 5040
   attccacctc cggacttttt tatttggtag tatgtgccag agtgccacca catctccctt 5100
   cacataaaga taacacgaag caggagacct ttcaaaaaca cctgctaatt cctttgaaaa 5160
   ctggactgaa gtacagactt gatatctcgg tctataccct tatagtaaaa atacaggaaa 5220
   ggaaatctaa ttgttgggcg agatttgggg aggggaaaaa cagcaagtaa ttaaagtaac 5280
   cttaatttga agtgaggaaa ctcaggccac ttttggaact gaatagtgtc tcctttcaaa 5340
   tccccctgct ttctactggg ctcctatatc gaacactctc taaatggctg tttgttttga 5400
   aagcaaacaa acagacctat gctgccctta tcatccgact ttcattctgt actcccactc 5460
   ccactcactc catcctgcac tgtaggaatc tttcttttct aaagttaaaa agaagctctg 5520
   ctttttgtct ctgagctcca gtttgtgtcc tatagccaaa gccactgaca gagacagatg 5580
   tgcccaggtg gcagaggggt cggctccgca agtcaagcaa gccatgaaga gcttccgtgg 5640
   ggctgaagca tgggcagcct aaggaggcct ctttctcttc ctgatgggtg tagggaaggc 5700
   acacagcagc cagtccccag gagtaattct ttaggaccct ggtcagttcc agtgaagttt 5760
   ctttcctcag ttaggtgagc acactcttgg tctttttagc agtgtgcaac tcaaagcaca 5820
   ggtcaagttt ctctgtgccc tctgcttgtt cctagggtag ggcactgtaa acaatgggtt 5880
   aggaagatgg gttgggctaa gacactcatc tggatcagtt tcctgctttc cattaagaac 5940
   aaccctggct cttaccccca tcctaagaca ccccacaatc accataacct tagagccgca 6000
   gtttctatgt cttctttgat taaggaatca gacatggatt agacgtgaag gtgtttattg 6060
   tgtgttgtta gcatgctgaa cttgagatgg ctataggtgg tttatttgat gttctttttt 6120
   ctgagataga atcttgagat gtctgtagtc ggaatgcagg ccacaagctg ctgatcttcc 6180
   tgcatgctat gattagaggc atgcaccaca ccacacctag tgagacaggt gatttcgaag 6240
   ctacagggtt cctctaacct cagaatgctg aattgtgtgt ctctgtttac ctatcagtgt 6300
   gaatttcctc gtgttcttca gctctgtgta tagtaggaat tttaaatatc aaggtcctat 6360
   tgtactcata aaaacaatct aatattgcat tttggtctgg ttatatttcc cagactgacc 6420
   ttggactcaa taatctcttg cctctgcctc agagtggctg ggacttgaag ccaccccaga 6480
   atttctggtt actccttttt ctctcattca catgacttgt ctgttgagtt gcatttgtaa 6540
   ggttaaagat ggcaggcgct ccaaacactg acttaaacag ctacagtgcc aactccatag 6600
   ttgtgcctaa aaggtcctag atagcttgta ccttctttct ttttctcccc cccccccccc 6660
   ccaagattct aaataacttc cttttggcca caggacaatg tgttttgtta gcgttaaaat 6720
   cggtaggtga aaaccaagcg tctcttctgt agagagaatg gcaatctgga agggaagctg 6780
   tgacctcatt gtactgctcc ttgttggtaa tcaagctctg actcccaaga aactgtgcgc 6840
   aggaggctat aatttaaaac aaagttgatg aaccagtcga gtgcctttct taaaattact 6900
   gctttaaagt ggatatgttg aaattatcag ctgctaatta ttggctccct gacaaatggc 6960
   attatttgtt tttcctgctt ggcattttaa tattatggaa taagcattca aatgtaaatg 7020
   tctaataatt tgtgtattat agaagacaat tccatggatt ttacagagtg ggttcaataa 7080
   ttcacccgaa caagcctggg accggaaagt gtagtcaagc attctgtgta aaaatttatc 7140
   tccagagtct ctgctctgag atactcttgt tcccccaaag ctaggctacc agcagacacc 7200
   accaatgagg aggtgtcttg gaagcataga caggtgtgac gagggcagag gaggagacga 7260
   acatctgctt tcttcagacc tttgccacaa attcaaacag gaccaaaaat aacaccttta 7320
   actcaacgaa tctgtttcga attgtgctgg atatagagaa tttctcctct cccctttcct 7380
   gtcttaagaa tgccctgtta acatgtattg aatattaaat tatttaacaa aatggcatct 7440
   gacgataaaa ggacacctgt catgagagtg gcctgtcccc actcttcatg tgtgtttatt 7500
   ccacactaga cccgagtttt aactatggat gaggctggat accagtgtgt atcacagtcc 7560
   aagtctcaga tttccactca aactacctga agagacattg atttgcttct ctgcacctac 7620
   cattcaggaa tataatcata ggagaaccgt caacaaaaaa agccaagtaa agataaatgc 7680
   tgtgcattgc cttgccctgg ctcacctgcc actgtgaaag gggtacagag tcactctggg 7740
   ctcatttgaa ttacttcctg gtttgtgtct gagttgtgtt gttatttttt tttttttttt 7800
   gataccgtaa aaagaaagtt atgctcaagg aagaatgcca tagttaatgt gtttcttcag 7860
   caaaccccac agagtcagag tgtgtgaggc cttcagtttg aaaaggtcat ttcctcagat 7920
   cttagaagcc acatctttta gaacccacag tctcattaca tattccattt caaagaggga 7980
   cttctgacca ccattgagta gatgttggag aagaacaaag ttacttgaaa gatctttcta 8040
   gtaaagagcc cttatgagct ataagccaaa ggggatggag atgtcaactg gaattttaaa 8100
   aaaacaacaa aaaccttaat gtgtttggtt tctggttccc tcggcctcta tggaacagct 8160
   aaagagcatt attttggttt ctgaggttaa aactcttgca ttttcctcaa gcatggtgtt 8220
   tatggtttga gggagaagac tggaactagg gaactgaggg cagtaatctt gaccccctct 8280
   ctctctctct ctctctctct ctctctctct ctctctctct gccttctgat tctcttagaa 8340
   cggaacatct tctggtcttc caacatgaga ggccatagag ccgagtcagt cactagaaag 8400
   cagtccgtcc ccgggactat ttcagtttca gcacttggat gactgtaaac gatgactgtc 8460
   acgaagctta cggctaactg tggaggcatg gatggtgccg tgctgggtta tcaattgaca 8520
   gttcaattca ctcagctagc tagcttgcca tctcagagtc tgactaatga cctcatattt 8580
   tttttttccc ctttaggtta ttggcgtggt aagtaaggag tacataccga agggaacacg 8640
   ctttggaccc ctcatcggtg aagtctacac taatgacaca gttcccaaga atgccaacag 8700
   gaagtatttt tggcgggtaa gtaagaaaac ctttttttta agacttttca ctataggggg 8760
   taaatggagc ttaaagaaca ggctcagttc cctttcaaca cagcagggct cacccaggga 8820
   aacactggaa ttctgagcaa gttccctaga actggttaaa cgctctgcct agaatattag 8880
   ctggaggtgg ttagatgtgg actacctgtg aatctcaatt tttgcaactc attgcataca 8940
   ggtctgggag ggaaggaaac agtttgccgc ttgcttcaag ggcaggctca tttgcatttc 9000
   tcttcgagga agtagtaatg agtcacggag acttacattt caccctttct tgatttcttg 9060
   ctgagttaac ttcatttgaa tggaagagtt atcctgagtg aacttgatgt cgaagacaaa 9120
   tgtcactaag agaggtagat ggtgggttag tggaactggg aaggatcatg gagctagttt 9180
   tatttaaatc catctgcacc ataaaacggt tacatttgac agtatcatag ttgttaagca 9240
   tgaggaaaat cctcgtgtgc ctatgagatt gtagactcag gtagaataac tattctaaag 9300
   gtctggccta tgctatctcc tttggagtgt cagggttagc gaggattctg aggtgacctg 9360
   ggagatggga ttcatgggta aaaattgttc tctgagatgt ctctggcatg ttcagttttc 9420
   ctcagtgtag aaatgaagag ctatttatac aaatttagtg agctgttttc ctcacacaga 9480
   catgaaatat acaccaccca gagaaatggt aatatccaca actggattac atgagaaaaa 9540
   gactttggtt aaaaaattac ttattcctga gagagtctgt cttcagctag gaagtctttg 9600
   tttccagaaa cgtactactt ctacaactgc atctgtagtc ttgttaagta tttgttctca 9660
   atttttattt atttaatact tagttggtgt taaattatat tgctccaaga tcgcagtctg 9720
   agattatgag cggtttcgcc cttattgctg ttctcagatg ggagccacca gtggtagatt 9780
   aatcttggcc tcagctggta tgaatgaaga caatccgaca ctgtcgtttt gaaaacggtc 9840
   aagaagcacc aaaggctctc ccattactgt ccccactgtc ccatttaaag atttacaaaa 9900
   agaattagac taaataacta gaaggcttcc cttgggagga attatttaag tcagttgtcc 9960
   acatgcaagg aagacaggaa tgaatctttt cacaggttgg aagatcctga tttgtcaagc 10020
   aggaatagga acatctctgt gttgtgagga atgaaaggtt gtcatgcaaa ttacacagtc 10080
   agagatgctc aggttgagaa agcagtgaca tttcttgtaa ctgtagtatg aatcagcttg 10140
   tgtttagtct tcttgatact ggatggaaag gctggtataa gtgtgccttt tacaaaagca 10200
   tgatgatagt ttcttggggt gcgtgtgact ttcacgacat ccaaggtcct tttttttaaa 10260
   acaaggatac agtaaaccgt agccatgaaa ggcctactgg gatcggcaca ccctctgcta 10320
   gctgtttcca ccctggtgta agggcgatgg aaccccttgt tcctggaagt ttgcgcgtca 10380
   gagtaaacaa acttgaaaac ccctcttgat agcagaatcc agtcggtctt gttacatttt 10440
   ctcttaacaa gatacaccgc ggaagctctc gcaggctgct ttgatgaagc cacacgcacc 10500
   ccccacacac acacacacac acacatacaa ttcacaggaa gtctctctta aaagaaactg 10560
   attctgctgt ttactgcctg tgttaaaggg acagagttcc ttttttattt ctgataacgt 10620
   tagagggaaa tacagaaacg ttcacacagc ctgtgtgtga ctaagaatac agcaaatagc 10680
   cctgtagagc aaactccctg aggtgagcat ggaagcgccg tacctcttgg agactgtctt 10740
   gtgtgtaaag agggctctct gctctgaagg aaggcggtgg ttcttcaagg agaggagctt 10800
   ttctggagag gatgaggagt gagtctttga ccccttggtt tcagtaggag tgtatttctc 10860
   ccctgctctt aactatgcct ttaaccaagc actctgagta cagctgtgag tcagaggtag 10920
   cattgctgaa gaagaaccat atattttctt tctcttcttt ttccaaaagg ccaaactcgt 10980
   ggctcttgtg tgtgtagacc gtgtatgcca gcctcctcac agatatgggc aaatctctct 11040
   actccttttc aagagagagg cacagggtgg ccgcctgtgt ttaccaagag gaaaagttac 11100
   ttctcgatag gctgtcaaac tttggcctcc gtgccagtgc ctcactctgt tatggcaggt 11160
   gaagttcacc tttgccccac ccagtgtttc cacaaaaagg cagggttcca agtattcatc 11220
   tgaacaagtg ttactgtggg actcagggtt gggggtggag gatgtttgca gagcctaagc 11280
   cccgggcggg cggaggtgta ggaaacacaa gtacagaggc catagaaaaa aggtgagact 11340
   cagtttgacg cagtcctctc ggctgctgtg cccagtgact caaagcacta gaagtcagca 11400
   gagttggaac tctgggctga gcagagtcgc ctgatcgata ttcgctactg tagcaagagt 11460
   acctctttat ggtagtttca cccactctcg gctgttgtta attggaatat tattattatt 11520
   attattatta ttttgctatc cactgccctc cccaacatga gaagaccata aaattgaaat 11580
   ggaaaggtaa ctagcacaat gtgccctgtt tcctccccca tttctgctga ttcagcgtga 11640
   gtcccaccgg atcagcaatg aggcctggag tcatgggtac agcgttggtt gctcgcctgt 11700
   gttccttctg agccattcag ggaagcttcc cggtcgcttt gggctggccg gctgtctttc 11760
   acactgcatc tatcctctcc ttttgaacag atctattcca gagaggagtt ccaccacttc 11820
   attgatggct ttaatgagga gaaaagcaac tggatgcgct acgtgaatcc agctcactct 11880
   gcccgggagc aaaacctggc tgcctgtcag aacgggatga acatctactt ctacactatt 11940
   aagcctatcc ctgccaacca ggaacttctt gtgtggtatt gtcgggactt tgcggagagg 12000
   ctccactacc cttatcctgg agagctcaca gtgataaatc tcagtaagtg gattccagac 12060
   caaaaaaaaa aaaaaaaaat taaaaatgct agtaatgtca gttctgcccc tgtgagctaa 12120
   taacatgttg tctaattata cggcttcgtc atgtgttgga ctaagtaggt ggctttagct 12180
   aagacgagga agaggaaaaa cattctttaa tgtccctact tcttattata aaacataatc 12240
   atcaaagata tacatattac atatattgta taaaataacc agtacagaat gttgttttcg 12300
   gaaagttgca ggaagaagta tatttccgat tctaatttat gcaagcggct gtaggcacaa 12360
   tcccaatggg tatggacctg tggaacaggc cagctgcagt cccttcctgc tgtgctgggt 12420
   cagagctttg agttttcact gaaatcttgt gaagatacgt gtgcctgtaa agccatgatc 12480
   taatgtggaa agctgttttc tagaaaaaaa aaaaaaaact gtcataattg ttcaagtagt 12540
   ctaagtgaat aaccctaaga gatgtcatat ctgagcttcc ttccttatgg taaaggggac 12600
   tgatctcatc tttcaatcag gcttacggta accgcctatc tctttatctt gacaaattct 12660
   tgcttccttg ggtttataag cttttacttt ttcttttctt ctttttaaat tttggctaaa 12720
   gttactgtca tgccctctag caagctttcc ctcttcagtc agtctcaggt agctttagga 12780
   acaatttaag aataaaaaaa aaaaaattct aacttctatt ttaactcagg gtgtgtgttc 12840
   tgggttatac tgccgaatct tacagcactt tttcaaatga ccatcttccc atgaaagcta 12900
   aatgttgaag gtttaaaagt tttcatttta atagtctctg aaatttgagt aaacatttcc 12960
   agaaatctat agagagttcc aagctagact ttacccagtt tctactcttc agtctcattt 13020
   gctttccctg gagactaaat gtagttcata ttttaccact gaagcactag aaatattaat 13080
   tttagtattt taacttttaa gaccaaggac agtgtctcgc tagccatgtt cattctacaa 13140
   tgcttgtgct ctcaggaatt ttcagttttc tgaaaatctt agcttcagta cctttcctgt 13200
   aggctcacaa tatagtgttt gtgcccgggc cctcagctca gcaacgtaca cccttgagct 13260
   aagcatggtg ggtaggtacc tgcccaatag cagcaagccc tctcctcgtg tttgggtttg 13320
   ctttgggtgt ttgtttgttt gtttgtttgt ttatggacaa ggtctcgtgc tgcttaggtt 13380
   cagttcagac ttgatgtagt cagggaaagc cttgagccac tgtcacctcc aaaatgctag 13440
   gatcacaggc ctgtgccctc cccccacccc tgcctcccac catcccccca atgcctgttc 13500
   cgagtgttta ctctttgtgt ccagaagtaa gtttcattat gctatgaaat gacagctttg 13560
   ctcttcagac accccccccc ttttgactga tgcaggagtc ttctgagggt cacaggaaca 13620
   cctcctttgt ctgacattcc taggacagaa agagagttaa ccattcagct gccgtgcaag 13680
   gctcttgctc ctgattgtga aacctgttgg cccaggtgtg gccactgatg actgacactc 13740
   tgatcaggaa aatttccagc atttcatcag gcctaatagg cagatcgagt gtccaagatg 13800
   ggctgtgcta gatttccagg cttaaagcac aatagaggtc tgtccagaat ctccgtaagg 13860
   acttccatca tggggtgcag gggatggaaa cctaatgaaa gaatgtaagt ccccagaaat 13920
   cacaaactga caggaaagag aagggagaga ggaatgtgga aaagaactgt taaatttagc 13980
   tcctggccct cccaaccttt ttggtaattt ttttttctat ctatctaact aacccatcta 14040
   gaaatcagtt gaccaaatta tagacttctg aatgttaatc tgctttctcg gtttcagttg 14100
   aaaacagact ttgttttgcc tactgcagaa cttctaggtt ctttcttgta gtcttagggg 14160
   tgcttattat agatcgaaaa tgtgagtcgg cataattaag ccattcagaa ccttccaaag 14220
   cagctcactc ttgaaatgac tctgtccgcc tacagccatt taagatttaa gaacaaaaac 14280
   agatcttgat tttctttttc atgttagctc aagctgctaa gtgggagagt tagaaatgat 14340
   atcagctcct gtgattagtc agctgctgaa ggatgagttt ttaaaaatgt accttcatat 14400
   acagtctata atttccagct gtaaagtatt ttagagactg acattttgct gcggatattc 14460
   cttcaggata agttctcagc ctggttgttt gtttgtttgt ttgtttgttt tctgaagaca 14520
   gagccaccaa acgctaaatt atgcatgtca cggagaaaat gaaaagctct gacttcattg 14580
   tttcttggtt cagtcattag cttcacagta gttcagtaac taaagtgctt agcaagaaga 14640
   gagccgatta aacctgtgct ctacactgga agaaagccca attctttata cttaacagct 14700
   ttcatttgtt aagtttccac tgtgggacta ctacaaaaac attatgagtc tttgatgtga 14760
   tttgccacat taaaaaaatg gcacagacag gggtgtggtg gcacacaccc tcaatcccag 14820
   tgatcgagag gcagacgctg gtggatctct ttattcttgg ccagtccaat ctatataaca 14880
   agttccagac ctactgagac tgcacaataa gtcttccccc agaaccccca tcaaaaaaag 14940
   agcagagtta ggaaggccgt acacaagcag gcttgcacac tctcacgcgc tcgctctcgc 15000
   gcgcccacac acacacatac ataggcatac gcacatgcgc acacaacttc ttttctctta 15060
   acctgagggt gcttctaaaa tcattatctt tttgtcttac ctccagtaaa tcccttcctg 15120
   tgactgtggg atgcctcgcc tgtcagcctt ccagcttaac ctgtctctct cttcctttac 15180
   cattttagct ttaaaaaaca aaagtgacaa tttgaacttc ctgcctgctg ggcctcaccc 15240
   gaaggactga tattgggctg ataaggggat atttattttg gttgagaggc ttgagaaatt 15300
   gctctccccc agaaagcttt ctgtcactga ccccatcaac atctcccctg atagtgttgt 15360
   ccacggtgtt tattctgggg ctctggctta cccaggagta actgataaca gccagcagga 15420
   gataacgtcc tgtaaagcgc tttccgactg gcatcaaatc cctccagcct gtcagcctgg 15480
   agaatggatc tgaaagcttt agttctgggc ttccacagag ttcatcttca gacctatcag 15540
   gtagcaagct tggagttcct tctcagttaa gcccaaaagg gctgttttat aagagcacaa 15600
   aggatacttc tttacattgt cttaagtgtc attccaaacc tgccagatct tggaggtcaa 15660
   gaatcttgtt tctactccga gcatgtgcac cccccaacta atgatgctct cagcatcctg 15720
   gggagaagtg cctgtttgaa tgagcatccc agaaacacaa ctcagcctgt gcatcggatg 15780
   tgttttatct ttggcccagg aaagctgagc tgaggctttt cctgcgaaat agggctacat 15840
   aactatggac agtttaggac agtattctcc ttgtctgagc ttgaccaggg catatatgct 15900
   gtctctagga gtaaatgttt gtctcttagc tgcctctgtc ttctttggtg ctgtaagtaa 15960
   ttgaactagc ttgggaagta cctgtcgtgg tttggcagag gtgactgtca cacctcacga 16020
   ttccaggaga cagcccagat ggtagtctgg ttagaccaga accttggtga aatgctcgca 16080
   ctgccgagca atggctagaa ggggcagccg ccatgccctt ctagttgata caggcaattc 16140
   gaacagggct catgaagttc ctatgtaaag agaatcgagt tggaaattga tgacagttca 16200
   ttacttaaaa ctagtcttaa tctttcatct aagtttgcac agcactctga tttcctctag 16260
   gtaaactgcg aatgacttat taacccgtga caacccccca ccctgtattt tttccacccc 16320
   atcttagtga acgctctgcc cgttccagtt tgaacagcac ttttctatcc tagttctcac 16380
   taatggaaag gagatcatcc aaggggcact gggctctatg gaggctggca ttgtcccagg 16440
   gtttgatgtt attcccgatc cccccccccc gcccccggga ggaagtggag cagtgtttct 16500
   gagtgggtgg cccagagccc tccctccgga gtgagaggcg ttaggggcca ggtgtctagc 16560
   ctttgtattt gctgctgctc agggtttctc aagaagagag aatggctttc tgatttcact 16620
   tcagttctcc acagccctgt gagtaaccgc cctttcttct tcattttagc acaaacggaa 16680
   agcaacccaa agcaatacag tagtgagaaa aatgaactct acccaaagag tgtccccaag 16740
   agagagtaca gcgtgaaaga aattctaaaa ctggactcca atccctccaa aaggaaggac 16800
   atctaccgtt ccaacatttc acccttcact ttagaaaagg acatggatgg ctttcggaaa 16860
   aatgggagcc ccgacatgcc cttctaccct cgggtggttt atcctatccg ggcacctctg 16920
   ccagaagact ttttgaaagc gtccctggcc tatgggatgg agagacccac ctacataact 16980
   cacagtcccc ttccgtcttc cacaactcca agtccccctg cgagcagcag cccggagcag 17040
   agccttaaga gctccagccc ccacagcagc ccgggaaaca cggtgtcacc cctggcgcca 17100
   ggcctcccag aacaccggga ctcctactcc tacttgaatg tttcctatgg ttccgagggc 17160
   ctgggctcct accctggcta tgcacctgcc ccccacctcc caccagcttt cattccttct 17220
   tacaatgctc actaccccaa gttcctgttg ccaccgtacg gcattagttc caatggcttg 17280
   agcaccatga acaacatcaa tggtatcaac aacttcagcc tcttccctag gttgtatccc 17340
   gtctacagta acctccttag tggcagcagc ctgcctcatc ccatgctcaa tccagcttcc 17400
   ctaccgagtt ccctgcctac cgatggagcc cggaggctgc ttccaccgga gcaccccaaa 17460
   gaggtgctta tcccagcacc ccacagtgcc ttctccctta ccggggctgc agccagcatg 17520
   aaggacgaga gtagtccccc cagcggatct ccaacggcgg gaactgcagc cacgtcagaa 17580
   cacgtggtac aacccaaagc tacctcatca gtgatggcgg cccccagcac tgacggagcc 17640
   atgaatctca ttaaaaacaa acgaaacatg actggttaca agactcttcc ttaccctctg 17700
   aagaaacaga atggcaagat caagtatgag tgcaatgtct gtgccaagac gttcggtcag 17760
   ctctccaacc tgaaggtagg tctccagacc cccgcgggtt tctgcccaca gacccgtgct 17820
   ggtttgccct tggctgccag gcagtgcatt gttgagtgca cttgagccat aggagaccca 17880
   gcttgagcct gaactggggt ctgctgaata ctgaaaatac agggtttatc tcagtgtctt 17940
   tcctaagagg cttgcatctg cattgtacat acctggctct gggaaaccta gcaggcaggc 18000
   aggcctcatt gcaaccccag agttcacccc tgtggtttct tcccaggtcc acctgagagt 18060
   gcacagtgga gaacgacctt tcaagtgcca gacctgcaac aagggtttta ctcagctcgc 18120
   ccacctgcag aaacactact tggtacacac aggagagaag ccacatgagt gccaggtggg 18180
   cagtattctc tgggtagaac tcttgacctc tgtggaaaag tagctgtaga attgtcttcc 18240
   tgtgttgttt caacaataca aaaaatatgg tcttgtacta ggctgctggc cctgcacagc 18300
   tcctgggtac tctgtgacta ctcacaggct atactgagga tggctgggtg gatgtcagtc 18360
   aagtttcagt gggtggggac atgtcctcan ataaacagta cctcagagta ctgtgtgccc 18420
   agcttctccc cccccccccc ccccccccca ccgcgcatga gcattgttaa gaggcttctg 18480
   gtctcccgag gtttctggct attggcctgc ctttccccnt ccagctgcaa acaattaatc 18540
   ttggtcttcc cntgtgccct ttctctgtct tcccttgccc tcacacttta ggtctgccac 18600
   aagagattta gcagcacaag caatctcaag acccaccttc gattgcattc tggagaaaaa 18660
   ccttaccaat gtaaggtgtg ccctgccaag tttacgcaat ttgtgcacct gaagctgcac 18720
   aagcgactgc atacccggga gcggcctcac aagtgtgccc agtgtcacaa gagctacatc 18780
   catctctgca gcctcaaggt ccacctgaag ggcaactgcc ctgcgggccc agctgctggg 18840
   ctgcctttgg aggatctgac ccgaatcaat gaagaaattg agaggttcga catcagcgac 18900
   aatgcagacc gtcttgagga catggaggac agtgtcgatg tgacctccat ggtggagaag 18960
   gagattctag ctgtggtcag aaaagagaaa gaagaaacca gtctgaaagt gtctttgcaa 19020
   agaaacatgg ggaacggcct cctctcctca gggtgcagcc tctatgagtc atcggacctg 19080
   tccctcatga agttgcctca cagcaaccca ctacctctgg tgcctgtaaa ggtcaaacaa 19140
   gaaacagttg aaccgatgga tccttaagat tttcagaaaa taagtgtttc gtgttgcttc 19200
   ttagggtatg gcttggtgaa tcagggtgcc tttagcaaat tgcttgtaca tgactccaga 19260
   tctgcaaagc tccgctggca ccgggtgctt ccctgcacct ctctggaatt aaagaaggac 19320
   tccaatgtta ccaaaatctc agggcataaa tgaggcaaag actcactata tatacatata 19380
   tacatatata catattataa atatatatat acttatttac agccatgtct atatatttga 19440
   acctgtgtat tttgaatatt tgtgtggata tgtttgcata gcgccttcct attactaaaa 19500
   ctattgccta gccataatta ttttttcaat gataattctt cataatttat tatacagttt 19560
   atctttcaaa aagcaataat taaagaagtt tacaatgact ggaaagattc tttgtaattt 19620
   gagtataaat gttgtatctt tgtcctgtgg ccattctttg tagataattt ctgcacatct 19680
   gtttaaatgc ctgagactta gaagatagct ctgtgatttc aggcaacctt tctctatgat 19740
   aatgctttaa aatgaggttt tgatattgcc aaagtcatgt ggttggtgtg ttaactcaga 19800
   agatcacaca atctgagtga cattctctaa gttggggata catgtgcaga attgctcagc 19860
   aataatttga ggggaaggaa gaagaaaaat attttatgtt tcagaatgat ggtttggttt 19920
   tcctcctcct agtcacaatt ttaccaaaca gtgacaggaa ggctttgcca acctgtctcc 19980
   caatgtcaca tgaccattct gagtggccat atgactttgg catccctggg tgttatctga 20040
   aaatgtgaag aagataaaaa agccgtgttc agaagatctg tcgtaaagca cagatgttgt 20100
   gtgtgtgtgt gtgtgggttg gggggtttga gtctggctgt cattttgctg ttggcttgtt 20160
   tttgtttttt taatatcaaa attgcacaaa gctggtgccc taccaagaag gatttgatat 20220
   agaaaggctc aggccacact taaaatacaa gcaagcaaag agaacagaaa aaaataaaag 20280
   taaaaacggg tattcttatc atcttaggtt aagcgggtaa tgaacactcc tgtccccaac 20340
   gcatcaactg tattgtatct gtaaaactca gcttttctca gtattttgtt tttgcattgt 20400
   ataattaact taattaaaga tgaaagggca ttgcaaaagt gttcaacaat tacctcattg 20460
   agtgtatcca gtaggagtgc aggaattaat gtcgtatctc atgagttgct acccagctga 20520
   gcgtgtgtgc ttccaaatgg taggctgggt ggttcggtcc tgtattctcc taagcccaaa 20580
   ggttacctgt tggtgttcaa ggtgtaataa agaatgctgt atatttatga acctatttat 20640
   accagtatac catgtgtata tatgatatat ttataaccac ttaaattgtg agccaagcca 20700
   tgtaaaagaa cctatttttc ctaagagcaa aaagaatctc tctgaagttt tgcttaaaac 20760
   tccatgacct cgctatgact ttggtgcttg ggcaccaccc tgcctactac cagagagcag 20820
   agcacctcag tgcagaggtg agggtgtgta gcatcttggg atggatagaa acaccacacc 20880
   atccagtcgc attt 20894
<210> 6
   <211> 23615
   <212> DNA
   <213> human
<400> 6
   gggaagccag acggttaaca cagacaaagt gctgccgtga cactcggccc tccagtgttg 60
   cggagaggca agagcagcga ccgcggcacc tgtccgcccg gagctgggac gcgggcgccc 120
   gggcggccgg acgaagcgag gagggaccgc cgaggtgcgc gtctgtgcgg ctcagcctgg 180
   cgggggacgc ggggagaatg tggactgggt agagatgaac gagacttttc tcagatgttg 240
   gatatttgct tggaaaaacg tgtgggtacg accttggtaa ggaacttgaa tttttttttt 300
   ttaattctga aattgatctg aaaactttat tttcttttcc tttattgtta ttattattaa 360
   tttttttggg ctaatgtcgc agtagaaaca tgcttctgct ttagtgcact tagtgctgtc 420
   aaacatttgt gagactttcc ttatgaatca ttaacccttt cagttctaga cataattgcc 480
   aattcattga aatttcagta gtggttccag ctcacactcg tcaaactatt ccgggttggc 540
   tgaagttttc tattttattt tatttttaac atgtgtttgg cgtcatgact ctacatgttg 600
   gaactaaata aaaataagca ggtttgctta aatcataact gagggaaaaa caactttgca 660
   tccaactttt ttttttttaa gagcatccta tttagagaag tggaagaatg taaaaacctc 720
   cttgaaggac ttccacagaa tgttatgttt acatttgaac aaacacacat tcttacatgg 780
   aaatgatacc catattcctc atttttatca aacatgtcta tatgagaaaa cccttacaga 840
   agttgtttac ctttttttgc ctttggaaaa cagttttttt ctgagtgtga gggaggattt 900
   tgggggaata tcctcatcaa tgtacaagtg gaagcagagc ttgtcctcca agtcttctaa 960
   atttgttata actttagtta cagtaaactg tagtacatca gtgacttctg ggaattcata 1020
   cactttcaga tttaaatgga aagtgctatt tgtagctgag gactcctaaa ggaattctct 1080
   ccagggaatt ttattaaacg gttttatgtt ttgtttttgc cttttcaatt tggtatgaga 1140
   tgcttgcaag tcagaagaca ctgcaggctg ttttcccctt cacccatttt tcctcctctt 1200
   ttcctgtggt ccaagtgatt tctaagaggc cgtagctcag tgatgctggc aggatgcaac 1260
   ccttttcagt cttccatgtg agaggatgaa agagagctcg cagcagaggg aggaggcagt 1320
   tgttttgaaa gttgttttgc gttgggagct ggtgggaaag ttcggtcttc cccatttgga 1380
   aaaggcaggc tgggttccgc tcctgcacca cacgcgcttt ccatttttag cttcattcag 1440
   aggcagacag agctccttcc tcttcctctc cttgtttgtg acacttttct gaggcagctt 1500
   ttccacagtg ccgagggtct ggcggccatg accccaggca ttctgggaca ctggactgtg 1560
   tgcccagaac atttttctgc catgagaggt aaagccaggg attgttcaga ggtgattctt 1620
   tttttttttt tccttttccc cacagtgagg ttgccacatt cttttttttt tttttttttt 1680
   taactaagag tagcatttaa aaaccttgct tcttttcaag gcagtttact ttatacggct 1740
   tcttggctct ttctcaactg taccaagcac tctgcatctg cttttaaagt cttcagacta 1800
   ctgtattagt catagcctct cagaaggagc cacaggaacg gcgggacaat ggggattaaa 1860
   ggcctttcct ttctcttcca ggctgccccc aagtgtaact ccagcactgt gaggtttcag 1920
   ggattggcag aggggaccaa ggggaccatg aaaatggaca tggaggatgc ggatatgact 1980
   ctgtggacag aggctgagtt tgaagagaag tgtacataca ttgtgaacga ccacccctgg 2040
   gattctggtg ctgatggcgg tacttcggtt caggcggagg catccttacc aaggaatctg 2100
   cttttcaagt atgccaccaa cagtgaagag gtaagcctct ggtttattga caagaagatt 2160
   ggggacctgg tgccaaatct ccctacttgc ccttgaggcc ttgtatatct ctgaaaacct 2220
   ctgagaatct gtaagtatca gtaaataatt gattgctcta ttcaattctt gcattgcttt 2280
   ctctttccct aaaccatttc cttctcattt cttccagcct tcaactgttc ctcactaatt 2340
   agtaaacagt taaatatttt ggcaaattgg catgtcttag aaaagcaact tgcagcatag 2400
   ggtgggtgaa attgtcagtg aacttcaaga aagctctggg cccactggcc ctagtgtccc 2460
   tgttgtacaa tatctcttaa gggagaaaac tttttcttgg aaaaaaaagt tttaatattt 2520
   cttttgtctg tctttggtaa ttagttggct ttactctttt taacaagcca gctttattag 2580
   ctgggtttct aaaactatcc tcaaaacttt gacgtgttta tgaagtgaag tgatggtata 2640
   agccgagaaa gggggtcatg taaaagtgtc ctctgtctgg atgacttcag agctaaccac 2700
   tgtttatctg cagcagctcc tttgctgggg ctgggctggc aggccaattc cttactggcc 2760
   tctgaagtgg gtacactctt tgttgtttca aaggggatga aaacccaaac ttggaatgag 2820
   caactgattt gtgtttacct ttaatataaa gattatagct aggcgtgatg tttagctgtg 2880
   aaataacttg cagaaccacc tcttgttttc agaatttggc ttactgtagt tgcacttact 2940
   agtctactac tgggttgagt tgaatgaaag gacccctcag cagacttccc ataccaccat 3000
   tagcacagag cactgtcaat tctattactt ccttcagaaa gtagggaaag gggatggttg 3060
   gccgtagaaa tccagaattt gctactggga ctattcctta cagccgtgtc aaatcacttt 3120
   cccccttccc taaggaacag tatttgaagg aatccgggag actaggaaat gctttgtctc 3180
   tttcctgttt gtgtgtctga gtccagtgtt tttggtgagg gggagacaca gctgtctaaa 3240
   attaaataga acaacgatgc tcagtctttc ttttttcccc cactgctgcg tgaatggagg 3300
   cagattttat aaaaaataaa atttaaattc tagttgtagt tgaatgttac caaaaaattt 3360
   aaccccagag gtcagcctta attatacctc ttcctgaaag taatgctctt cttatagaaa 3420
   gtctctcaat atgtggccct tggaatgaat gtaggatttg tttgggtata tgaggtttga 3480
   ctatggcttt aaaaagtgta gtgtgttttt tccccatatc aagtttgacc aaatattcgt 3540
   ctgttcccaa gcttcgtttg tgctgtgttg ccctgtccag tcctttggga ctgaagtaaa 3600
   aatctccatt ctgtgggtca tctttgcctt ttaattctat aatgttctta tcgtcagttc 3660
   agctcttagc ttaagttttt caatttccgt cattctcctt taaaatcata gctaagtcaa 3720
   gtaatgctct tagatcattt agttcaaggt tcagaaagac agtcacttac ccaaaggcat 3780
   ataggtaaag ccagaccggg gacttgaagc aaagcttttc tgacttctcc aatgagtgct 3840
   tctcgattat gctacactgg cccctcttgg tgtcactgtg gttttagtta tcatcttaat 3900
   atatttcaga ccaatcctta agctattttg tgtttttttc cctctgaatg tataacttcc 3960
   tatttcaata tattaagcaa ccaaaaaaca gacaatgcct gtgttcctga agaaggtcct 4020
   tcgaaactaa cctaagaggt gagggttctc ttatcctatt tccctatctt gcatttaact 4080
   gaagcgcttg aatagaggat agtatgtctt taaaccctaa tcataaagtc aagcaaggag 4140
   taaacttgaa tgtaatactg tcttaagctt actctggaat ttgtacctgt ttttctgttt 4200
   ctctcctttg tttttgaact ttgggaaagg acagcttcct ttagaaggtg aaagaggatg 4260
   gtggagttga gccattaagg gtaggatagc tgcaaactag ttccgggaca gcaacattaa 4320
   ggattgatga aatgtttaca ttatctttat tcagttagtt ctttaagttc catgagccat 4380
   tgaaatagga agagtctttc tacatgtact ttttatctga cttattaatc ttgcccttct 4440
   ttaaatgttt tcgtttaaat agcaaaagat gttacaatat gtactattgt gctgacatac 4500
   cggggaattt tttttttaca agaccatttt tcattatgaa tttgtaccat gtgtcagcac 4560
   cttttttttt tttttggtta aaacaaaaca tacaaatcat atttctgatc ttgaccctca 4620
   tttttagatt gggtttagaa tcattctgca cctttaatga aaaactgttc ttacttccta 4680
   tttccagaac tgaaatcaaa tatagaccta ctattcaaaa ctttgaaaat gttttaaact 4740
   gatgggttat tcttttatgg gggttttccc cttgtcctcc tgaaataatc tcttatgact 4800
   acttgactac agaaaaatca gttcaagcat ttatatttta gaagcgttac cctgtagagt 4860
   ttctgccgtc ggttgagact gcttgcactc aagaggcact gtggctctgt gagtctcacc 4920
   tccctgtatc gtggctgata aacatcagac agattgtgag gactcctttg gtgtccattc 4980
   tgatcgaaat ccattattaa ctcaagccat ctgtaaaaag tatctcacca taacatgcaa 5040
   ttctctcttc atgttgcctt ttatttgaga aatgatgtac caaggcatca ccatgtctcc 5100
   tttaatagaa agataatgaa gaaggaaacc ttctccaaac aactttgttc acttcactcc 5160
   ttttaaaaat ggggtgattg ccgggcgcgg tggctcacgc ctgtaatccc agcactttgg 5220
   gaggctgagg acagcagatc acctgaggtc aggagttcaa gaccagccta gccaacatgg 5280
   tgaaacccca tctctactaa aaatacaaaa attagccggg cgtggtggtg ggcgcctgta 5340
   atcccagcta cctgagaggc tgaggtagga gaatcgcttg aacctgggag gtggagtgag 5400
   ccgagatcgt gccattgcac tccagcccag gcaacgagag tgaaactctg tttcaaaata 5460
   aataagtaaa taaatgccat tgcactccag cccaggcaac aagagcaaaa ccctgtttct 5520
   aaataaataa aataaaataa aaatgaggtg attgcaggct gaaggtcttg gtcactactg 5580
   agttctagtt ctctcaagac aaatattcat atggtcaaaa cagaattata atctaacgag 5640
   tcattcttat agagtgggat tcggggaggg aaaacagata gctagtaatt taaatgatct 5700
   taatgtaatt tggggagagg atgatcagat gacatttaga cttggatata gtttctgctt 5760
   caaacccacg ctgtgtgtgt ctgtttgcat actctctaaa gaaggaattt gaaaaaaaaa 5820
   aaaaaaaaca aacctatatt gtccttgtta tcagccttcc attaagcgct ccaactcaac 5880
   ccatcctaag ctgtcaaatc tccttcattg tgtaaaattg aaaagaagcc ctgtgttttg 5940
   tccggaagct gtggaaactg tggacggcgg ggtgtgttct gtaactgagg ccacagacag 6000
   aaacaaatct gctcaagtgg tggaggggtc tgcaccgcac ataaagagct tctgtgggag 6060
   caggtgaagc aaggaggtat tgaagccggg tgtcctcttg ctgttgagtt tatagaaggc 6120
   acagagcggc ctgtccccag cagtaattct ttaggaccct ggtcagttcc agtgaagttt 6180
   ccttcttgag ttaggtgaac acattctttg tctttttagc actgtgaagt ttaagctaca 6240
   caccaagttc ctccttagta tttgcttggt tcaaagataa actgttacaa actatgggtc 6300
   agggagatgg gtagtctaag atgcacattc tggatctttc taggaccaga gggagactta 6360
   gtacttggtt ttttcacttc cctttaaaaa caatcctggt tcctgttccc cctccaaagt 6420
   tacctggaca agcaccatag tcttagagtt tcagctttta tggcgtctgt gtctcagacg 6480
   tgcatttgag atgaagatgt ttactgtata ttagaacgct agacttagaa tggatatagg 6540
   tggtttgtaa gtgccttcca gttttgaaat tttatgattt tgtatcttta tctataggtg 6600
   tgagtttata tgtgccctat agcactgtat gtaattgaaa tttaaaatat tagggcgctc 6660
   tgtccctttc ctaatgataa gctaatattg aagctcttta atttgtctgg ggtttgagga 6720
   ttatcataga cctctcttct accacagagc cttctctttt taattgttct cctgtcttgc 6780
   ctgttgaatt gaatttgtaa ggttagagat ggcaagagct acttccggtg ctgctttata 6840
   gcttactagt gatgagtcta tagttgtgct caaataggta ttgggtagct tatgctttct 6900
   agttgacttc tttttgggca caagaaaatg tgtgtaagat ggataactga aaacctagag 6960
   gcccattctg taaaagaatg gcaatttgga agaagaacca cgacctcagt gcagtgctcc 7020
   gttttggtaa tcaagctctg actcccaaga caccatctgt aagagactat aatttaaaac 7080
   aaagttgatg aaccagttga gtgcctttct taaaattact gttgtaaagt agatatgttg 7140
   aaaatatcaa ctgctaatta ttggctcacc aacaaatggc atgatttgtt tttcctgttt 7200
   gtcattctaa tattatggaa taatcactga aatatgaatg ttaaatgatt tgcatgtcat 7260
   agaattcaat tccattaatt ttccaaatta attagattca ataataatat gtccaaaaca 7320
   atgagcctct gaataagtct actaaagctt taaatatata tatttatatc tcaagactgc 7380
   tctgagatac tcttgtcccc agtagtttta ctgccaacaa ataccaataa tgacaaacgg 7440
   tgattaaaag ggcagataga tgtaaatgaa ggcagaggag gaggatgaaa tgaacatctg 7500
   cttttttcag ctctctgaca gaaattctta aaggaccaaa aataacactc ttgtttcaac 7560
   ttatgttagg tgtaggaaaa ttttcttttt ttctctttcc agtctgagta tgcttcattg 7620
   atttgtatat tgaatactaa attgttttag aaaaaaaaat cattggctga taagacactt 7680
   aaatgagaaa agctagtctg tcacaggcat gtaggtgtgc catatctatt taaatagagg 7740
   tttaaattat tgatgagact taatgaggaa ctggtgtata ttgtaaccaa aattgtagat 7800
   tccactgtga acaatctgtg catggatcga ttcacatatt tgagtctacc atttcaggta 7860
   catgaatgta caggagctac tgggaatata aaaactggaa aataaacatt atcatctatt 7920
   accactccct cctccttggt ccattttagt aaagaattca gtaaagtgat aggtaggcta 7980
   tcttgaatga gaagaaaata aaactaggaa agtgagagaa taaaaaccaa aacaaaactg 8040
   taactgcaaa ctgttgtaaa ggactactca cagaggagtt ctattgaata gtgacgaatt 8100
   gcttcctgat tcacggttgg tgattttttt accccttcaa agagaaattt attattaagg 8160
   aggaactggt atagttaaca tgtttcttta gcaaggcccc acaaagttaa aatgtgtgat 8220
   gcatccagtc tgaggtcatt tcctcagatc ttagaaccta cagctttcct ccgtataaac 8280
   ttaatttcaa aggagggctt ttggccaggc atggtggctc agcctgtaat ctcagcactt 8340
   tgggaggccg aggtgggagg atcgcttgag tccaggagtt tgagacaagg ctgggcaaca 8400
   tggtgagacc ccttctcaac aataacaaca aaaattagct gggcgtgatg gtgcatgcct 8460
   gtagtcccag ctactcagga ggctgaggtg ggaggatctc ttgatcccag gaggtcaagg 8520
   ctgcaatgag ctaagatcaa gccactgcat tccagcctga gtgatagtgg gagaccttgt 8580
   ctttaaaaca cacacacaca cacacacaca cacgagggcc tttgaccact cttgagtaga 8640
   agactcgaga agaacaaagt agaaggccag agaagaacaa agttacttga aagatctctt 8700
   attaaagaga atgtacaagc tatgaaaaaa aaaaaacaca cacacacaca caaacctcat 8760
   ctggaatgaa aaaaacataa tgcatttggt ttctggttcc ttaggctgtt atggaacaac 8820
   caaagaacat tattttggtt tctgaggtca gaactatttt attcccctca agcacactat 8880
   gcttatggtt tgagggagaa tgagaaatag gaaactagga acaggctgaa atggtctaat 8940
   cttgaccatc taattctgca gtgtcttatt ctcattctaa aagagaatgg ttatattcgc 9000
   tgttctagca taaaaagtaa tgataaaaat aaaagatccc gtattaccag acaataatcc 9060
   cctagactgt tttaatgctt ggttgagtat ttgcttatga tctcagactt taaaagatgg 9120
   tctcccccta tggtgaagct tgttaattat gtaggcatca ttaatgtctg tttacttatc 9180
   aaaattttat cattgttagt tgtattacta cttgacagtc caatttattt aattgaaaag 9240
   attggttaac attttatagt caaagtaatt gtttcctgtg ttttttcctg tttaggttat 9300
   tggagtgatg agtaaagaat acataccaaa gggcacacgt tttggacccc taataggtga 9360
   aatctacacc aatgacacag ttcctaagaa cgccaacagg aaatattttt ggagggtaag 9420
   taagggaaat ttcttcagac ccattaaatg ttaggaaaaa atggagctaa aagagctggg 9480
   tggctcacct ttctcatcct gtgctgagaa atgctggggc tcacccataa gtatccagca 9540
   tccccatgga cacagggaat tctgaacaaa tgtgatgaaa ccgatgaaat gtctggcctg 9600
   taggtggtta gtgatggaga tacgggctat atgtgaatct tgatttttgc aattcattag 9660
   agctttgtaa tgaaaggaaa cagtttgttg cttgctttaa ggataggttc atttgcattt 9720
   ctccgcaagg aagtagtaat gagttaccaa gccttagatt tcaccccttt ttgatttctt 9780
   gctgacttaa ctttaattga atggaagagt tatcacaaat gaattatctt tttggttttt 9840
   ttttttttga gatggagtct cactctgtca ccaggctgga gtgcaatggc atgatctcgg 9900
   ctcactgcaa cctccgcctc ccaggttcaa gcaattgtcc tgcctcagcc tcccgagtag 9960
   ctgggactaa ggtgcgcgcc accatgccca gttaattttt gtatttttag tagagacggg 10020
   gttccactat gttggccatg atggtctcga tctctggacc tcgtgatccg cccaccttgg 10080
   cctcccaaag tgctggaatt acaggcaaga gccaccgcgc ccagccagga atgacaaatg 10140
   aattacctta taagtaaatg ccattaagga aggatagctg gaagatgggt tgaggggaat 10200
   ggaggaccac agaactagtc ctatttaaat acatgtgcat ggtaaaatga ttccatttga 10260
   caataggtta attatctcat agcataagga aaatgcttaa cagtcatatg caagatgata 10320
   agctttccta tagcatccaa ccaaaagatc tagccagtac aatttccttt gctatattag 10380
   ggttagaaag gcccccagag gtgaaccaat tagatggaat ccttgaataa aacactggat 10440
   tagcagtgaa cagaaaaaag tcagattgct ttccttcttc ccatagatgt ctcagggata 10500
   tttagtttcc tcagaagata aagaatttag taagcgtttt tttgtgcata cttacatgaa 10560
   atgtacatta tttgaattct ttaaaaagaa acagctgcat gataacaaaa attgtgttat 10620
   gcttgcttta gctggtattt ttgcctagaa cgattatatc gttcggacaa gaagctattc 10680
   ctaagaaaca atatttttaa tccaggaagt ttttcatttt tagaaattta tcttactatt 10740
   tcccaagcaa aagagggtag ttacagattc actaagaatc atgtgctcac aatttttatt 10800
   taataattat tcctccttaa aatatattaa tcacctgact tacaatggtg gaaccatgag 10860
   tgcatttttg cctttattgt caataacgtc ttctcagaag tgagccacaa aggtgcatag 10920
   ttcttggagt taaaggtctg aattaagaca atccagcata agtctcatta atgtgtgatt 10980
   attttgagaa aaggcaagaa gtacctaaga atctccccct cactgtccag ttccctgttt 11040
   catttaaaga ttcactgtaa gtaactgaaa ggctttcctt gggaggattt atttgaatca 11100
   gtctttcaca tgcaaaggat attgtagaac atctcgtttt tgctggcagg aatatgaaca 11160
   tctgttgtga ggaaagaaaa agtttcatgc aaattacact gccaaagaag ggatgttcaa 11220
   gttgagaaac cagtgacatt tcttgtaact gtactatgaa tcagcgcatt ttaatcttct 11280
   agataatata tggaagtgca ggaaggtggt aggaaacggt gttcatttta catatgcgtt 11340
   attttattct gtgtgagtga cttcatggca ccgacattgc tgtttttaaa tgaggataca 11400
   gtaaattgca gtccgaggaa ggctaactgg aatcaacata cccgtagctt tagaaagcag 11460
   tttccgcacc agcgaagagt acaagagcga tggaacccca tgttcctgga agtttgcaca 11520
   tcagagtaaa caaacttgaa aacccctctt gatagcagaa ttcacccagc cttgttccat 11580
   tttctcttaa caaaacacac cgcaaaagct ctcacaagct gctttgatga agccacatgt 11640
   atttccccct tcacaattta caggaagtta ctcttaaaag aaagtgattc tggtgtttac 11700
   cgcctgtgtt aaagggacag agttcctttt tatttctgat aacgtttgag cgaaatacag 11760
   aaactatctg tagactagca tagtcggtac gtgagtaagg aaaagcaata acctgctgtc 11820
   cggtgagcac aaaattcctg ctacgaacag tgccttactg ctgcttggag actgcaagtc 11880
   gcagatcaca ctaggtattg actgattgta taaggaaatt tcttaaagtc taaagtaaag 11940
   gtggtacctc ctaaaaagag gggaagagag aaaactttgt gtggaaggat aaggagtgtg 12000
   tttatagttt cagtaagagt gtacgtttta atttttcttc ttcctctgcc tctttgccaa 12060
   gtagcctgag tgcatctgtt atccagaagt agtattactc taggacaaac ttcaaattct 12120
   tcattctgcg ttgcctttaa ggaacaacat actttcttcc tgttcttttt ccaaaaacac 12180
   acgcctatgg ctctgtgtgt ggtgttttag ccagcctcct cccagataag gggttccctt 12240
   ccctcctttg cattgaaagg aaagtgcaag tctggacatg tttatcaaga ggaaaagtga 12300
   cttctcagta atagactgtc aaattcgggc tgctgcccga gtgttcgctt tgttatggca 12360
   ggtgaagttc acctttgccc cacccagtgt ttccacaaaa aggcaaggtt ccaagtattc 12420
   atatgaacaa gtgttacttt aggacttgga gggttggggg tggaggatgt ttgcatagtt 12480
   gaagccttgg gcgggggtgt aggaaacggc gagtacagag gccatagaaa aagctaagac 12540
   tcagtttgac gtcgtcagcc ggcttggtct tctacccagt gactcaaagc actaaaagtc 12600
   agcataatcg gaactgaagt cagtagcatc gcccatttgc cattcactgc agtagcaaaa 12660
   gtagtactct gtggtgggtt aatcggtttg aggcagctcc ttaaatgaac atttgtgttt 12720
   catttttctg ttattttccc gaacatgaaa agacgataaa actgaaatgg aaaaggtaac 12780
   tgacaaaagt gtgccttacc tgtttccgcc ctgatttctg ctgattcaag actattctgg 12840
   ctaaactgat tggattcttt ttctaactag gcagtagggg atcagaaatc acacacggta 12900
   ccggctgtgt ttattctgag aggtgctggg gagctttggg tctgacttcc ttttacatgc 12960
   ctgtcttctc ttttggacag atctattcca gaggggagct tcaccacttc attgacggct 13020
   ttaatgaaga gaaaagcaac tggatgcgct atgtgaatcc agcacactct ccccgggagc 13080
   aaaacctggc tgcgtgtcag aacgggatga acatctactt ctacaccatt aagcccatcc 13140
   ctgccaacca ggaacttctt gtgtggtatt gtcgggactt tgcagaaagg cttcactacc 13200
   cttatcccgg agagctgaca atgatgaatc tcagtaagtg gattacagaa caaaaaaata 13260
   aaaaatgcca gtaatgtcgg ttctgcccct ttgaactaat aacatgttgt ttaattatac 13320
   ggctttgtca tgtgttggat gaagtaggtg gcttaagcta gggactagga agaggaaaaa 13380
   cattttttga gtccctatta actattagga aacttgatca tttaaaagta tatatatata 13440
   tgaggagcta ccttgagttt tgaattcagg atgttacagg aagaaatata tgtccaattc 13500
   taatttatcc aaaagcagtt gggagaatta cagggattgg tccagacatg ctgcgtatgc 13560
   aaggtatagc cctcatctgt ggtactttgg cagggcttag actgcatcaa aatatttata 13620
   gatgtacatt tgagtgtaca gttaggatct gatgtggaac attgtaagat cattgctaga 13680
   aaaactttgt cataattttt caatattatt ctaagtgaat aaccgtaaag attttacatc 13740
   ttagcttcct tccttacagt aaaaaaacta tctgatctct tgatcagtat tatagtagcc 13800
   acctatcact ttatcttaac aaattctcaa ttccttaggt ttatgtgctt ttacttcttt 13860
   tatttgatta aaattgctgt catgacctct ctctgcagag ggctgcatca ttttggtcat 13920
   tctcaagtga tctctttgag caatttaaga attgccataa gattctaacc tctgctgtaa 13980
   ctatggttgt gtgttcttgg ttagaccact aaatcttatt agcagtttta aaaattattc 14040
   cttttggttt agaagttaag actaaatgct gaagtttttg taacttttgg ttttgatatc 14100
   atttcaaact taagaaaaca tttgaagaaa aggacaaaga atttccactt accctttacc 14160
   caggtttacc agttattgat aagtatatcc atttgcttta ccagaaggct aacttgtttt 14220
   agttctcatt ttcacctttg agacatttgg aataaatatc aatgttaaca taaattggaa 14280
   ttttgacttt gattttagga ccaatgaaca agccaagtac ttaccctagt catatataat 14340
   ccaactgtat ggttatttgg tattcattcc acacttcatt ttacttgatc tcccttaaga 14400
   ttgcaagatt gtgtttgcag tttttctgaa aatctggggc tataaaagca tcaggacctc 14460
   ccccgtaggg gaggtcgtgt gtttggggtc cttacacaac aggttaccct tgagcttcag 14520
   gaaaagaact ggctctcagt tccccagttc cagcttaatg ggtctaatta ggtcctgacc 14580
   aaaaaggtgg cagttctttt ccctcatgtc tcttcagcgc tccccgagac tctggagact 14640
   ctgtcatatc cctagggctg agcctcccag gaaccattcg gctgttgtgg catctgtgta 14700
   tgccatgccc agtgctgagg acctagtaac aaacgacaaa tgcacaggca cagtggcatt 14760
   tttgtggaac tcgtattcca gctgtgcgtc tcagaagaag cgcacagctc cctcctggct 14820
   ttcttaacat agtgagccac ttccacttaa gggtctcctt acattccttg agtttaatca 14880
   ttcatggatt cagaggaaag tcttttgatt tttgcttttc tttaaacagt tcatttgagg 14940
   tgacctaccc cagtgacttt gcaccaacca ccaagaaact tttttgcatg cttcccgcac 15000
   cctgtgccaa tcaagggaag ggtttaaagg cctggcgttt ttattcctca aagaaaggtt 15060
   ttgcacagta ttttaaggtt caagtgcttc tactttgtgt tcagaagcaa ctgtcatata 15120
   tactgtgaaa tgacaccttt tatttatccc tttttattta tgcagtatgt ccccttttat 15180
   tttggcagaa ttttttctaa atggtggttt aacattttca agcacatttc attgtccaat 15240
   attcatagta aagaatgaga gttaacaata accagtcaca ttaaaacaag attcctgctg 15300
   ccagttgtga aaccggttgt cttaggcgtg gcagctgatg attgagactg tgatcaggaa 15360
   aatttccact atttcatcag gcctaatagg tagattgtgt ctccaaatga actgtgttgg 15420
   gtttccatgc ttaaagcaca atagaggtgg tgcaagaatc tccatgaggg cttaaatggc 15480
   agtgatggtt caggcggtag agtttggaga agaagggatt tgaaacaaac caaaggaaag 15540
   aaaagtaagt agccagaaat cacaaaatgg catttttcta aaaacaaagg aaaaggaata 15600
   aaagaactaa taagtttgaa acccctaccc ctcccaaatt tggcaggggg ggaggtattt 15660
   tttttctatc tatctaacta acccatctag aaaacagttg accaaattat agacttctaa 15720
   atgttaatct gctttctcag tttcagttga aaagagactt tgttttgcct actgcagaac 15780
   ttctaggttc tttcttatag tcttggggtt cttattatag atcgaaaatg tgagtcggca 15840
   taattaagcc attcggagtc ttcagaagca gttcactctt gaaatgactc cgtccgccta 15900
   cagccattta agatttcaga acaaaaacag atcttgattt tctttttcat gttaactcaa 15960
   gctgttgctg agtgggagag tcagaaatga caccagctcc actgattact cagctgctga 16020
   aggatgattt tttaaaatgc acctttactg tatatggact tcctaatttc cacctgtaga 16080
   gcatcttagg gaggctaaca tgtcactctg gatgttcttt tagaataaga tgcaaatcta 16140
   tttttctgaa ggcattagag atagcaaaca tttattgtga gtttactata tactaggcac 16200
   tgtgctaagt gttttgcata gaaagtttaa aattctggct tttttgttgg cccaatcata 16260
   agtttcatat cagttcaaca ttcaaattat attaaggtac ttaagaagaa tccctggcta 16320
   aatgtgaggg gcagtgccac agatggactg aaactttatg cttattgcac atttatgcta 16380
   ttattatttg ttgaattata gaaccaaggg agtgtggaag ccactggaaa aaatatgaga 16440
   cttagataca taatttgagt aaaaatggct caaagtcatg agggtaaagt tttttgtatt 16500
   tccattttat tcgagcggca tcgtttttaa aaatcattat gaatttgacc ctatatagat 16560
   gtttccaaat aattcttttt caccttcata aaattccttc ctgtggctgt gagatgcctt 16620
   gcctatcagt tttcaagctt agttgtcttt ctcatccttt accattttag ctttaaaaaa 16680
   caaaagtgac aattagaact tcctgcctgc tgggcctcac tgaaagaccg atattggcct 16740
   gataaggaga tatttatttt gttttagtgg cttcagaaat ccctctccct cagcaagctt 16800
   tccatcacgg cccccccgtc agcatcttcc ctgatagcgt tcttctctgt gtttattctg 16860
   gggcttcagg ctcgcccagg aggaactgat aaccgctggc aggagataac attctctaag 16920
   gggctctcaa attggaatcg aatccctcaa gccagtcagc ctagagaata catttaaagg 16980
   gttcagttct ggagtttcac agagttcatt tctagaccta tcagatagca agtgtggagt 17040
   tctttctcaa ctaaattcaa gcagagacat tttttagacg atgaaggata tttgcacaaa 17100
   ggcttcagca tgatccccca aacctgctgc ctctgaaggc atctccacac attgacagcc 17160
   aatgccttca gtgcgttcct agggcaggtg tcctggcttg agtgactgtc ctccaataat 17220
   cagagctcaa actaaacatc gtatgtttta cttttggttt ccaggcaagg ctgagcaggg 17280
   aattttcagt tttccctgcc cagatgggtg ttttttcctg aaggcatcat ttattgtgta 17340
   gcgaggagac agggctggct gtggcaggga tagtctagaa ctgtcctcat tgctgctgtt 17400
   cctaaatagt atctttacca agtaataacg tgccgtcttt gggaataagt gctttcctct 17460
   tagcctgttc tgttttcttg ggtgcgctaa gtaattgaac tggctcagga agtacctatt 17520
   gtggtttggc agaggtgact gtcacgcctt gtgactccag gggccagcac tgctgggatc 17580
   ctggctagac cagacagagc cttggtgaag tgcttaggct gtctgcacat cgcgaggaag 17640
   gtggtattca cttcgctaag ctccttggca taggcagttt gaacagggct ttatcaaatt 17700
   cgtattcaac aagagtagaa gcgaaaattg atgactgtgt attacttgaa atgagtctta 17760
   atctttcaca tttagttctc agggtatgct gatttccttt aggtaaacca tgaacatcag 17820
   aaagactttt attaacctat gacagggtcc ccaccccagt atttttccac tccattaaaa 17880
   tggaagtttt tttttttttt ttcttttttg agacagagtt ttgctcttgt tgcccagtct 17940
   ggagtgcaat ggcacaatct cggctcacca caacctccac ctcccagatt caagcgattc 18000
   ttctgcctca gcctcccaag tagctgggat tacaggtgtg cgccaccacg cccagctaat 18060
   tttgtatttt tagtagagat ggggtttctc catgttggtc aggctggtct cgaacttccg 18120
   acctcaggtg atccgcccac ctcggcctcc caaagtgctg ggattacagg caagagccac 18180
   tgcatccagc ttaggctatc ttactccagc ctaaacagca attttctatc ataaggtctg 18240
   tactaatgaa aacagaatca cccaaggctg ctgtttgttc tgtctgtgct gccattgtcc 18300
   gcattttgct gaggaggaaa cggaactgca cttttgagtg agtggcccag agccttctag 18360
   aatgagagtg cgttggaagc cagatatgtg gcgattgtgt cgccagctgt tactcaggtt 18420
   ttctcaagaa ggaggagcaa ctttggcagt tttgcttcag ttctctctag ccctctgtgt 18480
   aatcgcccct ttttctttat ttcagcacaa acacagagca gtctaaagca accgagcact 18540
   gagaaaaatg aactctgccc aaagaatgtc ccaaagagag agtacagcgt gaaagaaatc 18600
   ctaaaattgg actccaaccc ctccaaagga aaggacctct accgttctaa catttcaccc 18660
   ctcacatcag aaaaggacct cgatgacttt agaagacgtg ggagccccga aatgcccttc 18720
   taccctcggg tcgtttaccc catccgggcc cctctgccag aagacttttt gaaagcttcc 18780
   ctggcctacg ggatcgagag acccacgtac atcactcgct cccccattcc atcctccacc 18840
   actccaagcc cctctgcaag aagcagcccc gaccaaagcc tcaagagctc cagccctcac 18900
   agcagccctg ggaatacggt gtcccctgtg ggccccggct ctcaagagca ccgggactcc 18960
   tacgcttact tgaacgcgtc ctacggcacg gaaggtttgg gctcctaccc tggctacgca 19020
   cccctgcccc acctcccgcc agctttcatc ccctcgtaca acgctcacta ccccaagttc 19080
   ctcttgcccc cctacggcat gaattgtaat ggcctgagcg ctgtgagcag catgaatggc 19140
   atcaacaact ttggcctctt cccgaggctg tgccctgtct acagcaatct cctcggtggg 19200
   ggcagcctgc cccaccccat gctcaacccc acttctctcc cgagctcgct gccctcagat 19260
   ggagcccgga ggttgctcca gccggagcat cccagggagg tgcttgtccc ggcgccccac 19320
   agtgccttct cctttaccgg ggccgccgcc agcatgaagg acaaggcctg tagccccaca 19380
   agcgggtctc ccacggcggg aacagccgcc acggcagaac atgtggtgca gcccaaagct 19440
   acctcagcag cgatggcagc ccccagcagc gacgaagcca tgaatctcat taaaaacaaa 19500
   agaaacatga ccggctacaa gacccttccc tacccgctga agaagcagaa cggcaagatc 19560
   aagtacgaat gcaacgtttg cgccaagact ttcggccagc tctccaatct gaaggtaggc 19620
   cttgagagag agcagtccaa ggggctgtga gtgcatgctt gtgtttgtat ttagcttgct 19680
   ttccatgggg tatcgattgc atttgcagta gtatgagccc ccggttgggg atagtgggta 19740
   tggattccgc ctggcttttg ccacttctag ctctttgact ttggacaagt gacttccctt 19800
   ctcctgattt tcttctgaat aataaaaaaa ttaggggttt ggactagaag attaggtgaa 19860
   actccctgct agcctgtgat ttttgtgctt ttaagaaaaa caccattctg aaaacatgaa 19920
   gatttcttct ttttaagact gtcttgatgc ttttcttaag atatttgcat caacacttga 19980
   gtcttggagc agaaatgtta ggtctcagag ccagcttgag agcagagcta acacatgtgg 20040
   cttcttccca ggtccacctg agagtgcaca gtggagaacg gcctttcaaa tgtcagactt 20100
   gcaacaaggg ctttactcag ctcgcccacc tgcagaaaca ctacctggta cacacgggag 20160
   aaaagccaca tgaatgccag gtgcgcagta ttttctgggt agaccttctg acctttgtag 20220
   aaaatgtctg tgagtcaccc tcccatgtcc tatatagccc gtagttaaag ccaacaccag 20280
   attctgcgtt gtcccatcct ggactgatgg cactatggtc cttcccagta ctttgtatct 20340
   gctgatgact tgagatggca cagccagctt ccagtgggtg ggaaaatggt aggggaaata 20400
   aacagcccct cgtgtgctgt gtgcccacat ccccccgttt gcttaatacc acactggagg 20460
   tgccacaagg aggcttctca cctcctaggt tgctgggcgt tggccggtaa gcctgcccct 20520
   cccgttggca actcttaatc ttctggcctt cctgtctccc ttccctgctg tctctctccc 20580
   ctacactgta ggtctgccac aagagattta gcagcaccag caatctcaag acccacctgc 20640
   gactccattc tggagagaaa ccataccaat gcaaggtgtg ccctgccaag ttcacccagt 20700
   ttgtgcacct gaaactgcac aagcgtctgc acacccggga gcggccccac aagtgctccc 20760
   agtgccacaa gaactacatc catctctgta gcctcaaggt tcacctgaaa gggaactgcg 20820
   ctgcggcccc ggcgcctggg ctgcccttgg aagatctgac ccgaatcaat gaagaaatcg 20880
   agaagtttga catcagtgac aatgctgacc ggctcgagga cgtggaggat gacatcagtg 20940
   tgatctctgt agtggagaag gaaattctgg ccgtggtcag aaaagagaaa gaagaaactg 21000
   gcctgaaagt gtctttgcaa agaaacatgg ggaatggact cctctcctca gggtgcagcc 21060
   tttatgagtc atcagatcta cccctcatga agttgcctcc cagcaaccca ctacctctgg 21120
   tacctgtaaa ggtcaaacaa gaaacagttg aaccaatgga tccttaagat tttcagaaaa 21180
   cacttatttt gtttcttaag ttatgacttg gtgagtcagg gtgcctgtag gaagtggctt 21240
   gtacataatc ccagctctgc aaagctctct cgacagcaaa tggtttcccc tcacctctgg 21300
   aattaaagaa ggaactccaa agttactgaa atctcagggc atgaacaagg caaaggccat 21360
   atatatatat atatatatat ctgtatacat attatatata cttatttaca cctgtgtcta 21420
   tatatttgcc cctgtgtatt ttgaatattt gtgtggacat gtttgcatag ccttcccatt 21480
   actaagacta ttacctagtc ataattattt tttcaatgat aatccttcat aatttattat 21540
   acaatttatc attcagaaag caataattaa aaaagtttac aatgactgga aagattcctt 21600
   gtaatttgag tataaatgta tttttgtctt gtggccattc tttgtagata atttctgcac 21660
   atctgtataa gtacctaaga tttagttaaa caaatatatg acttcagtca acctctctct 21720
   ctaataatgg tttgaaaatg aggtttgggt aattgccaat gttggacagt tgatgtgttc 21780
   attcctggga tcctatcatt tgaacagcat tgtacataac ttgggggtat gtgtgcagga 21840
   ttacccaaga ataacttaag tagaagaaac aagaaaggga atcttgtata tttttgttga 21900
   tagttcatgt ttttccccca gccacaattt taccggaagg gtgacaggaa ggctttacca 21960
   acctgtctct ccctccaaaa gagcagaatc ctcccaccgc cctgccctcc ccaccgagtc 22020
   ctgtggccat tcagagcggc cacatgactt ttgcatccat tgtattatca gaaaatgtga 22080
   agaagaaaaa aatgccatgt tttaaaacca ctgcgaaaat ttccccaaag cataggtggc 22140
   tttgtgtgtg tgcgatttgg gggcttgagt ctgggtggtg ttttgttgtt ggtttttgtt 22200
   gctttttttt tttttttttt tttaatgtca aaattgcaca aacatggtgc tctaccagga 22260
   aggattcgag gtagataggc tcaggccaca ctttaaaaac aaacacacaa acaacaaaaa 22320
   acgggtattc tagtcatctt ggggtaaaag cgggtaatga acattcctat ccccaacaca 22380
   tcaattgtat tttttctgta aaactcagat tttcctcagt atttgtgttt ttacatttta 22440
   tggttaattt aatggaagat gaaagggcat tgcaaagttg ttcaacaaca gttacctcat  22500
   tgagtgtgtc cagtagtgca ggaaatgatg tcttatctaa tgatttgctt ctctagagga  22560
   gaaaccgagt aaatgtgctc cagcaagata gactttgtgt tattctatct tttattctgc  22620
   taagcccaaa gattacatgt tggtgttcaa agtgtagcaa aaaatgatgt atatttataa  22680
   atctatttat accactatat catatgtata tatatttata accacttaaa ttgtgagcca  22740
   agccatgtaa aagatctact ttttctaagg gcaaaaaaaa aaaaaaaaaa aaaagaacac  22800
   tcctttctga gactttgctt aatacttggt gacctcacaa tcacgtcggt atgattgggc  22860
   acccttgcct actgtaagag accctaaaac cttggtgcag tggtggggac cacaaaacaa  22920
   ccagggagga agagatacat cattttttag tattaaggac catctaagac agctctattt  22980
   tttttttgcc actttatgat tatgtggtca cacccaagtc acagaaataa aaaactgact  23040
   ttaccgctgc aatttttctg ttttcctcct tactaaatac tgatacatta ctccaatcta  23100
   ttttataatt atatttgaca ttttgttcac atcaactaat gttcacctgt agaagagaac  23160
   aaatttcgaa taatccaggg aaacccaaga gccttactgg tcttctgtaa cttccaagac  23220
   tgacagcttt ttatgtatca gtgtttgata aacacagtcc ttaactgaag gtaaaccaaa  23280
   gcatcacgtt gacattagac caaatacttt tgattcccaa ctactcgttt gttctttttc  23340
   tccttttgtg ctttcccata gtgagaattt ttataaagac ttcttgcttc tctcaccatc  23400
   catccttctc ttttctgcct cttacatgtg aatgttgagc ccacaatcaa cagtggtttt  23460
   attttttcct ctactcaaag ttaaaactga ccaaagttac tggcttttta ctttgctaga  23520
   acaacaaact atcttatgtt tacatactgg tttacaatgt tatttatgtg caaattgtca  23580
   aaatgtaaat taaatataaa tgttcatgct ttacc                             23615

### SEQUENCE LISTING

<110> The Walter and Eliza Hall Institute of Medical Research
<120> Modified cells that co-express Blimp1 and a reporter molecule and methods of using the same
<130> 1.5.92528
<140> EP 05700191.9
   <141> 2005-02-11
<150> AU 2004900673
   <151> 2004-02-12
<160> 6
<170> PatentIn version 3.1
<210> 1
   <211> 2571
   <212> DNA
   <213> Mus musculus
<400> 1
   atgagagagg cttatctcag atgttggatc ttctcttgga aaaacgtgtg ggtacgacct      60
   tgccaaaggc tgcattttaa aaccgtgctt cttcaaggca gtctacttta cacggctttg     120
   gactcttact caactgtaca agctgccccc aagtctagct ccggctccgt gaagtttcaa     180
   ggactggcag agactgggat catgaaaatg gacatggagg acgctgatat gactttgtgg     240
   acagaggccg agtttgaaga gaagtgtaca tacatagtga acgaccaccc ctgggattcc     300
   ggcgctgacg ggggtacttc tgttcaagcc gaggcatcct taccaaggaa cctgcttttc      360
   aagtatgctg ccaacaacag caaagaggtt attggcgtgg taagtaagga gtacataccg     420
   aagggaacac gctttggacc cctcatcggt gaagtctaca ctaatgacac agttcccaag     480
   aatgccaaca ggaagtattt ttggcggatc tattccagag aggagttcca ccacttcatt     540
   gatggcttta atgaggagaa aagcaactgg atgcgctacg tgaatccagc tcactctgcc     600
   cgggagcaaa acctggctgc ctgtcagaac gggatgaaca tctacttcta cactattaag     660
   cctatccctg ccaaccagga acttcttgtg tggtattgtc gggactttgc ggagaggctc     720
   cactaccctt atcctggaga gctcacagtg ataaatctca cacaaacgga aagcaaccca     780
   aagcaataca gtagtgagaa aaatgaactc tacccaaaga gtgtccccaa gagagagtac     840
   agcgtgaaag aaattctaaa actggactcc aatccctcca aaaggaagga catctaccgt     900
   tccaacattt cacccttcac tttagaaaag gacatggatg gctttcggaa aaatgggagc    960
   cccgacatgc ccttctaccc tcgggtggtt tatcctatcc gggcacctct gccagaagac 1020
   tttttgaaag cgtccctggc ctatgggatg gagagaccca cctacataac tcacagtccc 1080
   cttccgtctt ccacaactcc aagtccccct gcgagcagca gcccggagca gagccttaag 1140
   agctccagcc cccacagcag cccgggaaac acggtgtcac ccctggcgcc aggcctccca 1200
   gaacaccggg actcctactc ctacttgaat gtttcctatg gttccgaggg cctgggctcc 1260
   taccctggct atgcacctgc cccccacctc ccaccagctt tcattccttc ttacaatgct 1320
   cactacccca agttcctgtt gccaccgtac ggcattagtt ccaatggctt gagcaccatg 1380
   aacaacatca atggtatcaa caacttcagc ctcttcccta ggttgtatcc cgtctacagt 1440
   aacctcctta gtggcagcag cctgcctcat cccatgctca atccagcttc cctaccgagt 1500
   tccctgccta ccgatggagc ccggaggctg cttccaccgg agcaccccaa agaggtgctt 1560
   atcccagcac cccacagtgc cttctccctt accggggctg cagccagcat gaaggacgag 1620
   agtagtcccc ccagcggatc tccaacggcg ggaactgcag ccacgtcaga acacgtggta 1680
   caacccaaag ctacctcatc agtgatggcg gcccccagca ctgacggagc catgaatctc 1740
   attaaaaaca aacgaaacat gactggttac aagactcttc cttaccctct gaagaaacag 1800
   aatggcaaga tcaagtatga gtgcaatgtc tgtgccaaga cgttcggtca gctctccaac 1860
   ctgaaggtcc acctgagagt gcacagtgga gaacggcctt tcaagtgcca gacctgcaac 1920
   aagggtttta ctcagctcgc ccacctgcag aaacactact tggtacacac aggagagaag 1980
   ccacatgagt gccaggtctg ccacaagaga tttagcagca caagcaatct caagacccac 2040
   cttcgattgc attctggaga aaaaccttac caatgtaagg tgtgccctgc caagtttacg 2100
   caatttgtgc acctgaagct gcacaagcga ctgcataccc gggagcggcc tcacaagtgt 2160
   gcccagtgtc acaagagcta catccatctc tgcagcctca aggtccacct gaagggcaac 2220
   tgccctgcgg gcccagctgc tgggctgcct ttggaggatc tgacccgaat caatgaagaa 2280
   attgagaggt tcgacatcag cgacaatgca gaccgtcttg aggacatgga ggacagtgtc 2340
   gatgtgacct ccatggtgga gaaggagatt ctagctgtgg tcagaaaaga gaaagaagaa 2400
   accagtctga aagtgtcttt gcaaagaaac atggggaacg gcctcctctc ctcagggtgc 2460
   agcctctatg agtcatcgga cctgtccctc atgaagttgc ctcacagcaa cccactacct 2520
   ctggtgcctg taaaggtcaa acaagaaaca gttgaaccga tggatcctta a 2571
<210> 2
   <211> 856
   <212> PRT
   <213> Mus musculus
<400> 2
   Met Arg Glu Ala Tyr Leu Arg Cys Trp Ile Phe Ser Trp Lys Asn Val
   1               5                   10                  15
   Trp Val Arg Pro Cys Gln Arg Leu His Phe Lys Thr Val Leu Leu Gln
               20                  25                  30
   Gly Ser Leu Leu Tyr Thr Ala Leu Asp Ser Tyr Ser Thr Val Gln Ala
           35                  40                  45
   Ala Pro Lys Ser Ser Ser Gly Ser Val Lys Phe Gln Gly Leu Ala Glu
       50                  55                  60
   Thr Gly Ile Met Lys Met Asp Met Glu Asp Ala Asp Met Thr Leu Trp
   65                  70                  75                  80
   Thr Glu Ala Glu Phe Glu Glu Lys Cys Thr Tyr Ile Val Asn Asp His
                   85                  90                  95
   Pro Trp Asp Ser Gly Ala Asp Gly Gly Thr Ser Val Gln Ala Glu Ala
               100                 105                 110
   Ser Leu Pro Arg Asn Leu Leu Phe Lys Tyr Ala Ala Asn Asn Ser Lys
           115                 120                 125
   Glu Val Ile Gly Val Val Ser Lys Glu Tyr Ile Pro Lys Gly Thr Arg
       130                 135                 140
   Phe Gly Pro Leu Ile Gly Glu Val Tyr Thr Asn Asp Thr Val Pro Lys
   145                 150                 155                 160
   Asn Ala Asn Arg Lys Tyr Phe Trp Arg Ile Tyr Ser Arg Glu Glu Phe
                   165                 170                 175
   His His Phe Ile Asp Gly Phe Asn Glu Glu Lys Ser Asn Trp Met Arg
               180                 185                 190
   Tyr Val Asn Pro Ala His Ser Ala Arg Glu Gln Asn Leu Ala Ala Cys
           195                 200                 205
   Gln Asn Gly Met Asn Ile Tyr Phe Tyr Thr Ile Lys Pro Ile Pro Ala
       210                 215                 220
   Asn Gln Glu Leu Leu Val Trp Tyr Cys Arg Asp Phe Ala Glu Arg Leu
   225                 230                 235                 240
   His Tyr Pro Tyr Pro Gly Glu Leu Thr Val Ile Asn Leu Thr Gln Thr
                   245                 250                 255
   Glu Ser Asn Pro Lys Gln Tyr Ser Ser Glu Lys Asn Glu Leu Tyr Pro
               260                 265                 270
   Lys Ser Val Pro Lys Arg Glu Tyr Ser Val Lys Glu Ile Leu Lys Leu
           275                 280                 285
   Asp Ser Asn Pro Ser Lys Arg Lys Asp Ile Tyr Arg Ser Asn Ile Ser
       290                 295                 300
   Pro Phe Thr Leu Glu Lys Asp Met Asp Gly Phe Arg Lys Asn Gly Ser
   305                 310                 315                 320
   Pro Asp Met Pro Phe Tyr Pro Arg Val Val Tyr Pro Ile Arg Ala Pro
                   325                 330                 335
   Leu Pro Glu Asp Phe Leu Lys Ala Ser Leu Ala Tyr Gly Met Glu Arg
               340                 345                 350
   Pro Thr Tyr Ile Thr His Ser Pro Leu Pro Ser Ser Thr Thr Pro Ser
           355                 360                 365
   Pro Pro Ala Ser Ser Ser Pro Glu Gln Ser Leu Lys Ser Ser Ser Pro
       370                 375                 380
   His Ser Ser Pro Gly Asn Thr Val Ser Pro Leu Ala Pro Gly Leu Pro
   385                 390                 395                 400
   Glu His Arg Asp Ser Tyr Ser Tyr Leu Asn Val Ser Tyr Gly Ser Glu
                   405                 410                 415
   Gly Leu Gly Ser Tyr Pro Gly Tyr Ala Pro Ala Pro His Leu Pro Pro
               420                 425                 430
   Ala Phe Ile Pro Ser Tyr Asn Ala His Tyr Pro Lys Phe Leu Leu Pro
   435 440 445
   Pro Tyr Gly Ile Ser Ser Asn Gly Leu Ser Thr Met Asn Asn Ile Asn
   450 455 460
   Gly Ile Asn Asn Phe Ser Leu Phe Pro Arg Leu Tyr Pro Val Tyr Ser
   465 470 475 480
   Asn Leu Leu Ser Gly Ser Ser Leu Pro His Pro Met Leu Asn Pro Ala
   485 490 495
   Ser Leu Pro Ser Ser Leu Pro Thr Asp Gly Ala Arg Arg Leu Leu Pro
   500 505 510
   Pro Glu His Pro Lys Glu Val Leu Ile Pro Ala Pro His Ser Ala Phe
   515 520 525
   Ser Leu Thr Gly Ala Ala Ala Ser Met Lys Asp Glu Ser Ser Pro Pro
   530 535 540
   Ser Gly Ser Pro Thr Ala Gly Thr Ala Ala Thr Ser Glu His Val Val
   545 550 555 560
   Gln Pro Lys Ala Thr Ser Ser Val Met Ala Ala Pro Ser Thr Asp Gly
   565 570 575
   Ala Met Asn Leu Ile Lys Asn Lys Arg Asn Met Thr Gly Tyr Lys Thr
   580 585 590
   Leu Pro Tyr Pro Leu Lys Lys Gln Asn Gly Lys Ile Lys Tyr Glu Cys
   595 600 605
   Asn Val Cys Ala Lys Thr Phe Gly Gln Leu Ser Asn Leu Lys Val His
   610 615 620
   Leu Arg Val His Ser Gly Glu Arg Pro Phe Lys Cys Gln Thr Cys Asn
   625 630 635 640
   Lys Gly Phe Thr Gln Leu Ala His Leu Gln Lys His Tyr Leu Val His
   645 650 655
   Thr Gly Glu Lys Pro His Glu Cys Gln Val Cys His Lys Arg Phe Ser
   660 665 670
   Ser Thr Ser Asn Leu Lys Thr His Leu Arg Leu His Ser Gly Glu Lys
           675                 680                 685
   Pro Tyr Gln Cys Lys Val Cys Pro Ala Lys Phe Thr Gln Phe Val His
       690                 695                 700
   Leu Lys Leu His Lys Arg Leu His Thr Arg Glu Arg Pro His Lys Cys
   705                 710                 715                 720
   Ala Gln Cys His Lys Ser Tyr Ile His Leu Cys Ser Leu Lys Val His
                   725                 730                 735
   Leu Lys Gly Asn Cys Pro Ala Gly Pro Ala Ala Gly Leu Pro Leu Glu
               740                 745                 750
   Asp Leu Thr Arg Ile Asn Glu Glu Ile Glu Arg Phe Asp Ile Ser Asp
           755                 760                 765
   Asn Ala Asp Arg Leu Glu Asp Met Glu Asp Ser Val Asp Val Thr Ser
       770                 775                 780
   Met Val Glu Lys Glu Ile Leu Ala Val Val Arg Lys Glu Lys Glu Glu
   785                 790                 795                 800
   Thr Ser Leu Lys Val Ser Leu Gln Arg Asn Met Gly Asn Gly Leu Leu
                   805                 810                 815
   Ser Ser Gly Cys Ser Leu Tyr Glu Ser Ser Asp Leu Ser Leu Met Lys
               820                 825                 830
   Leu Pro His Ser Asn Pro Leu Pro Leu Val Pro Val Lys Val Lys Gln
           835                 840                 845
   Glu Thr Val Glu Pro Met Asp Pro
       850                 855
<210> 3
   <211> 2370
   <212> DNA
   <213> Homo sapiens
<400> 3
   atgaaaatgg acatggagga tgcggatatg actctgtgga cagaggctga gtttgaagag      60
   aagtgtacat acattgtgaa cgaccacccc tgggattctg gtgctgatgg cggtacttcg 120
   gttcaggcgg aggcatcctt accaaggaat ctgcttttca agtatgccac caacagtgaa 180
   gaggttattg gagtgatgag taaagaatac ataccaaagg gcacacgttt tggaccccta 240
   ataggtgaaa tctacaccaa tgacacagtt cctaagaacg ccaacaggaa atatttttgg 300
   aggatctatt ccagagggga gcttcaccac ttcattgacg gctttaatga agagaaaagc 360
   aactggatgc gctatgtgaa tccagcacac tctccccggg agcaaaacct ggctgcgtgt 420
   cagaacggga tgaacatcta cttctacacc attaagccca tccctgccaa ccaggaactt 480
   cttgtgtggt attgtcggga ctttgcagaa aggcttcact acccttatcc cggagagctg 540
   acaatgatga atctcacaca aacacagagc agtctaaagc aaccgagcac tgagaaaaat 600
   gaactctgcc caaagaatgt cccaaagaga gagtacagcg tgaaagaaat cctaaaattg 660
   gactccaacc cctccaaagg aaaggacctc taccgttcta acatttcacc cctcacatca 720
   gaaaaggacc tcgatgactt tagaagacgt gggagccccg aaatgccctt ctaccctcgg 780
   gtcgtttacc ccatccgggc ccctctgcca gaagactttt tgaaagcttc cctggcctac 840
   gggatcgaga gacccacgta catcactcgc tcccccattc catcctccac cactccaagc 900
   ccctctgcaa gaagcagccc cgaccaaagc ctcaagagct ccagccctca cagcagccct 960
   gggaatacgg tgtcccctgt gggccccggc tctcaagagc accgggactc ctacgcttac 1020
   ttgaacgcgt cctacggcac ggaaggtttg ggctcctacc ctggctacgc acccctgccc 1080
   cacctcccgc cagctttcat cccctcgtac aacgctcact accccaagtt cctcttgccc 1140
   ccctacggca tgaattgtaa tggcctgagc gctgtgagca gcatgaatgg catcaacaac 1200
   tttggcctct tcccgaggct gtgccctgtc tacagcaatc tcctcggtgg gggcagcctg 1260
   ccccacccca tgctcaaccc cacttctctc ccgagctcgc tgccctcaga tggagcccgg 1320
   aggttgctcc agccggagca tcccagggag gtgcttgtcc cggcgcccca cagtgccttc 1380
   tcctttaccg gggccgccgc cagcatgaag gacaaggcct gtagccccac aagcgggtct 1440
   cccacggcgg gaacagccgc cacggcagaa catgtggtgc agcccaaagc tacctcagca 1500
   gcgatggcag cccccagcag cgacgaagcc atgaatctca ttaaaaacaa aagaaacatg 1560
   accggctaca agacccttcc ctacccgctg aagaagcaga acggcaagat caagtacgaa 1620
   tgcaacgttt gcgccaagac tttcggccag ctctccaatc tgaaggtcca cctgagagtg 1680
   cacagtggag aacggccttt caaatgtcag acttgcaaca agggctttac tcagctcgcc 1740
   cacctgcaga aacactacct ggtacacacg ggagaaaagc cacatgaatg ccaggtctgc   1800
   cacaagagat ttagcagcac cagcaatctc aagacccacc tgcgactcca ttctggagag   1860
   aaaccatacc aatgcaaggt gtgccctgcc aagttcaccc agtttgtgca cctgaaactg   1920
   cacaagcgtc tgcacacccg ggagcggccc cacaagtgct cccagtgcca caagaactac   1980
   atccatctct gtagcctcaa ggttcacctg aaagggaact gcgctgcggc cccggcgcct   2040
   gggctgccct tggaagatct gacccgaatc aatgaagaaa tcgagaagtt tgacatcagt   2100
   gacaatgctg accggctcga ggacgtggag gatgacatca gtgtgatctc tgtagtggag   2160
   aaggaaattc tggccgtggt cagaaaagag aaagaagaaa ctggcctgaa agtgtctttg   2220
   caaagaaaca tggggaatgg actcctctcc tcagggtgca gcctttatga gtcatcagat   2280
   ctacccctca tgaagttgcc tcccagcaac ccactacctc tggtacctgt aaaggtcaaa   2340
   caagaaacag ttgaaccaat ggatccttaa                                    2370
<210> 4
   <211> 789
   <212> PRT
   <213> Homo sapiens
<400> 4
   Met Lys Met Asp Met Glu Asp Ala Asp Met Thr Leu Trp Thr Glu Ala
   1               5                   10                  15
   Glu Phe Glu Glu Lys Cys Thr Tyr Ile Val Asn Asp His Pro Trp Asp
               20                  25                  30
   Ser Gly Ala Asp Gly Gly Thr Ser Val Gln Ala Glu Ala Ser Leu Pro
           35                  40                  45
   Arg Asn Leu Leu Phe Lys Tyr Ala Thr Asn Ser Glu Glu Val Ile Gly
       50                  55                  60
   Val Met Ser Lys Glu Tyr Ile Pro Lys Gly Thr Arg Phe Gly Pro Leu
   65                  70                  75                  80
   Ile Gly Glu Ile Tyr Thr Asn Asp Thr Val Pro Lys Asn Ala Asn Arg
                   85                  90                  95
   Lys Tyr Phe Trp Arg Ile Tyr Ser Arg Gly Glu Leu His His Phe Ile
               100                 105                 110
   Asp Gly Phe Asn Glu Glu Lys Ser Asn Trp Met Arg Tyr Val Asn Pro
           115                 120                 125
   Ala His Ser Pro Arg Glu Gln Asn Leu Ala Ala Cys Gln Asn Gly Met
       130                 135                 140
   Asn Ile Tyr Phe Tyr Thr Ile Lys Pro Ile Pro Ala Asn Gln Glu Leu
   145                 150                 155                 160
   Leu Val Trp Tyr Cys Arg Asp Phe Ala Glu Arg Leu His Tyr Pro Tyr
                   165                 170                 175
   Pro Gly Glu Leu Thr Met Met Asn Leu Thr Gln Thr Gln Ser Ser Leu
               180                 185                 190
   Lys Gln Pro Ser Thr Glu Lys Asn Glu Leu Cys Pro Lys Asn Val Pro
           195                 200                 205
   Lys Arg Glu Tyr Ser Val Lys Glu Ile Leu Lys Leu Asp Ser Asn Pro
       210                 215                 220
   Ser Lys Gly Lys Asp Leu Tyr Arg Ser Asn Ile Ser Pro Leu Thr Ser
   225                 230                 235                 240
   Glu Lys Asp Leu Asp Asp Phe Arg Arg Arg Gly Ser Pro Glu Met Pro
                   245                 250                 255
   Phe Tyr Pro Arg Val Val Tyr Pro Ile Arg Ala Pro Leu Pro Glu Asp
               260                 265                 270
   Phe Leu Lys Ala Ser Leu Ala Tyr Gly Ile Glu Arg Pro Thr Tyr Ile
           275                 280                 285
   Thr Arg Ser Pro Ile Pro Ser Ser Thr Thr Pro Ser Pro Ser Ala Arg
       290                 295                 300
   Ser Ser Pro Asp Gln Ser Leu Lys Ser Ser Ser Pro His Ser Ser Pro
   305                 310                 315                 320
   Gly Asn Thr Val Ser Pro Val Gly Pro Gly Ser Gln Glu His Arg Asp
                   325                 330                 335
   Ser Tyr Ala Tyr Leu Asn Ala Ser Tyr Gly Thr Glu Gly Leu Gly Ser
   340 345 350
   Tyr Pro Gly Tyr Ala Pro Leu Pro His Leu Pro Pro Ala Phe Ile Pro
   355 360 365
   Ser Tyr Asn Ala His Tyr Pro Lys Phe Leu Leu Pro Pro Tyr Gly Met
   370 375 380
   Asn Cys Asn Gly Leu Ser Ala Val Ser Ser Met Asn Gly Ile Asn Asn
   385 390 395 400
   Phe Gly Leu Phe Pro Arg Leu Cys Pro Val Tyr Ser Asn Leu Leu Gly
   405 410 415
   Gly Gly Ser Leu Pro His Pro Met Leu Asn Pro Thr Ser Leu Pro Ser
   420 425 430
   Ser Leu Pro Ser Asp Gly Ala Arg Arg Leu Leu Gln Pro Glu His Pro
   435 440 445
   Arg Glu Val Leu Val Pro Ala Pro His Ser Ala Phe Ser Phe Thr Gly
   450 455 460
   Ala Ala Ala Ser Met Lys Asp Lys Ala Cys Ser Pro Thr Ser Gly Ser
   465 470 475 480
   Pro Thr Ala Gly Thr Ala Ala Thr Ala Glu His Val Val Gln Pro Lys
   485 490 495
   Ala Thr Ser Ala Ala Met Ala Ala Pro Ser Ser Asp Glu Ala Met Asn
   500 505 510
   Leu Ile Lys Asn Lys Arg Asn Met Thr Gly Tyr Lys Thr Leu Pro Tyr
   515 520 525
   Pro Leu Lys Lys Gln Asn Gly Lys Ile Lys Tyr Glu Cys Asn Val Cys
   530 535 540
   Ala Lys Thr Phe Gly Gln Leu Ser Asn Leu Lys Val His Leu Arg Val
   545 550 555 560
   His Ser Gly Glu Arg Pro Phe Lys Cys Gln Thr Cys Asn Lys Gly Phe
                   565                 570                 575
   Thr Gln Leu Ala His Leu Gln Lys His Tyr Leu Val His Thr Gly Glu
               580                 585                 590
   Lys Pro His Glu Cys Gln Val Cys His Lys Arg Phe Ser Ser Thr Ser
           595                 600                 605
   Asn Leu Lys Thr His Leu Arg Leu His Ser Gly Glu Lys Pro Tyr Gln
       610                 615                 620
   Cys Lys Val Cys Pro Ala Lys Phe Thr Gln Phe Val His Leu Lys Leu
   625                 630                 635                 640
   His Lys Arg Leu His Thr Arg Glu Arg Pro His Lys Cys Ser Gln Cys
                   645                 650                 655
   His Lys Asn Tyr Ile His Leu Cys Ser Leu Lys Val His Leu Lys Gly
               660                 665                 670
   Asn Cys Ala Ala Ala Pro Ala Pro Gly Leu Pro Leu Glu Asp Leu Thr
           675                 680                 685
   Arg Ile Asn Glu Glu Ile Glu Lys Phe Asp Ile Ser Asp Asn Ala Asp
       690                 695                 700
   Arg Leu Glu Asp Val Glu Asp Asp Ile Ser Val Ile Ser Val Val Glu
   705                 710                 715                 720
   Lys Glu Ile Leu Ala Val Val Arg Lys Glu Lys Glu Glu Thr Gly Leu
                   725                 730                 735
   Lys Val Ser Leu Gln Arg Asn Met Gly Asn Gly Leu Leu Ser Ser Gly
               740                 745                 750
   Cys Ser Leu Tyr Glu Ser Ser Asp Leu Pro Leu Met Lys Leu Pro Pro
           755                 760                 765
   Ser Asn Pro Leu Pro Leu Val Pro Val Lys Val Lys Gln Glu Thr Val
       770                 775                 780
   Glu Pro Met Asp Pro
   785
<210> 5
   <211> 20894
   <212> DNA
   <213> Mus musculus
<220>
   <221> misc_feature
   <222> (2935)..(3024)
   <223> n is any nucleotide
<220>
   <221> misc_feature
   <222> (3125)..(3125)
   <223> n is any nucleotide
<220>
   <221> misc_feature
   <222> (18390)..(18390)
   <223> n is any nucleotide
<220>
   <221> misc_feature
   <222> (18519)..(18519)
   <223> n is any nucleotide
<220>
   <221> misc_feature
   <222> (18552)..(18552)
   <223> n is any nucleotide
<400> 5
   gggggaagag tagtcagtcg ctcgctcact cgctcgctcg cacagacact gctgcagtga     60
   cactcggccc tccagtgtcg cggagacgca agagcagcgc gcagcacctg tccgcccgga    120
   gcgagcccgg cccgcggccg tagaaaagga gggaccgccg aggtgcgcgt cagtactgct    180
   cagcccggca gggacgcggg aggatgtgga ctgggtggac atgagagagg cttatctcag    240
   atgttggatc ttctcttgga aaaacgtgtg ggtacgacct tggtaaggaa ccagattctg    300
   tctttaatac gatttgaaac cttttatccc tttttctttc ttcctttttt ttttaacttt    360
   tcttttctcc cccctccccc ttttaaaaaa aaaaaaagaa tgaagcctca gtagaaacca    420
   gcgcttctgt tttagtacgc ggagcactgt caaacattta gaagactttt ttcctccgta    480
   tgaatcatta accctttcag ttctagacat aattgtcaat tcactgaaat ttcagtagtg    540
   gttcttgtcc gcttcgccac tcgctgcctt tacattactg taactatccc gggttgactt    600
   aggttttcac ttgtatttaa catcgtttgt tccacatgga ccttacatgt tggaactaaa    660
   taagaatgag atagtttaag ttgtacccgg gacaaggaca agtaagcatc tttccccttc    720
   tcggagcgtc ctatctaggg acgaattgta aagaccagct ccggagaggg actcccgctg 780
   tactgtgttt acattttcac aagcgcgcgt tctaacatgg ttatccttat tcctaatttt 840
   tatctgcggc gtctatgtgg gaatacgttg cagaggctgt tttatctttc ttgcttttcc 900
   tctttggaaa ggactttttc cgagggcaga taagaggagg atccccaagt cttctgtata 960
   actttagtta cagtaaactg tgccacttca gtgacttctg ggaattcatg cactttcaca 1020
   tttaaataga aagtgctatt tgtggctgag ggctcctaaa ggaattctct tcagggaatt 1080
   ctattgactt tttttttaat atgttttgtt tttaattttt ctatctggct cgagatgccc 1140
   acggattaaa aaaacaaaca aaactgctgg gtgtttccct cttccccaat ttttcttttc 1200
   ctgtggtcca tgggagctcg ggaaggctgg tactcaagga tgctggcagg atgcaacccc 1260
   tctcaggctt gcctgctgga ggatgaaaga gactgaacgc gcgcgcgcgc gcggcagagg 1320
   gaggggacct agttgttttg aaagttgctt cgctagggag ctggtgggaa agttcagttt 1380
   tccccatttg gaaaaggcag actgggttcc gctcctgcac cacacgtgtt ttccattttt 1440
   agcttcattc agacgcaggc agcgcccctg cctcttcctc cccttgtttg tgacacttct 1500
   ctgagacagc ttttccacag ctctgagggt ctggcggcca tgaccccggg cgtcccggga 1560
   cacaggacgc agcagcgccc acaacacatt tctgccttga gtgataaagc caaggattgt 1620
   tcaaaggtag ctgttctttc tctcccgatg aggttaacat atacatatac gctttttttt 1680
   tttttttcag ccaaaggctg cattttaaaa ccgtgcttct tcaaggcagt ctactttaca 1740
   cggctttgga ctcttactca actgtacaag tactccaagc ttttaaagtc ttcagagcac 1800
   cgtgttagtc atagcctcta agagggaggc acaggagcgc cggacaatgg ggattaaaag 1860
   cctttccctt ctcttccagg ctgcccccaa gtctagctcc ggctccgtga agtttcaagg 1920
   actggcagag actgggatca tgaaaatgga catggaggac gctgatatga ctttgtggac 1980
   agaggccgag tttgaagaga agtgtacata catagtgaac gaccacccct gggattccgg 2040
   cgctgacggg ggtacttctg ttcaagccga ggcatcctta ccaaggaacc tgcttttcaa 2100
   gtatgctgcc aacaacagca aagaggtaag ccggctgcct tcttgaagtc tgactggcaa 2160
   ttgggccagc tctcctacta ctatctctga gaaccgtgag aatttatatg cattggcaaa 2220
   taattgatcg ctccagtggc tgttttcctt gctttctctt caaaccaatt cctattcatt 2280
   tcttcctccc ttcagctgtc ctatactaat tagtaaacag ttaaattttt tggcaagttg 2340
   acatgtcttg ggaaagctaa ctggcagcac tggtgggcag catggtaaag ggctcagtgc 2400
   ttcaccccct ggccctcttg gatgacagtt ttaaaggaaa gaaacttcct tagaaaaaga 2460
   agtttttcct ctgctcatga gatggcttta ttcttttaac gagccagctt tattagctgg 2520
   gtttctaaaa ttattctcaa aaccttgacg tgtttatgaa ctgaagagat ggcattaacc 2580
   aggaagaggg tcacgtaaaa gtgtcctctg tcaggatgac ttcactaacc accctttacc 2640
   tgtggcagct ccctggcctg ggccaggccg gcaggtccat gttttatggc ttctgaagtg 2700
   ggtacactct ttgtatcaaa gacacagaac acctgaggag cacctgattt gtgtttatat 2760
   aacaattaga gtcggctgtg aagtgatttg caaaataact ccttgctctg agaatctggc 2820
   tgctgcagtt gctctcctga tggcttaagt tgctgaggct agccctgagg agacttccca 2880
   ccatcaccat tgcccacagt gctgtggttt ctgatccttg ctgtctttgg ggagnnnnnn 2940
   nnnnnnnnnn nnnnnnnnnn nnnnnnnnnn nnnnnnnnnn nnnnnnnnnn nnnnnnnnnn 3000
   nnnnnnnnnn nnnnnnnnnn nnnnaaggag ggattctgtg ttgtaaggat tccagatttg 3060
   ctcctaggac tatgcattat gaaagtgcca ggtcactttc ctccttccct aagcgacaac 3120
   atggngaaag aaactccaga gttggggaaa cactgtctct ttcctgtttg tgtgtctcag 3180
   ttcagggttt ggggggggaa ggagggaggt ggcaaagacc gagagctaaa cttaaagaga 3240
   ggaattctac cttgtgtttc actaaagctc cactctttgt tctatttctg atgggagact 3300
   tatttgtttt atgaaaagca aattttaaat tgtagtttgt gtataaatac caccaaaaaa 3360
   aattgtaacc ccagaggtca cccttaatta tacctctttc tgaaaacaat gttcttctca 3420
   acagaaagcc tctgagtatg taacccgtgg agtcaatgtg gcgtttgttt gggtatttga 3480
   ggtctggctg tggctttaaa aggtgtagtg tgttctcccc ccatcagatt tgcctaaata 3540
   ttggtccctt caaaaagggc cattcatgtc ctgttgtctc tgtagcctgt tgggatagaa 3600
   ataaagactc ctggtttttt gtcatcggct ctgttctcct tcagagtcct tctcagctca 3660
   gccctataaa gtttttcaat ttttgttgtt ctactgaaaa ctatggctga acggagttat 3720
   gctattagat catttagttc aaggttcaga tagtcaactt ccccgaaggc tagagttaca 3780
   gccaggccca gactgggagc aaagttctct gattgctcca ggtgtttcta gatcatgcta 3840
   ctctggtccc tcttggtgtc actgtggctt tatttatctt agcataattc aggcacagct 3900
   tttcactaag cctgtttgat tctgtttctt ttctgctgaa ttccttccca tctctgtatg 3960
   ttaagcaacc agaaagaggc agctcttgtg tttctgacag ggtgcccttc acaacacaca 4020
   gaaagtcagg gttctttgag cctactctat gttttaccga aagaaaagtc aaatcgttgt 4080
   ggtagcacct cttcggtcct caatcatgaa agccaccagg aaacaaaatt taagcatcat 4140
   cctatcctaa gcatacacca aaatgtgggc ctgtttttcc cttctctcca gcgccttctg 4200
   ttttgaactt tgggacaggt cccttggggt ggtgaaagag aaggacagaa ttgagccatt 4260
   gagggtgtga tagctgcaaa ccagttccag gccagaagca tctaggatgg aggaagtgtt 4320
   tacattacca ttgttcagtt agttcggtga gttccatgag ctgtagaaac agggaaaggc 4380
   ctttctacgg gtagctttta tctgactgat ctggcttgcc catctttaaa tgttttcatt 4440
   aaaatagcaa aagctgttac tttatgtact gtgtgctgac atcctgggga cttttttttt 4500
   aaaaaaaaaa aaaacaagat catttttcat tatgaatttg tgctatgtgt cagcaccttc 4560
   ccacccccac ccccaccccc ttgatttaaa aacaaaacat gcaaactgta tttctgatct 4620
   taacatcaat ttttagctct ggctcagaat cattctgttc ttttattggg gccgtttgat 4680
   cattcttact tcctatttcc agatctaaaa ccaagtacaa gcacactctt caaaactttt 4740
   agaacgttta accgggtggg ttcttcttcc gtgggatttc cccctcgtcc tcctgaaata 4800
   atctctgaat atttgactgc attaaaaaaa aatcagtttt gacattggga gagcagtatc 4860
   caggacaact ttctgctgtc agctgaggtg gcttgtcctc aggcactgtg tggctcctct 4920
   gactctggtc tgaagttgag tggtgacaaa catcaggcag atctagggac atggtgtcca 4980
   ttctgattga agtccattat taaattgagc catctgtatg gaatctcagc acaacatgca 5040
   attccacctc cggacttttt tatttggtag tatgtgccag agtgccacca catctccctt 5100
   cacataaaga taacacgaag caggagacct ttcaaaaaca cctgctaatt cctttgaaaa 5160
   ctggactgaa gtacagactt gatatctcgg tctataccct tatagtaaaa atacaggaaa 5220
   ggaaatctaa ttgttgggcg agatttgggg aggggaaaaa cagcaagtaa ttaaagtaac 5280
   cttaatttga agtgaggaaa ctcaggccac ttttggaact gaatagtgtc tcctttcaaa 5340
   tccccctgct ttctactggg ctcctatatc gaacactctc taaatggctg tttgttttga 5400
   aagcaaacaa acagacctat gctgccctta tcatccgact ttcattctgt actcccactc 5460
   ccactcactc catcctgcac tgtaggaatc tttcttttct aaagttaaaa agaagctctg 5520
   ctttttgtct ctgagctcca gtttgtgtcc tatagccaaa gccactgaca gagacagatg 5580
   tgcccaggtg gcagaggggt cggctccgca agtcaagcaa gccatgaaga gcttccgtgg 5640
   ggctgaagca tgggcagcct aaggaggcct ctttctcttc ctgatgggtg tagggaaggc 5700
   acacagcagc cagtccccag gagtaattct ttaggaccct ggtcagttcc agtgaagttt 5760
   ctttcctcag ttaggtgagc acactcttgg tctttttagc agtgtgcaac tcaaagcaca 5820
   ggtcaagttt ctctgtgccc tctgcttgtt cctagggtag ggcactgtaa acaatgggtt 5880
   aggaagatgg gttgggctaa gacactcatc tggatcagtt tcctgctttc cattaagaac 5940
   aaccctggct cttaccccca tcctaagaca ccccacaatc accataacct tagagccgca 6000
   gtttctatgt cttctttgat taaggaatca gacatggatt agacgtgaag gtgtttattg 6060
   tgtgttgtta gcatgctgaa cttgagatgg ctataggtgg tttatttgat gttctttttt 6120
   ctgagataga atcttgagat gtctgtagtc ggaatgcagg ccacaagctg ctgatcttcc 6180
   tgcatgctat gattagaggc atgcaccaca ccacacctag tgagacaggt gatttcgaag 6240
   ctacagggtt cctctaacct cagaatgctg aattgtgtgt ctctgtttac ctatcagtgt 6300
   gaatttcctc gtgttcttca gctctgtgta tagtaggaat tttaaatatc aaggtcctat 6360
   tgtactcata aaaacaatct aatattgcat tttggtctgg ttatatttcc cagactgacc 6420
   ttggactcaa taatctcttg cctctgcctc agagtggctg ggacttgaag ccaccccaga 6480
   atttctggtt actccttttt ctctcattca catgacttgt ctgttgagtt gcatttgtaa 6540
   ggttaaagat ggcaggcgct ccaaacactg acttaaacag ctacagtgcc aactccatag 6600
   ttgtgcctaa aaggtcctag atagcttgta ccttctttct ttttctcccc cccccccccc 6660
   ccaagattct aaataacttc cttttggcca caggacaatg tgttttgtta gcgttaaaat 6720
   cggtaggtga aaaccaagcg tctcttctgt agagagaatg gcaatctgga agggaagctg 6780
   tgacctcatt gtactgctcc ttgttggtaa tcaagctctg actcccaaga aactgtgcgc 6840
   aggaggctat aatttaaaac aaagttgatg aaccagtcga gtgcctttct taaaattact 6900
   gctttaaagt ggatatgttg aaattatcag ctgctaatta ttggctccct gacaaatggc 6960
   attatttgtt tttcctgctt ggcattttaa tattatggaa taagcattca aatgtaaatg 7020
   tctaataatt tgtgtattat agaagacaat tccatggatt ttacagagtg ggttcaataa 7080
   ttcacccgaa caagcctggg accggaaagt gtagtcaagc attctgtgta aaaatttatc 7140
   tccagagtct ctgctctgag atactcttgt tcccccaaag ctaggctacc agcagacacc 7200
   accaatgagg aggtgtcttg gaagcataga caggtgtgac gagggcagag gaggagacga 7260
   acatctgctt tcttcagacc tttgccacaa attcaaacag gaccaaaaat aacaccttta 7320
   actcaacgaa tctgtttcga attgtgctgg atatagagaa tttctcctct cccctttcct 7380
   gtcttaagaa tgccctgtta acatgtattg aatattaaat tatttaacaa aatggcatct 7440
   gacgataaaa ggacacctgt catgagagtg gcctgtcccc actcttcatg tgtgtttatt 7500
   ccacactaga cccgagtttt aactatggat gaggctggat accagtgtgt atcacagtcc 7560
   aagtctcaga tttccactca aactacctga agagacattg atttgcttct ctgcacctac 7620
   cattcaggaa tataatcata ggagaaccgt caacaaaaaa agccaagtaa agataaatgc 7680
   tgtgcattgc cttgccctgg ctcacctgcc actgtgaaag gggtacagag tcactctggg 7740
   ctcatttgaa ttacttcctg gtttgtgtct gagttgtgtt gttatttttt tttttttttt 7800
   gataccgtaa aaagaaagtt atgctcaagg aagaatgcca tagttaatgt gtttcttcag 7860
   caaaccccac agagtcagag tgtgtgaggc cttcagtttg aaaaggtcat ttcctcagat 7920
   cttagaagcc acatctttta gaacccacag tctcattaca tattccattt caaagaggga 7980
   cttctgacca ccattgagta gatgttggag aagaacaaag ttacttgaaa gatctttcta 8040
   gtaaagagcc cttatgagct ataagccaaa ggggatggag atgtcaactg gaattttaaa 8100
   aaaacaacaa aaaccttaat gtgtttggtt tctggttccc tcggcctcta tggaacagct 8160
   aaagagcatt attttggttt ctgaggttaa aactcttgca ttttcctcaa gcatggtgtt 8220
   tatggtttga gggagaagac tggaactagg gaactgaggg cagtaatctt gaccccctct 8280
   ctctctctct ctctctctct ctctctctct ctctctctct gccttctgat tctcttagaa 8340
   cggaacatct tctggtcttc caacatgaga ggccatagag ccgagtcagt cactagaaag 8400
   cagtccgtcc ccgggactat ttcagtttca gcacttggat gactgtaaac gatgactgtc 8460
   acgaagctta cggctaactg tggaggcatg gatggtgccg tgctgggtta tcaattgaca 8520
   gttcaattca ctcagctagc tagcttgcca tctcagagtc tgactaatga cctcatattt 8580
   tttttttccc ctttaggtta ttggcgtggt aagtaaggag tacataccga agggaacacg 8640
   ctttggaccc ctcatcggtg aagtctacac taatgacaca gttcccaaga atgccaacag 8700
   gaagtatttt tggcgggtaa gtaagaaaac ctttttttta agacttttca ctataggggg 8760
   taaatggagc ttaaagaaca ggctcagttc cctttcaaca cagcagggct cacccaggga 8820
   aacactggaa ttctgagcaa gttccctaga actggttaaa cgctctgcct agaatattag 8880
   ctggaggtgg ttagatgtgg actacctgtg aatctcaatt tttgcaactc attgcataca 8940
   ggtctgggag ggaaggaaac agtttgccgc ttgcttcaag ggcaggctca tttgcatttc 9000
   tcttcgagga agtagtaatg agtcacggag acttacattt caccctttct tgatttcttg 9060
   ctgagttaac ttcatttgaa tggaagagtt atcctgagtg aacttgatgt cgaagacaaa 9120
   tgtcactaag agaggtagat ggtgggttag tggaactggg aaggatcatg gagctagttt 9180
   tatttaaatc catctgcacc ataaaacggt tacatttgac agtatcatag ttgttaagca 9240
   tgaggaaaat cctcgtgtgc ctatgagatt gtagactcag gtagaataac tattctaaag 9300
   gtctggccta tgctatctcc tttggagtgt cagggttagc gaggattctg aggtgacctg 9360
   ggagatggga ttcatgggta aaaattgttc tctgagatgt ctctggcatg ttcagttttc 9420
   ctcagtgtag aaatgaagag ctatttatac aaatttagtg agctgttttc ctcacacaga 9480
   catgaaatat acaccaccca gagaaatggt aatatccaca actggattac atgagaaaaa 9540
   gactttggtt aaaaaattac ttattcctga gagagtctgt cttcagctag gaagtctttg 9600
   tttccagaaa cgtactactt ctacaactgc atctgtagtc ttgttaagta tttgttctca 9660
   atttttattt atttaatact tagttggtgt taaattatat tgctccaaga tcgcagtctg 9720
   agattatgag cggtttcgcc cttattgctg ttctcagatg ggagccacca gtggtagatt 9780
   aatcttggcc tcagctggta tgaatgaaga caatccgaca ctgtcgtttt gaaaacggtc 9840
   aagaagcacc aaaggctctc ccattactgt ccccactgtc ccatttaaag atttacaaaa 9900
   agaattagac taaataacta gaaggcttcc cttgggagga attatttaag tcagttgtcc 9960
   acatgcaagg aagacaggaa tgaatctttt cacaggttgg aagatcctga tttgtcaagc 10020
   aggaatagga acatctctgt gttgtgagga atgaaaggtt gtcatgcaaa ttacacagtc 10080
   agagatgctc aggttgagaa agcagtgaca tttcttgtaa ctgtagtatg aatcagcttg 10140
   tgtttagtct tcttgatact ggatggaaag gctggtataa gtgtgccttt tacaaaagca 10200
   tgatgatagt ttcttggggt gcgtgtgact ttcacgacat ccaaggtcct tttttttaaa 10260
   acaaggatac agtaaaccgt agccatgaaa ggcctactgg gatcggcaca ccctctgcta 10320
   gctgtttcca ccctggtgta agggcgatgg aaccccttgt tcctggaagt ttgcgcgtca 10380
   gagtaaacaa acttgaaaac ccctcttgat agcagaatcc agtcggtctt gttacatttt 10440
   ctcttaacaa gatacaccgc ggaagctctc gcaggctgct ttgatgaagc cacacgcacc 10500
   ccccacacac acacacacac acacatacaa ttcacaggaa gtctctctta aaagaaactg 10560
   attctgctgt ttactgcctg tgttaaaggg acagagttcc ttttttattt ctgataacgt 10620
   tagagggaaa tacagaaacg ttcacacagc ctgtgtgtga ctaagaatac agcaaatagc 10680
   cctgtagagc aaactccctg aggtgagcat ggaagcgccg tacctcttgg agactgtctt 10740
   gtgtgtaaag agggctctct gctctgaagg aaggcggtgg ttcttcaagg agaggagctt 10800
   ttctggagag gatgaggagt gagtctttga ccccttggtt tcagtaggag tgtatttctc 10860
   ccctgctctt aactatgcct ttaaccaagc actctgagta cagctgtgag tcagaggtag 10920
   cattgctgaa gaagaaccat atattttctt tctcttcttt ttccaaaagg ccaaactcgt 10980
   ggctcttgtg tgtgtagacc gtgtatgcca gcctcctcac agatatgggc aaatctctct 11040
   actccttttc aagagagagg cacagggtgg ccgcctgtgt ttaccaagag gaaaagttac 11100
   ttctcgatag gctgtcaaac tttggcctcc gtgccagtgc ctcactctgt tatggcaggt 11160
   gaagttcacc tttgccccac ccagtgtttc cacaaaaagg cagggttcca agtattcatc 11220
   tgaacaagtg ttactgtggg actcagggtt gggggtggag gatgtttgca gagcctaagc 11280
   cccgggcggg cggaggtgta ggaaacacaa gtacagaggc catagaaaaa aggtgagact 11340
   cagtttgacg cagtcctctc ggctgctgtg cccagtgact caaagcacta gaagtcagca 11400
   gagttggaac tctgggctga gcagagtcgc ctgatcgata ttcgctactg tagcaagagt 11460
   acctctttat ggtagtttca cccactctcg gctgttgtta attggaatat tattattatt 11520
   attattatta ttttgctatc cactgccctc cccaacatga gaagaccata aaattgaaat 11580
   ggaaaggtaa ctagcacaat gtgccctgtt tcctccccca tttctgctga ttcagcgtga 11640
   gtcccaccgg atcagcaatg aggcctggag tcatgggtac agcgttggtt gctcgcctgt 11700
   gttccttctg agccattcag ggaagcttcc cggtcgcttt gggctggccg gctgtctttc 11760
   acactgcatc tatcctctcc ttttgaacag atctattcca gagaggagtt ccaccacttc 11820
   attgatggct ttaatgagga gaaaagcaac tggatgcgct acgtgaatcc agctcactct 11880
   gcccgggagc aaaacctggc tgcctgtcag aacgggatga acatctactt ctacactatt 11940
   aagcctatcc ctgccaacca ggaacttctt gtgtggtatt gtcgggactt tgcggagagg 12000
   ctccactacc cttatcctgg agagctcaca gtgataaatc tcagtaagtg gattccagac 12060
   caaaaaaaaa aaaaaaaaat taaaaatgct agtaatgtca gttctgcccc tgtgagctaa 12120
   taacatgttg tctaattata cggcttcgtc atgtgttgga ctaagtaggt ggctttagct 12180
   aagacgagga agaggaaaaa cattctttaa tgtccctact tcttattata aaacataatc 12240
   atcaaagata tacatattac atatattgta taaaataacc agtacagaat gttgttttcg 12300
   gaaagttgca ggaagaagta tatttccgat tctaatttat gcaagcggct gtaggcacaa 12360
   tcccaatggg tatggacctg tggaacaggc cagctgcagt cccttcctgc tgtgctgggt 12420
   cagagctttg agttttcact gaaatcttgt gaagatacgt gtgcctgtaa agccatgatc 12480
   taatgtggaa agctgttttc tagaaaaaaa aaaaaaaact gtcataattg ttcaagtagt 12540
   ctaagtgaat aaccctaaga gatgtcatat ctgagcttcc ttccttatgg taaaggggac 12600
   tgatctcatc tttcaatcag gcttacggta accgcctatc tctttatctt gacaaattct 12660
   tgcttccttg ggtttataag cttttacttt ttcttttctt ctttttaaat tttggctaaa 12720
   gttactgtca tgccctctag caagctttcc ctcttcagtc agtctcaggt agctttagga 12780
   acaatttaag aataaaaaaa aaaaaattct aacttctatt ttaactcagg gtgtgtgttc 12840
   tgggttatac tgccgaatct tacagcactt tttcaaatga ccatcttccc atgaaagcta 12900
   aatgttgaag gtttaaaagt tttcatttta atagtctctg aaatttgagt aaacatttcc 12960
   agaaatctat agagagttcc aagctagact ttacccagtt tctactcttc agtctcattt 13020
   gctttccctg gagactaaat gtagttcata ttttaccact gaagcactag aaatattaat 13080
   tttagtattt taacttttaa gaccaaggac agtgtctcgc tagccatgtt cattctacaa 13140
   tgcttgtgct ctcaggaatt ttcagttttc tgaaaatctt agcttcagta cctttcctgt 13200
   aggctcacaa tatagtgttt gtgcccgggc cctcagctca gcaacgtaca cccttgagct 13260
   aagcatggtg ggtaggtacc tgcccaatag cagcaagccc tctcctcgtg tttgggtttg 13320
   ctttgggtgt ttgtttgttt gtttgtttgt ttatggacaa ggtctcgtgc tgcttaggtt 13380
   cagttcagac ttgatgtagt cagggaaagc cttgagccac tgtcacctcc aaaatgctag 13440
   gatcacaggc ctgtgccctc cccccacccc tgcctcccac catcccccca atgcctgttc 13500
   cgagtgttta ctctttgtgt ccagaagtaa gtttcattat gctatgaaat gacagctttg 13560
   ctcttcagac accccccccc ttttgactga tgcaggagtc ttctgagggt cacaggaaca 13620
   cctcctttgt ctgacattcc taggacagaa agagagttaa ccattcagct gccgtgcaag 13680
   gctcttgctc ctgattgtga aacctgttgg cccaggtgtg gccactgatg actgacactc 13740
   tgatcaggaa aatttccagc atttcatcag gcctaatagg cagatcgagt gtccaagatg 13800
   ggctgtgcta gatttccagg cttaaagcac aatagaggtc tgtccagaat ctccgtaagg 13860
   acttccatca tggggtgcag gggatggaaa cctaatgaaa gaatgtaagt ccccagaaat 13920
   cacaaactga caggaaagag aagggagaga ggaatgtgga aaagaactgt taaatttagc 13980
   tcctggccct cccaaccttt ttggtaattt ttttttctat ctatctaact aacccatcta 14040
   gaaatcagtt gaccaaatta tagacttctg aatgttaatc tgctttctcg gtttcagttg 14100
   aaaacagact ttgttttgcc tactgcagaa cttctaggtt ctttcttgta gtcttagggg 14160
   tgcttattat agatcgaaaa tgtgagtcgg cataattaag ccattcagaa ccttccaaag 14220
   cagctcactc ttgaaatgac tctgtccgcc tacagccatt taagatttaa gaacaaaaac 14280
   agatcttgat tttctttttc atgttagctc aagctgctaa gtgggagagt tagaaatgat 14340
   atcagctcct gtgattagtc agctgctgaa ggatgagttt ttaaaaatgt accttcatat 14400
   acagtctata atttccagct gtaaagtatt ttagagactg acattttgct gcggatattc 14460
   cttcaggata agttctcagc ctggttgttt gtttgtttgt ttgtttgttt tctgaagaca 14520
   gagccaccaa acgctaaatt atgcatgtca cggagaaaat gaaaagctct gacttcattg 14580
   tttcttggtt cagtcattag cttcacagta gttcagtaac taaagtgctt agcaagaaga 14640
   gagccgatta aacctgtgct ctacactgga agaaagccca attctttata cttaacagct 14700
   ttcatttgtt aagtttccac tgtgggacta ctacaaaaac attatgagtc tttgatgtga 14760
   tttgccacat taaaaaaatg gcacagacag gggtgtggtg gcacacaccc tcaatcccag 14820
   tgatcgagag gcagacgctg gtggatctct ttattcttgg ccagtccaat ctatataaca 14880
   agttccagac ctactgagac tgcacaataa gtcttccccc agaaccccca tcaaaaaaag 14940
   agcagagtta ggaaggccgt acacaagcag gcttgcacac tctcacgcgc tcgctctcgc 15000
   gcgcccacac acacacatac ataggcatac gcacatgcgc acacaacttc ttttctctta 15060
   acctgagggt gcttctaaaa tcattatctt tttgtcttac ctccagtaaa tcccttcctg 15120
   tgactgtggg atgcctcgcc tgtcagcctt ccagcttaac ctgtctctct cttcctttac 15180
   cattttagct ttaaaaaaca aaagtgacaa tttgaacttc ctgcctgctg ggcctcaccc 15240
   gaaggactga tattgggctg ataaggggat atttattttg gttgagaggc ttgagaaatt 15300
   gctctccccc agaaagcttt ctgtcactga ccccatcaac atctcccctg atagtgttgt 15360
   ccacggtgtt tattctgggg ctctggctta cccaggagta actgataaca gccagcagga 15420
   gataacgtcc tgtaaagcgc tttccgactg gcatcaaatc cctccagcct gtcagcctgg 15480
   agaatggatc tgaaagcttt agttctgggc ttccacagag ttcatcttca gacctatcag 15540
   gtagcaagct tggagttcct tctcagttaa gcccaaaagg gctgttttat aagagcacaa 15600
   aggatacttc tttacattgt cttaagtgtc attccaaacc tgccagatct tggaggtcaa 15660
   gaatcttgtt tctactccga gcatgtgcac cccccaacta atgatgctct cagcatcctg 15720
   gggagaagtg cctgtttgaa tgagcatccc agaaacacaa ctcagcctgt gcatcggatg 15780
   tgttttatct ttggcccagg aaagctgagc tgaggctttt cctgcgaaat agggctacat 15840
   aactatggac agtttaggac agtattctcc ttgtctgagc ttgaccaggg catatatgct 15900
   gtctctagga gtaaatgttt gtctcttagc tgcctctgtc ttctttggtg ctgtaagtaa 15960
   ttgaactagc ttgggaagta cctgtcgtgg tttggcagag gtgactgtca cacctcacga 16020
   ttccaggaga cagcccagat ggtagtctgg ttagaccaga accttggtga aatgctcgca 16080
   ctgccgagca atggctagaa ggggcagccg ccatgccctt ctagttgata caggcaattc 16140
   gaacagggct catgaagttc ctatgtaaag agaatcgagt tggaaattga tgacagttca 16200
   ttacttaaaa ctagtcttaa tctttcatct aagtttgcac agcactctga tttcctctag 16260
   gtaaactgcg aatgacttat taacccgtga caacccccca ccctgtattt tttccacccc 16320
   atcttagtga acgctctgcc cgttccagtt tgaacagcac ttttctatcc tagttctcac 16380
   taatggaaag gagatcatcc aaggggcact gggctctatg gaggctggca ttgtcccagg 16440
   gtttgatgtt attcccgatc cccccccccc gccccccgga ggaagtggag cagtgtttct 16500
   gagtgggtgg cccagagccc tccctccgga gtgagaggcg ttaggggcca ggtgtctagc 16560
   ctttgtattt gctgctgctc agggtttctc aagaagagag aatggctttc tgatttcact 16620
   tcagttctcc acagccctgt gagtaaccgc cctttcttct tcattttagc acaaacggaa 16680
   agcaacccaa agcaatacag tagtgagaaa aatgaactct acccaaagag tgtccccaag 16740
   agagagtaca gcgtgaaaga aattctaaaa ctggactcca atccctccaa aaggaaggac 16800
   atctaccgtt ccaacatttc acccttcact ttagaaaagg acatggatgg ctttcggaaa 16860
   aatgggagcc ccgacatgcc cttctaccct cgggtggttt atcctatccg ggcacctctg 16920
   ccagaagact ttttgaaagc gtccctggcc tatgggatgg agagacccac ctacataact 16980
   cacagtcccc ttccgtcttc cacaactcca agtccccctg cgagcagcag cccggagcag 17040
   agccttaaga gctccagccc ccacagcagc ccgggaaaca cggtgtcacc cctggcgcca 17100
   ggcctcccag aacaccggga ctcctactcc tacttgaatg tttcctatgg ttccgagggc 17160
   ctgggctcct accctggcta tgcacctgcc ccccacctcc caccagcttt cattccttct 17220
   tacaatgctc actaccccaa gttcctgttg ccaccgtacg gcattagttc caatggcttg 17280
   agcaccatga acaacatcaa tggtatcaac aacttcagcc tcttccctag gttgtatccc 17340
   gtctacagta acctccttag tggcagcagc ctgcctcatc ccatgctcaa tccagcttcc 17400
   ctaccgagtt ccctgcctac cgatggagcc cggaggctgc ttccaccgga gcaccccaaa 17460
   gaggtgctta tcccagcacc ccacagtgcc ttctccctta ccggggctgc agccagcatg 17520
   aaggacgaga gtagtccccc cagcggatct ccaacggcgg gaactgcagc cacgtcagaa 17580
   cacgtggtac aacccaaagc tacctcatca gtgatggcgg cccccagcac tgacggagcc 17640
   atgaatctca ttaaaaacaa acgaaacatg actggttaca agactcttcc ttaccctctg 17700
   aagaaacaga atggcaagat caagtatgag tgcaatgtct gtgccaagac gttcggtcag 17760
   ctctccaacc tgaaggtagg tctccagacc cccgcgggtt tctgcccaca gacccgtgct 17820
   ggtttgccct tggctgccag gcagtgcatt gttgagtgca cttgagccat aggagaccca 17880
   gcttgagcct gaactggggt ctgctgaata ctgaaaatac agggtttatc tcagtgtctt 17940
   tcctaagagg cttgcatctg cattgtacat acctggctct gggaaaccta gcaggcaggc 18000
   aggcctcatt gcaaccccag agttcacccc tgtggtttct tcccaggtcc acctgagagt 18060
   gcacagtgga gaacgacctt tcaagtgcca gacctgcaac aagggtttta ctcagctcgc 18120
   ccacctgcag aaacactact tggtacacac aggagagaag ccacatgagt gccaggtggg 18180
   cagtattctc tgggtagaac tcttgacctc tgtggaaaag tagctgtaga attgtcttcc 18240
   tgtgttgttt caacaataca aaaaatatgg tcttgtacta ggctgctggc cctgcacagc 18300
   tcctgggtac tctgtgacta ctcacaggct atactgagga tggctgggtg gatgtcagtc 18360
   aagtttcagt gggtggggac atgtcctcan ataaacagta cctcagagta ctgtgtgccc 18420
   agcttctccc cccccccccc ccccccccca ccgcgcatga gcattgttaa gaggcttctg 18480
   gtctcccgag gtttctggct attggcctgc ctttccccnt ccagctgcaa acaattaatc 18540
   ttggtcttcc cntgtgccct ttctctgtct tcccttgccc tcacacttta ggtctgccac 18600
   aagagattta gcagcacaag caatctcaag acccaccttc gattgcattc tggagaaaaa 18660
   ccttaccaat gtaaggtgtg ccctgccaag tttacgcaat ttgtgcacct gaagctgcac 18720
   aagcgactgc atacccggga gcggcctcac aagtgtgccc agtgtcacaa gagctacatc 18780
   catctctgca gcctcaaggt ccacctgaag ggcaactgcc ctgcgggccc agctgctggg 18840
   ctgcctttgg aggatctgac ccgaatcaat gaagaaattg agaggttcga catcagcgac 18900
   aatgcagacc gtcttgagga catggaggac agtgtcgatg tgacctccat ggtggagaag 18960
   gagattctag ctgtggtcag aaaagagaaa gaagaaacca gtctgaaagt gtctttgcaa 19020
   agaaacatgg ggaacggcct cctctcctca gggtgcagcc tctatgagtc atcggacctg 19080
   tccctcatga agttgcctca cagcaaccca ctacctctgg tgcctgtaaa ggtcaaacaa 19140
   gaaacagttg aaccgatgga tccttaagat tttcagaaaa taagtgtttc gtgttgcttc 19200
   ttagggtatg gcttggtgaa tcagggtgcc tttagcaaat tgcttgtaca tgactccaga 19260
   tctgcaaagc tccgctggca ccgggtgctt ccctgcacct ctctggaatt aaagaaggac 19320
   tccaatgtta ccaaaatctc agggcataaa tgaggcaaag actcactata tatacatata 19380
   tacatatata catattataa atatatatat acttatttac agccatgtct atatatttga 19440
   acctgtgtat tttgaatatt tgtgtggata tgtttgcata gcgccttcct attactaaaa 19500
   ctattgccta gccataatta ttttttcaat gataattctt cataatttat tatacagttt 19560
   atctttcaaa aagcaataat taaagaagtt tacaatgact ggaaagattc tttgtaattt 19620
   gagtataaat gttgtatctt tgtcctgtgg ccattctttg tagataattt ctgcacatct 19680
   gtttaaatgc ctgagactta gaagatagct ctgtgatttc aggcaacctt tctctatgat 19740
   aatgctttaa aatgaggttt tgatattgcc aaagtcatgt ggttggtgtg ttaactcaga 19800
   agatcacaca atctgagtga cattctctaa gttggggata catgtgcaga attgctcagc 19860
   aataatttga ggggaaggaa gaagaaaaat attttatgtt tcagaatgat ggtttggttt 19920
   tcctcctcct agtcacaatt ttaccaaaca gtgacaggaa ggctttgcca acctgtctcc 19980
   caatgtcaca tgaccattct gagtggccat atgactttgg catccctggg tgttatctga 20040
   aaatgtgaag aagataaaaa agccgtgttc agaagatctg tcgtaaagca cagatgttgt 20100
   gtgtgtgtgt gtgtgggttg gggggtttga gtctggctgt cattttgctg ttggcttgtt 20160
   tttgtttttt taatatcaaa attgcacaaa gctggtgccc taccaagaag gatttgatat 20220
   agaaaggctc aggccacact taaaatacaa gcaagcaaag agaacagaaa aaaataaaag 20280
   taaaaacggg tattcttatc atcttaggtt aagcgggtaa tgaacactcc tgtccccaac 20340
   gcatcaactg tattgtatct gtaaaactca gcttttctca gtattttgtt tttgcattgt 20400
   ataattaact taattaaaga tgaaagggca ttgcaaaagt gttcaacaat tacctcattg 20460
   agtgtatcca gtaggagtgc aggaattaat gtcgtatctc atgagttgct acccagctga 20520
   gcgtgtgtgc ttccaaatgg taggctgggt ggttcggtcc tgtattctcc taagcccaaa 20580
   ggttacctgt tggtgttcaa ggtgtaataa agaatgctgt atatttatga acctatttat 20640
   accagtatac catgtgtata tatgatatat ttataaccac ttaaattgtg agccaagcca 20700
   tgtaaaagaa cctatttttc ctaagagcaa aaagaatctc tctgaagttt tgcttaaaac 20760
   tccatgacct cgctatgact ttggtgcttg ggcaccaccc tgcctactac cagagagcag 20820
   agcacctcag tgcagaggtg agggtgtgta gcatcttggg atggatagaa acaccacacc 20880
   atccagtcgc attt 20894
<210> 6
   <211> 23615
   <212> DNA
   <213> Homo sapiens
<400> 6
   gggaagccag acggttaaca cagacaaagt gctgccgtga cactcggccc tccagtgttg 60
   cggagaggca agagcagcga ccgcggcacc tgtccgcccg gagctgggac gcgggcgccc 120
   gggcggccgg acgaagcgag gagggaccgc cgaggtgcgc gtctgtgcgg ctcagcctgg     180
   cgggggacgc ggggagaatg tggactgggt agagatgaac gagacttttc tcagatgttg     240
   gatatttgct tggaaaaacg tgtgggtacg accttggtaa ggaacttgaa tttttttttt     300
   ttaattctga aattgatctg aaaactttat tttcttttcc tttattgtta ttattattaa     360
   ttttttttgg ctaatgtcgc agtagaaaca tgcttctgct ttagtgcact tagtgctgtc     420
   aaacatttgt gagactttcc ttatgaatca ttaacccttt cagttctaga cataattgcc     480
   aattcattga aatttcagta gtggttccag ctcacactcg tcaaactatt ccgggttggc     540
   tgaagttttc tattttattt tatttttaac atgtgtttgg cgtcatgact ctacatgttg     600
   gaactaaata aaaataagca ggtttgctta aatcataact gagggaaaaa caactttgca     660
   tccaactttt ttttttttaa gagcatccta tttagagaag tggaagaatg taaaaacctc     720
   cttgaaggac ttccacagaa tgttatgttt acatttgaac aaacacacat tcttacatgg     780
   aaatgatacc catattcctc atttttatca aacatgtcta tatgagaaaa cccttacaga     840
   agttgtttac ctttttttgc ctttggaaaa cagttttttt ctgagtgtga gggaggattt     900
   tgggggaata tcctcatcaa tgtacaagtg gaagcagagc ttgtcctcca agtcttctaa     960
   atttgttata actttagtta cagtaaactg tagtacatca gtgacttctg ggaattcata    1020
   cactttcaga tttaaatgga aagtgctatt tgtagctgag gactcctaaa ggaattctct    1080
   ccagggaatt ttattaaacg gttttatgtt ttgtttttgc cttttcaatt tggtatgaga    1140
   tgcttgcaag tcagaagaca ctgcaggctg ttttcccctt cacccatttt tcctcctctt    1200
   ttcctgtggt ccaagtgatt tctaagaggc cgtagctcag tgatgctggc aggatgcaac    1260
   ccttttcagt cttccatgtg agaggatgaa agagagctcg cagcagaggg aggaggcagt    1320
   tgttttgaaa gttgttttgc gttgggagct ggtgggaaag ttcggtcttc cccatttgga    1380
   aaaggcaggc tgggttccgc tcctgcacca cacgcgcttt ccatttttag cttcattcag    1440
   aggcagacag agctccttcc tcttcctctc cttgtttgtg acacttttct gaggcagctt    1500
   ttccacagtg ccgagggtct ggcggccatg accccaggca ttctgggaca ctggactgtg    1560
   tgcccagaac atttttctgc catgagaggt aaagccaggg attgttcaga ggtgattctt    1620
   tttttttttt tccttttccc cacagtgagg ttgccacatt cttttttttt tttttttttt    1680
   taactaagag tagcatttaa aaaccttgct tcttttcaag gcagtttact ttatacggct    1740
   tcttggctct ttctcaactg taccaagcac tctgcatctg cttttaaagt cttcagacta    1800
   ctgtattagt catagcctct cagaaggagc cacaggaacg gcgggacaat ggggattaaa 1860
   ggcctttcct ttctcttcca ggctgccccc aagtgtaact ccagcactgt gaggtttcag 1920
   ggattggcag aggggaccaa ggggaccatg aaaatggaca tggaggatgc ggatatgact 1980
   ctgtggacag aggctgagtt tgaagagaag tgtacataca ttgtgaacga ccacccctgg 2040
   gattctggtg ctgatggcgg tacttcggtt caggcggagg catccttacc aaggaatctg 2100
   cttttcaagt atgccaccaa cagtgaagag gtaagcctct ggtttattga caagaagatt 2160
   ggggacctgg tgccaaatct ccctacttgc ccttgaggcc ttgtatatct ctgaaaacct 2220
   ctgagaatct gtaagtatca gtaaataatt gattgctcta ttcaattctt gcattgcttt 2280
   ctctttccct aaaccatttc cttctcattt cttccagcct tcaactgttc ctcactaatt 2340
   agtaaacagt taaatatttt ggcaaattgg catgtcttag aaaagcaact tgcagcatag 2400
   ggtgggtgaa attgtcagtg aacttcaaga aagctctggg cccactggcc ctagtgtccc 2460
   tgttgtacaa tatctcttaa gggagaaaac tttttcttgg aaaaaaaagt tttaatattt 2520
   cttttgtctg tctttggtaa ttagttggct ttactctttt taacaagcca gctttattag 2580
   ctgggtttct aaaactatcc tcaaaacttt gacgtgttta tgaagtgaag tgatggtata 2640
   agccgagaaa gggggtcatg taaaagtgtc ctctgtctgg atgacttcag agctaaccac 2700
   tgtttatctg cagcagctcc tttgctgggg ctgggctggc aggccaattc cttactggcc 2760
   tctgaagtgg gtacactctt tgttgtttca aaggggatga aaacccaaac ttggaatgag 2820
   caactgattt gtgtttacct ttaatataaa gattatagct aggcgtgatg tttagctgtg 2880
   aaataacttg cagaaccacc tcttgttttc agaatttggc ttactgtagt tgcacttact 2940
   agtctactac tgggttgagt tgaatgaaag gacccctcag cagacttccc ataccaccat 3000
   tagcacagag cactgtcaat tctattactt ccttcagaaa gtagggaaag gggatggttg 3060
   gccgtagaaa tccagaattt gctactggga ctattcctta cagccgtgtc aaatcacttt 3120
   ccccctcccc taaggaacag tatttgaagg aatccgggag actaggaaat gctttgtctc 3180
   tttcctgttt gtgtgtctga gtccagtgtt tttggtgagg gggagacaca gctgtctaaa 3240
   attaaataga acaacgatgc tcagtctttc ttttttcccc cactgctgcg tgaatggagg 3300
   cagattttat aaaaaataaa atttaaattc tagttgtagt tgaatgttac caaaaaattt 3360
   aaccccagag gtcagcctta attatacctc ttcctgaaag taatgctctt cttatagaaa 3420
   gtctctcaat atgtggccct tggaatgaat gtaggatttg tttgggtata tgaggtttga 3480
   ctatggcttt aaaaagtgta gtgtgttttt tccccatatc aagtttgacc aaatattcgt 3540
   ctgttcccaa gcttcgtttg tgctgtgttg ccctgtccag tcctttggga ctgaagtaaa 3600
   aatctccatt ctgtgggtca tctttgcctt ttaattctat aatgttctta tcgtcagttc 3660
   agctcttagc ttaagttttt caatttccgt cattctcctt taaaatcata gctaagtcaa 3720
   gtaatgctct tagatcattt agttcaaggt tcagaaagac agtcacttac ccaaaggcat 3780
   ataggtaaag ccagaccggg gacttgaagc aaagcttttc tgacttctcc aatgagtgct 3840
   tctcgattat gctacactgg cccctcttgg tgtcactgtg gttttagtta tcatcttaat 3900
   atatttcaga ccaatcctta agctattttg tgtttttttc cctctgaatg tataacttcc 3960
   tatttcaata tattaagcaa ccaaaaaaca gacaatgcct gtgttcctga agaaggtcct 4020
   tcgaaactaa cctaagaggt gagggttctc ttatcctatt tccctatctt gcatttaact 4080
   gaagcgcttg aatagaggat agtatgtctt taaaccctaa tcataaagtc aagcaaggag 4140
   taaacttgaa tgtaatactg tcttaagctt actctggaat ttgtacctgt ttttctgttt 4200
   ctctcctttg tttttgaact ttgggaaagg acagcttcct ttagaaggtg aaagaggatg 4260
   gtggagttga gccattaagg gtaggatagc tgcaaactag ttccgggaca gcaacattaa 4320
   ggattgatga aatgtttaca ttatctttat tcagttagtt ctttaagttc catgagccat 4380
   tgaaatagga agagtctttc tacatgtact ttttatctga cttattaatc ttgcccttct 4440
   ttaaatgttt tcgtttaaat agcaaaagat gttacaatat gtactattgt gctgacatac 4500
   cggggaattt tttttttaca agaccatttt tcattatgaa tttgtaccat gtgtcagcac 4560
   cttttttttt tttttggtta aaacaaaaca tacaaatcat atttctgatc ttgaccctca 4620
   tttttagatt gggtttagaa tcattctgca cctttaatga aaaactgttc ttacttccta 4680
   tttccagaac tgaaatcaaa tatagaccta ctattcaaaa ctttgaaaat gttttaaact 4740
   gatgggttat tcttttatgg gggttttccc cttgtcctcc tgaaataatc tcttatgact 4800
   acttgactac agaaaaatca gttcaagcat ttatatttta gaagcgttac cctgtagagt 4860
   ttctgccgtc ggttgagact gcttgcactc aagaggcact gtggctctgt gagtctcacc 4920
   tccctgtatc gtggctgata aacatcagac agattgtgag gactcctttg gtgtccattc 4980
   tgatcgaaat ccattattaa ctcaagccat ctgtaaaaag tatctcacca taacatgcaa 5040
   ttctctcttc atgttgcctt ttatttgaga aatgatgtac caaggcatca ccatgtctcc 5100
   tttaatagaa agataatgaa gaaggaaacc ttctccaaac aactttgttc acttcactcc 5160
   ttttaaaaat ggggtgattg ccgggcgcgg tggctcacgc ctgtaatccc agcactttgg 5220
   gaggctgagg acagcagatc acctgaggtc aggagttcaa gaccagccta gccaacatgg 5280
   tgaaacccca tctctactaa aaatacaaaa attagccggg cgtggtggtg ggcgcctgta 5340
   atcccagcta cctgagaggc tgaggtagga gaatcgcttg aacctgggag gtggagtgag 5400
   ccgagatcgt gccattgcac tccagcccag gcaacgagag tgaaactctg tttcaaaata 5460
   aataagtaaa taaatgccat tgcactccag cccaggcaac aagagcaaaa ccctgtttct 5520
   aaataaataa aataaaataa aaatgaggtg attgcaggct gaaggtcttg gtcactactg 5580
   agttctagtt ctctcaagac aaatattcat atggtcaaaa cagaattata atctaacgag 5640
   tcattcttat agagtgggat tcggggaggg aaaacagata gctagtaatt taaatgatct 5700
   taatgtaatt tggggagagg atgatcagat gacatttaga cttggatata gtttctgctt 5760
   caaacccacg ctgtgtgtgt ctgtttgcat actctctaaa gaaggaattt gaaaaaaaaa 5820
   aaaaaaaaca aacctatatt gtccttgtta tcagccttcc attaagcgct ccaactcaac 5880
   ccatcctaag ctgtcaaatc tccttcattg tgtaaaattg aaaagaagcc ctgtgttttg 5940
   tccggaagct gtggaaactg tggacggcgg ggtgtgttct gtaactgagg ccacagacag 6000
   aaacaaatct gctcaagtgg tggaggggtc tgcaccgcac ataaagagct tctgtgggag 6060
   caggtgaagc aaggaggtat tgaagccggg tgtcctcttg ctgttgagtt tatagaaggc 6120
   acagagcggc ctgtccccag cagtaattct ttaggaccct ggtcagttcc agtgaagttt 6180
   ccttcttgag ttaggtgaac acattctttg tctttttagc actgtgaagt ttaagctaca 6240
   caccaagttc ctccttagta tttgcttggt tcaaagataa actgttacaa actatgggtc 6300
   agggagatgg gtagtctaag atgcacattc tggatctttc taggaccaga gggagactta 6360
   gtacttggtt ttttcacttc cctttaaaaa caatcctggt tcctgttccc cctccaaagt 6420
   tacctggaca agcaccatag tcttagagtt tcagctttta tggcgtctgt gtctcagacg 6480
   tgcatttgag atgaagatgt ttactgtata ttagaacgct agacttagaa tggatatagg 6540
   tggtttgtaa gtgccttcca gttttgaaat tttatgattt tgtatcttta tctataggtg 6600
   tgagtttata tgtgccctat agcactgtat gtaattgaaa tttaaaatat tagggcgctc 6660
   tgtccctttc ctaatgataa gctaatattg aagctcttta atttgtctgg ggtttgagga 6720
   ttatcataga cctctcttct accacagagc cttctctttt taattgttct cctgtcttgc 6780
   ctgttgaatt gaatttgtaa ggttagagat ggcaagagct acttccggtg ctgctttata 6840
   gcttactagt gatgagtcta tagttgtgct caaataggta ttgggtagct tatgctttct 6900
   agttgacttc tttttgggca caagaaaatg tgtgtaagat ggataactga aaacctagag 6960
   gcccattctg taaaagaatg gcaatttgga agaagaacca cgacctcagt gcagtgctcc 7020
   gttttggtaa tcaagctctg actcccaaga caccatctgt aagagactat aatttaaaac 7080
   aaagttgatg aaccagttga gtgcctttct taaaattact gttgtaaagt agatatgttg 7140
   aaaatatcaa ctgctaatta ttggctcacc aacaaatggc atgatttgtt tttcctgttt 7200
   gtcattctaa tattatggaa taatcactga aatatgaatg ttaaatgatt tgcatgtcat 7260
   agaattcaat tccattaatt ttccaaatta attagattca ataataatat gtccaaaaca 7320
   atgagcctct gaataagtct actaaagctt taaatatata tatttatatc tcaagactgc 7380
   tctgagatac tcttgtcccc agtagtttta ctgccaacaa ataccaataa tgacaaacgg 7440
   tgattaaaag ggcagataga tgtaaatgaa ggcagaggag gaggatgaaa tgaacatctg 7500
   cttttttcag ctctctgaca gaaattctta aaggaccaaa aataacactc ttgtttcaac 7560
   ttatgttagg tgtaggaaaa ttttcttttt ttctctttcc agtctgagta tgcttcattg 7620
   atttgtatat tgaatactaa attgttttag aaaaaaaaat cattggctga taagacactt 7680
   aaatgagaaa agctagtctg tcacaggcat gtaggtgtgc catatctatt taaatagagg 7740
   tttaaattat tgatgagact taatgaggaa ctggtgtata ttgtaaccaa aattgtagat 7800
   tccactgtga acaatctgtg catggatcga ttcacatatt tgagtctacc atttcaggta 7860
   catgaatgta caggagctac tgggaatata aaaactggaa aataaacatt atcatctatt 7920
   accactccct cctccttggt ccattttagt aaagaattca gtaaagtgat aggtaggcta 7980
   tcttgaatga gaagaaaata aaactaggaa agtgagagaa taaaaaccaa aacaaaactg 8040
   taactgcaaa ctgttgtaaa ggactactca cagaggagtt ctattgaata gtgacgaatt 8100
   gcttcctgat tcacggttgg tgattttttt accccttcaa agagaaattt attattaagg 8160
   aggaactggt atagttaaca tgtttcttta gcaaggcccc acaaagttaa aatgtgtgat 8220
   gcatccagtc tgaggtcatt tcctcagatc ttagaaccta cagctttcct ccgtataaac 8280
   ttaatttcaa aggagggctt ttggccaggc atggtggctc agcctgtaat ctcagcactt 8340
   tgggaggccg aggtgggagg atcgcttgag tccaggagtt tgagacaagg ctgggcaaca 8400
   tggtgagacc ccttctcaac aataacaaca aaaattagct gggcgtgatg gtgcatgcct 8460
   gtagtcccag ctactcagga ggctgaggtg ggaggatctc ttgatcccag gaggtcaagg 8520
   ctgcaatgag ctaagatcaa gccactgcat tccagcctga gtgatagtgg gagaccttgt 8580
   ctttaaaaca cacacacaca cacacacaca cacgagggcc tttgaccact cttgagtaga 8640
   agactcgaga agaacaaagt agaaggccag agaagaacaa agttacttga aagatctctt 8700
   attaaagaga atgtacaagc tatgaaaaaa aaaaaacaca cacacacaca caaacctcat 8760
   ctggaatgaa aaaaacataa tgcatttggt ttctggttcc ttaggctgtt atggaacaac 8820
   caaagaacat tattttggtt tctgaggtca gaactatttt attcccctca agcacactat 8880
   gcttatggtt tgagggagaa tgagaaatag gaaactagga acaggctgaa atggtctaat 8940
   cttgaccatc taattctgca gtgtcttatt ctcattctaa aagagaatgg ttatattcgc 9000
   tgttctagca taaaaagtaa tgataaaaat aaaagatccc gtattaccag acaataatcc 9060
   cctagactgt tttaatgctt ggttgagtat ttgcttatga tctcagactt taaaagatgg 9120
   tctcccccta tggtgaagct tgttaattat gtaggcatca ttaatgtctg tttacttatc 9180
   aaaattttat cattgttagt tgtattacta cttgacagtc caatttattt aattgaaaag 9240
   attggttaac attttatagt caaagtaatt gtttcctgtg ttttttcctg tttaggttat 9300
   tggagtgatg agtaaagaat acataccaaa gggcacacgt tttggacccc taataggtga 9360
   aatctacacc aatgacacag ttcctaagaa cgccaacagg aaatattttt ggagggtaag 9420
   taagggaaat ttcttcagac ccattaaatg ttaggaaaaa atggagctaa aagagctggg 9480
   tggctcacct ttctcatcct gtgctgagaa atgctggggc tcacccataa gtatccagca 9540
   tccccatgga cacagggaat tctgaacaaa tgtgatgaaa ccgatgaaat gtctggcctg 9600
   taggtggtta gtgatggaga tacgggctat atgtgaatct tgatttttgc aattcattag 9660
   agctttgtaa tgaaaggaaa cagtttgttg cttgctttaa ggataggttc atttgcattt 9720
   ctccgcaagg aagtagtaat gagttaccaa gccttagatt tcaccccttt ttgatttctt 9780
   gctgacttaa ctttaattga atggaagagt tatcacaaat gaattatctt tttggttttt 9840
   ttttttttga gatggagtct cactctgtca ccaggctgga gtgcaatggc atgatctcgg 9900
   ctcactgcaa cctccgcctc ccaggttcaa gcaattgtcc tgcctcagcc tcccgagtag 9960
   ctgggactaa ggtgcgcgcc accatgccca gttaattttt gtatttttag tagagacggg 10020
   gttccactat gttggccatg atggtctcga tctctggacc tcgtgatccg cccaccttgg 10080
   cctcccaaag tgctggaatt acaggcaaga gccaccgcgc ccagccagga atgacaaatg 10140
   aattacctta taagtaaatg ccattaagga aggatagctg gaagatgggt tgaggggaat 10200
   ggaggaccac agaactagtc ctatttaaat acatgtgcat ggtaaaatga ttccatttga 10260
   caataggtta attatctcat agcataagga aaatgcttaa cagtcatatg caagatgata 10320
   agctttccta tagcatccaa ccaaaagatc tagccagtac aatttccttt gctatattag 10380
   ggttagaaag gcccccagag gtgaaccaat tagatggaat ccttgaataa aacactggat 10440
   tagcagtgaa cagaaaaaag tcagattgct ttccttcttc ccatagatgt ctcagggata 10500
   tttagtttcc tcagaagata aagaatttag taagcgtttt tttgtgcata cttacatgaa 10560
   atgtacatta tttgaattct ttaaaaagaa acagctgcat gataacaaaa attgtgttat 10620
   gcttgcttta gctggtattt ttgcctagaa cgattatatc gttcggacaa gaagctattc 10680
   ctaagaaaca atatttttaa tccaggaagt ttttcatttt tagaaattta tcttactatt 10740
   tcccaagcaa aagagggtag ttacagattc actaagaatc atgtgctcac aatttttatt 10800
   taataattat tcctccttaa aatatattaa tcacctgact tacaatggtg gaaccatgag 10860
   tgcatttttg cctttattgt caataacgtc ttctcagaag tgagccacaa aggtgcatag 10920
   ttcttggagt taaaggtctg aattaagaca atccagcata agtctcatta atgtgtgatt 10980
   attttgagaa aaggcaagaa gtacctaaga atctccccct cactgtccag ttccctgttt 11040
   catttaaaga ttcactgtaa gtaactgaaa ggctttcctt gggaggattt atttgaatca 11100
   gtctttcaca tgcaaaggat attgtagaac atctcgtttt tgctggcagg aatatgaaca 11160
   tctgttgtga ggaaagaaaa agtttcatgc aaattacact gccaaagaag ggatgttcaa 11220
   gttgagaaac cagtgacatt tcttgtaact gtactatgaa tcagcgcatt ttaatcttct 11280
   agataatata tggaagtgca ggaaggtggt aggaaacggt gttcatttta catatgcgtt 11340
   attttattct gtgtgagtga cttcatggca ccgacattgc tgtttttaaa tgaggataca 11400
   gtaaattgca gtccgaggaa ggctaactgg aatcaacata cccgtagctt tagaaagcag 11460
   tttccgcacc agcgaagagt acaagagcga tggaacccca tgttcctgga agtttgcaca 11520
   tcagagtaaa caaacttgaa aacccctctt gatagcagaa ttcacccagc cttgttccat 11580
   tttctcttaa caaaacacac cgcaaaagct ctcacaagct gctttgatga agccacatgt 11640
   atttccccct tcacaattta caggaagtta ctcttaaaag aaagtgattc tggtgtttac 11700
   cgcctgtgtt aaagggacag agttcctttt tatttctgat aacgtttgag cgaaatacag 11760
   aaactatctg tagactagca tagtcggtac gtgagtaagg aaaagcaata acctgctgtc 11820
   cggtgagcac aaaattcctg ctacgaacag tgccttactg ctgcttggag actgcaagtc 11880
   gcagatcaca ctaggtattg actgattgta taaggaaatt tcttaaagtc taaagtaaag 11940
   gtggtacctc ctaaaaagag gggaagagag aaaactttgt gtggaaggat aaggagtgtg 12000
   tttatagttt cagtaagagt gtacgtttta atttttcttc ttcctctgcc tctttgccaa 12060
   gtagcctgag tgcatctgtt atccagaagt agtattactc taggacaaac ttcaaattct 12120
   tcattctgcg ttgcctttaa ggaacaacat actttcttcc tgttcttttt ccaaaaacac 12180
   acgcctatgg ctctgtgtgt ggtgttttag ccagcctcct cccagataag gggttccctt 12240
   ccctcctttg cattgaaagg aaagtgcaag tctggacatg tttatcaaga ggaaaagtga 12300
   cttctcagta atagactgtc aaattcgggc tgctgcccga gtgttcgctt tgttatggca 12360
   ggtgaagttc acctttgccc cacccagtgt ttccacaaaa aggcaaggtt ccaagtattc 12420
   atatgaacaa gtgttacttt aggacttgga gggttggggg tggaggatgt ttgcatagtt 12480
   gaagccttgg gcgggggtgt aggaaacggc gagtacagag gccatagaaa aagctaagac 12540
   tcagtttgac gtcgtcagcc ggcttggtct tctacccagt gactcaaagc actaaaagtc 12600
   agcataatcg gaactgaagt cagtagcatc gcccatttgc cattcactgc agtagcaaaa 12660
   gtagtactct gtggtgggtt aatcggtttg aggcagctcc ttaaatgaac atttgtgttt 12720
   catttttctg ttattttccc gaacatgaaa agacgataaa actgaaatgg aaaaggtaac 12780
   tgacaaaagt gtgccttacc tgtttccgcc ctgatttctg ctgattcaag actattctgg 12840
   ctaaactgat tggattcttt ttctaactag gcagtagggg atcagaaatc acacacggta 12900
   ccggctgtgt ttattctgag aggtgctggg gagctttggg tctgacttcc ttttacatgc 12960
   ctgtcttctc ttttggacag atctattcca gaggggagct tcaccacttc attgacggct 13020
   ttaatgaaga gaaaagcaac tggatgcgct atgtgaatcc agcacactct ccccgggagc 13080
   aaaacctggc tgcgtgtcag aacgggatga acatctactt ctacaccatt aagcccatcc 13140
   ctgccaacca ggaacttctt gtgtggtatt gtcgggactt tgcagaaagg cttcactacc 13200
   cttatcccgg agagctgaca atgatgaatc tcagtaagtg gattacagaa caaaaaaata 13260
   aaaaatgcca gtaatgtcgg ttctgcccct ttgaactaat aacatgttgt ttaattatac 13320
   ggctttgtca tgtgttggat gaagtaggtg gcttaagcta gggactagga agaggaaaaa 13380
   cattttttga gtccctatta actattagga aacttgatca tttaaaagta tatatatata 13440
   tgaggagcta ccttgagttt tgaattcagg atgttacagg aagaaatata tgtccaattc 13500
   taatttatcc aaaagcagtt gggagaatta cagggattgg tccagacatg ctgcgtatgc 13560
   aaggtatagc cctcatctgt ggtactttgg cagggcttag actgcatcaa aatatttata 13620
   gatgtacatt tgagtgtaca gttaggatct gatgtggaac attgtaagat cattgctaga 13680
   aaaactttgt cataattttt caatattatt ctaagtgaat aaccgtaaag attttacatc 13740
   ttagcttcct tccttacagt aaaaaaacta tctgatctct tgatcagtat tatagtagcc 13800
   acctatcact ttatcttaac aaattctcaa ttccttaggt ttatgtgctt ttacttcttt 13860
   tatttgatta aaattgctgt catgacctct ctctgcagag ggctgcatca ttttggtcat 13920
   tctcaagtga tctctttgag caatttaaga attgccataa gattctaacc tctgctgtaa 13980
   ctatggttgt gtgttcttgg ttagaccact aaatcttatt agcagtttta aaaattattc 14040
   cttttggttt agaagttaag actaaatgct gaagtttttg taacttttgg ttttgatatc 14100
   atttcaaact taagaaaaca tttgaagaaa aggacaaaga atttccactt accctttacc 14160
   caggtttacc agttattgat aagtatatcc atttgcttta ccagaaggct aacttgtttt 14220
   agttctcatt ttcacctttg agacatttgg aataaatatc aatgttaaca taaattggaa 14280
   ttttgacttt gattttagga ccaatgaaca agccaagtac ttaccctagt catatataat 14340
   ccaactgtat ggttatttgg tattcattcc acacttcatt ttacttgatc tcccttaaga 14400
   ttgcaagatt gtgtttgcag tttttctgaa aatctggggc tataaaagca tcaggacctc 14460
   ccccgtaggg gaggtcgtgt gtttggggtc cttacacaac aggttaccct tgagcttcag 14520
   gaaaagaact ggctctcagt tccccagttc cagcttaatg ggtctaatta ggtcctgacc 14580
   aaaaaggtgg cagttctttt ccctcatgtc tcttcagcgc tccccgagac tctggagact 14640
   ctgtcatatc cctagggctg agcctcccag gaaccattcg gctgttgtgg catctgtgta 14700
   tgccatgccc agtgctgagg acctagtaac aaacgacaaa tgcacaggca cagtggcatt 14760
   tttgtggaac tcgtattcca gctgtgcgtc tcagaagaag cgcacagctc cctcctggct 14820
   ttcttaacat agtgagccac ttccacttaa gggtctcctt acattccttg agtttaatca 14880
   ttcatggatt cagaggaaag tcttttgatt tttgcttttc tttaaacagt tcatttgagg 14940
   tgacctaccc cagtgacttt gcaccaacca ccaagaaact tttttgcatg cttcccgcac 15000
   cctgtgccaa tcaagggaag ggtttaaagg cctggcgttt ttattcctca aagaaaggtt 15060
   ttgcacagta ttttaaggtt caagtgcttc tactttgtgt tcagaagcaa ctgtcatata 15120
   tactgtgaaa tgacaccttt tatttatccc tttttattta tgcagtatgt ccccttttat 15180
   tttggcagaa ttttttctaa atggtggttt aacattttca agcacatttc attgtccaat 15240
   attcatagta aagaatgaga gttaacaata accagtcaca ttaaaacaag attcctgctg 15300
   ccagttgtga aaccggttgt cttaggcgtg gcagctgatg attgagactg tgatcaggaa 15360
   aatttccact atttcatcag gcctaatagg tagattgtgt ctccaaatga actgtgttgg 15420
   gtttccatgc ttaaagcaca atagaggtgg tgcaagaatc tccatgaggg cttaaatggc 15480
   agtgatggtt caggcggtag agtttggaga agaagggatt tgaaacaaac caaaggaaag 15540
   aaaagtaagt agccagaaat cacaaaatgg catttttcta aaaacaaagg aaaaggaata 15600
   aaagaactaa taagtttgaa acccctaccc ctcccaaatt tggcaggggg ggaggtattt 15660
   tttttctatc tatctaacta acccatctag aaaacagttg accaaattat agacttctaa 15720
   atgttaatct gctttctcag tttcagttga aaagagactt tgttttgcct actgcagaac 15780
   ttctaggttc tttcttatag tcttggggtt cttattatag atcgaaaatg tgagtcggca 15840
   taattaagcc attcggagtc ttcagaagca gttcactctt gaaatgactc cgtccgccta 15900
   cagccattta agatttcaga acaaaaacag atcttgattt tctttttcat gttaactcaa 15960
   gctgttgctg agtgggagag tcagaaatga caccagctcc actgattact cagctgctga 16020
   aggatgattt tttaaaatgc acctttactg tatatggact tcctaatttc cacctgtaga 16080
   gcatcttagg gaggctaaca tgtcactctg gatgttcttt tagaataaga tgcaaatcta 16140
   tttttctgaa ggcattagag atagcaaaca tttattgtga gtttactata tactaggcac 16200
   tgtgctaagt gttttgcata gaaagtttaa aattctggct tttttgttgg cccaatcata 16260
   agtttcatat cagttcaaca ttcaaattat attaaggtac ttaagaagaa tccctggcta 16320
   aatgtgaggg gcagtgccac agatggactg aaactttatg cttattgcac atttatgcta 16380
   ttattatttg ttgaattata gaaccaaggg agtgtggaag ccactggaaa aaatatgaga 16440
   cttagataca taatttgagt aaaaatggct caaagtcatg agggtaaagt tttttgtatt 16500
   tccattttat tcgagcggca tcgtttttaa aaatcattat gaatttgacc ctatatagat 16560
   gtttccaaat aattcttttt caccttcata aaattccttc ctgtggctgt gagatgcctt 16620
   gcctatcagt tttcaagctt agttgtcttt ctcatccttt accattttag ctttaaaaaa 16680
   caaaagtgac aattagaact tcctgcctgc tgggcctcac tgaaagaccg atattggcct 16740
   gataaggaga tatttatttt gttttagtgg cttcagaaat ccctctccct cagcaagctt 16800
   tccatcacgg cccccccgtc agcatcttcc ctgatagcgt tcttctctgt gtttattctg 16860
   gggcttcagg ctcgcccagg aggaactgat aaccgctggc aggagataac attctctaag 16920
   gggctctcaa attggaatcg aatccctcaa gccagtcagc ctagagaata catttaaagg 16980
   gttcagttct ggagtttcac agagttcatt tctagaccta tcagatagca agtgtggagt 17040
   tctttctcaa ctaaattcaa gcagagacat tttttagacg atgaaggata tttgcacaaa 17100
   ggcttcagca tgatccccca aacctgctgc ctctgaaggc atctccacac attgacagcc 17160
   aatgccttca gtgcgttcct agggcaggtg tcctggcttg agtgactgtc ctccaataat 17220
   cagagctcaa actaaacatc gtatgtttta cttttggttt ccaggcaagg ctgagcaggg 17280
   aattttcagt tttccctgcc cagatgggtg ttttttcctg aaggcatcat ttattgtgta 17340
   gcgaggagac agggctggct gtggcaggga tagtctagaa ctgtcctcat tgctgctgtt 17400
   cctaaatagt atctttacca agtaataacg tgccgtcttt gggaataagt gctttcctct 17460
   tagcctgttc tgttttcttg ggtgcgctaa gtaattgaac tggctcagga agtacctatt 17520
   gtggtttggc agaggtgact gtcacgcctt gtgactccag gggccagcac tgctgggatc 17580
   ctggctagac cagacagagc cttggtgaag tgcttaggct gtctgcacat cgcgaggaag 17640
   gtggtattca cttcgctaag ctccttggca taggcagttt gaacagggct ttatcaaatt 17700
   cgtattcaac aagagtagaa gcgaaaattg atgactgtgt attacttgaa atgagtctta 17760
   atctttcaca tttagttctc agggtatgct gatttccttt aggtaaacca tgaacatcag 17820
   aaagactttt attaacctat gacagggtcc ccaccccagt atttttccac tccattaaaa 17880
   tggaagtttt tttttttttt ttcttttttg agacagagtt ttgctcttgt tgcccagtct 17940
   ggagtgcaat ggcacaatct cggctcacca caacctccac ctcccagatt caagcgattc 18000
   ttctgcctca gcctcccaag tagctgggat tacaggtgtg cgccaccacg cccagctaat 18060
   tttgtatttt tagtagagat ggggtttctc catgttggtc aggctggtct cgaacttccg 18120
   acctcaggtg atccgcccac ctcggcctcc caaagtgctg ggattacagg caagagccac 18180
   tgcatccagc ttaggctatc ttactccagc ctaaacagca attttctatc ataaggtctg 18240
   tactaatgaa aacagaatca cccaaggctg ctgtttgttc tgtctgtgct gccattgtcc 18300
   gcattttgct gaggaggaaa cggaactgca cttttgagtg agtggcccag agccttctag 18360
   aatgagagtg cgttggaagc cagatatgtg gcgattgtgt cgccagctgt tactcaggtt 18420
   ttctcaagaa ggaggagcaa ctttggcagt tttgcttcag ttctctctag ccctctgtgt 18480
   aatcgcccct ttttctttat ttcagcacaa acacagagca gtctaaagca accgagcact 18540
   gagaaaaatg aactctgccc aaagaatgtc ccaaagagag agtacagcgt gaaagaaatc 18600
   ctaaaattgg actccaaccc ctccaaagga aaggacctct accgttctaa catttcaccc 18660
   ctcacatcag aaaaggacct cgatgacttt agaagacgtg ggagccccga aatgcccttc 18720
   taccctcggg tcgtttaccc catccgggcc cctctgccag aagacttttt gaaagcttcc 18780
   ctggcctacg ggatcgagag acccacgtac atcactcgct cccccattcc atcctccacc 18840
   actccaagcc cctctgcaag aagcagcccc gaccaaagcc tcaagagctc cagccctcac 18900
   agcagccctg ggaatacggt gtcccctgtg ggccccggct ctcaagagca ccgggactcc 18960
   tacgcttact tgaacgcgtc ctacggcacg gaaggtttgg gctcctaccc tggctacgca 19020
   cccctgcccc acctcccgcc agctttcatc ccctcgtaca acgctcacta ccccaagttc 19080
   ctcttgcccc cctacggcat gaattgtaat ggcctgagcg ctgtgagcag catgaatggc 19140
   atcaacaact ttggcctctt cccgaggctg tgccctgtct acagcaatct cctcggtggg 19200
   ggcagcctgc cccaccccat gctcaacccc acttctctcc cgagctcgct gccctcagat 19260
   ggagcccgga ggttgctcca gccggagcat cccagggagg tgcttgtccc ggcgccccac 19320
   agtgccttct cctttaccgg ggccgccgcc agcatgaagg acaaggcctg tagccccaca 19380
   agcgggtctc ccacggcggg aacagccgcc acggcagaac atgtggtgca gcccaaagct 19440
   acctcagcag cgatggcagc ccccagcagc gacgaagcca tgaatctcat taaaaacaaa 19500
   agaaacatga ccggctacaa gacccttccc tacccgctga agaagcagaa cggcaagatc 19560
   aagtacgaat gcaacgtttg cgccaagact ttcggccagc tctccaatct gaaggtaggc 19620
   cttgagagag agcagtccaa ggggctgtga gtgcatgctt gtgtttgtat ttagcttgct 19680
   ttccatgggg tatcgattgc atttgcagta gtatgagccc ccggttgggg atagtgggta 19740
   tggattccgc ctggcttttg ccacttctag ctctttgact ttggacaagt gacttccctt 19800
   ctcctgattt tcttctgaat aataaaaaaa ttaggggttt ggactagaag attaggtgaa 19860
   actccctgct agcctgtgat ttttgtgctt ttaagaaaaa caccattctg aaaacatgaa 19920
   gatttcttct ttttaagact gtcttgatgc ttttcttaag atatttgcat caacacttga 19980
   gtcttggagc agaaatgtta ggtctcagag ccagcttgag agcagagcta acacatgtgg 20040
   cttcttccca ggtccacctg agagtgcaca gtggagaacg gcctttcaaa tgtcagactt 20100
   gcaacaaggg ctttactcag ctcgcccacc tgcagaaaca ctacctggta cacacgggag 20160
   aaaagccaca tgaatgccag gtgcgcagta ttttctgggt agaccttctg acctttgtag 20220
   aaaatgtctg tgagtcaccc tcccatgtcc tatatagccc gtagttaaag ccaacaccag 20280
   attctgcgtt gtcccatcct ggactgatgg cactatggtc cttcccagta ctttgtatct 20340
   gctgatgact tgagatggca cagccagctt ccagtgggtg ggaaaatggt aggggaaata 20400
   aacagcccct cgtgtgctgt gtgcccacat ccccccgttt gcttaatacc acactggagg 20460
   tgccacaagg aggcttctca cctcctaggt tgctgggcgt tggccggtaa gcctgcccct 20520
   cccgttggca actcttaatc ttctggcctt cctgtctccc ttccctgctg tctctctccc 20580
   ctacactgta ggtctgccac aagagattta gcagcaccag caatctcaag acccacctgc 20640
   gactccattc tggagagaaa ccataccaat gcaaggtgtg ccctgccaag ttcacccagt 20700
   ttgtgcacct gaaactgcac aagcgtctgc acacccggga gcggccccac aagtgctccc 20760
   agtgccacaa gaactacatc catctctgta gcctcaaggt tcacctgaaa gggaactgcg 20820
   ctgcggcccc ggcgcctggg ctgcccttgg aagatctgac ccgaatcaat gaagaaatcg 20880
   agaagtttga catcagtgac aatgctgacc ggctcgagga cgtggaggat gacatcagtg 20940
   tgatctctgt agtggagaag gaaattctgg ccgtggtcag aaaagagaaa gaagaaactg 21000
   gcctgaaagt gtctttgcaa agaaacatgg ggaatggact cctctcctca gggtgcagcc 21060
   tttatgagtc atcagatcta cccctcatga agttgcctcc cagcaaccca ctacctctgg 21120
   tacctgtaaa ggtcaaacaa gaaacagttg aaccaatgga tccttaagat tttcagaaaa 21180
   cacttatttt gtttcttaag ttatgacttg gtgagtcagg gtgcctgtag gaagtggctt 21240
   gtacataatc ccagctctgc aaagctctct cgacagcaaa tggtttcccc tcacctctgg 21300
   aattaaagaa ggaactccaa agttactgaa atctcagggc atgaacaagg caaaggccat 21360
   atatatatat atatatatat ctgtatacat attatatata cttatttaca cctgtgtcta 21420
   tatatttgcc cctgtgtatt ttgaatattt gtgtggacat gtttgcatag ccttcccatt 21480
   actaagacta ttacctagtc ataattattt tttcaatgat aatccttcat aatttattat 21540
   acaatttatc attcagaaag caataattaa aaaagtttac aatgactgga aagattcctt 21600
   gtaatttgag tataaatgta tttttgtctt gtggccattc tttgtagata atttctgcac 21660
   atctgtataa gtacctaaga tttagttaaa caaatatatg acttcagtca acctctctct 21720
   ctaataatgg tttgaaaatg aggtttgggt aattgccaat gttggacagt tgatgtgttc 21780
   attcctggga tcctatcatt tgaacagcat tgtacataac ttgggggtat gtgtgcagga 21840
   ttacccaaga ataacttaag tagaagaaac aagaaaggga atcttgtata tttttgttga 21900
   tagttcatgt ttttccccca gccacaattt taccggaagg gtgacaggaa ggctttacca 21960
   acctgtctct ccctccaaaa gagcagaatc ctcccaccgc cctgccctcc ccaccgagtc 22020
   ctgtggccat tcagagcggc cacatgactt ttgcatccat tgtattatca gaaaatgtga 22080
   agaagaaaaa aatgccatgt tttaaaacca ctgcgaaaat ttccccaaag cataggtggc 22140
   tttgtgtgtg tgcgatttgg gggcttgagt ctgggtggtg ttttgttgtt ggtttttgtt 22200
   gctttttttt tttttttttt tttaatgtca aaattgcaca aacatggtgc tctaccagga 22260
   aggattcgag gtagataggc tcaggccaca ctttaaaaac aaacacacaa acaacaaaaa 22320
   acgggtattc tagtcatctt ggggtaaaag cgggtaatga acattcctat ccccaacaca  22380
   tcaattgtat tttttctgta aaactcagat tttcctcagt atttgtgttt ttacatttta  22440
   tggttaattt aatggaagat gaaagggcat tgcaaagttg ttcaacaaca gttacctcat  22500
   tgagtgtgtc cagtagtgca ggaaatgatg tcttatctaa tgatttgctt ctctagagga  22560
   gaaaccgagt aaatgtgctc cagcaagata gactttgtgt tattctatct tttattctgc  22620
   taagcccaaa gattacatgt tggtgttcaa agtgtagcaa aaaatgatgt atatttataa  22680
   atctatttat accactatat catatgtata tatatttata accacttaaa ttgtgagcca  22740
   agccatgtaa aagatctact ttttctaagg gcaaaaaaaa aaaaaaaaaa aaaagaacac  22800
   tcctttctga gactttgctt aatacttggt gacctcacaa tcacgtcggt atgattgggc  22860
   acccttgcct actgtaagag accctaaaac cttggtgcag tggtggggac cacaaaacaa  22920
   ccagggagga agagatacat cattttttag tattaaggac catctaagac agctctattt  22980
   tttttttgcc actttatgat tatgtggtca cacccaagtc acagaaataa aaaactgact  23040
   ttaccgctgc aatttttctg ttttcctcct tactaaatac tgatacatta ctccaatcta  23100
   ttttataatt atatttgaca ttttgttcac atcaactaat gttcacctgt agaagagaac  23160
   aaatttcgaa taatccaggg aaacccaaga gccttactgg tcttctgtaa cttccaagac  23220
   tgacagcttt ttatgtatca gtgtttgata aacacagtcc ttaactgaag gtaaaccaaa  23280
   gcatcacgtt gacattagac caaatacttt tgattcccaa ctactcgttt gttctttttc  23340
   tccttttgtg ctttcccata gtgagaattt ttataaagac ttcttgcttc tctcaccatc  23400
   catccttctc ttttctgcct cttacatgtg aatgttgagc ccacaatcaa cagtggtttt  23460
   attttttcct ctactcaaag ttaaaactga ccaaagttac tggcttttta ctttgctaga  23520
   acaacaaact atcttatgtt tacatactgg tttacaatgt tatttatgtg caaattgtca  23580
   aaatgtaaat taaatataaa tgttcatgct ttacc                             23615